(19) Europäisches Patentamt
European Patent Office
Office européen des brevets

(11) EP 0 779 888 B1

(12) **EUROPÄISCHE PATENTSCHRIFT**

(45) Veröffentlichungstag und Bekanntmachung des Hinweises auf die Patenterteilung:
**28.04.1999 Patentblatt 1999/17**

(21) Anmeldenummer: **95931988.0**

(22) Anmeldetag: **05.09.1995**

(51) Int Cl.[6]: **C07D 487/04**, A61K 31/505

(86) Internationale Anmeldenummer:
**PCT/EP95/03482**

(87) Internationale Veröffentlichungsnummer:
**WO 96/07657 (14.03.1996 Gazette 1996/12)**

(54) **PYRIMIDO [5,4-D]PYRIMIDINE, DIESE VERBINDUNGEN ENTHALTENDE ARZNEIMITTEL, DEREN VERWENDUNG UND VERFAHREN ZU IHRER HERSTELLUNG**

PYRIMIDO [5,4-D]PYRIMIDINES, DRUGS CONTAINING THESE COMPOUNDS, THEIR USE, AND PROCESS FOR PREPARING THEM

PYRIMIDO [5,4-D]PYRIMIDINES, MEDICAMENTS CONTENANT CES COMPOSES, LEUR UTILISATION ET LEUR PROCEDE DE FABRICATION

(84) Benannte Vertragsstaaten:
**AT BE CH DE DK ES FR GB GR IE IT LI LU MC NL PT SE**

(30) Priorität: **07.09.1994 DE 4431867**
**01.02.1995 DE 19503151**
**13.06.1995 DE 19521386**
**04.08.1995 DE 19528672**

(43) Veröffentlichungstag der Anmeldung:
**25.06.1997 Patentblatt 1997/26**

(73) Patentinhaber: **Boehringer Ingelheim Pharma KG**
**55216 Ingelheim am Rhein (DE)**

(72) Erfinder:
- **HIMMELSBACH, Frank**
  **D-88441 Mittelbiberach (DE)**
- **RÜDEN VON, Thomas**
  **A-1201 Wien (AT)**
- **DAHMANN, Georg**
  **D-88400 Biberach (DE)**
- **METZ, Thomas**
  **A-1010 Wien (AT)**

(74) Vertreter: **Laudien, Dieter, Dr.**
**c/o Boehringer Ingelheim GmbH**
**55216 Ingelheim (DE)**

(56) Entgegenhaltungen:
**EP-A- 0 167 817**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

In der EP-A-0,167,817 werden 8-Alkylthio-2-piperazino-pyrimido-[5,4-d]pyrimidine beschrieben, welche wertvolle pharmakologische Eigenschaften aufweisen, nämlich neben einer antithrombotischen, insbesondere aufgrund ihrer selektiven Tumor-PDE-hemmenden Wirkung, eine metastasenhemmende und tumorwachstumshemmende Wirkung.

Es wurde nun gefunden, daß die Pyrimido[5,4-d]pyrimidine der allgemeinen Formel

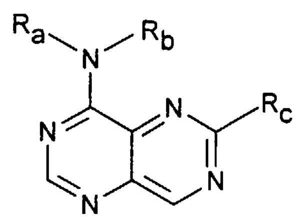

, (I)

deren Tautomeren, deren Stereoisomere und deren Salze, insbesonders deren physiologisch verträgliche Salze mit anorganischen oder organischen Säuren oder Basen, ebenfalls wertvolle pharmakologische Eigenschaften aufweisen, insbesondere jedoch eine Hemmwirkung auf die durch Tyrosinkinasen vermittelte Signaltransduktion.

Gegenstand der vorliegenden Erfindung sind die Verbindungen der obigen allgemeinen Formel I, diese Verbindungen enthaltende Arzneimittel, deren Verwendung zur Behandlung von Krankheiten, insbesondere von Tumorerkrankungen, und deren Herstellung.

In der obigen allgemeinen Formel I bedeutet

$R_a$ ein Wasserstoffatom oder eine Alkylgruppe,

$R_b$ eine durch die Reste $R_1$ bis $R_3$ substituierte Phenylgruppe, wobei

$R_1$ und $R_2$, die gleich oder verschieden sein können, jeweils ein Wasserstoff-, Fluor-, Chlor-, Brom- oder Jodatom,

eine $C_{1-6}$-Alkyl-, Hydroxy- oder $C_{1-6}$-Alkoxygruppe,

eine $C_{3-7}$-Cycloalkyl- oder $C_{4-7}$-Cycloalkoxygruppe, die jeweils durch eine oder zwei Alkylgruppen oder durch eine Arylgruppe substituiert sein können,

eine gegebenenfalls durch eine Arylgruppe substituierte $C_{2-5}$-Alkenyl- oder $C_{3-5}$-Alkenyloxygruppe, wobei der Vinylteil nicht mit dem Sauerstoffatom verknüpft sein kann,

eine gegebenenfalls durch eine Arylgruppe substituierte $C_{2-5}$-Alkinyl- oder $C_{3-5}$-Alkinyloxygruppe, wobei der Ethinylteil nicht mit dem Sauerstoffatom verknüpft sein kann,

eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfinyl-, Trifluormethylsulfonyl-, Arylsulfenyl-, Arylsulfinyl-, Arylsulfonyl-, Aralkylsulfenyl-, Aralkylsulfinyl- oder Aralkylsulfonylgruppe,

eine durch 1 bis 3 Fluoratome substituierte Methyl- oder Methoxygruppe,

eine durch 1 bis 5 Fluoratome substituierte $C_{2-4}$-Alkyl- oder $C_{2-4}$-Alkoxygruppe,

eine Nitro-, Amino-, Alkylamino-, Dialkylamino-, $C_{3-7}$-Cycloalkylamino-, N-Alkyl-$C_{3-7}$-cycloalkylamino-, Arylamino-, N-Alkyl-arylamino-, Aralkylamino- oder N-Alkyl-aralkylaminogruppe,

eine gegebenenfalls durch 1 bis 4 Alkylgruppen substituierte 4-bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-,

N-Aryl-imino- oder N-Aralkyl-iminogruppe ersetzt sein können,

eine gegebenenfalls durch 1 bis 4 Alkylgruppen substituierte (Alkylenimino)carbonyl- oder (Alkylenimino)sulfonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,

eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Perfluoralkylsulfonylamino-, N-Alkyl-perfluoralkylsulfonylamino-, Alkylcarbonyl-, Arylcarbonyl-, Aralkylcarbonyl-, Aryl-hydroxymethyl-, Aralkyl-hydroxymethyl-, Carboxy-, Alkoxycarbonyl-, Aralkoxycarbonyl-, Aminocarbonyl-, Alkylaminocarbonyl-, Dialkylaminocarbonyl-, Arylaminocarbonyl-, N-Alkyl-arylaminocarbonyl-, Aralkylaminocarbonyl-, N-Alkylaralkylaminocarbonyl-, N-Hydroxy-aminocarbonyl-, N-Hydroxyalkylaminocarbonyl-, N-Alkoxy-aminocarbonyl-, N-Alkoxy-alkylaminocarbonyl-, Cyano-, Azido-, N-Cyano-amino- oder N-Cyanoalkylaminogruppe,

eine Sulfo-, Alkoxysulfonyl-, Aminosulfonyl-, Alkylaminosulfonyl-, Dialkylaminosulfonyl-, Arylaminosulfonyl-, N-Alkylarylaminosulfonyl-, Aralkylaminosulfonyl- oder N-Alkyl-aralkylaminosulfonylgruppe,

eine Phosphono-, O-Alkyl-phosphono-, O,O'-Dialkyl-phosphono-, O-Aralkyl-phosphono- oder O,O'-Diaralkylphosphonogruppe,

eine durch $R_4$ substituierte Alkyl-oder Alkoxygruppe, wobei $R_4$ eine Hydroxy-, Alkoxy-, Aryloxy-, Aralkoxy-, Amino-, Alkylamino-, Dialkylamino-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, Carboxy-, Alkoxycarbonyl-, Aminocarbonyl-, Alkylaminocarbonyl-, Dialkylaminocarbonyl- oder Cyanogruppe,

eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe oder in den vorstehend erwähnten 6- bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein können, oder

eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann, darstellt,

$R_3$ ein Wasserstoff-, Fluor-, Chlor- oder Bromatom, eine Alkyl-, Alkoxy- oder Trifluormethylgruppe oder

$R_2$ zusammen mit $R_3$, sofern diese an benachbarte Kohlenstoffatome gebunden sind, auch eine gegebenenfalls durch eine oder zwei Alkylgruppen substituierte Methylendioxygruppe, eine gegebenenfalls durch ein oder zwei Alkylgruppen substituierte n-$C_{3-6}$-Alkylengruppe oder eine gegebenenfalls durch ein oder zwei Fluor-, Chlor-, Brom- oder Jodatome, durch eine oder zwei Hydroxy-, Alkyl-, Alkoxy-, Trifluormethyl- oder Cyangruppen substituierte 1,3-Butadien-1,4-diylengruppe, wobei die Substituenten gleich oder verschieden sein können, oder

$R_a$ zusammen mit $R_1$, sofern $R_1$ in o-Stellung zu dem durch $R_a$ substituierten Stickstoffatom steht, auch eine gegebenenfalls durch eine oder zwei Alkylgruppen substituierte n-$C_{2-4}$-Alkylengruppe darstellen, und

$R_c$ ein Wasserstoff-, Fluor-, Chlor-, Brom- oder Jodatom,

eine Alkyl-, $C_{3-7}$-Cycloalkyl-, $C_{3-7}$-Cycloalkyl-$C_{1-3}$-alkyl-, Aryl-, Aralkyl-, Hydroxy-, Aryloxy-, Aralkoxy-, Mercapto-, $C_{1-8}$-Alkylsulfenyl-, $C_{1-8}$-Alkylsulfinyl-, $C_{1-8}$-Alkylsulfonyl-, $C_{4-7}$-Cycloalkylsulfenyl-, $C_{4-7}$-Cycloalkylsulfinyl-, $C_{4-7}$-Cycloalkylsulfonyl-, $C_{3-7}$-Cycloalkyl-$C_{1-3}$-alkylsulfenyl-, $C_{3-7}$-Cycloalkyl-$C_{1-3}$-alkylsulfinyl-, $C_{3-7}$-Cycloalkyl-$C_{1-3}$-al-

kylsulfonyl-, Arylsulfenyl-, Arylsulfinyl-, Arylsulfonyl-, Aralkylsulfenyl-, Aralkylsulfinyl- oder Aralkylsulfonylgruppe,

eine $C_{1-8}$-Alkoxygruppe, die durch eine Alkoxycarbonyl-, Cyano-, Carboxy-, Aminocarbonyl-, Alkylaminocarbonyl- oder Dialkylaminocarbonylgruppe substituiert sein kann,

eine $C_{2-8}$-Alkoxygruppe, die durch eine Hydroxy-, Alkoxy-, Hydroxy-$C_{2-4}$-alkylamino-, Amino-, Alkylamino-, Dialkylamino-, Alkylcarbonylamino-, N-Alkyl-N-(alkylcarbonyl)amino-, Alkylsulfonylamino-, N-Alkyl-N-(alkylsulfonyl) amino-, Alkoxycarbonylamino- oder N-Alkyl-N-(alkoxycarbonyl)aminogruppe, durch eine gegebenenfalls durch 1 bis 4 Alkylgruppen substituierte 5- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe und zusätzlich in den vorstehend erwähnten 6- bis 7-gliedrigen Alkyleniminogruppen eine Methylengruppe in 4-Stellung durch ein Sauerstoff- oder Schwefelatom, durch eine Carbonyl-, Sulfinyl-, Sulfonyl-, Imino-, N-Alkyl-imino-, N-Alkylcarbonyl-imino-, N-Formylimino-, N-Dialkylaminocarbonyl-imino-, N-Alkoxycarbonyl-imino-, N-Alkylsulfonyl-imino-, N-Aryl-imino- oder N-Aralkyl-iminogruppe ersetzt sein kann, substituiert ist,

eine durch zwei Hydroxy- oder Alkoxygruppen substituierte $C_{3-8}$-Alkoxygruppe,

eine durch eine $C_{3-7}$-Cycloalkylgruppe substituierte $C_{1-8}$-Alkoxygruppe, wobei der Cycloalkylteil jeweils durch 1 bis 4 Alkylgruppen substituiert sein kann und wobei in den vorstehend erwähnten $C_{4-7}$-Cycloalkylteilen jeweils eine Methylengruppe durch ein Sauerstoff- oder Schwefelatom, durch eine Carbonyl-, Sulfinyl-, Sulfonyl-, Imino-, N-Alkyl-imino-, N-Alkylcarbonyl-imino-, N-Alkoxycarbonyl-imino-, N-Alkylsulfonyl-imino-, N-Aryl-imino- oder N-Aralkyl-iminogruppe ersetzt sein kann,

eine gegebenenfalls durch eine oder zwei Hydroxygruppen oder durch eine Alkoxy-, Alkoxycarbonyl-, Cyano-, Carboxy-, Aminocarbonyl-, Alkylaminocarbonyl-, Dialkylaminocarbonyl-, Hydroxy-$C_{2-4}$-alkylamino-, Amino-, Alkylamino-, Dialkylamino-, Alkylcarbonylamino-, N-Alkyl-N-(alkylcarbonyl)amino-, Alkylsulfonylamino-, N-Alkyl-N-(alkylsulfonyl)amino-, Alkoxycarbonylamino- oder N-Alkyl-N-(alkoxycarbonyl)aminogruppe substituierte $C_{4-7}$-Cycloalkoxygruppe, wobei in den vorstehend erwähnten $C_{5-7}$-Cycloalkoxygruppen jeweils eine Methylengruppe durch ein Sauerstoff- oder Schwefelatom, durch eine Sulfinyl-, Sulfonyl-, Imino-, N-Alkyl-imino-, N-Alkylcarbonyl-imino-, N-Alkoxycarbonyl-imino-, N-Alkylsulfonyl-imino-, N-Aryl-imino- oder N-Aralkyl-iminogruppe ersetzt sein kann,

eine gegebenenfalls durch eine Arylgruppe oder $C_{3-7}$-Cycloalkylgruppe substituierte $C_{3-8}$-Alkenyloxygruppe, wobei der Vinylteil nicht mit dem Sauerstoffatom verknüpft sein kann, eine gegebenenfalls durch eine Arylgruppe oder $C_{3-7}$-Cycloalkylgruppe substituierte $C_{3-8}$-Alkinyloxygruppe, wobei der Ethinylteil nicht mit dem Sauerstoffatom verknüpft sein kann,

eine gegebenenfalls durch 1 bis 4 Alkylgruppen oder 1 bis 2 Arylgruppen substituierte 4- bis 8-gliedrige Alkyleniminogruppe, die zusätzlich durch den Rest $R_5$ substituiert sein kann, wobei

$R_5$ eine Aryl-, Aralkyl-, Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonyloxy-, Arylcarbonyloxy-, Amino-, Alkylamino-, Hydroxy-$C_{2-4}$-alkylamino-, Dialkylamino-, Cyanamino-, Formylamino-, N-(Alkyl)-N-(hydroxy-$C_{2-4}$-alkyl) amino- oder Bis-(hydroxy-$C_{2-4}$-alkyl)aminogruppe,

eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,

eine gegebenenfalls durch 1 bis 4 Alkylgruppen oder eine Hydroxyalkylgruppe substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe ersetzt sein können,

eine gegebenenfalls durch 1 bis 4 Alkylgruppen oder eine Hydroxyalkylgruppe substituierte 6- oder 7-gliedrige Alkyleniminogruppe, wobei jeweils eine Methylengruppe in 4-Stellung des Alkyleniminoteils durch ein Sauerstoff- oder Schwefelatom, durch eine Carbonyl-, Sulfinyl-, Sulfonyl-, Imino-, N-Alkylimino-, N-Alkylcarbonylimino-, N-Alkylsulfonyl-imino-, N-Arylimino- oder N-Aralkyl-iminogruppe ersetzt ist und zusätzlich im Alkyleniminoteil der vorstehend erwähnten Gruppen jeweils eine oder zwei der zu den Stickstoffatomen benachbarten Methylengruppen durch eine Carbonylgruppe ersetzt sein können,

eine durch eine Hydroxy-, Alkoxy-, Amino-, Alkylamino-, Dialkylamino-,Alkylcarbonylamino-, N-Alkyl-alkylcarbonylamino-, Alkylsulfonylamino-, N-Alkyl-alkylsulfonylamino- oder eine Hydroxyalkylgruppe substituierte 4- bis 7-gliedrige Alkyleniminogruppe,

eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe,

eine $(NR_7R_8)CONR_6$- oder $(NR_7R_8)SO_2NR_6$-Gruppe, in denen

> $R_6$, $R_7$ und $R_8$, die gleich oder verschieden sein können, jeweils ein Wasserstoffatom oder eine Alkylgruppe oder $R_6$ und $R_7$ zusammen eine n-$C_{2-4}$-Alkylengruppe und $R_8$ ein Wasserstoffatom oder eine Alkylgruppe darstellen,

eine Carboxyalkyl-, Alkoxycarbonylalkyl-, Aminocarbonylalkyl-, Alkylaminocarbonylalkyl- oder Dialkylaminocarbonylalkylgruppe,

eine gegebenenfalls durch 1 bis 4 Alkylgruppen substituierte (Alkylenimino)carbonylalkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,

eine (Carboxyalkyl)oxy-, (Alkoxycarbonylalkyl)oxy-, (Aminocarbonylalkyl)oxy-, (Alkylaminocarbonylalkyl)oxy- oder (Dialkylaminocarbonylalkyl)oxy-gruppe,

eine gegebenenfalls durch 1 bis 4 Alkylgruppen substituierte [(Alkylenimino)carbonylalkyl]oxy-gruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,

eine Cyanoalkyl-, Hydroxyalkyl-, Alkoxyalkyl-, Aryloxyalkyl-Aminoalkyl-, Alkylaminoalkyl- oder Dialkylaminoalkylgruppe,

eine gegebenenfalls durch 1 bis 4 Alkylgruppen substituierte (Alkylenimino)alkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,

eine Alkylcarbonylaminoalkyl-, N-Alkyl-alkylcarbonylaminoalkyl-, Alkylsulfonylaminoalkyl-, N-Alkyl-alkylsulfonylaminoalkyl-, Arylcarbonylaminoalkyl-, N-Alkyl-arylcarbonylaminoalkyl-, Arylsulfonylaminoalkyl-, N-Alkyl-arylsulfonylaminoalkyl-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, Alkylsulfenylalkyl-, Alkylsulfinylalkyl-, Alkylsulfonylalkyl-, Arylsulfenylalkyl-, Arylsulfinylalkyl- oder Arylsulfonylalkylgruppe oder eine $C_{3-7}$-Cycloalkylgruppe, wobei in einer $C_{5-7}$-Cycloalkylgruppe eine Methylengruppe durch ein Sauerstoff- oder Schwefelatom, durch eine Sulfinyl-, Sulfonyl-, Imino- oder N-Alkyl-iminogruppe ersetzt sein kann, darstellt,

oder $R_c$ eine gegebenenfalls durch 1 bis 4 Alkylgruppen oder durch eine Arylgruppe substituierte 6- bis 8-gliedrige Alkyleniminogruppe, die zusätzlich durch den Rest $R_5$ substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl-, N-Oxido-N-alkylimino- oder $R_9$N-Gruppe ersetzt ist, wobei

$R_9$ ein Wasserstoffatom, eine Alkyl-, Hydroxy-$C_{2-4}$-alkyl-, Alkoxy-$C_{2-4}$-alkyl-, Amino-$C_{2-4}$-alkyl-, Alkylamino-$C_{2-4}$-alkyl-, Dialkylamino-$C_{2-4}$-alkyl-, (Hydroxy-$C_{2-4}$-alkoxy)$C_{2-4}$-alkyl-, Aminocarbonylalkyl-, Alkylaminocarbonylalkyl-, Dialkylaminocarbonylalkyl-, Aryl-, Aralkyl-, Formyl-, Alkylcarbonyl-, Alkylsulfonyl-, Arylcarbonyl-, Aryl-sulfonyl-, Aralkylcarbonyl-, Aralkylsulfonyl-, Alkoxycarbonyl-, Cyano-, Aminocarbonyl-, Alkylaminocarbonyl- oder Dialkylaminocarbonylgruppe oder eine (Alkylenimino)carbonylgruppe mit jeweils 4 bis 7 Ringatomen im Alkyleniminoteil, wobei in einem 6- bis 7-gliedrigen Alkyleniminoteil eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann, darstellt,

oder $R_c$ eine gegebenenfalls durch 1 bis 4 Alkylgruppen substituierte 1-Pyrrolidinylgruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 2 bis 4 Kohlenstoffatome enthält, wenn sich die zwei Wasserstoffatome an Kohlenstoffatomen befinden, die durch ein Atom getrennt sind, wobei die vorstehend erwähnten 1-Pyrrolidinylgruppen zusätzlich durch den Rest $R_5$, der wie vorstehend erwähnt definiert ist, substituiert sein können,

eine gegebenenfalls durch 1 bis 4 Alkylgruppen substituierte 1-Piperidinyl- oder 1-Azacyclohept-l-yl-gruppe, in denen zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 1 bis 4 Kohlenstoffatome enthält, wenn sich die zwei Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind, wobei die vorstehend erwähnten 1-Piperidinyl- und 1-Azacyclohept-1-yl-gruppen zusätzlich durch den Rest $R_5$, der wie vorstehend erwähnt definiert ist, substituiert sein können,

eine gegebenenfalls durch 1 bis 4 Alkylgruppen substituierte 1-Pyrrolidinylgruppe, in der zwei Wasserstoffatome in 3-Stellung durch eine $-O-CH_2CH_2-O-$ oder $-O-CH_2CH_2CH_2-O-$Gruppe substituiert sind,

eine gegebenenfalls durch 1 bis 4 Alkylgruppen substituierte 1-Piperidinyl- oder 1-Azacyclohept-1-yl-gruppe, in denen in 3-Stellung oder in 4-Stellung jeweils zwei Wasserstoffatome durch eine $-O-CH_2CH_2-O-$ oder $-O-CH_2CH_2CH_2-O-$Gruppe substituiert sind,

eine Gruppe der Formel

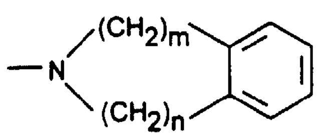

in der

m und n, die gleich oder verschieden sein können, die Zahlen 1 bis 3 oder
m die Zahl 0 und n die Zahl 2, 3 oder 4 bedeuten, wobei zusätzlich der obige Benzoteil durch Fluor-, Chlor-, Brom- oder Jodatome, durch Alkyl-, Trifluormethyl-, Hydroxy-, Alkoxy- oder Cyanogruppen und der obige gesättigte cyclische Iminoteil durch 1 oder 2 Alkylgruppen, wobei die Substituenten jeweils gleich oder verschieden sein können, mono- oder disubstituiert sein kann, oder

eine ($R_{10}NR_{11}$)-Gruppe, in der

$R_{10}$ und $R_{11}$ die gleich oder verschieden sein können, jeweils ein Wasserstoffatom,

eine $C_{1-16}$-Alkylgruppe, die durch 1 oder 2 Aryl- oder $C_{3-7}$-Cycloalkylgruppen, durch eine Carboxy-, Alkoxycarbonyl-, Aminocarbonyl-, Alkylaminocarbonyl-, Dialkylaminocärbonyl-, Hydroxy-$C_{2-4}$-alkylaminocarbonyl-, Cyano-, Hydroxy-, Alkoxy-, Aryloxy-, Aralkoxy-, $C_{2-4}$-Alkylendioxy-, Alkylcarbonyloxy-, Arylcarbonyloxy-, Formylamino-,

Amino-, Alkylamino- oder Dialkylaminogruppe, durch eine gegebenenfalls durch 1 bis 4 Alkylgruppen substituierte (Alkylenimino)carbonylgruppe mit jeweils 4 bis 7 Ringatomen im Alkyleniminoteil, wobei in einem 6- oder 7-gliedrigen Alkyleniminoteil jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,

durch eine gegebenenfalls durch 1 bis 4 Alkylgruppen substituierte 4- bis 7-gliedrige Alkyleniminogruppe, wobei in den vorstehend erwähnten 6- oder 7-gliedrigen Alkyleniminogruppen jeweils eine Methylengruppe in 4-Stellung durch ein Sauerstoff- oder Schwefelatom, durch eine Sulfinyl-, Sulfonyl- oder $R_9$N-Gruppe ersetzt sein kann, wobei $R_9$ wie eingangs definiert ist, und zusätzlich in den vorstehend erwähnten 5- bis 7-gliedrigen Alkyleniminogruppen jeweils eine oder zwei zu den Stickstoffatomen benachbarte Methylengruppen durch eine Carbonylgruppe ersetzt sein können,

durch eine Alkylsulfonylamino-, N-Alkyl-alkylsulfonylamino-, Arylcarbonylamino-, N-Alkyl-arylcarbonylamino-, Alkylcarbonylamino-, N-Alkyl-alkylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe, durch eine $(R_8NR_7)$-CO-$NR_6$- oder $(R_8NR_7)$-$SO_2$-$NR_6$-Gruppe, wobei $R_6$, $R_7$ und $R_8$ wie eingangs definiert sind, durch eine Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe, durch eine durch $R_5$ und gegebenenfalls zusätzlich durch 1 bis 4 Alkylgruppen substituierte $C_{4-7}$-Cycloalkylgruppe, wobei $R_5$ wie eingangs definiert ist, durch eine gegebenenfalls durch 1 bis 4 Alkylgruppen substituierte $C_{5-7}$-Cycloalkylgruppe, in der eine Methylengruppe durch ein Sauerstoff- oder Schwefelatom, durch eine Sulfinyl-, Sulfonyl- oder $NR_9$-Gruppe ersetzt ist oder durch ein Fluor-, Chlor-, Brom- oder Jodatom substituiert sein kann,

eine durch 2 oder 3 Fluoratome substituierte $C_{2-10}$-Alkylgruppe,

eine durch 4 oder 5 Fluoratome substituierte $C_{3-10}$-Alkylgruppe,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine durch 2 bis 5 Hydroxy- oder Alkoxygruppen substituierte $C_{3-10}$-Alkylgruppe,

eine durch eine Arylgruppe und eine Hydroxygruppe substituierte $C_{2-6}$-Alkylgruppe, die zusätzlich durch eine Amino-, Alkylamino-, Dialkylamino-, Hydroxy- oder Alkoxygruppe substituiert sein kann,

eine durch eine Amino-, Alkylamino-, Dialkylamino-, Alkylcarbonylamino- oder Alkoxycarbonylaminogruppe und zusätzlich durch eine Hydroxy- oder Alkoxygruppe substituierte $C_{3-6}$-Alkylgruppe,

eine gegebenenfalls durch eine Arylgruppe oder $C_{3-7}$-Cycloalkylgruppe substituierte Alkenyl- oder Alkinylgruppe mit jeweils 3 bis 6 Kohlenstoffatomen, wobei der Vinyl- oder Ethinylteil nicht mit dem Stickstoffatom verknüpft sein kann,

eine $C_{2-4}$-Alkylgruppe, die durch eine $C_{2-4}$-Alkoxygruppe substituiert ist, welche in ω-Position durch eine Hydroxy- oder Alkoxygruppe substituiert ist,

eine Arylgruppe,

eine Cyclopropylgruppe, die durch 1 oder 2 Alkylgruppen, durch eine Aryl-, Carboxy-, Alkoxycarbonyl-, Aminocarbonyl-, Alkylaminocarbonyl- oder Dialkylaminocarbonylgruppe oder durch eine gegebenenfalls durch 1 bis 4 Alkylgruppen substituierte (Alkylenimino)carbonylgruppe mit jeweils 4 bis 7 Ringatomen im Alkyleniminoteil substituiert sein kann, wobei in den vorstehend erwähnten 6- oder 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkylimino-, N-Alkylcarbonyl-imino-, N-Alkylsulfonyl-imino-, N-Aryl-imino- oder N-Aralkyl-iminogruppe ersetzt sein kann,

eine gegebenenfalls durch 1 bis 4 Alkylgruppen substituierte $C_{4-7}$-Cycloalkylgruppe, die zusätzlich durch $R_5$, der

wie eingangs definiert ist, substituiert sein kann,

eine gegebenenfalls durch 1 bis 2 Alkylgruppen substituierte $C_{5-7}$-Cycloalkylgruppe, die zusätzlich durch eine N,N-Dialkyl-N-oxido-aminogruppe substituiert ist,

eine gegebenenfalls durch 1 bis 4 Alkylgruppen substituierte $C_{5-7}$-Cycloalkenylgruppe, wobei der Vinylteil nicht mit dem Stickstoffatom der ($R_{11}NR_{10}$)-Grupppe verknüpft sein kann,

eine gegebenenfalls durch 1 bis 4 Alkylgruppen substituierte $C_{4-7}$-Cycloalkylgruppe, die zusätzlich durch $R_5$ substituiert sein kann, wobei in dem Cycloalkylteil eine Methylengruppe durch ein Sauerstoff- oder Schwefelatom, durch eine Sulfinyl-, Sulfonyl-, N-Alkyl-N-oxido-imino- oder $R_9$N-Gruppe ersetzt ist, wobei $R_5$ und $R_9$ wie eingangs definiert sind,

eine gegebenenfalls durch 1 bis 4 Alkylgruppen substituierte $C_5$-$C_7$-Cycloalkyl- oder $C_5$-$C_7$-Cycloalkylalkylgruppe, in denen jeweils eine Methylengruppe im Cycloalkylteil durch eine Carbonylgruppe ersetzt ist,

eine gegebenenfalls durch 1 bis 4 Alkylgruppen substituierte Cyclopentyl- oder Cyclopentylalkylgruppe, in denen jeweils zwei Wasserstoffatome im Cyclopentylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 2 bis 4 Kohlenstoffatome enthält, wenn sich die zwei Wasserstoffatome an Kohlenstoffatomen befinden, die durch ein Kohlenstoffatom getrennt sind, wobei die vorstehend erwähnten Ringe zusätzlich durch den Rest $R_5$, der wie eingangs definiert ist, substituiert sein können,

eine gegebenenfalls durch 1 bis 4 Alkylgruppen substituierte Cyclohexyl-, Cyclohexylalkyl-, Cycloheptyl- oder Cycloheptylalkylgruppe, in denen jeweils zwei Wasserstoffatome im Cycloalkylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 1 bis 4 Kohlenstoffatome enthält, wenn sich die zwei Wasserstoffatome an Kohlenstoffatomen befinden, die durch ein Kohlenstoffatom getrennt sind, oder 1 bis 3 Kohlenstoffatome enthält, wenn sich die zwei Wasserstoffatome an Kohlenstoffatomen befinden, die durch zwei Kohlenstoffatome getrennt sind, wobei die vorstehend erwähnten Ringe zusätzlich durch den Rest $R_5$, der wie eingangs definiert ist, substituiert sein können,

eine gegebenenfalls durch 1 bis 4 Alkylgruppen substituierte 3-Cyclohexen-1-yl- oder 3-Cyclohexen-l-yl-alkylgruppe, in denen im Cyclohexenylteil zwei Wasserstoffatome in 2,5-Stellung durch eine n-$C_{1-3}$-Alkylenbrücke ersetzt sind,

eine 3-Chinuclidinyl-, 4-Chinuclidinyl-, 2-Chinuclidinylalkyl-, 3-Chinuclidinyl-alkyl-, 4-Chinuclidinyl-alkyl-, Azabicyclo[2.2.1]hept-4-yl-, Azabicyclo[2.2.1]hept-4-yl-alkyl- oder Adamantylgruppe, oder

$R_{10}$ ein Wasserstoffatom oder eine Alkylgruppe und $R_{11}$ eine Hydroxy-, Alkoxy- oder Cyanogruppe darstellen,

wobei, sofern nichts anderes erwähnt wurde,

unter den bei der Definition der vorstehend erwähnten Reste erwähnten Arylteilen eine Phenylgruppe zu verstehen ist, die jeweils durch $R_{12}$ monosubstituiert, durch $R_{13}$ mono-, di- oder trisubstituiert oder durch $R_{12}$ monosubstituiert und zusätzlich durch $R_{13}$ mono- oder disubstituiert sein kann, wobei die Substituenten gleich oder verschieden sein können, und

$R_{12}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Perfluoralkoxy-, Nitro-, Amino-, Alkylcarbonylamino-, N-Alkyl-alkylcarbonylamino-, Alkylamino-, Dialkylamino-, Hydroxy-$C_{2-4}$-alkylamino-, N-Alkyl- (hydroxy-$C_{2-4}$-alkyl)amino-, Bis-(hydroxy-$C_{2-4}$-alkyl)amino-, Phenylalkylcarbonylamino-, Phenylcarbonylamino-, Alkylsulfonylamino-, Phenylalkylsulfonylamino-, Phenylsulfonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl-, Dialkylaminosulfonyl-, ($R_8NR_7$)-CO-$NR_6$- oder ($R_8NR_7$)-$SO_2$-$NR_6$-Gruppe, wobei $R_6$, $R_7$ und $R_8$ wie eingangs definiert sind,

eine gegebenenfalls durch 1 bis 4 Alkylgruppen oder eine Hydroxyalkylgruppe substituierte 5- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoffatom oder eine $R_9$N-Gruppe ersetzt sein kann, wobei $R_9$ wie eingangs definiert ist,

eine gegebenenfalls durch 1 bis 4 Alkylgruppen oder eine Hydroxyalkylgruppe substituierte 5- bis 7-gliedrige Alkyleniminogruppe, wobei jeweils eine oder zwei zu dem Stickstoffatom benachbarte Methylengruppen jeweils durch eine Carbonylgruppe ersetzt ist, und

$R_{13}$ eine Alkyl-, Hydroxy- oder Alkoxygruppe, ein Fluor-, Chlor-, Brom- oder Jodatom darstellen, wobei zwei Reste $R_{13,}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

sowie, soweit nichts anderes erwähnt wurde, die vorstehend erwähnten Alkyl-, Alkylen- und Alkoxyteile jeweils 1 bis 4 Kohlenstoffatome enthalten,

insbesondere diejenigen der vorstehend erwähnten Verbindungen der allgemeinen Formel I mit der Maßgabe, daß, sofern nichts anderes erwähnt wurde, jedes Kohlenstoffatom in den vorstehend erwähnten Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

Bevorzugte Verbindungen der obigen allgemeinen Formel I sind diejenigen, in denen

$R_a$ ein Wasserstoffatom oder eine Alkylgruppe,

$R_b$ eine durch die Reste $R_1$ bis $R_3$ substituierte Phenylgruppe, wobei

$R_1$ ein Wasserstoff-, Fluor-, Chlor-, Brom- oder Jodatom,

eine $C_{1-6}$-Alkyl-, Hydroxy- oder $C_{1-6}$-Alkoxygruppe,

eine $C_{3-6}$-Cycloalkyl- oder $C_{5-6}$-Cycloalkoxygruppe,

eine $C_{2-5}$-Alkenyl- oder $C_{3-5}$-Alkenyloxygruppe, wobei der Vinylteil nicht mit dem Sauerstoffatom verknüpft sein kann,

eine $C_{2-5}$-Alkinyl- oder $C_{3-5}$-Alkinyloxygruppe, wobei der Ethinylteil nicht mit dem Sauerstoffatom verknüpft sein kann,

eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfonyl-, Arylsulfenyl-, Arylsulfinyl-, Arylsulfonyl-, Aralkylsulfenyl-, Aralkylsulfinyl- oder Aralkylsulfonylgruppe,

eine durch 1 bis 3 Fluoratome substituierte Methyl- oder Methoxygruppe,

eine durch 1 bis 5 Fluoratome substituierte $C_{2-4}$-Alkyl- oder $C_{2-4}$-Alkoxygruppe,

eine Nitro-, Amino-, Alkylamino-, Dialkylamino-, $C_{3-6}$-Cycloalkylamino-, N-Alkyl-$C_{3-6}$-cycloalkylamino-, Arylamino-, N-Alkyl-arylamino-, Aralkylamino- oder N-Alkyl-aralkylaminogruppe,

eine 5- bis 7-gliedrige Alkyleniminogruppe, wobei jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoffatom, durch eine Imino- oder N-Alkyl-iminogruppe ersetzt sein können,

eine (Alkylenimino) carbonyl- oder (Alkylenimino) sulfonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoffatom, durch eine Imino- oder N-Alkyl-iminogruppe ersetzt sein kann,

eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralakylcarbonylamino-, Aralkylsulfonylamino-, N-Alkyl-aralkylsulfonylamino-, Trifluormethylsulfonylamino-, N-Alkyl-trifluormethylsulfonylamino-, Alkylcarbonyl-, Arylcarbonyl-, Aralkylcarbonyl-, Carboxy-, Alkoxycarbonyl-, Aminocarbonyl-, Alkylaminocarbonyl-, Dialkylaminocarbonyl-, Arylaminocarbonyl-, N-Alkylarylaminocarbonyl-, Aralkylaminocarbonyl-, N-Alkyl-aralkylaminocarbonyl-, N-Hydroxy-aminocarbonyl-, N-Hydroxy-alkylaminocarbonyl-, N-Alkoxy-aminocarbonyl-, N-Alkoxy-alkylaminocarbonyl-, Cyano-, Azido-, N-Cyano-amino- oder N-Cyano-alkylaminogruppe,

eine Sulfo-, Aminosulfonyl-, Alkylaminosulfonyl-, Dialkylaminosulfonyl-, Arylaminosulfonyl-, N-Alkyl-arylaminosulfonyl-, Aralkylaminosulfonyl- oder N-Alkyl-aralkylaminosulfonylgruppe,

eine Phosphono-, O-Alkyl-phosphono-, O,O'-Dialkyl-phosphonooder O,O'-Diaralkyl-phosphonogruppe,

eine durch $R_4$ substituierte Alkyl- oder Alkoxygruppe, wobei

$R_4$ eine Hydroxy-, Alkoxy-, Aryloxy-, Amino-, Alkylamino-, Dialkylamino-, Alkylsulfenyl-, Alkylsulfinyl-, Alkylsulfonyl-, Arylsulfenyl-, Arylsulfinyl-, Arylsulfonyl-, Carboxy-, Alkoxycarbonyl-, Aminocarbonyl-, Alkylaminocarbonyl-, Dialkylaminocarbonyl- oder Cyanogruppe oder

eine (Alkylenimino)carbonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoffatom, durch eine Imino- oder N-Alkyl-iminogruppe ersetzt sein kann, darstellt,

$R_2$ ein Wasserstoff-, Fluor-, Chlor- oder Bromatom, eine Alkyl-, Trifluormethyl-, Hydroxy-, Alkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Trifluormethylsulfonylamino-, N-Alkyl-trifluormethylsulfonylamino- oder Cyanogruppe und

$R_3$ ein Wasserstoff-, Fluor-, Chlor- oder Bromatom, eine Alkyl-, Trifluormethyl- oder Alkoxygruppe oder

$R_2$ zusammen mit $R_3$, sofern diese an benachbarte Kohlenstoffatome gebunden sind, auch eine gegebenenfalls durch eine oder zwei Alkylgruppen substituierte Methylendioxygruppe, eine gegebenenfalls durch eine oder zwei Alkylgruppen substituierte n-$C_{3-6}$-Alkylengruppe oder eine gegebenenfalls durch ein Fluor-, Chlor- oder Bromatom, durch eine Hydroxy-, Alkyl-, Alkoxy-, Trifluormethyl- oder Cyangruppe substituierte 1,3-Butadien-1,4-diylengruppe oder

$R_a$ zusammen mit $R_1$, sofern $R_1$ in o-Stellung zu dem durch $R_a$ substituierten Stickstoffatom steht, auch eine n-$C_{2-3}$-Alkylengruppe darstellen, und

$R_c$ ein Wasserstoff- oder Chloratom,

eine Alkyl-, Aryl-, Aralkyl-, Mercapto-, Alkylsulfenyl-, Alkylsulfinyl- oder Alkylsulfonylgruppe,

eine Hydroxy-, Aryloxy- oder Aralkoxygruppe,

eine $C_{1-6}$-Alkoxygruppe, die durch eine Alkoxycarbonyl-, Cyano-, Carboxy-, Aminocarbonyl-, Alkylaminocarbonyl- oder Dialkylaminocarbonylgruppe substituiert sein kann,

eine $C_{2-6}$-Alkoxygruppe, die durch eine Hydroxy-, Alkoxy-, Hydroxy-$C_{2-4}$-alkylamino-, Amino-, Alkylamino-, Dialkylamino-, Alkylcarbonylamino-, Alkylsulfonylamino- oder Alkoxycarbonylaminogruppe, durch eine gegebenenfalls durch 1 bis 2 Alkylgruppen substituierte 5- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoffatom oder durch eine Carbonyl-, Imino-, Alkyl-imino-, Alkylcarbonyl-imino-, Alkoxycarbonyl-imino-, Alkylsulfonyl-imino-, Formyl-imino-, Dialkylaminocarbonyl-imino-, Aryl-imino- oder Aralkyl-iminogruppe ersetzt sein kann, substituiert ist,

eine durch zwei Hydroxy- oder Alkoxygruppen substituierte $C_{3-6}$-Alkoxygruppe,

eine durch eine $C_{3-7}$-Cycloalkylgruppe substituierte Alkoxygruppe, wobei der Cycloalkylteil jeweils durch 1 oder 2 Alkylgruppen substituiert sein kann und wobei in den vorstehend erwähnten $C_{4-7}$-Cycloalkylteilen jeweils eine Methylengruppe durch ein Sauerstoffatom, durch eine Imino-, N-Alkyl-imino-, N-Alkylcarbonyl-imino-, N-Alkoxycarbonyl-imino- oder N-Aryl-iminogruppe ersetzt sein kann,

eine gegebenenfalls durch eine Hydroxy-, Alkoxy-, Alkoxycarbonyl-, Cyano-, Carboxy-, Aminocarbonyl-, Alkylaminocarbonyl-, Dialkylaminocarbonyl-, Hydroxy-$C_{2-4}$-alkylamino-, Amino-, Alkylamino-, Dialkylamino-, Alkylcarbonylamino-, Alkylsulfonylamino- oder Alkoxycarbonylaminogruppe substituierte $C_{4-7}$-Cycloalkoxygruppe,

eine $C_{5-7}$-Cycloalkoxygruppe, wobei in der vorstehend erwähnten Cyclopentyloxygruppe jeweils eine Methylengruppe in 3-Stellung, in den vorstehend erwähnten $C_{6-7}$-Cycloalkoxygruppen jeweils eine Methylengruppe in 3- oder 4-Stellung durch ein Sauerstoffatom oder durch eine Imino-, Alkyl-imino-, Alkylcarbonyl-imino-, Alkoxycarbonyl-imino-, Alkylsulfonyl-imino-, Aryl-imino- oder Aralkyl-iminogruppe ersetzt ist,

eine $C_{3-6}$-Alkenyloxygruppe, wobei der Vinylteil nicht mit dem Sauerstoffatom verknüpft sein kann,

eine $c_{3-6}$-Alkinyloxygruppe, wobei der Ethinylteil nicht mit dem Sauerstoffatom verknüpft sein kann,

eine gegebenenfalls durch 1 bis 4 Alkylgruppen oder eine Arylgruppe substituierte 4- bis 8-gliedrige Alkyleniminogruppe, die zusätzlich durch den Rest $R_5$ substituiert sein kann, wobei

$R_5$ eine Aryl-, Aralkyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl-, 4-Alkyl-piperazinocarbonyl-, Cyano-, Hydroxy-, Alkoxy-, Aryloxy-, Amino-, Alkylamino-, Dialkylamino-, Hydroxy-$C_{2-4}$-alkylamino-, N-Alkyl-hydroxy-$C_{2-4}$-alkylamino-, Di(hydroxy-$C_{2-4}$-alkyl)amino-, Formylamino-, Cyanamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Carboxyalkyl-, Alkoxycarbonylalkyl-, Aminocarbonylalkyl-, Alkylaminocarbonylalkyl-, Dialkylaminocarbonylalkyl-, Pyrrolidinocarbonylalkyl-, Piperidinocarbonylalkyl-, Morpholinocarbonylalkyl-, Piperazinocarbonylalkyl-, 4-Alkyl-piperazinocarbonylalkyl-, Cyanoalkyl-, Hydroxyalkyl-, Alkoxyalkyl-, Aryloxyalkyl-, Aminoalkyl-, Alkylaminoalkyl-, Dialkylaminoalkyl-, Alkylcarbonylaminoalkyl-, N-Alkyl-alkylcarbonylaminoalkyl-, Alkylsulfonylaminoalkyl-, N-Alkyl-alkylsulfonylaminoalkyl-, Arylcarbonylaminoalkyl-, N-Alkyl-arylcarbonylaminoalkyl-, Arylsulfonylaminoalkyl-, N-Alkyl-arylsulfonylaminoalkyl-, Alkylsulfenyl-, Alkylsulfinyl-, Alkylsulfonyl-, Arylsulfenyl-, Arylsulfinyl-, Arylsulfonyl-, Alkylsulfenylalkyl-, Alkylsulfinylalkyl-, Alkylsulfonylalkyl-, Arylsulfenylalkyl-, Arylsulfinylalkyl-, Arylsulfonylalkyl-, Carboxyalkoxy-, Alkoxycarbonylalkoxy-, Aminocarbonylalkoxy-, Alkylaminocarbonylalkoxy-, Dialkylaminocarbonylalkoxy-, Pyrrolidinocarbonylalkoxy-, Piperidinocarbonylalkoxy-, Morpholinocarbonylalkoxygruppe oder eine $(R_8NR_7)$-CO-$NR_6$-Gruppe, wobei
$R_6$, $R_7$ und $R_8$, die gleich oder verschieden sein können, jeweils ein Wasserstoffatom oder eine Alkylgruppe oder $R_6$ und $R_7$ zusammen eine n-$C_{2-3}$-Alkylengruppe und
$R_8$ ein Wasserstoffatom oder eine Alkylgruppe darstellen,

eine gegebenenfalls durch eine oder zwei Alkylgruppen oder eine Hydroxymethylgruppe substituierte Pyrrolidino-, Piperidino-, Morpholino-, Piperazino-, 4-Alkylpiperazino- oder 4-Alkylcarbonylpiperazinogruppe, wobei im heterocyclischen Teil der vorstehend erwähnten Gruppen jeweils eine oder zwei der zu den Stickstoffatomen benachbarten Methylengruppen durch eine Carbonylgruppe ersetzt sein können,

oder $R_c$ eine gegebenenfalls durch 1 bis 4 Alkylgruppen oder durch eine Arylgruppe substituierte 6- bis 8-gliedrige Alkyleniminogruppe, die zusätzlich durch den Rest $R_5$ substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl-, N-Oxido-N-alkylimino- oder $R_9N$-Gruppe ersetzt ist, wobei

$R_9$ ein Wasserstoffatom, eine Alkyl-, Hydroxy-$C_{2-4}$-alkyl-, Alkoxy-$C_{2-4}$-alkyl-, Hydroxy-$C_{2-4}$-alkoxy-$C_{2-4}$-alkyl-, Aminocarbonylalkyl-, Alkylaminocarbonylalkyl-, Dialkylaminocarbonylalkyl-, Amino-$C_{2-4}$-alkyl-, Alkylamino-$C_{2-4}$-alkyl-, Dialkylamino-$C_{2-4}$-alkyl-, Aryl-, Aralkyl-, Formyl-, Alkycarbonyl-, Alkylsulfonyl-, Arylcarbonyl-, Arylsulfonyl-, Aralkylcarbonyl-, Aralkylsulfonyl-, Alkoxycarbonyl-, Cyano-, Aminocarbonyl-, Alkylaminocarbonyl- oder Dialkylaminocarbonylgruppe darstellt,

oder $R_c$ eine gegebenenfalls durch 1 oder 2 Alkylgruppen substituierte 1-Pyrrolidinylgruppe, in der zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind,

eine gegebenenfalls durch 1 oder 2 Alkylgruppen substituierte 1-Piperidinyl- oder 1-Azacyclohept-1-yl-gruppe, in denen zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind, oder 1 oder 2 Kohlenstoffatome enthält, wenn sich die zwei Wasserstoffatome an Kohlenstoffatomen befinden, die durch zwei Atome getrennt sind,

eine 1-Pyrrolidinylgruppe, in der zwei Wasserstoffatome in 3-Stellung durch eine $-O-CH_2CH_2-O-$ oder $-O-CH_2CH_2CH_2-O-$Gruppe ersetzt sind,

eine 1-Piperidinyl- oder 1-Azacyclohept-1-yl-gruppe, in denen zwei Wasserstoffatome in 3-Stellung oder in 4-Stellung durch eine $-O-CH_2CH_2-O-$ oder $-O-CH_2CH_2CH_2-O-$Gruppe ersetzt sind,

eine 2-Isoindolinyl-, 1,2,3,4-Tetrahydro-isochinolin-2-yl- oder 2,3,4,5-Tetrahydro-IH-3-benzazepin-3-yl-Gruppe, wobei der Benzoteil der vorstehend erwähnten Gruppen jeweils durch ein Fluor-, Chlor-, Brom- oder Jodatom, durch eine Trifluormethylgruppe oder durch eine oder zwei Alkyl- oder Alkoxygruppen substituiert sein kann, oder

eine $(R_{10}NR_{11})$-Gruppe darstellt, in der

$R_{10}$ ein Wasserstoffatom oder eine $C_{1-8}$-Alkylgruppe, die ab Position 2 durch eine Hydroxy- oder Alkoxygruppe substituiert sein kann,

$R_{11}$ ein Wasserstoffatom,

eine $C_{1-10}$-Alkylgruppe, die durch eine Aryl-, $C_{3-7}$-Cycloalkyl-, Hydroxy-, Alkoxy-, Aryloxy-, Carboxy-, Alkoxycarbonyl-, Aminocarbonyl-, Alkyl aminocarbonyl - , Dialkylaminocarbonyl-, Hydroxy-$C_{2-4}$-alkylaminocarbonyl-, Cyano-, Formylamino-, Amino-, Alkylamino- oder Dialkylaminogruppe, durch eine Pyrrolidinocarbonyl-, Piperidinocarbonyl-, Morpholinocarbonyl-, Piperazinocarbonyl- oder 4-Alkyl-piperazinocarbonylgruppe,

durch eine gegebenenfalls durch 1 oder 2 Alkylgruppen substituierte 5- bis 7-gliedrige Alkyleniminogruppe, wobei in den vorstehend erwähnten 6- oder 7-gliedrigen Alkyleniminoteilen jeweils eine Methylengruppe in 4-Stellung durch ein Sauerstoff- oder Schwefelatom, durch eine Sulfinyl-, Sulfonyl- oder $R_9N$-Gruppe ersetzt sein kann, wobei $R_9$ wie eingangs definiert ist,

durch eine Pyrrolidino-, Piperidino-, Piperazino- oder 4-Alkylpiperazinogruppe, wobei jeweils eine oder zwei der zu den Stickstoffatomen benachbarten Methylengruppen durch eine Carbonylgruppe ersetzt sind,

durch eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino- oder N-Alkyl-alkoxycarbonylaminogruppe, durch eine $(R_8NR_7)-CO-NR_6$-Gruppe, wobei $R_6$ bis $R_8$ wie eingangs definiert sind, durch eine Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe oder durch eine $C_{5-7}$-cycloalkylgruppe, in der eine Methylengruppe durch ein Sauerstoffatom oder durch eine Imino- oder Alkyliminogruppe ersetzt ist, substituiert sein kann,

eine durch ein Chloratom oder ein bis drei Fluoratome substituierte $C_{2-4}$-Alkylgruppe,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine durch 2 bis 5 Hydroxygruppen substituierte $C_{3-10}$-Alkylgruppe,

eine durch eine Hydroxy- und zusätzlich durch eine Arylgruppe substituierte $C_{2-6}$-Alkylgruppe, die gegebenenfalls zusätzlich durch eine Hydroxy- oder Alkoxygruppe substituiert sein kann,

eine durch eine Hydroxy- und zusätzlich durch eine Amino-, Alkylamino- oder Dialkylaminogruppe substituierte $C_{3-6}$-Alkylgruppe,

eine gegebenenfalls durch eine Arylgruppe substituierte Alkenyl- oder Alkinylgruppe mit jeweils 3 bis 6 Kohlenstoffatomen, wobei der Vinyl- oder Ethinylteil nicht mit dem Stickstoffatom verknüpft sein kann,

eine $C_{2-4}$-Alkylgruppe, die durch eine $C_{2-4}$-Alkoxygruppe substituiert ist, welche in ω-Position durch eine Hydroxy- oder Alkoxygruppe substituiert ist,

eine Arylgruppe,

eine Cyclopropylgruppe, die durch 1 oder 2 Alkylgruppen, durch eine Aryl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonylgruppe substituiert sein kann,

eine gegebenenfalls durch 1 oder 2 Alkylgruppen substituierte $C_{4-7}$-Cycloalkylgruppe, die zusätzlich durch $R_5$ substituiert sein kann, wobei $R_5$ wie eingangs definiert ist,

eine gegebenenfalls durch 1 oder 2 Alkylgruppen substituierte $C_{5-7}$-Cycloalkylgruppe, die zusätzlich durch eine N,N-Dialkyl-N-oxido-aminogruppe substituiert ist,

eine gegebenenfalls durch 1 oder 2 Alkylgruppen substituierte $C_{5-7}$-Cycloalkenylgruppe, wobei der Vinylteil nicht mit dem Stickstoffatom der $(R_{11}NR_{10})$-Grupppe verknüpft sein kann,

eine gegebenenfalls durch 1 oder 2 Alkylgruppen substituierte $C_{5-7}$-Cycloalkylgruppe, wobei in dem Cycloalkylteil jeweils eine Methylengruppe durch ein Sauerstoff- oder Schwefelatom, durch eine Sulfinyl-, Sulfonyl-, N-Alkyl-N-oxido-imino- oder $R_9$N-Gruppe ersetzt ist, wobei $R_9$ wie eingangs definiert ist,

eine gegebenenfalls durch 1 bis 4 Alkylgruppen substituierte $C_{5-7}$-Cycloalkylgruppe, in denen jeweils eine Methylengruppe durch eine Carbonylgruppe ersetzt ist,

eine gegebenenfalls durch 1 oder 2 Alkylgruppen substituierte $C_{4-7}$-Cycloalkylmethylgruppe, die im Cycloalkylteil zusätzlich durch $R_5$ substituiert ist, wobei $R_5$ wie vorstehend erwähnt definiert ist,

eine gegebenenfalls durch 1 bis 4 Alkylgruppen substituierte Cyclopentyl- oder Cyclopentylalkylgruppe, in denen jeweils zwei Wasserstoffatome im Cyclopentylteil durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Kohlenstoffatom getrennt sind,

eine gegebenenfalls durch 1 bis 4 Alkylgruppen substituierte Cyclohexyl-, Cyclohexylalkyl-, Cycloheptyl- oder Cycloheptylalkylgruppe, in denen jeweils zwei Wasserstoffatome im Cycloalkylteil durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Kohlenstoffatom getrennt sind, oder 1 oder 2 Kohlenstoffatome enthält, wenn sich die zwei Wasserstoffatome an Kohlenstoffatomen befinden, die durch zwei Kohlenstoffatome getrennt sind,

eine gegebenenfalls durch 1 bis 4 Alkylgruppen substituierte 5-Norbornen-2-yl- oder 5-Norbornen-2-yl-alkylgruppe,

eine 3-Chinuclidinyl-, 4-Chinuclidinyl-, 3-Chinuclidinyl-alkyl-, 4-Chinuclidinyl-alkyl-, Azabicyclo[2.2.1]hept-4-yl-, Azabicyclo[2.2.1]hept-4-yl-alkyl- oder Adamantylgruppe, oder

$R_{10}$ ein Wasserstoffatom oder eine Alkylgruppe und $R_{11}$ eine Hydroxy- oder Alkoxygruppe darstellen,

deren Tautomere, deren Stereoisomere und deren Salze,

wobei, sofern nichts anderes erwähnt wurde,

unter den bei der Definition der vorstehend erwähnten Reste erwähnten Arylteilen eine Phenylgruppe zu verstehen ist, die jeweils durch $R_{12}$ monosubstituiert, durch $R_{13}$ mono-, di- oder trisubstituiert oder durch $R_{12}$ monosubstituiert und zusätzlich durch $R_{13}$ mono- oder disubstituiert sein kann, wobei die Substituenten gleich oder verschieden sein können, und

$R_{12}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Perfluoralkoxy-, Nitro-, Amino-, Alkylamino-, Hydroxy-$C_{2-4}$-alkylamino-, N-Alkyl-hydroxy-$C_{2-4}$-alkylamino-, Di(hydroxy-$C_{2-4}$-alkyl)amino-, Dialkylamino-, Alkylcarbonylamino-, Phenylalkylcarbonylamino-, Phenylcarbonylamino-, Alkylsulfonylamino-, Phenylalkylsulfonylamino-, Phenylsulfonylamino-, N-Alkyl-alkylcarbonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkylphenylsulfonylamino-, Aminosulfonyl-, Alkylaminosulfonyl- oder Dialkylaminosulfonylgruppe,

eine gegebenenfalls durch 1 bis 2 Alkylgruppen oder eine Hydroxyalkylgruppe substituierte 5- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoffatom oder eine $R_9$N-Gruppe ersetzt sein kann,

eine gegebenenfalls durch 1 bis 2 Alkylgruppen oder eine Hydroxyalkylgruppe substituierte 5- bis 7-gliedrige Alkyleniminogruppe, wobei jeweils eine oder zwei zu dem Stickstoffatom benachbarte Methylengruppen durch jeweils eine Carbonylgruppe ersetzt sind,

eine $(R_8NR_7)$-CO-$NR_6$-Gruppe, wobei $R_6$ bis $R_8$ wie vorstehend erwähnt definiert sind,

$R_{13}$ eine Alkyl-, Hydroxy- oder Alkoxygruppe, ein Fluor-, Chlor-, Brom- oder Jodatom darstellen, wobei zwei Reste $R_{13}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

sowie, soweit nichts anderes erwähnt wurde, die vorstehend erwähnten Alkyl-, Alkylen- und Alkoxyteile jeweils 1 bis 4 Kohlenstoffatome enthalten sowie, sofern nichts anderes erwähnt wurde, jedes Kohlenstoffatom in den vorstehend erwähnten Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

Besonders bevorzugte Verbindungen der allgemeinen Formel I sind diejenigen, in denen

$R_a$ ein Wasserstoffatom oder eine Alkylgruppe,

$R_b$ eine durch die Reste $R_1$ bis $R_3$ substituierte Phenylgruppe, wobei

$R_1$ ein Wasserstoff-, Fluor-, Chlor-, Brom- oder Jodatom,

eine Alkyl-, Hydroxy-, Alkoxy-, $C_{3-6}$-Cycloalkyl- oder $C_{5-6}$-Cycloalkoxygruppe,

eine in 2-Stellung durch eine Hydroxy-, Alkoxy- oder Phenoxygruppe substituierte Ethoxygruppe,

eine $C_{2-5}$-Alkenyl- oder $C_{3-5}$-Alkenyloxygruppe, wobei der Vinylteil nicht mit dem Sauerstoffatom verknüpft sein kann,

eine $C_{2-5}$-Alkinyl- oder $C_{3-5}$-Alkinyloxygruppe, wobei der Ethinylteil nicht mit dem Sauerstoffatom verknüpft sein kann,

eine Phenyl-, Phenoxy-, Phenylalkyl-, Phenylalkoxy-, Alkoxyalkyl-, Phenoxyalkyl-, Carboxyalkyl-, Alkoxycarbonylalkyl-, Aminocarbonylalkyl-, Alkylaminocarbonylalkyl-, Dialkylaminocarbonylalkyl-, Cyanoalkyl-, Alkylsulfenyl-, Alkylsulfinyl-, Alkylsulfonyl-, Alkylsulfonyloxy-, Trifluormethylsulfenyl-, Trifluormethylsulfonyl-, Nitro-, Amino-, Alkylamino-, Dialkylamino-, Pyrrolidino-, Piperidino-, Morpholino-, Alkylcarbonylamino-, N-Alkyl-alkylcarbonylamino-, Alkylsulfonylamino-, N-Alkyl-alkylsulfonylamino-, Trifluormethylsulfonylamino-, N-Alkyl-trifluormethylsulfonylamino-, Alkylcarbonyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl- oder Cyanogruppe,

eine durch 1 bis 3 Fluoratome substituierte Methyl- oder Methoxygruppe,

eine durch 1 bis 5 Fluoratome substituierte Ethyl- oder Ethoxygruppe,

$R_2$ ein Wasserstoff-, Fluor-, Chlor- oder Bromatom, eine Alkyl-, Trifluormethyl-, Amino-, Alkylamino-, Dialkylamino-, Alkylcarbonylamino-, Alkylsulfonylamino-, Trifluormethylsulfonylamino-, Hydroxy- oder Alkoxygruppe,

$R_3$ ein Wasserstoff-, Fluor-, Chlor- oder Bromatom oder eine Alkylgruppe, oder

$R_2$ zusammen mit $R_3$, sofern diese an benachbarte Kohlenstoffatome gebunden sind, auch eine Methylendioxy- oder n-$C_{3-6}$-Alkylengruppe oder eine gegebenenfalls durch ein Fluor-, Chlor- oder Bromatom, durch eine Alkyl-, Alkoxy- oder Trifluormethylgruppe substituierte 1,3-Butadien-1,4-diylengruppe darstellen, bedeuten, und

$R_c$ ein Wasserstoff- oder Chloratom,

eine Alkyl-, Phenyl-, Mercapto-, Alkylsulfenyl-, Alkylsulfinyl- oder Alkylsulfonylgruppe,

eine Hydroxy-, Phenoxy- oder Phenyl-$C_{1-2}$-alkoxygruppe,

eine Alkoxygruppe,

eine $C_{2-4}$-Alkoxygruppe, die durch eine Hydroxy-, Alkoxy-, (2-Hydroxyethyl)amino-, Dialkylamino-, Morpholino-, 1-Pyrrolidinyl-, 1-Piperidinyl-, 4-Methyl-1-piperazinyl-, 4-Acetyl-1-piperazinyl-, 4-Methylsulfonyl-1-piperazinyl-, 4-Methoxycarbonyl- 1-piperazinyl-, 4-Formyl-1-piperazinyl- oder 4-Dimethylaminocarbonyl-1-piperazinylgruppe substituiert ist,

eine $C_{3-4}$-Alkoxygruppe, die durch zwei Hydroxygruppen substituiert ist,

eine $C_{1-2}$-Alkoxygruppe, die durch eine gegebenenfalls durch eine oder zwei Methylgruppen substituierte $C_{3-7}$-Cycloalkylgruppe substituiert ist, wobei in den vorstehend erwähnten $C_{4-6}$-Cycloalkylgruppen jeweils eine Methylengruppe durch ein Sauerstoffatom ersetzt sein kann,

eine gegebenenfalls durch eine Hydroxy-, Dialkylamino-, Alkoxy-, Carboxy-, Alkoxycarbonyl-, Aminocarbonyl-, Alkylaminocarbonyl-, Dialkylaminocarbonyl-, Alkylcarbonylamino-, Alkylsulfonylamino- oder Alkoxycarbonylaminogruppe substituierte $C_{4-6}$-Cycloalkoxygruppe,

eine Cyclopentyloxygruppe, in der die Methylengruppe in 3-Stellung durch ein Sauerstoffatom oder durch eine Alkyliminogruppe ersetzt ist,

eine Cyclohexyloxygruppe, in der die Methylengruppe in 3- oder 4-Stellung durch ein Sauerstoffatom oder durch eine Alkylimino-, Alkylcarbonyl-imino-, Alkoxycarbonyl-imino- oder Alkylsulfonyl-iminogruppe ersetzt ist,

eine gegebenenfalls durch eine oder zwei Methylgruppen substituierte Allyloxy- oder Propargyloxygruppe,

eine 1-Azetidinylgruppe,

eine 1-Pyrrolidinylgruppe, die durch 1 bis 2 Alkylgruppen, durch eine Phenyl-, Carboxy-, Hydroxyalkyl-, Aminoalkyl-, Alkylaminoalkyl-, Dialkylaminoalkyl-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonyl-Gruppe oder in 3-Stellung auch durch eine Hydroxy-, Alkoxy-, Amino-, Alkylamino-, Dialkylamino-,

Alkylcarbonylamino-, Alkoxycarbonylamino-, Formylamino-, Cyanamino-, Alkylsulfonylamino-, Dialkylaminocarbonylamino-, N-Alkyl-dialkylaminocarbonylamino-, N-Alkyl-dialkylaminocarbonylamino- oder Cyanogruppe substituiert sein kann,

eine 1-Pyrrolidinylgruppe, in der zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind,

eine 1-Piperidinylgruppe, die durch 1 bis 4 Alkylgruppen, durch eine Phenyl-, Hydroxyalkyl-, Aminoalkyl-, Alkylaminoalkyl-, Dialkylaminoalkyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonyl-Gruppe oder in 3-oder 4-Stellung auch durch eine Hydroxy-, Alkoxy-, Amino-, Alkylamino-, Dialkylamino-, Alkylcarbonylamino-, Alkoxycarbonylamino-, Formylamino-, Cyanamino-, Alkylsulfonylamino-, Dialkylaminocarbonylamino-, N-Alkyl-dialkylaminocarbonylamino- oder Cyanogruppe substituiert sein kann,

eine 1-Piperidinylgruppe, die durch 1 bis 2 Alkylgruppen oder eine Phenylgruppe und zusätzlich durch eine Hydroxygruppe substituiert ist,

eine 1-Piperidinylgruppe, in der in 3-Stellung oder in 4-Stellung zwei Wasserstoffatome durch eine $-O-CH_2CH_2-O-$ oder $-O-CH_2CH_2CH_2-O-$Gruppe ersetzt sind,

eine 1-Piperidinylgruppe, in der zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind, oder 1 oder 2 Kohlenstoffatome enthält, wenn sich die zwei Wasserstoffatome an Kohlenstoffatomen befinden, die durch zwei Atome getrennt sind,

eine gegebenenfalls durch 1 oder 2 Alkylgruppen substituierte 1-Piperidinylgruppe, in der die Methylengruppe in 4-Stellung durch ein Sauerstoff- oder Schwefelatom, durch eine Carbonyl-, Sulfinyl-, Sulfonyl-, Imino-, Alkyl-imino-, Hydroxy-$C_{2-4}$-alkylimino-, Alkoxy-$C_{2-4}$-alkyl-imino-, Aminocarbonylalkyl-imino-, Alkylaminocarbonylalkyl-imino-, Dialkylaminocarbonylalkylimino-, Amino-$C_{2-4}$-alkyl-iminö-, Alkylamino-$C_{2-4}$-alkyl-imino-, Dialkylamino-$C_{2-4}$-alkylimino-, Hydroxy-$C_{2-4}$-alkoxy-$C_{2-4}$-alkylimino-, Phenyl-imino-, Phenylalkyl-imino-, Alkylcarbonylimino-, Alkylsulfonyl-imino-, Phenylcarbonyl-imino-, Phenylsulfonyl-imino- oder N-Oxido-N-alkyl-imino-Gruppe ersetzt ist,

eine gegebenenfalls durch 1 oder 2 Alkylgruppen substituierte 1-Azacyclohept-1-yl-Gruppe, in der jeweils die Methylengruppe in 4-Stellung durch ein Sauerstoffatom, durch eine Imino-, N-Alkyl-imino-, N-Phenyl-imino-, N-Phenylalkyl-imino-, N-Alkylcarbonyl-imino-, N-Alkylsulfonyl-imino-, N-Phenylcarbonyl-imino- oder N-Phenylsulfonylimino-Gruppe ersetzt sein kann oder zwei Wasserstoffatome in 3,6-Stellung durch eine $-CH_2CH_2-$Gruppe ersetzt sein können,

eine 2-Isoindolinyl-, 1,2,3,4-Tetrahydro-isochinolin-2-yl- oder 2,3,4,5-Tetrahydro-lH-3-benzazepin-3-yl-Gruppe, die jeweils im Benzoteil durch ein Fluor-, Chlor- oder Bromatom, durch eine Alkyl-, Trifluormethyl- oder Alkoxygruppe substituiert sein können, oder

eine $(R_{10}NR_{11})$-Gruppe darstellen, in der

$R_{10}$ ein Wasserstoffatom oder eine $C_{1-6}$-Alkylgruppe, die ab Position 2 durch eine Hydroxy- oder Alkoxygruppe substituiert sein kann, und

$R_{11}$ ein Wasserstoffatom,

eine $C_{1-8}$-Alkylgruppe, die durch eine Phenyl-, $C_{3-6}$-Cycloalkyl-, Hydroxy-, Alkoxy-, Cyano-, Carboxy-, Alkoxycarbonyl-, Aminocarbonyl-, Alkylaminocarbonyl-, Dialkylaminocarbonyl-, (2-Hydroxyethyl)aminocarbonyl-, Pyrrolidinocarbonyl-, Piperidinocarbonyl-, Morpholinocarbonyl-, 1-Piperazinylcarbonyl-, 4-Alkyl-1-piperazinylcarbonyl-,

Amino-, Formylamino-, Alkylamino-, Dialkylamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Phenylcarbonylamino-, N-Alkyl-phenylcarbonylamino-, Phenylsulfonylamino-, N-Alkyl-phenylsulfonylamino-, Alkylsulfenyl-, Alkylsulfinyl-, Alkylsulfonyl-, 1-Pyrrolidinyl-, 2-Oxo-1-pyrrolidinyl-, 1-Piperidinyl-, 2-Oxo-1-piperidinyl-, Morpholino-, 1-Piperazinyl-, 4-Alkyl-1-piperazinyl-, 4-Alkylcarbonyl-1-piperazinyl-, 4-Alkylsulfonyl-1-piperazinyl-, 4-Alkoxycarbonyl-1-piperazinyl-, 4-Cyano-1-piperazinyl-, 4-Formyl-1-piperazinyl-, 4-Aminocarbonyl-1-piperazinyl-, 4-Alkylaminocarbonyl-1-piperazinyl- oder 4-Dialkylaminocarbonyl-1-piperazinyl- oder eine $(R_8NR_7)$-CO-$NR_6$-Gruppe substituiert sein kann, wobei

$R_6$ und $R_7$ zusammen eine n-$C_{2-3}$-Alkylenbrücke und
$R_8$ ein Wasserstoffatom oder eine Alkylgruppe darstellen,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine 2,2,2-Trifluorethylgruppe,

eine durch 2 bis 5 Hydroxygruppen substituierte $C_{3-10}$-Alkylgruppe,

eine durch eine Hydroxy- und zusätzlich durch eine Aminogruppe substituierte $C_{3-5}$-Alkylgruppe,

eine durch eine Phenylgruppe und zusätzlich durch eine Hydroxygruppe substituierte $C_{2-4}$-Alkylgruppe, die gegebenenfalls zusätzlich durch eine Hydroxy- oder Alkoxygruppe substituiert sein kann,

eine gegebenenfalls durch eine Phenylgruppe substituierte Alkenyl- oder Alkinylgruppe mit jeweils 3 bis 6 Kohlenstoffatomen, wobei der Vinyl- oder Ethinylteil nicht mit dem Stickstoffatom verknüpft sein kann,

eine $C_{2-4}$-Alkylgruppe, die durch eine $C_{2-4}$-Alkoxygruppe substituiert ist, welche in $\omega$-Position durch eine Hydroxy- oder Alkoxygruppe substituiert ist,

eine Phenylgruppe,

eine Phenylgruppe, die durch eine Alkylcarbonylamino-, N-Alkylalkylcarbonylamino-, (2-Hydroxyethyl)amino-, Di-(2-hydroxyethyl)amino-, N-Alkyl-(2-hydroxyethyl)amino-, Amino-, Alkylamino- oder Dialkylaminogruppe oder durch eine $(R_8NR_7)$-CO-$NR_6$-Gruppe substituiert ist, wobei $R_6$ bis $R_8$ wie vorstehend erwähnt definiert sind,

eine Phenylgruppe, die durch eine Pyrrolidino-, Piperidino-, 2-Oxo-pyrrolidino-, 2-Oxo-piperidino-, Morpholino-, 1-Piperazinyl- oder 4-Alkyl-1-piperazinyl-Gruppe substituiert ist, wobei die vorstehend erwähnten heterocyclischen Teile am Kohlenstoffgerüst jeweils durch 1 oder 2 Alkylgruppen oder durch eine Hydroxyalkylgruppe substituiert sein können,

eine $C_{3-7}$-Cycloalkylgruppe, die durch 1 oder 2 Alkylgruppen, durch eine Phenyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonylgruppe substituiert sein kann,

eine gegebenenfalls durch 1 oder 2 Methylgruppen substituierte $C_{5-7}$-Cycloalkylgruppe, die durch eine Hydroxymethyl-, Cyano-, Hydroxy-, Alkoxy-, Amino-, Alkylamino-, Dialkylamino-, 2-Hydroxyethylamino-, Di-(2-hydroxyethyl)amino-, N-Alkyl-2-hydroxyethylamino-, N,N-Dialkyl-N-oxido-amino-, Alkoxycarbonylamino-, N-Alkyl-alkoxycarbonylamino-, Alkylcarbonylamino-, N-Alkylalkylcarbonylamino-, Alkylsulfonylamino-, N-Alkyl-alkylsulfonylamino-, Phenylcarbonylamino-, N-Alkyl-phenylcarbonylamino-, Phenylsulfonylamino-, N-Alkylphenylsulfonylamino- oder durch eine $(R_8NR_7)$-CO-$NR_6$-Gruppe substituiert ist, wobei $R_6$ bis $R_8$ wie vorstehend erwähnt definiert sind,

eine gegebenenfalls durch 1 oder 2 Methylgruppen substituierte $C_{5-7}$-Cycloalkylgruppe, die durch eine Pyrrolidino-, Piperidino-, 2-Oxo-pyrrolidino-, 2-Oxo-piperidino-, Morpholino-, 1-Piperazinyl-, 4-Alkyl-1-piperazinyl- oder 4-Alkylcarbonyl-1-piperazinyl-Gruppe substituiert ist, wobei die vorstehend erwähnten heterocyclischen Teile am Kohlenstoffgerüst jeweils durch 1 oder 2 Alkylgruppen oder durch eine Hydroxymethylgruppe substituiert sein können,

eine gegebenenfalls durch 1 oder 2 Alkylgruppen substituierte $C_{5-7}$-Cycloalkenylgruppe, wobei der Vinylteil nicht

an das Stickstoffatom der ($R_{11}NR_{10}$)-Gruppe gebunden sein kann,

eine Tetrahydrofurfurylgruppe,

eine Cyclopentylgruppe, in der die Methylengruppe in 3-Stellung durch ein Sauerstoffatom, eine Imino-, Alkylimino-, Alkylcarbonylimino-, Formylimino-, Aminocarbonylimino-, Alkylaminocarbonylimino-, Alkoxycarbonylimino-, Alkylsulfonylimino-, Dialkylaminocarbonylimino- oder Cyaniminogruppe ersetzt ist,

eine Cyclohexylgruppe, in der die Methylengruppe in 3-Stellung durch eine Imino-, Alkyl-imino-, Alkylcarbonyl-imino-, Alkoxycarbonyl-imino- oder Alkylsulfonyl-imino-Gruppe ersetzt ist,

eine Cyclohexylgruppe, in der die Methylengruppe in 4-Stellung durch ein Sauerstoffatom, eine Imino-, N-Alkyl-imino-, N-Phenyl-imino-, N-Phenylalkyl-imino-, N-Formyl-imino-, N-Alkylcarbonyl-imino-, N-Phenylcarbonyl-imino-, N-Alkoxycarbonyl-imino-, N-Cyan-imino-, N-Aminocarbonyl-imino-, N-Alkylaminocarbonyl-imino-, N,N-Dialkylaminocarbonyl-imino-, N-Alkyl-N-oxido-imino-, N-Alkylsulfonyl-imino- oder N-Phenylsulfonyl-imino-Gruppe ersetzt ist,

eine Cyclohexylgruppe, in der eine Methylengruppe durch eine Carbonylgruppe ersetzt ist,

eine gegebenenfalls durch 1 bis 2 Methylgruppen substituierte Cyclopentyl- oder Cyclohexylgruppe, die durch eine Carboxyalkoxy-, Alkoxycarbonylalkoxy-, Aminocarbonylalkoxy-, Alkylaminocarbonylalkoxy-, Dialkylaminocarbonylalkoxy-, Pyrrolidinocarbonylalkoxy-, Piperidinocarbonylalkoxy-, Morpholinocarbonylalkoxy-, Carboxyalkyl-, Alkoxycarbonylalkyl-, Aminocarbonylalkyl-, Alkylaminocarbonylalkyl-, Dialkylaminocarbonylalkyl-, Pyrrolidinocarbonylalkyl-, Piperidinocarbonylalkyl- oder Morpholinocarbonylalkylgruppe substituiert ist,

eine Cyclohexylmethylgruppe, wobei der Cyclohexylteil durch eine Carboxy-, Aminocarbonyl-, Alkylaminocarbonyl-, Dialkylaminocarbonyl-, Pyrrolidinocarbonyl-, Piperidinocarbonyl-, Morpholinocarbonyl-, Alkoxycarbonyl- oder Hydroxymethylgruppe substituiert ist,

eine gegebenenfalls durch 1 bis 3 Methylgruppen substituierte Cyclohexyl- oder Cyclohexylmethylgruppe, in denen jeweils im Cyclohexylteil zwei Wasserstoffatome durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Kohlenstoffatom getrennt sind, oder 1 oder 2 Kohlenstoffatome enthält, wenn sich die zwei Wasserstoffatome an Kohlenstoffatomen befinden, die durch zwei Kohlenstoffatome getrennt sind,

eine gegebenenfalls durch 1 bis 3 Methylgruppen substituierte 5-Norbornen-2-yl- oder 5-Norbornen-2-yl-methylgruppe,

eine 3-Chinuclidinyl-, 4-Chinuclidinyl- oder Adamantylgruppe bedeuten, oder

$R_{10}$ ein Wasserstoffatom oder eine Alkylgruppe und $R_{11}$ eine Hydroxy- oder Alkoxygruppe bedeuten,

deren Tautomere, deren Stereoisomere und deren Salze,

wobei die vorstehend erwähnten Phenylreste jeweils durch ein Fluor-, Chlor- oder Bromatom, durch eine Nitro-, Alkyl-, Alkoxy-, Trifluormethyl- oder Hydroxygruppe substituiert sein können und, sofern nichts anderes erwähnt wurde, die vorstehend erwähnten Alkyl-, Alkylen- und Alkoxyteile jeweils 1 bis 4 Kohlenstoffatome enthalten sowie, sofern nichts anderes erwähnt wurde, jedes Kohlenstoffatom in den vorstehend erwähnten Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

Ganz besonders bevorzugte Verbindungen der allgemeinen Formel I sind diejenigen, in denen

$R_a$ ein Wasserstoffatom oder eine Methylgruppe,

$R_b$ eine 2-Naphthyl-, 1,2,3,4-Tetrahydro-6-naphthyl- oder 5-Indanylgruppe oder eine durch die Reste $R_1$ bis $R_3$

substituierte Phenylgruppe, wobei

$R_1$ ein Wasserstoff-, Fluor-, Chlor-, Brom- oder Jodatom,

eine $C_{1-4}$-Alkyl-, $C_{1-4}$-Alkoxy-, $C_{3-6}$-Cycloalkyl-, $C_{5-6}$-Cycloalkoxy-, Cyano-, Methoxycarbonyl-, Ethoxycarbonyl-, Ethinyl- oder Nitrogruppe,

eine durch 1 bis 3 Fluoratome substituierte Methyl- oder Methoxygruppe,

eine durch 1 bis 5 Fluoratome substituierte Ethyl- oder Ethoxygruppe,

$R_2$ ein Wasserstoff-, Fluor- oder Chloratom, eine Methyl-, Hydroxy-, Methoxy-, Amino-, $C_{1-2}$-Alkylamino-, Di-$C_{1-2}$-alkylamino-, $C_{1-2}$-Alkylcarbonylamino-, $C_{1-2}$-Alkylsulfonylamino-, Trifluormethylsulfonylamino- oder Trifluormethylgruppe,

$R_3$ ein Wasserstoff-, Fluor-, Chlor- oder Bromatom oder eine Methylgruppe und

$R_c$ ein Wasserstoffatom, eine Methyl-, Phenyl-, 4-Methoxyphenyl-, Methylsulfenyl-, Methylsulfinyl- oder Methylsulfonylgruppe,

eine Hydroxygruppe,

eine $C_{1-4}$-Alkoxygruppe,

eine Ethoxygruppe, die in 2-Stellung durch eine Hydroxy-, Methoxy-, Morpholino- oder (2-Hydroxyethyl)aminogruppe substituiert ist,

eine 2-Propyloxygruppe, die in 1-Stellung durch eine Methoxy- oder Dimethylaminogruppe substituiert ist,

eine Methoxygruppe, die durch eine 2-Tetrahydrofuryl-, 2-Tetrahydropyranyl- oder 3-Methyl-3-oxetanylgruppe substituiert ist,

eine Cyclobutyloxygruppe,

eine gegebenenfalls in 3-Stellung durch eine Hydroxygruppe substituierte Cyclopentyloxygruppe,

eine Cyclopentyloxygruppe, in der die Methylengruppe in 3-Stellung durch ein Sauerstoffatom oder durch eine Methyl-iminogruppe ersetzt ist,

eine Cyclohexyloxygruppe, die in 2-,3- oder 4-Stellung durch eine Hydroxygruppe oder in 4-Stellung auch durch eine Di-($C_{1-2}$-alkyl)amino-, Methoxy-, Carboxy-, Methoxycarbonyl-, Dimethylaminocarbonyl-, Methylaminocarbonyl-, Aminocarbonyl-, Acetylamino-, Methylsulfonylamino-, Methoxycarbonylamino-oder tert.Butyloxycarbonylaminogruppe substituiert sein kann,

eine Cyclohexyloxygruppe, in der die Methylengruppe in 3-Stellung durch eine Methyl-iminogruppe oder die Methylengruppe in 4-Stellung durch ein Sauerstoffatom oder durch eine Methylimino-, Acetyl-imino-, tert.Butyloxycarbonyl-imino-, Methoxycarbonyl-imino- oder Methylsulfonyl-iminogruppe ersetzt ist,

eine Allyloxygruppe,

eine 1-Azetidinylgruppe,

eine 1-Pyrrolidinylgruppe, die durch 1 oder 2 Methylgruppen, durch eine Carboxy-, Methoxycarbonyl-, Ethoxycarbonyl-, Aminocarbonyl-, $C_{1-2}$-Alkylaminocarbonyl- oder Di-($C_{1-2}$)-alkylaminocarbonylgruppe oder in 3-Stellung durch eine Amino-, $C_{1-2}$-Alkylamino-, Di-($C_{1-2}$-alkyl) amino-, $C_{1-2}$-Alkoxycarbonylamino-, $C_{1-2}$-Alkylcarbonylamino-, $C_{1-2}$-Alkylsulfonylamino-, Cyanamino-, Formylamino- oder Dimethylaminocarbonylaminogruppe substituiert sein kann,

eine 1-Pyrrolidinylgruppe, in der in 3-Stellung zwei Wasserstoffatome durch eine n-$C_{4-5}$-Alkylenbriicke ersetzt sind,

eine 1-Piperidinylgruppe, die durch 1 bis 4 Methylgruppen, durch eine Phenyl-, Hydroxy-$C_{1-2}$-alkyl-, Carboxy-, $C_{1-2}$-Alkoxycarbonyl-, Aminocarbonyl-, $C_{1-2}$-Alkylaminocarbonyl-, Di($C_{1-2}$)-alkylaminocarbonyl-, Pyrrolidinocarbonyl- oder Morpholinocarbonylgruppe oder in 3- oder 4-Stellung durch eine Hydroxy-, $C_{1-2}$-Alkoxy-, Amino-, $C_{1-2}$-Alkylamino-, Di-($C_{1-2}$)-Alkylamino-, $C_{1-2}$-Alkylcarbonylamino-, $C_{1-2}$-Alkoxycarbonylamino-, Formylamino-, Cyanamino-, Di-($C_{1-2}$-alkyl)aminocarbonylamino-, $C_{1-2}$-Alkylsulfonylamino- oder Cyanogruppe substituiert sein kann,

eine 1-Piperidinylgruppe, in der in 3-Stellung oder in 4-Stellung zwei Wasserstoffatome durch eine n-$C_{4-5}$-Alkylenbrücke oder durch eine -O-$CH_2CH_2$-O-Brücke ersetzt sind,

eine 1-Piperidinylgruppe, die durch 1 oder 2 Methylgruppen oder eine Phenylgruppe und zusätzlich in 3- oder 4-Stellung durch eine Hydroxygruppe substituiert ist,

eine 1-Piperidinylgruppe, in der in 2,5-Stellung zwei Wasserstoffatome durch eine -$CH_2$- oder -$CH_2CH_2$-Brücke ersetzt sind,

eine gegebenenfalls durch 1 bis 2 Methylgruppen substituierte 1-Piperidinylgruppe, in der die Methylengruppe in 4-Stellung durch ein Sauerstoff- oder Schwefelatom, durch eine Carbonyl-, Sulfinyl-, Sulfonyl-, Imino-, $C_{1-2}$-Alkylimino-, (2-Hydroxyethyl)-imino-, 2- (2-Hydroxyethoxy) ethyl-imino-, (2-Aminoethyl)imino-, $C_{1-3}$-Alkylaminocarbonylmethyl-imino-, N-Oxido-N-$C_{1-2}$-alkylimino-, Phenyl-imino-, Benzyl-imino-, Acetyl-imino- oder Methansulfonyliminogruppe ersetzt ist,

eine 1-Azacyclohept-1-yl-Gruppe, in der zwei Wasserstoffatome in 3,6-Stellung durch eine -$CH_2CH_2$-Gruppe ersetzt sein können,

eine 1,2,3,4-Tetrahydro-isochinolin-2-yl- oder 2,3,4,5-Tetrahydro-1H-3-benzazepin-3-yl-Gruppe oder

eine ($R_{10}NR_{11}$)-Gruppe darstellen, in der

$R_{10}$ ein Wasserstoffatom, eine $C_{1-4}$-Alkylgruppe oder eine 2-Hydroxyethylgruppe und

$R_{11}$ ein Wasserstoffatom,

eine $C_{1-6}$-Alkyl-, $C_{3-6}$-Cycloalkyl-methyl- oder Phenyl-$C_{1-3}$-alkylgruppe,

eine gegebenenfalls durch 1 oder 2 Methylgruppen substituierte $C_{3-6}$-Cycloalkyl-, Allyl- oder Propargylgruppe,

eine Phenylgruppe, die durch eine Hydroxy- oder Methylgruppe oder in 4-Stellung durch eine N-$C_{1-2}$-Alkyl-$C_{1-2}$-alkylcarbonylamino-, N-$C_{1-2}$-Alkyl- (2-hydroxyethyl)amino-, Di-(2-hydroxyethyl)amino-, 1-Pyrrolidinyl-, 2-Oxo-1-pyrrolidinyl-, 2-Hydroxymethyl-1-pyrrolidinyl-, 2-Oxo-1-imidazolidinyl-, 3-Methyl-2-oxo-1-imidazolidinyl- oder Morpholinogruppe substituiert sein kann,

eine durch eine Carboxy-, $C_{1-2}$-Alkoxycarbonyl-, Aminocarbonyl-, $C_{1-2}$-Alkylaminocarbonyl-, Di-$C_{1-2}$-alkylaminocarbonyl-, Pyrrolidinocarbonyl-, Piperidinocarbonyl- oder Morpholinocarbonylgruppe substituierte Methylgruppe,

eine gegebenenfalls durch eine Methylgruppe substituierte Ethylgruppe, die in 2-Stellung durch eine Hydroxy-, $C_{1-2}$-Alkoxy-, Amino-, $C_{1-2}$-Alkylamino-, Di-$C_{1-2}$-alkylamino-, Acetylamino-, 1-Pyrrolidinyl-, Morpholino-, 1-Piperazinyl-, 4-Methyl-1-piperazinyl-, 4-Acetyl-1-piperazinyl-, 4-Aminocarbonyl-1-piperazinyl-, 4-Dimethylaminocarbonyl-1-piperazinyl-, 4-Methylaminocarbonyl-1-piperazinyl-, 4-Methylsulfonyl-1-piperazinyl-, 4-Methoxycarbonyl-1-piperazinyl-oder 4-Cyano-1-piperazinylgruppe substituiert ist,

eine 2-Hydroxyethylgruppe, die im Ethylteil durch eine Phenyl-, 3-Hydroxyphenyl-, 4-Hydroxyphenyl-, 4-Nitrophenyl- oder Benzylgruppe substituiert ist, wobei der Ethylteil der vorstehend erwähnten Gruppen zusätzlich durch eine Methyl-, Hydroxymethyl- oder Methoxymethylgruppe substituiert sein kann,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine 2,2,2-Trifluorethylgruppe,

eine in 3-Stellung durch eine Hydroxy-, Cyano-, Carboxy-, Aminocarbonyl-, Methylaminocarbonyl-, Dimethylaminocarbonyl-, Formylamino, Methoxycarbonylamino-, Morpholino- oder 2-Oxo-1-pyrrolidinylgruppe substituierte Propylgruppe,

eine in 4-Stellung durch eine Hydroxygruppe substituierte Butylgruppe,

eine in 1-Stellung durch eine Hydroxygruppe und zusätzlich in 3-Stellung durch eine Methylgruppe substituierte 3-Butylgruppe,

eine in 1-Stellung durch eine Hydroxygruppe und zusätzlich in 3-Stellung durch eine Methylgruppe substituierte 2-Butylgruppe,

eine in 1-Stellung durch eine Hydroxygruppe und zusätzlich in 4-Stellung durch eine Methylgruppe substituierte 4-Pentylgruppe,

eine 2,3-Dihydroxypropyl-, 3-Amino-2-hydroxy-propyl-, Tris-(3-hydroxypropyl)methyl-, 1,3-Dihydroxy-2-propyl-, 1,3-Dihydroxy-2-methyl-2-propyl- oder Tris-(hydroxymethyl)methylgruppe,

eine 2-Propylgruppe, die in 2-Stellung durch eine Hydroxymethyl-, $C_{1-2}$-Alkoxymethyl-, Carboxy-, $C_{1-2}$-Alkoxycarbonyl-, Aminocarbonyl-, N-$C_{1-2}$-Alkylaminocarbonyl-, N,N-Di-$C_{1-2}$-alkylaminocarbonyl-, Pyrrolidinocarbonyl-, Morpholinocarbonyl- oder (2-Hydroxyethyl)aminocarbonylgruppe substituiert ist,

eine 4-Tetrahydropyranyl-, Tetrahydrofurfuryl-, 1-Desoxy-1-D-sorbityl- oder 2-(2-Hydroxyethyloxy)ethylgruppe,

eine in 2- oder 3-Stellung durch eine Hydroxygruppe oder in 1-Stellung durch eine Hydroxymethylgruppe substituierte Cyclopentylgruppe,

eine in 2-, 3- oder 4-Stellung durch eine Hydroxymethyl-, Hydroxy-, $C_{1-2}$-Alkoxy-, ($C_{1-4}$-Alkoxy)carbonylamino-, Amino-, $C_{1-2}$-Alkylamino-, Di-$C_{1-2}$-alkylamino-, Carboxy-, $C_{1-2}$-Alkoxycarbonyl-, Aminocarbonyl-, $C_{1-2}$-Alkylaminocarbonyl-, Di-$C_{1-2}$-Alkylaminocarbonyl-, N-Oxido-di-$C_{1-2}$-alkylamino-, pyrrolidinocarbonyl-, Morpholinocarbonyl-, $C_{1-2}$-Alkylcarbonylamino- oder $C_{1-2}$-Alkylsulfonylaminogruppe substituierte Cyclohexylgruppe, die zusätzlich durch eine Methylgruppe substituiert sein kann,

eine Cyclohexylgruppe, die in 4-Stellung durch eine 1-Pyrrolidinyl-, 2-Oxo-1-pyrrolidinyl-, 2-Hydroxymethyl-1-pyrrolidinyl-N-$C_{1-2}$-Alkyl- (2-hydroxyethyl)amino-, Di-(2-hydroxyethyl)amino-, N-$C_{1-2}$-Alkyl-$C_{1-2}$-alkylcarbonylamino-, Morpholino-, 2-Oxo-1-imidazolidinyl-, 3-Methyl-2-oxo-1-imidazolidinyl-, Carboxy-$C_{1-2}$-alkyl-, Carboxy-$C_{1-2}$-alkoxy-, $C_{1-2}$-Alkoxycarbonyl-$C_{1-2}$-alkyl-, $C_{1-2}$-Alkoxycarbonyl-$C_{1-2}$-alkoxy-, Aminocarbonyl-$C_{1-2}$-alkyl-, Aminocarbonyl-$C_{1-2}$-alkoxy-, $C_{1-2}$-Alkylaminocarbonyl-$C_{1-2}$-alkyl-, $C_{1-2}$-Alkylaminocarbonyl-$C_{1-2}$-alkoxy-, Di-$C_{1-2}$-Alkylaminocarbonyl-$C_{1-2}$-alkyl-, Di-$C_{1-2}$-Alkylaminocarbonyl-$C_{1-2}$-alkoxy-, Pyrrolidinocarbonyl-$C_{1-2}$-alkyl-, Pyrrolidinocarbonyl-$C_{1-2}$-alkoxy-, Morpholinocarbonyl-$C_{1-2}$-alkyl-, Morpholinocarbonyl-$C_{1-2}$-alkoxy-, Piperidinocarbonyl-$C_{1-2}$-alkyl- oder Piperidinocarbonyl-$C_{1-2}$-alkoxygruppe substituiert ist,

eine Cyclohexylgruppe, in der zwei Wasserstoffatome in 4-Stellung durch eine Oxo-Gruppe oder eine n-$C_{4-5}$-Alkylenbrücke ersetzt sind,

eine Cyclohexylgruppe, in der die Methylengruppe in 4-Stellung durch eine Imino-, $C_{1-2}$-Alkyl-imino-, Phenyl-$C_{1-2}$-alkyl-imino-, N-Methyl-N-oxido-imino-, Formyl-imino-, $C_{1-2}$-Alkylcarbonylimino-, $C_{1-2}$-Alkylsulfonyl-imino-, $C_{1-2}$-Alkoxycarbonyl-imino-, Cyan-imino-, Aminocarbonyl-imino-, $C_{1-2}$-Alkylaminocarbonylimino- oder N,N-Di-$C_{1-2}$-alkylaminocarbonyliminogruppe ersetzt ist,

eine Cyclohexylgruppe, in der die Methylengruppe in 3-Stellung durch eine Imino-, $C_{1-2}$-Alkyl-imino-, $C_{1-2}$-Alkylcarbonylimino-, $C_{1-2}$-Alkylsulfonyl-imino- oder $C_{1-2}$-Alkoxycarbonyl-iminogruppe ersetzt ist,

eine Cyclopentylgruppe, in der die Methylengruppe in 3-Stellung durch ein Sauerstoffatom oder eine Imino-, $C_{1-2}$-Alkyl-imino-, Formyl-imino-, $C_{1-2}$-Alkylcarbonyl-imino-, $C_{1-2}$-Alkylsulfonylimino-, $C_{1-2}$-Alkoxycarbonyl-imino-, Cyan-imino- oder N,N-Di-$C_{1-2}$-alkylaminocarbonyliminogruppe ersetzt ist,

eine Cyclohexylmethylgruppe, wobei der Cyclohexylteil in 4-Stellung durch eine Carboxy-, $C_{1-2}$-Alkoxycarbonyl-, N,N-Di-$C_{1-2}$-Alkylaminocarbonyl- oder Morpholinocarbonylgruppe substituiert ist,

eine Norbornan-2-yl-, Norbornan-2-yl-methyl-, 5-Norbornen-2-yl-methyl-, Bornyl-, 3-Chinuclidinyl- oder Adamantylgruppe oder

$R_{10}$ ein Wasserstoffatom oder eine Methylgruppe und $R_{11}$ eine Hydroxy- oder Methoxygruppe bedeuten,

insbesondere diejenigen Verbindungen, in denen

$R_a$ ein Wasserstoffatom oder eine Methylgruppe,

$R_b$ eine 2-Naphthyl- oder 5-Indanylgruppe oder eine durch die Reste $R_1$ bis $R_3$ substituierte Phenylgruppe, wobei

$R_1$ ein Wasserstoff-, Fluor-, Chlor-, Brom- oder Jodatom, eine Methyl-, Ethyl-, tert.Butyl-, Trifluormethyl-, Ethinyl-, Methoxy-, Cyclopropyl-, Trifluormethoxy-, Cyano-, Ethoxycarbonyl- oder Nitrogruppe,

$R_2$ ein Wasserstoff-, Fluor- oder Chloratom, eine Amino-, Methyl- oder Trifluormethylgruppe und

$R_3$ ein Wasserstoff-, Chlor- oder Bromatom darstellen, und

$R_c$ ein Wasserstoffatom, eine Hydroxy-, Methoxy-, Butyloxy-, Cyclopentyloxy-, 2-[(2-Hydroxyethyl)amino]-ethoxy-, Methylsulfenyl-, Methylsulfinyl- oder Methylsulfonylgruppe, eine 1-Azetidinyl- oder eine gegebenenfalls durch eine oder zwei Methylgruppen substituierte 1-Pyrrolidinylgruppe,

eine durch eine Hydroxymethylgruppe substituierte 1-Piperidinylgruppe,

eine gegebenenfalls durch eine oder zwei Methylgruppen substituierte 1-Piperidinylgruppe, in der die Methylengruppe in 4-Stellung durch ein Sauerstoff- oder Schwefelatom, durch eine Carbonyl-, Sulfinyl-, Sulfonyl-, Imino-, Methyl-imino-, N-Oxido-N-methyl-imino-, 2-Propylaminocarbonyl-methyl-imino-, Phenyl-imino-, Benzyl-imino-, Acetyl-imino- oder Methylsulfonyl-iminogruppe ersetzt sein kann,

eine in 3-Stellung durch eine Hydroxy- oder Diethylaminocarbonylgruppe oder in 4-Stellung durch eine Hydroxy-, Carboxy-, Methoxycarbonyl-, Aminocarbonyl-, Methylaminocarbonyl-, Dimethylaminocarbonyl-, Pyrrolidinocarbonyl-, Morpholinocarbonyl-, Amino-, Acetylamino-, Methoxycarbonylamino-, Formylamino-, Cyanamino-, Dimethylaminocarbonylamino-, Methylsulfonylamino- oder Phenylgruppe substituierte 1-Piperidinylgruppe,

eine 4-Hydroxy-4-phenyl-1-piperidinylgruppe,

eine 1,2,3,4-Tetrahydro-isochinolin-2-yl- oder 2,3,4,5-Tetrahydro-1H-3-benzazepin-3-yl-Gruppe,

eine 1-Piperidinylgruppe, in der in 4-Stellung zwei Wasserstoffatome durch eine $-OCH_2CH_2$-O-Brücke ersetzt sind,

eine 1-Azacyclohept-1-yl-Gruppe, in der zwei Wasserstoffatome in 3- und 6-Stellung durch eine $-CH_2-CH_2$-Gruppe ersetzt sind, oder

eine ($R_{10}NR_{11}$)-Gruppe darstellen, in der

$R_{10}$ ein Wasserstoffatom, eine $C_{1-4}$-Alkylgruppe oder eine 2-Hydroxyethylgruppe und

$R_{11}$ ein Wasserstoffatom,

eine gegebenenfalls durch eine Methylgruppe substituierte Phenylgruppe,

eine Phenylgruppe, die in 4-Stellung durch eine Morpholino- oder 2-(Hydroxymethyl)-1-pyrrolidinylgruppe substituiert ist,

eine $C_{1-6}$-Alkyl-, $C_{3-6}$-Cycloalkyl-, Phenyl-$C_{1-3}$-alkyl-, Cyclopropylmethyl-, Allyl-, Propargyl-, 2-Hydroxyethyl-, 1-Hydroxy-2-propyl-, 3-Hydroxypropyl-, 4-Hydroxybutyl-, 2-Methoxyethyl-, 1-Adamantyl-, Norbornan-2-yl-, Aminocarbonylmethyl-, 2-(Dimethylamino)ethyl-, 3-Chinuclidinyl-, 2,2,2-Trifluorethyl-, 4-Piperidinyl-, 1-Methyl-4-piperidinyl-, 1-Methyl-1-oxido-4-piperidinyl-, 1-Ethoxycarbonyl-4-piperidinyl-, 1-Benzyl-4-piperidinyl-, 2-(2-Hydroxyethoxy) ethyl-, 4-Tetrahydropyranyl-, 1-Hydroxy-2-methyl-2-propyl-, 1-Methoxy-2-methyl-2-propyl-, 2-(Methylaminocarbonyl)-2-propyl-, 2,3-Dihydroxy-1-propyl-, 2-(Morpholino)ethyl-, 1-Desoxy-1-D-sorbityl-, 3-(2-Oxo-1-pyrrolidinyl)-propyl-, Tris-(hydroxymethyl)methyl-, 1,3-Dihydroxy-2-propyl-, 1,3-Dihydroxy-2-methyl-2-propyl- oder Bornylgruppe,

eine 2-Hydroxyethylgruppe, die in 2-Stellung durch eine Phenylgruppe und in 1-Stellung zusätzlich durch eine Methyl- oder Hydroxymethylgruppe substituiert ist,

eine Methylcyclohexyl-, 4-Carboxy-cyclohexyl-, 4-Methoxycarbonyl-cyclohexyl-, 4-Dimethylaminocarbonyl-cyclohexyl-, 4-(1-pyrrolidinylcarbonyl)-cyclohexyl-, 4-(Morpholinocarbonyl)-cyclohexyl-, 4-[2-(Methoxycarbonyl)ethyl] cyclohexyl-, 4-(2-Carboxy-ethyl)cyclohexyl-, 4-(tert.Butyloxycarbonylamino)-cyclohexyl-, 4-Methoxycyclohexyl-, 4-Aminocyclohexyl-, 4-(Dimethylamino)cyclohexyl-, 4-(N,N-Dimethyl-N-oxido-amino)cyclohexyl-, 4-(Acetylamino)-cyclohexyl-, 4-(Methylsulfonylamino)-cyclohexyl-, 2-Hydroxycyclohexyl-, 4-Hydroxycyclohexyl-, 4-(Hydroxymethyl)-cyclohexyl-, 4-Hydroxy-4-methyl-cyclohexyl- oder 4-Oxocyclohexylgruppe,

eine durch eine 1,4,7,10,13-Pentaoxacyclopentadecyl- oder eine 1,4,7,10,13,16-Hexaoxacyclooctadecylgruppe substituierte Methylgruppe, oder

$R_{10}$ eine Methylgruppe und $R_{11}$ eine Methoxygruppe darstellen, bedeuten,

deren Tautomere, deren Stereoisomere und deren Salze.

Als besonders bevorzugte Verbindungen der allgemeinen Formel I seien folgende erwähnt:

(1) 4-[(3,4-Dichlorphenyl)amino]-6-[(trans-4-hydroxycyclohexyl)-amino]-pyrimido [5,4-d]pyrimidin,

(2) 4-[(3-Chlor-4-fluor-phenyl)amino]-6-[(trans-4-hydroxycyclohexyl)-amino]-pyrimido [5,4-d]pyrimidin,

(3) 4-[(3-Bromphenyl)amino]-6-[(trans-4-hydroxycyclohexyl)amino]pyrimido [5,4-d]pyrimidin,

(4) 4-[(3-Chlorphenyl)amino]-6-(cyclopropylamino)-pyrimido-[5,4-d]pyrimidin,

(5) 4-[(3-Methylphenyl)amino]-6-(4-amino-1-piperidinyl)-pyrimido[5,4-d]pyrimidin,

(6) 4-[(3-Methylphenyl)amino]-6-[(trans-4-aminocyclohexyl)amino]pyrimido [5,4-d]pyrimidin,

(7) 4-[(3-Methylphenyl)amino]-6-(N-(trans-4-hydroxycyclohexyl)-N-methyl-amino)-pyrimido [5,4-d]pyrimidin,

(8) 4-[(3-Methylphenyl)amino]-6-(4-methoxycarbonylamino-1-piperidinyl)-pyrimido[5,4-d]pyrimidin,

(9) 4-[(3-Methylphenyl)amino]-6-[trans-4-(morpholinocarbonyl)cyclohexylamino]-pyrimido[5, 4-d]pyrimidin,

(10) 4-[(3-Methylphenyl)amino]-6-[trans-4-(pyrrolidinocarbonyl)cyclohexylamino]-pyrimido [5,4-d]pyrimidin,

(11) 4-[(3-Chlor-4-fluor-phenyl)amino]-6- [N-(trans-4-hydroxycyclohexyl)-N-methyl-amino]-pyrimido [5,4-d]pyrimidin,

(12) 4-[(4-Amino-3,5-dichlor-phenyl)amino]-6-[(trans-4-hydroxycyclohexyl)-amino]-pyrimido [5,4-d]pyrimidin,

(13) 4-[(3-Chlor-4-fluor-phenyl)amino]-6-[2-(morpholino)ethylamino]-pyrimido [5,4-d]pyrimidin,

(14) 4-[(4-Amino-3,5-dibrom-phenyl)amino]-6-[(trans-4-hydroxycyclohexyl)amino]-pyrimido[5,4-d]pyrimidin,

(15) 4-[(3-Chlor-4-fluor-phenyl)amino]-6-morpholino-pyrimido[5,4-d]pyrimidin,

(16) 4- [(3-Chlor-4-fluor-phenyl)amino]-6-(1-hydroxy-2-methyl-2-propylamino)-pyrimido [5,4-d]pyrimidin,

(17) 4-[(3-Chlor-4-fluor-phenyl)amino]-6-(1-hydroxy-2-propylamino)-pyrimido[5,4-d]pyrimidin,

(18) 4-[(3-Chlor-4-fluor-phenyl)amino]-6-(1,3-dihydroxy-2-propylamino)-pyrimido[5,4-d]pyrimidin,

(19) 4- [(3-Chlor-4-fluor-phenyl)amino]-6-[4-amino-1-piperidinyl]-pyrimido [5,4-d]pyrimidin,

(20) 4-[(3-Methylphenyl)amino]-6-(4-piperidinyl-amino)pyrimido[5,4-d]pyrimidin,

(21) 4-[(3-Methylphenyl)amino]-6-(4-formylamino-1-piperidinyl)pyrimido [5,4-d]pyrimidin,

(22) 4-[(3-Methylphenyl)amino]-6-[(1-ethoxycarbonyl-4-piperidinyl)amino]-pyrimido[5,4-d]pyrimidin,

(23) 4-[(3-Methylphenyl)amino]-6-[(3-chinuclidinyl)amino]pyrimido[5,4-d]pyrimidin,

(24) 4-[(3-Chlor-4-fluor-phenyl)amino]-6-[(4-aminocyclohexyl)amino]-pyrimido[5,4-d]pyrimidin,

(25) 4-[(3-Chlor-4-fluor-phenyl)amino]-6-(4-piperidinyl-amino)pyrimido[5,4-d]pyrimidin,

(26) 4-[(3-Chlor-4-fluor-phenyl)amino]-6-[trans-4-(morpholinocarbonyl)cyclohexylamino]-pyrimido [5,4-d]pyrimidin,

(27) 4-[(3-Chlor-4-fluor-phenyl)amino]-6-[trans-4-(pyrrolidinocarbonyl)cyclohexylamino]-pyrimido[5,4-d]pyrimidin,

(28) 4-[(4-Fluorphenyl)amino)-6-(cyclopropylamino)-pyrimido[5,4-d]pyrimidin,

(29) 4-[(3-Chlor-4-fluor-phenyl)amino]-6-(cyclopropylamino)pyrimido[5,4-d]pyrimidin,

(30) 4-[(3,4-Dichlorphenyl)amino]-6-(cyclopropylamino)pyrimido [5,4-d]pyrimidin,

(31) 4-[(3-Chlor-4-fluor-phenyl)amino]-6-[(trans-4-dimethylaminocyclohexyl)amino]-pyrimido[5,4-d]pyrimidin und

(32) 4-[(3-Chlor-4-fluor-phenyl)amino]-6-(1-methyl-4-piperidinyl-amino)-pyrimido[5,4-d]pyrimidin sowie deren Salze.

Die Verbindungen der allgemeinen Formel I lassen sich beispielsweise nach folgenden Verfahren herstellen:
a) Umsetzung einer Verbindung der allgemeinen Formel

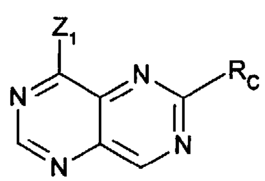

, (II)

in der

$R_c$ wie eingangs definiert ist und
$Z_1$ eine Austrittsgruppe wie ein Halogenatom, z.B. ein Chlor- oder Bromatom oder eine Methylsulfonyl- oder eine Hydroxygruppe darstellt, mit einem Amin der allgemeinen Formel

$$H\text{-}(R_aNR_b)\ ,\qquad (III)$$

in der

$R_a$ und $R_b$ wie eingangs definiert sind.

Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Ethylenglycolmonomethylether, Ethylenglycoldiethylether oder Sulfolan gegebenenfalls in Gegenwart einer anorganischen Base, z.B. Natriumcarbonat oder Kaliumhydroxid, oder einer tertiären organischen Base, z.B. Triethylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 200°C, vorzugsweise jedoch bei Temperaturen zwischen 60 und 150°C, durchgeführt. Die Umsetzung kann jedoch auch ohne Lösungsmittel oder in einem Überschuß der eingesetzten Verbindung der allgemeinen Formel III durchgeführt werden. Handelt es sich bei $Z_1$ um eine Hydroxygruppe, so wird die Umsetzung zweckmäßigerweise in Gegenwart von Hexamethyldisilazan, vorzugsweise ohne weitere Lösungsmittel und gegebenenfalls in Gegenwart eines Reaktionsbeschleunigers wie einer organischen Säure wie z.B. Toluolsulfonsäure bei Temperaturen zwischen 0 und 200°C, vorzugsweise jedoch bei Temperaturen zwischen 60 und 180°C, durchgeführt.

b) Zur Herstellung von Verbindungen der allgemeinen Formel I, in der $R_c$ einen der für $R_c$ eingangs erwähnten über ein Sauerstoff- oder Stickstoffatom, über eine Mercapto- oder Sulfenylgruppe mit dem Pyrimido[5,4-d]pyrimidin verknüpften Reste darstellt:

Umsetzung einer Verbindung der allgemeinen Formel

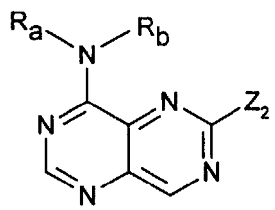

, (IV)

in der

$R_a$ und $R_b$ wie eingangs definiert sind und
$Z_2$ eine Austrittsgruppe wie ein Halogenatom, eine substituierte Hydroxy-, Mercapto-, Sulfinyl- oder Sulfonylgruppe wie ein Chlor- oder Bromatom, eine Methoxy-, Ethoxy-, Phenoxy-, Methylsulfinyl-, Ethylsulfinyl-, Methylsulfonyl- oder Ethylsulfonylgruppe darstellt, mit einer Verbindung der allgemeinen Formel

$$X_1 - H, \qquad (V)$$

in der

$X_1$ einen der für $R_c$ eingangs erwähnten über ein Sauerstoff- oder Stickstoffatom, über eine Mercapto- oder Sulfenylgruppe mit dem Pyrimido[5,4-d]pyrimidin verknüpften Reste darstellt.

Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Ethylenglycolmonomethylether, Ethylenglycoldiethylether oder Sulfolan gegebenenfalls in Gegenwart einer anorganischen Base, z.B. Natriumcarbonat oder Kaliumhydroxid, oder einer tertiären organischen Base, z.B. Triethylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 150°C, vorzugsweise jedoch bei Temperaturen zwischen 20 und 120°C, durchgeführt. Die Umsetzung kann jedoch auch ohne Lösungsmittel oder in einem Überschuß der eingesetzten Verbindung der allgemeinen Formel V durchgeführt werden.

Mit einem Alkohol der allgemeinen Formel V wird die Umsetzung vorzugsweise in einem entsprechenden Alkohol und gegebenenfalls in Gegenwart einer organischen oder anorganischen Base wie mit dem entsprechenden Alkalimetallalkoholat bei Temperaturen zwischen 0 und 100°C durchgeführt.

Mit einer Mercaptoverbindung der allgemeinen Formel V wird die Umsetzung vorzugsweise in einem Lösungsmittel mit dem entsprechenden Alkalimetallthiolat oder dem entsprechenden Thiol und einer organischen oder anorganischen Base bei Temperaturen zwischen 0 und 80°C durchgeführt.

Mit Wasser wird die Umsetzung vorzugsweise in Wasser oder in einem Gemisch aus Wasser und einem organischen Lösungsmittel in Gegenwart eines Alkalihydroxids oder einer Mineralsäure wie Salzsäure oder Schwefelsäure bei Temperaturen zwischen 20 und 100°C durchgeführt.

c) Zur Herstellung von Verbindungen der allgemeinen Formel I, in der $R_c$ einen der für $R_c$ eingangs erwähnten über eine Sulfinyl- oder Sulfonylgruppe mit dem Pyrimido[5,4-d]pyrimidin verknüpften Reste darstellt:

Oxidation einer Verbindung der allgemeinen Formel

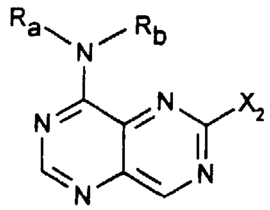

, (VI)

in der

$R_a$ und $R_b$ wie eingangs definiert sind und

$X_2$ einen der für $R_c$ eingangs erwähnten über ein Schwefelatom mit dem Pyrimido[5,4-d]pyrimidin verknüpften Reste darstellt.

Die Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid, Chloroform oder Dioxan bei -20 bis 80°C, mit Natriummetaperjodat in wäßrigem Methanol oder Ethanol bei -15 bis 25°C, mit Brom in Eisessig oder wäßriger Essigsäure gegebenenfalls in Gegenwart einer schwachen Base wie Natriumacetat, mit N-Bromsuccinimid in Ethanol, mit tert.Butylhypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer Sulfonylverbindung der allgemeinen Formel I wird die Oxidation ausgehend von einer entsprechenden Sulfinylverbindung zweckmäßigerweise mit einem oder mehr Äquivalenten des verwendeten Oxidationsmittels oder ausgehend von einer entsprechenden Sulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

Zur Herstellung von Gemischen aus einer Sulfinyl- und Sulfonylverbindung der allgemeinen Formel I wird die Oxidation ausgehend von einer entsprechenden Sulfenylverbindung vorzugsweise in Methylenchlorid durch Behandlung mit einer entsprechenden Menge von m-Chlorperbenzoesäure bei Temperaturen zwischen 20°C und der Rückflußtemperatur des Reaktionsgemisches durchgeführt.

d) Zur Herstellung einer Verbindung der allgemeinen Formel I, in der $R_c$ ein Wasserstoffatom darstellt:

Enthalogenierung einer Verbindung der allgemeinen Formel

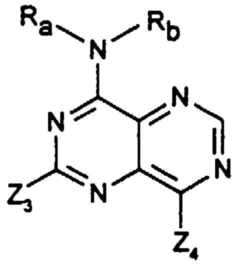

, (VII)

in der

$R_a$ und $R_b$ wie eingangs definiert sind,
$Z_3$ und $Z_4$, die gleich oder verschieden sein können, jeweils ein Halogenatom wie ein Chlor- oder Bromatom darstellen.

Die Enthalogenierung wird mit Jodwasserstoffsäure und Diphosphortetrajodid, wobei die Jodwasserstoffsäure gleichzeitig als Lösungsmittel dienen kann, bei Temperaturen zwischen 20 und 100°C, vorzugsweise bei 50°C, und durch anschließendes Erhitzen mit einem Hydrierungskatalysator wie Palladium/Kohle in einem geeigneten Lösungsmittel wie Dioxan, Essigester oder Ethylglycoldiethylether auf 70 bis 125°C, vorzugsweise auf die Rückflußtemperatur des verwendeten Lösungsmittels, durchgeführt.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden oder

eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden oder

eine Verbindung der allgemeinen Formel I, die eine Carboxygruppe enthält, so kann diese mittels Veresterung in einen entsprechenden Ester der allgemeinen Formel I übergeführt werden oder

eine Verbindung der allgemeinen Formel I, die eine Carboxyoder Estergruppe enthält, so kann diese mittels Amidierung in ein entsprechendes Amid der allgemeinen Formel I übergeführt werden oder

eine Verbindung der allgemeinen Formel I, die eine primäre oder sekundäre Hydroxygruppe enthält, so kann diese mittels Oxidation in eine entsprechende Carbonylverbindung der allgemeinen Formel I übergeführt werden.

Die nachträgliche Veresterung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenyl-phosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Acylierung oder Sulfonylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem entsprechenden Acyl- oder Sulfonylderivat gegebenenfalls in Gegenwart einer tertiären organischen Base oder in Gegenwart einer anorganischen Base oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Alkylierung wird gegebenenfalls in einem Lösungsmittel oder Lösungsmittelgemisch wie

Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyljodid, Ethylbromid, Dimethylsulfat oder Benzylchlorid, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton oder Butyraldehyd in Gegenwart eines komplexen Metallhydrids wie Natriumborhydrid, Lithiumborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung wird jedoch vorzugsweise in Gegenwart von Ameisensäure als Reduktionsmittel bei erhöhten Temperaturen, z.B. bei Temperaturen zwischen 60 und 120°C, durchgeführt.

Die nachträgliche Amidierung wird durch Umsetzung eines entsprechenden reaktionsfähigen Carbonsäurederivates mit einem entsprechenden Amin gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, wobei das eingesetzte Amin gleichzeitig als Lösungsmittel dienen kann, gegebenenfalls in Gegenwart einer tertiären organischen Base oder in Gegenwart einer anorganischen Base oder mit einer entsprechenden Carbonsäure in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Oxidation wird gegebenenfalls in einem Lösungsmittel wie Methylenchlorid, Wasser, Dimethylformamid, Benzol, Chlorbenzol, Tetrahydrofuran oder Dioxan mit einem Oxidationsmittel wie Chromschwefelsäure, Chromtrioxid und Pyridin, Pyridiniumdichromat, Pyridiniumchlorochromat, Oxalylchlorid/Dimethylsulfoxid/Triethylamin, Tetra-n-propylperruthenat/N-Methylmorpholin-N-oxid oder Rutheniumtrichlorid/Natriummetaperiodat zweckmäßigerweise bei Temperaturen zwischen -80 und 100°C, vorzugsweise bei Temperaturen zwischen -80°C und Raumtemperatur, durchgeführt.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Trityl-, Benzyl- oder Tetrahydropyranylgruppe,

als Schutzreste für eine Carboxygruppe die Trimethylsilyl-, Methyl-, Ethyl-, tert.Butyl-, Benzyl- oder Tetrahydropyranylgruppe,

als Schutzreste für eine Phosphonogruppe eine Alkylgruppe wie die Methyl-, Ethyl-, Isopropyl- oder n-Butylgruppe, die Phenyl- oder Benzylgruppe,

als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe und

als Schutzreste für das Stickstoffatom einer 1-Aza-bicycloalkylgruppe wie der Chinuclidinylgruppe die Benzylgruppe oder Boran in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines

2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Spaltung des Komplexes einer 1-Aza-bicycloalkylgruppe wie der Chinuclidinylgruppe mit Boran erfolgt vorzugsweise durch Behandlung mit einer Säure wie Salzsäure und gegebenenfalls in Gegenwart eines Lösungsmittels wie Methanol, Ethanol, Essigsäure oder Dioxan bei Temperaturen zwischen 0°C und der Siedetemperatur des Reaktionsgemisches. Bei dieser Umsetzung kann eine gegebenenfalls vorhandene Estergruppe gleichzeitig in die entsprechende Carboxygruppe übergeführt werden.

Die Spaltung nur eines Alkylrestes von einer O,O'-Dialkylphosphonogruppe erfolgt beispielsweise mit Natriumiodid in einem Lösungsmittel wie Aceton, Ethyl-methylketon, Acetonitril oder Dimethylformamid bei Temperaturen zwischen 40 und 150°C, vorzugsweise jedoch bei Temperaturen zwischen 60 und 100°C.

Die Abspaltung beider Alkylreste von einer O,O'-Dialkyl-phosphonogruppe erfolgt beispielsweise mit Jodtrimethylsilan,

Bromtrimethylsilan oder Chlortrimethylsilan/Natriumiodid in einem Lösungsmittel wie Methylchlorid, Chloroform oder Acetonitril bei Temperaturen zwischen 0°C und der Siedetemperatur des Reaktionsgemisches, vorzugsweise jedoch bei Temperaturen zwischen 20 und 60°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxy-, Phosphono-, O-Alkylphosphono-, Sulfo- oder 5-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II bis V sind teilweise literaturbekannt oder man erhält diese nach an sich literaturbekannten Verfahren (siehe Beispiele I bis XVII).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine spezifische

Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Hier wurde eine Interleukin-3- (IL-3) abhängige Zellinie murinen Ursprungs verwendet, die derart genetisch verändert wurde, daß sie funktionellen humanen EGF-R exprimiert. Die Proliferation dieser F/L-HERc genannten Zellen kann daher entweder durch murines IL-3 oder durch EGF stimuliert werden (siehe von Rüden, T. et al. in EMBO J. 7, 2749-2756 (1988) und Pierce, J. H. et al. in Science 239, 628-631 (1988)).

Als Ausgangsmaterial für die F/L-HERc Zellen diente die Zelllinie FDC-$P_1$, deren Herstellung von Dexter, T. M. et al. in J. Exp. Med. 152, 1036-1047 (1980) beschrieben wurde. Alternativ können aber auch andere Wachstumsfaktor-abhängige Zellen verwendet werden (siehe beispielsweise Pierce, J. H. et al. in Science 239, 628-631 (1988), Shibuya, H. et al. in Cell 70, 57-67 (1992) und Alexander, W. S. et al. in EMBO J. 10, 3683-3691 (1991)). Zur Expression der humanen EGF-R cDNA (siehe Ullrich, A. et al. in Nature 309, 418-425 (1984)) wurden rekombinante Retroviren verwendet, wie in von Rüden, T. et al., EMBO J. 7, 2749-2756 (1988) beschrieben, mit dem Unterschied, daß zur Expression der EGF-R cDNA der retrovirale Vektor LXSN (siehe Miller, A. D. et al. in BioTechniques 7, 980-990 (1989)) eingesetzt wurde und als Verpackungszelle die Linie GP+E86 (siehe Markowitz, D. et al. in J. Virol. 62, 1120-1124 (1988)) diente.

Der Test wurde wie folgt durchgeführt:

F/L-HERc Zellen wurden in RPMI/1640 Medium (BioWhittaker), supplementiert mit 10 % foetalem Rinderserum (FCS, Boehringer Mannheim), 2 mM Glutamin (BioWhittaker), Standardantibiotika und 20 ng/ml humanem EGF (Promega), bei 37°C und 5% $CO_2$ kultiviert. Zur Untersuchung der inhibitorischen Aktivität der erfindungsgemäßen Verbindungen wurden 1,5 x $10^4$ Zellen pro Vertiefung in Triplikaten in 96-Loch-Platten in obigem Medium (200µl) kultiviert, wobei die Proliferation der Zellen entweder mit EGF (20 ng/ml) oder murinem IL-3 stimuliert wurde. Als Quelle für IL-3 dienten Kulturüberstände der Zellinie X63/0 mIL-3 (siehe Karasuyama, H. et al.in Eur. J. Immunol. 18, 97-104 (1988)). Die erfindungsgemäßen Verbindungen wurden in 100% Dimethylsulfoxid (DMSO) gelöst und in verschiedenen Verdünnungen den Kulturen zugefügt, wobei die maximale DMSO Konzentration 1% betrug. Die Kulturen wurden für 48 Stunden bei 37°C inkubiert.

Zur Bestimmung der inhibitorischen Aktivität der erfindungsgemäßen Verbindungen wurde die relative Zellzahl mit dem Cell Titer 96™ AQueous Non-Radioactive Cell Proliferation Assay (Promega) in O.D. Einheiten gemessen. Die relative Zellzahl wurde in Prozent der Kontrolle (F/LHERc Zellen ohne Inhibitor) berechnet und die Wirkstoffkonzentration, die die Proliferation der Zellen zu 50% hemmt ($IC_{50}$), abgeleitet. Hierbei wurden folgende Ergebnisse erhalten:

| Verbindung (Beispiel Nr.) | Hemmung der EGF-abhängigen Proliferation $IC_{50}$ [µM] | Hemmung der IL-3-abhängigen Proliferation $IC_{50}$ [µM] |
|---|---|---|
| 1(3) | 2,5 | >10 |
| 1(5) | 1,0 | >10 |
| 1(6) | 0,005 | >10 |
| 1(10) | 0,5 | >10 |
| 1(13) | 0,3 | >10 |
| 1(17) | 0,04 | > 3 |
| 1(19) | 0,05 | >10 |
| 1(23) | 0,025 | > 3 |
| 1(58) | 0,02 | > 3 |
| 1(62) | 0,040 | > 1 |
| 1(72) | 0,003 | > 3 |
| 1(108) | 0,050 | >10 |
| 1(109) | 0,015 | 10 |

(fortgesetzt)

| Verbindung (Beispiel Nr.) | Hemmung der EGF-abhängigen Proliferation $IC_{50}$ [μM] | Hemmung der IL-3-abhängigen Proliferation $IC_{50}$ [μM] |
|---|---|---|
| 1(110) | 0,002 | >10 |
| 3(5) | 0,05 | > 3 |
| 3(18) | 0,05 | > 3 |
| 3(19) | 0,015 | > 3 |
| 3(45) | 0,020 | > 1 |
| 4(1) | 2 | >10 |
| 1(129) | 0.008 | >20 |
| 1(183) | 0.005 | >20 |
| 1(204) | 0.0008 | >10 |
| 1(134) | 0.013 | >10 |
| 1(207) | 0.002 | >10 |
| 1(201) | 0.032 | >10 |
| 1(61) | 0.032 | 9 |
| 1(140) | 0.005 | >10 |
| 1(170) | 0.015 | >10 |
| 1(197) | 0.9 | 10 |
| 1(193) | 0.004 | 10 |

Die erfindungsgemäßen Verbindungen inhibieren auch die EGF-stimulierte Proliferation der menschlichen Tumorzellinie KB, die von einem oralen epidermoiden Karzinom stammt und den EGF-Rezeptor überexprimiert (z.B. Aboud-Pirak, E. et al, J. Natl. Cancer. Inst. 80, 1605-11 (1988)). KB-Zellen (bezogen von ATCC) wurden in DMEM (BioWhittaker) in Anwesenheit von 10% FCS (Boehringer Mannheim), 50μM beta-Mercaptoethanol und Standardantibiotika passagiert. Als Indikator für die EGF/TGF-alpha-stimulierte Zellproliferation wurde die EGF-induzierte DNA-Synthese durch Messung des Einbaus radioaktiv markierten Thymidins bestimmt. Dazu wurden die Zellen zweimal gewaschen und 1500 Zellen pro Vertiefung einer 96-Loch-Platte in 200μl IMDM (Bio-Whittaker) ohne Serum in Anwesenheit von 50μM beta-Mercaptoethanol, Standardantibiotika, TGF-alpha [10ng/ml] oder EGF [20ng/ml] und von verschiedenen Konzentrationen der erfindungsgemäßen Substanzen ausplattiert (Triplikate, maximale DMSO-Konzentration 1%, siehe Proliferations-Test mit F/L-HERc-Zellen). Nach 60 Stunden wurde für etwa 16 - 18h [$^3$H]-Thymidin (0.1μCi in 10μl) zugegeben. Die anschließende Messung des Thymidin-Einbaus ergab für die Verbindungen 2, 6, 17, 18, 19, 72, 83, 93, 95, 96 und 104 des Beispiels 1 $IC_{50}$-Werte von 0.1 - 1 μM für die Hemmung der EGF/TGF-alpha-stimulierten KB-Zell-Proliferation.

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc. .

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Diese Kombinationen können

entweder simultan oder sequentiell verabreicht werden.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

Beispiel I

4-[(3-Methylphenyl)amino]-2,8-dichlor-pyrimido[5,4-d]pyrimidin

Zu 1,0 g 2,4,8-Trichlor-pyrimido[5,4-d]pyrimidin in 20 ml Methylenchlorid werden bei 0 bis -10°C 0,45 g 3-Methylanilin und 0,42 g Triethylamin zugegeben und 1,5 Stunden bei dieser Temperatur gerührt. Anschließend wird mit Wasser versetzt, die organische Phase abgetrennt, getrocknet und eingeengt. Der Rückstand wird durch Chromatographie über eine Aluminiumoxid-Säule mit Petrolether/Essigester (10:2) gereinigt.
Ausbeute: 0,51 g (39 % der Theorie),
Schmelzpunkt: 180-181°C

Analog Beispiel I werden folgende Verbindungen erhalten:

(1) 4-[(3-Trifluormethylphenyl)amino]-2,8-dichlor-pyrimido[5,4-d]pyrimidin
$R_f$-Wert: 0,56 (Aluminiumoxid; Petrolether/Essigester = 2:1)

(2) 4-[(3-Bromphenyl)amino]-2,8-dichlor-pyrimido[5,4-d]pyrimidin
Schmelzpunkt: 205-208°C
$R_f$-Wert: 0,50 (Aluminiumoxid; Petrolether/Essigester = 2:1)

(3) 4-[(3-Chlorphenyl)amino]-2,8-dichlor-pyrimido[5,4-d]pyrimidin
$R_f$-Wert: 0,67 (Aluminiumoxid; Petrolether/Essigester = 2:1)

(4) 4-(Phenylamino]-2,8-dichlor-pyrimido[5,4-d]pyrimidin $R_f$-Wert: 0,50 (Aluminiumoxid; Petrolether/Essigester = 2:1)

Beispiel II

4-Hydroxy-6-methylsulfinyl-pyrimido[5,4-d]pyrimidin und 4-Hydroxy-6-methylsulfonyl-pyrimido[5,4-d]pyrimidin

2,0 g 4-Hydroxy-6-methylthio-pyrimido[5,4-d]pyrimidin und 8 g 3-Chlorperoxybenzoesäure (Gehalt: 50 %) werden in 50 ml Methylenchlorid 3 Stunden kräftig gerührt. Der Niederschlag wird abgesaugt, mit Essigester gewaschen und getrocknet.
Ausbeute: 2,2 g,
$R_f$-Wert: 0,27 und 0,50 (Kieselgel; Methylenchlorid/Essigester/Methanol = 10:4:3)

Beispiel III

4-Hydroxy-6-(cyclopropylamino)-pyrimido[5,4-d]pyrimidin

2,2 g eines Gemisches aus 4-Hydroxy-6-methylsulfinyl-pyrimido[5,4-d]pyrimidin und 4-Hydroxy-6-methylsulfonyl-pyrimido[5,4-d]pyrimidin werden in 10 ml Cyclopropylamin 3 Stunden unter Rückfluß erhitzt. Nach dem Abkühlen wird eingeengt, der Rückstand wird mit Wasser gerührt und der Feststoff abgesaugt und getrocknet.
Ausbeute: 1,7 g,
Schmelzpunkt: >240°C
$R_f$-Wert: 0,45 (Kieselgel; Methylenchlorid/Essigester/Methanol = 10:4:3)

Beispiel IV

4-Chlor-6-(cyclopropylamino)-pyrimido[5,4-d]pyrimidin

1,7 g 4-Hydroxy-6-(cyclopropylamino)-pyrimido[5,4-d]pyrimidin werden mit 50 ml Thionylchlorid unter Zusatz von 4 Tropfen Dimethylformamid 1,5 Stunden unter Rückfluß erhitzt. Das Reaktionsgemisch wird eingeengt, mit Methylenchlorid versetzt und nochmals eingeengt. Der Rückstand wird dann zwischen Methylenchlorid und einer wäßrigen Kaliumcarbonatlösung verteilt. Die wäßrige Phase wird noch zweimal mit Methylenchlorid extrahiert und die vereinigten organischen Phasen getrocknet und eingeengt.Das Produkt wird ohne weitere Reinigung weiter umgesetzt. Schmelzpunkt: 135°C (Zers.)
$R_f$-Wert: 0,53 (Kieselgel; Petrolether/Essigester = 2:1)
Analog Beispiel IV wird folgende Verbindung erhalten:

(1) 4-Chlor-6-methylthio-pyrimido[5,4-d]pyrimidin
Schmelzpunkt: 90-92°C
$R_f$-Wert: 0,63 (Kieselgel; Petrolether/Essigester = 7:3)

Beispiel V

5-Amino-2-methylthio-pyrimidin-4-carbonsäure

131,4 g 5-Brom-2-methylthio-pyrimidin-4-carbonsäure, 860 ml konz. wäßriges Ammoniak und 2,42 g Kupfer (II)sulfat, gelöst in 34 ml Wasser, werden in einem Bombenrohr 4 Stunden bei 95°C geschüttelt. Nach dem Abkühlen wird der Niederschlag abgesaugt. Der Niederschlag wird in 600 ml heißem Wasser gelöst und die Lösung über Aktivkohle filtriert. Das Filtrat wird im Eisbad abgekühlt und mit konzentrierter Salzsäure auf einen pH von 3 gebracht. Der Niederschlag wird abgesaugt und durch Lösen in verdünnter Natronlauge und Ausfällen mit Salzsäure gereinigt.
Ausbeute: 54,6 g (56 % der Theorie),
Schmelzpunkt: 187°C
$R_f$-Wert: 0,35 (Kieselgel; Essigester/Methanol = 2:1)

Beispiel VI

4-Hydroxy-6-methylthio-pyrimido [5,4-d]pyrimidin

Methode A:

25 g 5-Amino-2-methylthio-pyrimidin-4-carbonsäure und 150 ml Formamid werden in einem Ölbad gerührt, wobei innerhalb einer halben Stunde die Ölbadtemperatur auf 180°C gesteigert wird. Es wird noch 1,5 Stunden bei dieser Temperatur gerührt. Dann wird das Reaktionsgemisch heiß auf 750 ml eines Eis/Wasser-Gemisches gegeben. Nach 2 Stunden wird abgesaugt, mit Wasser gewaschen und getrocknet.

Methode B:

Eine Mischung von 69 g 5-Amino-2-methylthio-pyrimidin-4-carbonsäure, 155 g Formamidinacetat und 300 ml Ethoxyethanol wird 2 Stunden zum Sieden erhitzt. Dann wird das Reaktionsgemisch auf 10°C abgekühlt, mit 250 ml Wasser versetzt und eine Stunde bei 10°C stehen gelassen. Dann wird abgesaugt, mit Wasser gewaschen und getrocknet.
Ausbeute: 59 g (82 % d. Theorie).
Schmelzpunkt: >240°C
$R_f$-Wert: 0,63 (Kieselgel; Methylenchlorid/Essigester/Methanol = 10:4:3)

Beispiel VII

4-Dibenzylamino-cyclohexanon

1,0 ml Oxalylchlorid werden in 40 ml Methylenchlorid gelöst und auf -60°C abgekühlt. Man tropft 1,7 ml Dimethylsulfoxid in 20 ml Methylenchlorid zu, rührt noch zwei Minuten und tropft dann langsam 2,95 g 4-N,N-Dibenzylamino-cyclohexanol in 20 ml Methylenchlorid hinzu. Nach 15 Minuten werden 7,0 ml Triethylamin zugetropft. Nach

weiteren 5 Minuten wird auf Raumtemperatur erwärmt und 12 Stunden gerührt. Die Reaktionsmischung wird einmal mit je 100 ml Wasser und gesättigter Natriumchlorid-Lösung gewaschen. Nach dem Trocknen über Natriumsulfat wird das Lösungsmittel im Rotationsverdampfer abdestilliert und das Rohprodukt über eine Kieselgelsäule mit Petrolether/Essigester (10:3, dann 10:5, dann 10:10) gereinigt.
Ausbeute: 2,91 g (99 % der Theorie),
Schmelzpunkt: 63-64°C

Beispiel VIII

4-Dibenzylamino-1-methyl-cyclohexanol

In eine Lösung von 10,7 g 4-Dibenzylamino-cyclohexanon in 200 ml Ether wird eine Lösung von 15,1 ml 3,0 molarem Methylmagnesiumbromid in 200 ml Ether getropft. Die Mischung wird dann 45 Minuten zum Sieden erhitzt, auf 0°C abgekühlt und vorsichtig mit 300 ml gesättigter Ammoniumchloridlösung versetzt. Die Etherphase wird abgetrennt, mit je 100 ml gesättigter Natriumhydrogencarbonat-Lösung und gesättigter Natriumchloridlösung gewaschen und über Natriumsulfat getrocknet. Nach dem Abdestillieren des Lösungsmittels im Rotationsverdampfer wird das Rohprodukt über eine Aluminiumoxid-Säule mit Petrolether/Essigester (10:1, dann 10:3) gereinigt; dabei werden die Diastereomeren getrennt.
cis-Diastereomer:
Ausbeute: 3,73 g (33 % der Theorie),
Schmelzpunkt: 91-95°C
$R_f$-Wert: 0,52 (Aluminiumoxid; Petrolether/Essigester = 10:3)
trans-Diastereomer:
Ausbeute: 2,33 g (21 % der Theorie),
Schmelzpunkt: 111-115°C
$R_f$-Wert: 0,29 (Aluminiumoxid; Petrolether/Essigester = 10:3)

Beispiel IX

cis-4-Amino-1-methylcyclohexanol

Eine Lösung von 4,2 g cis-4-Dibenzylamino-methyl-cyclohexanol in 30 ml Methanol wird mit 1,5 g Palladium auf Kohle (10 %) versetzt und bei Raumtemperatur und 3,5 bar hydriert, bis kein Wasserstoff mehr aufgenommen wird. Nach Filtration und Verdampfen des Lösungsmittels im Rotationsverdampfer erhält man 2,75 g eines öligen Rückstands, der ohne weitere Reinigung eingesetzt wird.
$R_f$-Wert: 0,06 (Aluminiumoxid; Petrolether/Essigester = 10:4)
Analog Beispiel IX wird folgende Verbindung erhalten:

(1) trans-4-Amino-1-methylcyclohexanol
Schmelzpunkt: 225-230°C
$R_f$-Wert: 0,13 (Aluminiumoxid; Petrolether/Essigester = 10:4)

Beispiel X

trans-4-(Hydroxymethyl)-cyclohexylamin

Zu 0,9 g Lithiumaluminiumhydrid in 70 ml Tetrahydrofuran wird bei Raumtemperatur unter Rühren portionsweise eine Lösung von 1,4 g trans-4-Amino-cyclohexancarbonsäure-methylester in 30 ml Tetrahydrofuran getropft. Danach wird noch eine Stunde zum Sieden erhitzt. Man kühlt auf 0°C und tropft vorsichtig solange 10%ige Kalilauge hinzu, bis sich ein weißer Niederschlag gebildet hat. Man dekantiert und wäscht den Niederschlag viermal durch Zugabe von je 50 ml Tetrahydrofuran und Abdekantieren. Die organischen Phasen werden vereinigt, das Lösungsmittel im Rotationsverdampfer abdestilliert und der Rückstand an Aluminiumoxid mit einem Essigester/Methanol/konz.Ammoniak-Gemisch (500:180:1) säulenchromatographisch gereinigt.
Schmelzpunkt: 135-139°C
Ausbeute: 0.99 g (84% der Theorie),
$R_f$-Wert: 0,48 (Aluminiumoxid; Essigester/Methanol/konz.Ammoniak = 500:180:1)

Beispiel XI

4-Tetrahydropyranon-oxim

Zu einer Mischung von 5.2 g Hydroxylamin-hydrochlorid und 4.8 g Natriumacetat in 50 ml Wasser wird bei 60°C unter Rühren 5.0 g 4-Tetrahydropyranon getropft. Nach einer weiteren Stunde bei 60°C wird abkühlen gelassen und die Lösung dreimal mit je 50 ml Ether extrahiert. Dann werden die vereinigten organischen Phasen über Natriumsulfat getrocknet, das Solvens im Rotationsverdampfer abdestilliert und der Rückstand ohne weitere Reinigung in die nächste Reaktion eingesetzt.
Schmelzpunkt: 50-52°C
Ausbeute: 4,2 g (74% der Theorie),
$R_f$-Wert: 0,30 (Kieselgel; Petrolether/Essigester = 1:1)

Beispiel XII

4-Amino-tetrahydropyran

4.2 g 4-Tetrahydropyranon-oxim werden in 100 ml Ethanol gelöst und nach Zugabe von 0.5 g Palladium auf Kohle (10%) in einer Parr-Apparatur 2.5 Stunden bei 90°C und 5 bar Wasserstoffdruck hydriert. Nach dem Abkühlen wird das Solvens im Rotationsverdampfer abdestilliert und der Rückstand ohne weitere Reinigung weiter verwendet.
Ausbeute: 0.7 g (19%der Theorie) eines farblosen Öls,
$R_f$-Wert: 0,45 (Kieselgel; Methylenchlorid/Essigester/Methanol = 10:4:2)

Beispiel XIII

4-Brom-cyclopropylbenzol

Zu einer Mischung von 11.8 g Cyclopropylbenzol, 11 g Kaliumacetat und 100 ml Eisessig werden bei 5°C 16 g Brom langsam zugetropft. Nach 5 Stunden bei 5°C und 2 Stunden bei 10°C wird die Mischung auf Eiswasser gegossen und dreimal mit je 100 ml Ether extrahiert. Die vereinigten organischen Phasen werden je einmal mit Natriumthiosulfat-Lösung, Wasser und gesättigter Natriumchlorid-Lösung gewaschen und über Natriumsulfat getrocknet. Das Solvens wird im Rotationsverdampfer abdestilliert und der Rückstand mit Petrolether über Aluminiumoxid filtriert. Die Destillation des Rückstandes ergibt 6,2 g eines farblosen Öls
($Kp_{12}$ = 108 - 112°C).
$R_f$-Wert: 0,65 (Aluminiumoxid ; Petrolether)

Beispiel XIV

4-Brom-2-nitro-cyclopropylbenzol

Zu einer Mischung von 12 g 4-Brom-cyclopropylbenzol und 20 ml konzentrierter Schwefelsäure wird bei 0°C eine Mischung von 4.3 ml 65%iger Salpetersäure und 5 ml konzentrierter Schwefelsäure innerhalb 30 Minuten zugegeben. Dann wird die Mischung auf Eiswasser gegossen und dreimal mit je 100 ml Methylenchlorid extrahiert. Die vereinigten organischen Phasen werden über Natriumsulfat getrocknet. Das Solvens wird im Rotationsverdampfer abdestilliert und der Rückstand mit Petrolether an Aluminiumoxid säulenchromatographisch gereinigt.
Ausbeute: 3,6 g (25% der Theorie) eines farblosen Öls,
$R_f$-Wert: 0,30 (Aluminiumoxid ; Petrolether)

Beispiel XV

2-Amino-cyclopropylbenzol

3,5 g 4-Brom-2-nitro-cyclopropylbenzol werden in 30 ml Ethanol gelöst und nach Zugabe von 0.5 g Palladium auf Kohle (10%) in einer Parr-Apparatur 2.5 Stunden bei Raumtemperatur und 5 bar Wasserstoffdruck hydriert. Nach dem Abkühlen wird das Solvens im Rotationsverdampfer abdestilliert, der Rückstand mit 1N Natronlauge alkalisch gestellt und dreimal mit je 50 ml Ether extrahiert. Die vereinigten organischen Phasen werden über Natriumsulfat getrocknet, das Solvens im Rotationsverdampfer abdestilliert und der Rückstand säulenchromatographisch gereinigt.
Ausbeute: 1,3 g (72% der Theorie) eines farblosen Öls,

$R_f$-Wert: 0,43 (Kieselgel; Petrolether/Essigester = 2:1)

Beispiel XVI

2,5-Diamino-benzonitril

10 g 2-Cyano-4-nitro-anilin werden in 50 ml Dimethylformamid gelöst und nach Zugabe von 0.5 g Palladium auf Kohle (10%) in einer Parr-Apparatur 2 Stunden bei Raumtemperatur und 3 bar Wasserstoffdruck hydriert. Nach dem Abkühlen wird filtriert, das Solvens im Rotationsverdampfer abdestilliert und der Rückstand ohne weitere Reinigung weiter verwendet.
Ausbeute: 10,2 g (100% der Theorie) eines braunen, chromatographisch einheitlichen Öls,
$R_f$-Wert: 0,63 (Kieselgel; Methylenchlorid/Essigester/Methanol = 10:4:2)

Beispiel XVII

1,4-Diamino-2,6-dibrom-benzol

3.0 g 2,6-Dibrom-4-nitro-anilin werden in 150 ml Ethanol, 150 ml Essigester und 30 ml Dimethylformamid gelöst und nach Zugabe von 0.5 g Platin auf Kohle (5%) in einer Parr-Apparatur 1 Stunde bei Raumtemperatur und 1,5 bar Wasserstoffdruck hydriert. Nach dem Abkühlen wird filtriert, das Solvens im Rotationsverdampfer abdestilliert und der Rückstand säulenchromatographisch gereinigt.
Ausbeute: 1,4 g eines farblosen Öls,
$R_f$-Wert: 0,47 (Kieselgel; Petrolether/Essigester = 2:1)

Beispiel XVIII

3-(N-Benzyloxycarbonyl)amino-tetrahydrofuran

Eine Lösung von 0,74 g 3-Tetrahydrofuran-carbonsäure, 2 ml Triethylamin und 2,2 g Diphenylphosphorylazid in 10 ml Dioxan wird eine Stunde zum Sieden erhitzt. Nach Zugabe von 2,7 g Benzylalkohol wird weitere 12 h zum Sieden erhitzt. Nach Verdampfen des Lösungsmittels im Rotationsverdampfer wird der Rückstand durch Chromatographie über eine Kieselgelsäule mit Petrolether/Essigester (10:3) gereinigt.
Ausbeute: 2,24 g (86 % der Theorie),
Schmelzpunkt: 54-56°C
$R_f$-Wert: 0,53 (Kieselgel; Petrolether/Essigester = 1:1)

Beispiel XIX

3-Amino-tetrahydrofuran

Eine Lösung von 2,2 g 3-(N-Benzyloxycarbonyl)aminotetrahydrofuran in 30 ml Methanol wird mit 0,4 g Palladium auf Kohle (10 %) versetzt und bei Raumtemperatur und 5 bar hydriert, bis kein Wasserstoff mehr aufgenommen wird. Nach Filtration und Verdampfen des Lösungsmittels im Rotationsverdampfer erhält man 0,31 g eines öligen Rückstands, der ohne weitere Reinigung eingesetzt wird. Ausbeute: 0,31 g (33 % der Theorie),
$R_f$-Wert: 0,45 (Aluminiumoxid;
Methylenchlorid/Essigester/Methanol = 10:4:3)
Massenspektrum: $M^+$ = 87

Beispiel XX

4-(2-Hydroxymethyl-1-pyrrolidinyl)anilin

Eine Lösung von 1,0 g 1-(4-Nitrophenyl)-2-hydroxymethylpyrrolidin in 40 ml Methanol wird mit 0,5 g Palladium auf Kohle (5 %) versetzt und bei Raumtemperatur und 5 bar hydriert, bis kein Wasserstoff mehr aufgenommen wird. Nach Filtration und Verdampfen des Lösungsmittels im Rotationsverdampfer erhält man einen dunkel gefärbten Rückstand, der ohne weitere Reinigung eingesetzt wird.
Ausbeute: 1,1 g (quant.),
$R_f$-Wert: 0,81 (Kieselgel; Methylenchlorid/Methanol = 10:3)

Beispiel XXI

3-(4-Amino-phenyl)-propionsäure

Eine Mischung von 155 g 4-Nitro-zimtsäure in 1 l Methanol wird mit 15 g Palladium auf Kohle (10 %) und 30 ml Wasser versetzt und bei Raumtemperatur und 3 bar hydriert, bis kein Wasserstoff mehr aufgenommen wird (2 h). Nach Filtration und Verdampfen des Lösungsmittels im Rotationsverdampfer wird zur Entfernung der Wasser-Reste zweimal mit je 300 ml Toluol versetzt und das Lösungsmittel im Rotationsverdampfer abdestilliert. Man erhält so 132 g (quant.) 3-(4-Amino-phenyl)-propionsäure.
Schmelzpunkt: 124 - 128°C.

Beispiel XXII

3-(trans-4-Acetylamino-cyclohexyl)-propionsäure

Eine Mischung von 397 g 3-(4-Amino-phenyl)-propionsäure, 125 g NaOH und 160 g Raney-Nickel in 5,7 1 Wasser wird bei 170°C und 100 bar hydriert, bis kein Wasserstoff mehr aufgenommen wird (30 h). Nach Filtration und Waschen des Rückstands mit Wasser erhält man als Filtrat 6,3 l einer farblosen Lösung, die mit einer Lösung von 192 g NaOH in 400 ml Wasser und danach tropfenweise innerhalb 35 min. mit 454 ml Acetanhydrid versetzt wird. Nach fünf Stunden wird vom Niederschlag abfiltriert, das Filtrat durch Zugabe konzentrierter Salzsäure auf pH4 gebracht und drei Stunden bei O°C gerührt. Dann wird abgesaugt, mit 250 ml Eiswasser nachgewaschen und bei 70°C getrocknet. Man erhält so 216 g 3-(trans-4-Acetylamino-cyclohexyl)-propionsäure.
Ausbeute: 216 g (42 % d. Theorie).
Schmelzpunkt: 193 - 196°C

Beispiel XXIII

3-(trans-4-Amino-cyclohexyl)-propionsäuremethylesterhydrochlorid

Eine Mischung von 185 g 3-(trans-4-Acetylamino-cyclohexyl)propionsäure, 500 ml Wasser und 500 ml konz. Salzsäure wird 68 h zum Sieden erhitzt. Dann wird im Rotationsverdampfer zur Trockne eingedampft, fünfmal mit je 300 ml einer Methanol/Toluol-2:1-Mischung versetzt und jeweils erneut eingedampft. Der Rückstand wird mit 450 ml einer Aceton/tert.-Butylmethylether-1:2-Mischung verrührt, abgesaugt und im Vakuum über NaOH getrocknet. Zur Vervollständigung der Veresterung wird in 1 l Methanol gelöst und unter Eiskühlung tropfenweise mit 50 ml Thionylchlorid versetzt. Nach 30 min. wird das Lösungsmittel im Rotationsverdampfer abdestilliert, der Rückstand mit 300 ml Methanol versetzt und erneut eingedampft. Der Rückstand wird mit 450 ml einer Aceton/tert.-Butylmethylether-1:2-Mischung verrührt, abgesaugt und getrocknet.
Ausbeute: 178 g (92 % d. Theorie).
Schmelzpunkt: 196 - 198°C

Beispiel XXIV

trans-4-Dibenzylamino-cyclohexanol

Zu einer Mischung von 61 g trans-4-Amino-cyclohexanol, 350 ml Wasser, 350 ml Ethanol und 110 g Kaliumcarbonat werden 140 ml Benzylbromid zugetropft. Dann wird eine Stunde zum Sieden erhitzt, abgekühlt und der kristalline Niederschlag abgesaugt. Der Rückstand wird in 1,5 l Cyclohexan aufgenommen, die Mischung aufgekocht, abgekühlt und abgesaugt. Nachwaschen mit Cyclohexan und Trocknen im Vakuum ergibt 108 g 192 % d. Theorie) trans-4-Dibenzylamino-cyclohexanol.
Schmelzpunkt: 97 - 99°C

Beispiel XXV

α-(trans-4-Amino-cyclohexyloxy)-essigsäure-tert.-butylester

Zu einer Mischung von 76 g trans-4-Dibenzylamino-cyclohexanol, 57 ml α-Bromessigsäure-tert.-butylester, 700 ml Toluol und 2,8 g Tetrabutylammoniumhydrogensulfat wird bei Raumtemperatur eine Lösung von 262 g NaOH in 260 ml Wasser zugetropft. Nach 20 h wird die organische Phase abgetrennt, zweimal mit je 100 ml Wasser und

einmal mit gesättigter Natriumchlorid-Lösung gewaschen, über Magnesiumsulfat getrocknet und das Lösungsmittel im Rotationsverdampfer abdestilliert.
Man erhält so 116 g eines leicht gelben Feststoffs, der ohne weitere Reinigung in 1,5 l Methanol aufgenommen, mit 20 g Palladium auf Kohle (10%) versetzt und bei Raumtemperatur und 5 bar hydriert wird, bis kein Wasserstoff mehr aufgenommen wird (3 h). Nach Filtration wird das Lösungsmittel im Rotationsverdampfer abdestilliert.
Ausbeute: 68 g (quant.) eines gelben Öls, das ohne weitere Reinigung in die nächste Reaktion eingesetzt wird.

Beispiel XXVI

α-(trans-4-Amino-cyclohexyloxy)-essigsäure-methylesterhydrochlorid

59 g des oben erhaltenen Öls werden in 500 ml Methanol gelöst. Eine Stunde lang wird bei 0°C HCl-Gas eingeleitet und weitere 12 h bei Raumtemperatur gerührt. Das Lösungsmittel wird im Rotationsverdampfer abdestilliert, der Rückstand mit Aceton verrieben und abgesaugt. Nach dem Trocknen im Vakuum erhält man 34 g (59 % der Theorie) α-(trans-4-Amino-cyclohexyloxy)essigsäure-methylester-hydrochlorid als farblose Kristalle. Schmelzpunkt: 157 - 160°C

Beispiel XXVII

trans-4-Aminomethyl-cyclohexancarbonsäuremethylesterhydrochlorid

103 g trans-4-Aminomethyl-cyclohexancarbonsäure werden in 1 l Methanol gelöst und unter Eiskühlung mit 48 ml Thionylchlorid tropfenweise versetzt. Nach 2 h bei Raumtemperatur wird das Lösungsmittel im Rotationsverdampfer abdestilliert, der Rückstand mit 300 ml tert.-Butylmethylether verrührt, abgesaugt und getrocknet.
Ausbeute: 137 g (quant.).
Schmelzpunkt: 170 - 172°C

Biespiel 1

4-[(3-Methylphenyl)amino]-6-(cyclohexylamino)-pyrimido-[5,4-d]pyrimidin

Zu 0,4 g eines Gemisches aus 4-[(3-Methylphenyl)amino]-6-methylsulfinyl-pyrimido [5,4-d]pyrimidin und 4-[(3-Methylphenyl)amino]-6-methylsulfonyl-pyrimido[5,4-d]pyrimidin in 10 ml Dioxan werden 1,3 ml Cyclohexylamin gegeben und über Nacht bei Raumtemperatur gerührt. Das Reaktionsgemisch wird eingedampft, mit Wasser versetzt und der Feststoff abgesaugt. Das Rohprodukt wird durch Chromatographie über eine Kieselgelsäule mit Petrolether/Essigester (2:1) gereinigt.
Ausbeute: 0,23 g (54 % der Theorie),
Schmelzpunkt: 165-167°C
$R_f$-Wert: 0,51 (Kieselgel; Petrolether/Essigester = 2:1)

| | | | |
|---|---|---|---|
| Ber. | C 68,24 | H 6,63 | N 25,13 |
| Gef. | 68,44 | 6,79 | 25,01 |

Analog Beispiel 1 werden folgende Verbindungen erhalten:

(1) 4-[(3-Methylphenyl)amino]-6-(isobutylamino)-pyrimido-[5,4-d]pyrimidin
Schmelzpunkt: 164-166°C

| | | | |
|---|---|---|---|
| Ber. | C 66,21 | H 6,54 | N 27,25 |
| Gef. | 66,28 | 6,64 | 27,13 |

(2) 4-[(3-Methylphenyl)amino]-6-(isopropylamino)-pyrimido [5,4-d]pyrimidin
Schmelzpunkt: 176-178°C
$R_f$-Wert: 0,60 (Kieselgel; Petrolether/Essigester = 1:1)

| | | | |
|---|---|---|---|
| Ber. | C 65,29 | H 6,16 | N 28,55 |

(fortgesetzt)

| | | | |
|---|---|---|---|
| Gef. | 65,04 | 6,17 | 28,26 |

(3) 4-[(3-Methylphenyl)amino]-6-(1-phenyl-4-piperazinyl)-pyrimido[5,4-d]pyrimidin
Schmelzpunkt: 190-192°C
$R_f$-Wert: 0,43 (Kieselgel; Petrolether/Essigester = 2:1)

| | | | |
|---|---|---|---|
| Ber. | C 69,50 | H 5,83 | N 24,67 |
| Gef. | 69,70 | 6,01 | 24,31 |

(4) 4-[(3-Methylphenyl)amino]-6-(piperidinyl)-pyrimido[5,4-d]pyrimidin
Schmelzpunkt: 111-113°C
$R_f$-Wert: 0,84 (Kieselgel; Petrolether/Essigester = 1:1)

| | | | |
|---|---|---|---|
| Ber. | C 67,48 | H 6,29 | N 26,23 |
| Gef. | 67,51 | 6,36 | 26,26 |

(5) 4-[(3-Methylphenyl)amino]-6-methoxy-pyrimido[5,4-d]pyrimidin
Durchführung in Methanol mit Natriummethylat
Schmelzpunkt: 121-123°C
$R_f$-Wert: 0,31 (Kieselgel; Petrolether/Essigester = 2:1)

| | | | |
|---|---|---|---|
| Ber. | C 62,91 | H 4,90 | N 26,20 |
| Gef. | 62,90 | 4,99 | 26,13 |

(6) 4-[(3-Methylphenyl)amino]-6-(tert.butylamino)-pyrimido[5,4-d]pyrimidin
Schmelzpunkt: 206-208°C
$R_f$-Wert: 0,50 (Kieselgel; Petrolether/Essigester = 2:1)

| | | | |
|---|---|---|---|
| Ber. | C 66,21 | H 6,54 | N 27,25 |
| Gef. | 66,17 | 6,59 | 27,12 |

(7) 4-[(3-Methylphenyl)amino]-6-(2,3,4,5-tetrahydro-1H-3-benzazepin-3-yl)-pyrimido [5,4-d]pyrimidin
Schmelzpunkt: 154-156°C
$R_f$-Wert: 0,86 (Kieselgel; Petrolether/Essigester = 1:1)

| | | | |
|---|---|---|---|
| Ber. | C 72,23 | H 5,80 | N 21,97 |
| Gef. | 71,93 | 5,82 | 21,93 |

(8) 4-[(3-Methylphenyl)amino]-6-(ethylamino)-pyrimido[5,4-d]pyrimidin
Schmelzpunkt: 160°C
$R_f$-Wert: 0,44 (Kieselgel; Petrolether/Essigester = 1:1)

| | | | |
|---|---|---|---|
| Ber. | C 64,27 | H 5,75 | N 29,98 |
| Gef. | 64,22 | 5,91 | 29,99 |

(9) 4-[(3-Methylphenyl)amino]-6-(n-hexylamino)-pyrimido[5,4-d]pyrimidin
Schmelzpunkt: 116-118°C
$R_f$-Wert: 0,46 (Kieselgel; Petrolether/Essigester = 1:1)

| | | | |
|---|---|---|---|
| Ber. | C 67,83 | H 7,19 | N 24,98 |
| Gef. | 67,77 | 7,19 | 24,99 |

(10) 4-[(3-Methylphenyl)amino] -6-(diethylamino)-pyrimido[5,4-d]pyrimidin

Schmelzpunkt: 119-121°C
$R_f$-Wert: 0,62 (Kieselgel; Petrolether/Essigester = 2:1)

| | | | |
|---|---|---|---|
| Ber. | C 66,21 | H 6,49 | N 27,25 |
| Gef. | 66,27 | 6,67 | 27,31 |

(11) 4-[(3-Methylphenyl)amino]-6-(dimethylamino)-pyrimido[5,4-d]pyrimidin
Schmelzpunkt: 120-121°C
$R_f$-Wert: 0,57 (Kieselgel; Petrolether/Essigester = 1:1)

| | | | |
|---|---|---|---|
| Ber. | C 64,27 | H 5,75 | N 29,98 |
| Gef. | 64,17 | 5,77 | 30,22 |

(12) 4-[(3-Methylphenyl)amino]-6-(benzylamino)-pyrimido[5,4-d]pyrimidin
Schmelzpunkt: 198-204°C
$R_f$-Wert: 0,66 (Kieselgel; Petrolether/Essigester/Methanol = 10:8:1)
Massenspektrum: $M^+$ = 342

(13) 4-[(3-Methylphenyl)amino]-6-amino-pyrimido[5,4-d]pyrimidin
Schmelzpunkt: >260°C
$R_f$-Wert: 0,67 (Kieselgel; Petrolether/Essigester/Methanol = 10:8:1,5; Auftrag in Dimethylformamid)
Massenspektrum: $M^+$ = 252

(14) 4-(Phenylamino)-6-(1-pyrrolidinyl)-pyrimido[5,4-d]pyrimidin
Schmelzpunkt: 172-174°C
Massenspektrum: $M^+$ = 292

(15) 4-[(3-Methylphenyl)amino]-6-(methylamino)-pyrimido[5,4-d]pyrimidin
Schmelzpunkt: 195-197°C
$R_f$-Wert: 0,31 (Kieselgel; Petrolether/Essigester = 1:1)

| | | | |
|---|---|---|---|
| Ber. | C 63,14 | H 5,29 | N 31,55 |
| Gef. | 62,74 | 5,31 | 31,09 |

(16) 4-[(3-Methylphenyl)amino]-6-(4-methyl-1-piperazinyl)-pyrimido [5,4-d]pyrimidin
Schmelzpunkt: 133-135°C
$R_f$-Wert: 0,53 (Aluminiumoxid; Petrolether/Essigester = 1:1)

| | | | |
|---|---|---|---|
| Ber. | C 64,45 | H 6,31 | N 29,23 |
| Gef. | 64,36 | 6,39 | 29,03 |

(17) 4-[(3-Methylphenyl)amino]-6-(cyclopropylamino)-pyrimido[5,4-d]pyrimidin
Schmelzpunkt: 168-170°C
$R_f$-Wert: 0,48 (Kieselgel; Petrolether/Essigester = 1:1)

| | | | |
|---|---|---|---|
| Ber. | C 65,96 | H 5,18 | N 28,84 |
| Gef. | 65,48 | 5,69 | 28,22 |

(18) 4-[(3-Methylphenyl)amino]-6-(1-pyrrolidinyl)-pyrimido[5,4-d]pyrimidin
Schmelzpunkt: 145-147°C
$R_f$-Wert: 0,65 (Kieselgel; Petrolether/Essigester/Methanol = 10:5:1)

(19) 4-[(3-Methylphenyl)amino]-6-(morpholino)-pyrimido[5,4-d]pyrimidin
Schmelzpunkt: 125-127°C
$R_f$-Wert: 0,53 (Kieselgel; Petrolether/Essigester/Methanol = 10:5:1)

(20) 4-(N-Methyl-N-phenyl-amino]-6-(1-pyrrolidinyl)-pyrimido[5,4-d]pyrimidin
Schmelzpunkt: 128-130°C
$R_f$-Wert: 0,45 (Kieselgel; Petrolether/Essigester = 1:1)

| | | | |
|---|---|---|---|
| Ber. | C 66,64 | H 5,92 | N 27,43 |
| Gef. | 66,54 | 5,83 | 27,11 |

(21) 4-[(3-Methylphenyl)amino]-6-(allylamino)-pyrimido[5,4-d]pyrimidin
Schmelzpunkt: 152-156°C
$R_f$-Wert: 0,63 (Kieselgel; Petrolether/Essigester/Methanol = 10:8:1)

(22) 4-[(3-Methylphenyl)amino]-6-[(2-methyl-2-buten-4-yl)amino]-pyrimido [5,4-d]pyrimidin

(23) 4-[(3-Methylphenyl)amino]-6-(propargylamino)-pyrimido[5,4-d]pyrimidin
Schmelzpunkt: 185-187°C
$R_f$-Wert: 0,68 (Kieselgel; Petrolether/Essigester/Methanol = 10:8:1)

(24) 4-[(3-Methylphenyl)amino]-6-[(cyclopropylmethyl)amino)-pyrimido-[5,4-d]pyrimidin
Schmelzpunkt: 141-144°C
$R_f$-Wert: 0,71 (Kieselgel; Petrolether/Essigester/Methanol = 10:8:1)

(25) 4-[(3-Methylphenyl)amino]-6-(cyclobutylamino)-pyrimido[5,4-d]pyrimidin
Schmelzpunkt: 184-186°C
$R_f$-Wert: 0,54 (Kieselgel; Petrolether/Essigester/Methanol = 10:8:1)

(26) 4-[(3-Methylphenyl)amino]-6-[(2-hydroxyethyl)amino]-pyrimido[5,4-d]pyrimidin
Schmelzpunkt: 167-171°C
$R_f$-Wert: 0,42 (Kieselgel; Petrolether/Essigester/Methanol = 10:8:2)

(27) 4-[(3-Methylphenyl)amino]-6-[(2-methoxyethyl)amino]-pyrimido[5,4-d]pyrimidin
Schmelzpunkt: 128-131°C
$R_f$-Wert: 0,56 (Kieselgel; Petrolether/Essigester/Methanol = 10:8:1)

(28) 4-[(3-Methylphenyl)amino]-6-(1-piperazinyl)-pyrimido[5,4-d]pyrimidin
Schmelzpunkt: 125-128°C
$R_f$-Wert: 0,37 (Kieselgel; Methylenchlorid/Methanol = 6:1) Massenspektrum: $M^+$ = 321

(29) 4-[(3-Methylphenyl)amino]-6-(1-acetyl-4-piperazinyl)-pyrimido[5,4-d]pyrimidin
Schmelzpunkt: 180-182°C
$R_f$-Wert: 0,38 (Kieselgel; Essigester/Methanol = 15:1) Massenspektrum: $M^+$ = 363

(30) 4-[(3-Methylphenyl)amino]-6-(1-methansulfonyl-4-piperazinyl)-pyrimido [5,4-d]pyrimidin
Hergestellt aus den Verbindungen des Beispiels 2 durch Umsetzung mit Piperazin und anschließender Umsetzung mit Methansulfonylchlorid in Gegenwart von Triethylamin.
Schmelzpunkt: 248-250°C
$R_f$-Wert: 0,53 (Kieselgel; Methylenchlorid/Methanol = 30:1)
Massenspektrum: $M^+$ = 399

(31) 4-[(3-Methylphenyl)amino]-6-(4-hydroxy-1-piperidinyl)-pyrimido [5,4-d]pyrimidin
Schmelzpunkt: 205-207°C
$R_f$-Wert: 0,33 (Kieselgel; Petrolether/Essigester/Methanol = 10:10:1)

(32) 4-[(3-Methylphenyl)amino]-6-(3-hydroxy-1-piperidinyl)-pyrimido [5,4-d] pyrimidin
Schmelzpunkt: 171-173°C
$R_f$-Wert: 0,46 (Kieselgel; Methylenchlorid/Methanol = 9:1)

(33) 4-[(3-Methylphenyl)amino]-6-(4-phenyl-1-piperidinyl)-pyrimido [5,4-d]pyrimidin

Schmelzpunkt: 148-150°C
$R_f$-Wert: 0,77 (Kieselgel; Petrolether/Essigester = 1:1)

(34) 4-[(3-Methylphenyl)amino]-6-(1-benzyl-4-piperazinyl)-pyrimido [5,4-d]pyrimidin
Schmelzpunkt: 132-134°C

(35) 4-[(3-Methylphenyl)amino]-6-[(2-phenylethyl)amino]-pyrimido[5,4-d]pyrimidin
Schmelzpunkt: 150-151°C
$R_f$-Wert: 0,56 (Kieselgel; Petrolether/Essigester = 1:1)

| | | | |
|---|---|---|---|
| Ber. | C 70,77 | H 5,66 | N 23,58 |
| Gef. | 70,85 | 5,82 | 23,52 |

(36) 4-[(3-Methylphenyl)amino]-6-(cyclopentylamino)-pyrimido[5,4-d]pyrimidin
Schmelzpunkt: 178-180°C
$R_f$-Wert: 0,49 (Kieselgel; Petrolether/Essigester = 1:1)

| | | | |
|---|---|---|---|
| Ber. | C 67,48 | H 6,29 | N 26,23 |
| Gef. | 67,42 | 6,33 | 25,70 |

(37) 4-[(3-Methylphenyl)amino]-6-(thiomorpholino)-pyrimido[5,4-d]pyrimidin
Schmelzpunkt: 125-127°C
$R_f$-Wert: 0,81 (Kieselgel; Petrolether/Essigester = 1:1)

(38) 4-[(3-Methylphenyl)amino]-6-(S-oxido-thiomorpholino)-pyrimido [5,4-d]pyrimidin
Schmelzpunkt: 212°C
$R_f$-Wert: 0,40 (Kieselgel; Essigester/Methanol = 10:1)

(39) 4-[(3-Methylphenyl)amino]-6-(S,S-dioxido-thiomorpholino)-pyrimido [5,4-d]pyrimidin
Schmelzpunkt: 240°C
$R_f$-Wert: 0,30 (Kieselgel; Petrolether/Essigester = 1:1)

(40) 4-[(3-Methylphenyl)amino]-6-[(carboxymethyl)amino]-pyrimido[5,4 -d]pyrimidin

(41) 4-[(3-Methylphenyl)amino]-6-[(aminocarbonylmethyl)amino]-pyrimido[5,4-d]pyrimidin
Schmelzpunkt: 290°C (Zers.)
$R_f$-Wert: 0,26 (Aluminiumoxid; Methylenchlorid/Methanol = 9:1)

(42) 4-[(3-Methylphenyl)amino]-6-[(methylaminocarbonylmethyl)amino]-pyrimido [5,4-d]pyrimidin

(43) 4-[(3-Methylphenyl)amino]-6-[(dimethylaminocarbonylmethyl)amino]-pyrimido[5,4-d]pyrimidin

(44) 4-[(3-Methylphenyl)amino]-6-[[(1-pyrrolidinyl)carbonylmethyl]amino]-pyrimido [5,4-d]pyrimidin

(45) 4-[(3-Methylphenyl)amino]-6-[[(morpholinocarbonyl)-methyl]amino]-pyrimido [5,4-d]pyrimidin

(46) 4-[(3-Methylphenyl)amino]-6-(4-carboxy-1-piperidinyl)-pyrimido[5,4-d]pyrimidin
Hergestellt aus den Verbindungen des Beispiels 2 durch Umsetzung mit Piperidin-4-carbonsäure in einem Dioxan/Natronlauge-Gemisch.
Schmelzpunkt: 255-258°C (Zers.)
$R_f$-Wert: 0,21 (Kieselgel; Petrolether/Essigester/Methanol = 10:10:1)
Massenspektrum: $M^+$ = 364

(47) 4-[(3-Methylphenyl)amino]-6-(4-aminocarbonyl-1-piperidinyl)-pyrimido[5,4-d]pyrimidin
Schmelzpunkt: 242-244°C (Zers.)
$R_f$-Wert: 0,48 (Kieselgel; Petrolether/Essigester/Methanol = 10:10:5)
Massenspektrum: $M^+$ = 363

(48) 4-[(3-Methylphenyl)amino]-6-(3-diethylaminocarbonyl-1-piperidinyl)-pyrimido[5,4-d]pyrimidin
Schmelzpunkt: 119-121°C
$R_f$-Wert: 0,36 (Kieselgel; Petrolether/Essigester/Methanol = 10.10:1)

| | | | |
|---|---|---|---|
| Ber. | C 65,85 | H 6,97 | N 23,37 |
| Gef. | 65,80 | 7,07 | 23,17 |

(49) 4-[(3-Methylphenyl)amino]-6-(8-aza-1,4-dioxaspiro[4,5]decan-8-yl)-pyrimido [5,4-d]pyrimidin
Schmelzpunkt: 184-186°C
$R_f$-Wert: 0,56 (Kieselgel; Petrolether/Essigester = 1:1)

| | | | |
|---|---|---|---|
| Ber. | C 63,48 | H 5,86 | N 22,21 |
| Gef. | 63,35 | 6,00 | 21,89 |

(50) 4-[(3-Methylphenyl)amino]-6-[(2-dimethylaminoethyl)amino]-pyrimido [5,4-d]pyrimidin
Schmelzpunkt: 140-142°C
$R_f$-Wert: 0,66 (Aluminiumoxid; Petrolether/Essigester/Methanol = 10:10:1)
Massenspektrum: $M^+$ = 323

(51) 4-[(3-Methylphenyl)amino]-6-[bis-(2-hydroxyethyl)-amino]-pyrimido [5,4-d]pyrimidin
Schmelzpunkt: 180-182°C
$R_f$-Wert: 0,29 (Kieselgel; Petrolether/Essigester/Methanol/Ammoniak = 5:5:1.25:0.1)
Massenspektrum: $M^+$ = 340

(52) 4-[(3-Methylphenyl)amino]-6-(dibutylamino)-pyrimido[5,4-d]pyrimidin
Schmelzpunkt: 56-58°C
$R_f$-Wert: 0,57 (Kieselgel; Petrolether/Essigester = 1:1)

| | | | |
|---|---|---|---|
| Ber. | C 69,20 | H 7,74 | N 23,06 |
| Gef. | 69,38 | 7,80 | 22,91 |

(53) 4-[(3-Methylphenyl)amino]-6-(cyclopentyloxy)-pyrimido[5,4-d]pyrimidin
Schmelzpunkt: 83-85°C
$R_f$-Wert: 0,50 (Kieselgel; Petrolether/Essigester = 2:1)
Hergestellt aus den Verbindungen des Beispiels 2 unter Verwendung von Cyclopentanol und metallischem Natrium.

(54) 4-[(3-Methylphenyl)amino]-6-(4-cyan-1-piperidinyl)-pyrimido[5,4 -d]pyrimidin

(55) 4-[(3-Methylphenyl)amino]-6-(2-azaspiro[4,5]decan-2-yl)-pyrimido[5,4-d]pyrimidin

(56) 4-[(3-Methylphenyl)amino]-6-(7-azaspiro[4,5]decan-7-yl)-pyrimido [5,4-d]pyrimidin

(57) 4-[(3-Methylphenyl)amino]-6-(2-butylamino)-pyrimido[5,4-d]pyrimidin
Schmelzpunkt: 178-180°C
$R_f$-Wert: 0,67 (Kieselgel; Petrolether/Essigester = 1:1)

| | | | |
|---|---|---|---|
| Ber. | C 66,21 | H 6,54 | N 27,25 |
| Gef. | 66,23 | 6,59 | 27,19 |

Massenspektrum: $M^+$ = 308

(58) 4-[(3-Methylphenyl)amino]-6-(1-hydroxy-2-propylamino)-pyrimido [5,4-d]pyrimidin
Schmelzpunkt: 176-178°C
$R_f$-Wert: 0,33 (Kieselgel; Petrolether/Essigester/Methanol = 10:10:1)

| | | | |
|---|---|---|---|
| Ber. | C 61,92 | H 5,85 | N 27,08 |
| Gef. | 61,60 | 5,97 | 26,72 |

(59) 4-[(3-Methylphenyl)amino]-6-(2-carboxy-1-pyrrolidinyl)-pyrimido[5,4-d]pyrimidin

(60) 4-[(3-Methylphenyl)amino]-6-(2-aminocarbonyl-1-pyrrolidinyl)-pyrimido [5,4-d]pyrimidin

(61) 4-[(3-Methylphenyl)amino]-6-(4-amino-1-piperidinyl)-pyrimido[5,4-d]pyrimidin
Schmelzpunkt: 105-110°C
$R_f$-Wert: 0,12 (Kieselgel; Methylenchlorid/Methanol = 1:1)

(62) 4-[(3-Methylphenyl)amino]-6-[(1-methyl-4-piperidinyl)amino]-pyrimido[5,4-d]pyrimidin
Schmelzpunkt: 204-205°C
$R_f$-Wert: 0,37 (Aluminiumoxid; Petrolether/Essigester/Methanol = 10:10:0,5)

| | | | |
|---|---|---|---|
| Ber. | C 65,31 | H 6,63 | N 28,06 |
| Gef. | 65,23 | 6,68 | 27,72 |

(63) 4-[(3-Methylphenyl)amino]-6-(1,2,3,4-tetrahydro-2-iso-chinolinyl)-pyrimido [5,4-d]pyrimidin
Schmelzpunkt: 95-97°C
$R_f$-Wert: 0,38 (Kieselgel; Petrolether/Essigester = 7:3)
Massenspektrum: $M^+$ = 368

(64) 4-[(3-Methylphenyl)amino]-6-(2-aza-bicyclo[2.2.2]octan-2-yl)-pyrimido [5,4-d]pyrimidin

(65) 4-[(3-Methylphenyl)amino]-6-[(endo-2-norbornyl)amino]-pyrimido[5,4-d]pyrimidin
Schmelzpunkt: 149-154°C
$R_f$-Wert: 0,78 (Kieselgel; Petrolether/Essigester/Methanol = 10:8:2)

| | | | |
|---|---|---|---|
| Ber. | C 69,34 | H 6,40 | N 24,26 |
| Gef. | 69,65 | 6,49 | 24,23 |

(66) 4-[(3-Methylphenyl)amino]-6-[(norbornan-2-yl-methyl)amino]-pyrimido[5,4-d]pyrimidin

(67) 4-[(3-Methylphenyl)amino]-6-[(5-norbornen-2-yl-methyl)amino]-pyrimido [5,4-d]pyrimidin

(68) 4-[(3-Methylphenyl)amino]-6-(R(+)-bornylamino)-pyrimido[5,4-d]pyrimidin
Schmelzpunkt: 184-187°C
$R_f$-Wert: 0,80 (Kieselgel; Petrolether/Essigester/Methanol = 10:6:1)

(69) 4-[(3-Methylphenyl)amino]-6-[(3-chinuclidinyl)amino]-pyrimido [5,4-d]pyrimidin
Schmelzpunkt: 186-189°C
$R_f$-Wert: 0,48 (Kieselgel; Petrolether/Essigester/Methanol = 10:5:2)

| | | | |
|---|---|---|---|
| Ber. | C 66,46 | H 6,41 | N 27,13 |
| Gef. | 66,09 | 6,40 | 27,10 |

(70) 4-[(3-Methylphenyl)amino]-6-[(cyclopentylmethyl)amino]-pyrimido[5,4-d]pyrimidin

(71) 4-[(3-Methylphenyl)amino]-6-[(1-adamantyl)amino]-pyrimido[5,4-d]pyrimidin
Schmelzpunkt: 262-266°C
$R_f$-Wert: 0,69 (Kieselgel; Petrolether/Essigester/Methanol = 10:8:1)

(72) 4-[(3-Methylphenyl)amino]-6-[(trans-4-hydroxycyclohexyl)amino]-pyrimido [5,4-d]pyrimidin
Schmelzpunkt: 226-228°C

$R_f$-Wert: 0,30 (Kieselgel; Petrolether/Essigester/Methanol = 10:8:2)

(73) 4-[(3-Methylphenyl)amino]-6-[(2-hydroxycyclopentyl)amino]-pyrimido[5,4-d]pyrimidin

(74) 4-[(3-Methylphenyl)amino]-6-[(4-dimethylamino-cyclohexyl)amino]-pyrimido [5,4-d]pyrimidin

(75) 4-[(3-Methylphenyl)amino]-6-[(3-methylcyclohexyl)amino]-pyrimido [5,4-d]pyrimidin
Schmelzpunkt: 150-152°C
$R_f$-Wert: 0,76 (Kieselgel; Petrolether/Essigester/Methanol = 10:8:1)

(76) 4-[(3-Methylphenyl)amino]-6-[(spiro[5,5]undecan-3-yl)amino]-pyrimido [5,4-d]pyrimidin

(77) 4-[(3-Methylphenyl)amino]-6-[(3-cyanopropyl)amino]-pyrimido[5,4-d]pyrimidin

(78) 4-[(3-Methylphenyl)amino]-6-(2-aza-bicyclo[2.2.1]heptan-2-yl)-pyrimido[5,4-d]pyrimidin

(79) 4-[(3-Methylphenyl)amino]-6-(3-aza-bicyclo[3.2.2]nonan-3-yl)-pyrimido [5,4-d]pyrimidin
Schmelzpunkt: 116-119°C
$R_f$-Wert: 0,75 (Kieselgel; Petrolether/Essigester/Methanol = 10:6:1)

(80) 4-[(3-Methylphenyl)amino]-6-(exo-2-norbornylamino)-pyrimido[5,4-d]pyrimidin
Schmelzpunkt: 245-247°C
$R_f$-Wert: 0,70 (Kieselgel; Petrolether/Essigester/Methanol = 10:8:1)

(81) 4,6-Bis-[(3-Methylphenyl)amino]-pyrimido[5,4-d]pyrimidin Schmelzpunkt: 220-222°C
$R_f$-Wert: 0,37 (Kieselgel; Petrolether/Essigester = 1:2)

(82) 4-[(3-Fluorphenyl)amino]-6-(cyclopropylamino)-pyrimido[5,4-d]pyrimidin
Schmelzpunkt: 180-182°C
$R_f$-Wert: 0,45 (Kieselgel; Petrolether/Essigester = 1:1)

(83) 4-[(3-Chlorphenyl)amino]-6-(cyclopropylamino)-pyrimido[5,4-d]pyrimidin
Schmelzpunkt: 182-184°C

| | | | | |
|---|---|---|---|---|
| Ber. | C 57,60 | H 4,18 | N 26,87 | C1 11,33 |
| Gef. | 57,66 | 4,39 | 26,40 | 11,24 |

(84) 4-[(3-Bromphenyl)amino]-6-(cyclopropylamino)-pyrimido[5,4-d]pyrimidin
Schmelzpunkt: 205-207°C
$R_f$-Wert: 0,63 (Kieselgel; Petrolether/Essigester= 1:1)

| | | | | |
|---|---|---|---|---|
| Ber. | C 50,42 | H 3,66 | N 23,52 | Br 22,39 |
| Gef. | 50,29 | 3,82 | 23,42 | 22,65 |

(85) 4-[(3-Trifluormethylphenyl)amino]-6-(cyclopropylamino)-pyrimido [5,4-d]pyrimidin
Schmelzpunkt: 146-148°C
$R_f$-Wert: 0,22 (Kieselgel; Petrolether/Essigester= 2:1)

(86) 4-[(3-Methoxyphenyl)amino]-6-(cyclopropylamino)-pyrimido[5,4-d]pyrimidin
Schmelzpunkt: 143-145°C
$R_f$-Wert: 0,50 (Kieselgel; Petrolether/Essigester = 1:1)

(87) 4-[(3-Ethylphenyl)amino]-6-(cyclopropylamino)-pyrimido[5,4-d]pyrimidin
Schmelzpunkt: 140-142°C
$R_f$-Wert: 0,55 (Kieselgel; Petrolether/Essigester = 1:1)

| | | | |
|---|---|---|---|
| Ber. | C 66,86 | H 5,61 | N 27,52 |
| Gef. | 66,51 | 5,92 | 27,12 |

(88) 4-[(3-Methylphenyl)amino]-6-hydroxy-pyrimido[5,4-d]-pyrimidin
Schmelzpunkt: > 220°C
$R_f$-Wert: 0,28 (Kieselgel; Ammoniak/Methanol = 10:1)
Hergestellt aus den Verbindungen des Beispiels 2 und Natronlauge.

(89) 4-[(3-Methylphenyl)amino]-6- (hydroxyamino)-pyrimido[5,4-d]pyrimidin

(90) 4-[(3-Methylphenyl)amino]-6-(methoxyamino)-pyrimido[5,4-d]pyrimidin

(91) 4-[(3-Methylphenyl)amino]-6-(N-methyl-N-methoxy-amino)-pyrimido [5,4-d]pyrimidin
Schmelzpunkt: 118-121°C
$R_f$-Wert: 0,60 (Kieselgel; Petrolether/Essigester/Methanol = 10:7:1)

(92) 4-[(3-Methylphenyl)amino]-6-(2,2,2-trifluorethylamino)-pyrimido [5,4-d]pyrimidin
Schmelzpunkt: 172-175°C
$R_f$-Wert: 0,59 (Kieselgel; Petrolether/Essigester/Methanol = 10:8:1)

(93) 4-[(3-Chlorphenyl)amino]-6-(1-pyrrolidinyl)-pyrimido[5,4-d]pyrimidin
Schmelzpunkt: 170-173°C
$R_f$-Wert: 0,68 (Kieselgel; Petrolether/Essigester/Methanol = 10:5:1)

| | | | | |
|---|---|---|---|---|
| Ber. | C 58,81 | H 4,63 | N 25,72 | Cl 10,85 |
| Gef. | 58,93 | 4,77 | 25,52 | 11,10 |

(94) 4-[(3-Fluorphenyl)amino]-6-(1-pyrrolidinyl)-pyrimido[5,4-d]pyrimidin
Schmelzpunkt: 169-172°C
$R_f$-Wert: 0,60 (Kieselgel; Petrolether/Essigester/Methanol = 10:5:1)

| | | | |
|---|---|---|---|
| Ber. | C 61,93 | H 4,87 | N 27,08 |
| Gef. | 62,00 | 4,95 | 27,07 |

(95) 4-[(3-Bromphenyl)amino]-6-(isopropylamino)-pyrimido[5,4-d]pyrimidin
Schmelzpunkt: 181-184°C
$R_f$-Wert: 0,50 (Kieselgel; Petrolether/Essigester/Methanol = 10:5:1)

| | | | | |
|---|---|---|---|---|
| Ber. | C 50,15 | H 4,21 | N 23,39 | Br 22,24 |
| Gef. | 49,86 | 4,37 | 23,11 | 22,30 |

(96) 4-[(3-Chlorphenyl)amino]-6-(isopropylamino)-pyrimido[5,4-d]pyrimidin
Schmelzpunkt: 193-196°C
$R_f$-Wert: 0,53 (Kieselgel; Petrolether/Essigester/Methanol = 10:5:1)

| | | | | |
|---|---|---|---|---|
| Ber. | C 57,24 | H 4,80 | N 26,70 | Cl 11,26 |
| Gef. | 57,48 | 4,97 | 26,54 | 11,85 |

(97) 4-[(3-Fluorphenyl)amino]-6-(isopropylamino)-pyrimido[5,4-d]pyrimidin
Schmelzpunkt: 195-200°C
$R_f$-Wert: 0,50 (Kieselgel; Petrolether/Essigester/Methanol = 10:5:1)

| | | | |
|---|---|---|---|
| Ber. | C 60,39 | H 5,07 | N 28,17 |
| Gef. | 60,13 | 5,13 | 28,03 |

(98) 4-[(3-Bromphenyl)amino]-6-(morpholino)-pyrimido[5,4-d]-pyrimidin
Schmelzpunkt: 183-187°C
$R_f$-Wert: 0,40 (Kieselgel; Petrolether/Essigester/Methanol = 10:5:1)

| | | | |
|---|---|---|---|
| Ber. | C 49,63 | H 3,90 | N 21,70 |
| Gef. | 49,59 | 4,17 | 21,64 |

(99) 4-[(3-Chlorphenyl)amino]-6-(morpholino)-pyrimido[5,4-d]-pyrimidin
Schmelzpunkt: 188-192°C
$R_f$-Wert: 0,41 (Kieselgel; Petrolether/Essigester/Methanol = 10:5:1)

| | | | | |
|---|---|---|---|---|
| Ber. | C 56,06 | H 4,41 | N 24,52 | Cl 10,34 |
| Gef. | 55,75 | 4,67 | 24,43 | 10,97 |

(100) 4-[(3-Fluorphenyl)amino]-6-(morpholino)-pyrimido[5,4-d]pyrimidin
Schmelzpunkt: 166-169°C
$R_f$-Wert: 0,48 (Kieselgel; Petrolether/Essigester/Methanol = 10:5:1)

(101) 4-[(3-Methylphenyl)amino]-6-[2-(2'-hydroxyethylamino)ethyloxy]pyrimido[5,4-d]pyrimidin
$R_f$-Wert: 0,10 (Kieselgel; Petrolether/Essigester/Methanol/Ammoniak = 5:5:1,25:0,1)
Massenspektrum: $M^+$ = 340
(102) 4-[(3-Methylphenyl)amino]-6-(1-azetidinyl)-pyrimido[5,4-d]pyrimidin
Schmelzpunkt: 129-131°C
$R_f$-Wert: 0,51 (Kieselgel; Petrolether/Essigester/Methanol = 10:6:1)

(103) 4-[(3-Bromphenyl)amino]-6-(1-pyrrolidinyl)-pyrimido[5,4-d]pyrimidin
Schmelzpunkt: 206-208°C
$R_f$-Wert: 0,63 (Kieselgel; Petrolether/Essigester/Methanol = 10:5:1)

| | | | | |
|---|---|---|---|---|
| Ber. | C 51,76 | H 4,07 | N 22,64 | Br 21,52 |
| Gef. | 51,67 | 4,22 | 22,44 | 22,14 |

(104) 4-[(3-Bromphenyl)amino]-6-[(trans-4-hydroxycyclohexyl)amino]pyrimido [5,4-d]pyrimidin
Schmelzpunkt: 226-231°C
$R_f$-Wert: 0,43 (Kieselgel; Petrolether/Essigester/Methanol = 10:10:2)

| | | | | |
|---|---|---|---|---|
| Ber. | C 52,06 | H 4,61 | N 20,24 | Br 19,24 |
| Gef. | 52,23 | 4,83 | 20,30 | 19,38 |

(105) 4-[(3-Methylphenyl)amino]-6-[(3-hydroxypropyl)amino]-pyrimido[5,4 -d]pyrimidin
Schmelzpunkt: 186-190°C
$R_f$-Wert: 0,38 (Kieselgel; Petrolether/Essigester/Methanol = 10:10:2)

| | | | |
|---|---|---|---|
| Ber. | C 61,92 | H 5,84 | N 27,08 |
| Gef. | 61,90 | 6,08 | 26,66 |

(106) 4-[(3-Methylphenyl)amino]-6-[(4-hydroxybutyl)amino]-pyrimido [5,4-d]pyrimidin
Schmelzpunkt: 195-201°C
$R_f$-Wert: 0,32 (Kieselgel; Petrolether/Essigester/Methanol = 10:10:2)

| | | | |
|---|---|---|---|
| Ber. | C 62,95 | H 6,21 | N 25,91 |
| Gef. | 63,04 | 6,41 | 25,51 |

(107) 4-[(3-Methylphenyl)amino]-6-[(cis-4-hydroxycyclohexyl)amino]pyrimido [5,4-d]pyrimidin
Schmelzpunkt: 212-216°C

$R_f$-Wert: 0,48 (Kieselgel; Petrolether/Essigester/Methanol = 10:8:3)

| | | | |
|---|---|---|---|
| Ber. | C 65,12 | H 6,33 | N 23,98 |
| Gef. | 65,06 | 6,46 | 23,86 |

(108) 4-[(3-Methylphenyl)amino]-6-[(trans-4-tert.butyloxycarbonylamino-cyclohexyl)amino]pyrimido[5,4-d]pyrimidin
Schmelzpunkt: 198-200°C
$R_f$-Wert: 0,55 (Kieselgel; Petrolether/Essigester/Methanol = 10:8:1)
Massenspektrum: $M^+$ = 449

(109) 4-[(3-Methylphenyl)amino]-6-[(4-oxo-cyclohexyl)amino]-pyrimido[5,4-d]pyrimidin
Hergestellt aus den Verbindungen des Beispiels 2 durch Umsetzung mit trans-4-Hydroxycyclohexylamin und anschließender Umsetzung mit Pyridiniumchlorochromat.
Schmelzpunkt: 215-218°C
$R_f$-Wert: 0,50 (Kieselgel; Petrolether/Essigester/Methanol = 10:8:3)
Massenspektrum: $M^+$ = 348

(110) 4-[(3-Chlorphenyl)amino]-6-[(trans-4-hydroxycyclohexyl)amino]pyrimido[5,4-d]pyrimidin
Schmelzpunkt: 219-223°C
$R_f$-Wert: 0,34 (Kieselgel; Petrolether/Essigester/Methanol = 10:8:2)

| | | | | |
|---|---|---|---|---|
| Ber. | C 58,30 | H 5,16 | N 22,66 | Cl 9,56 |
| Gef. | 58,22 | 5,06 | 22,88 | 9,61 |

(111) 4-[(3-Fluorphenyl)amino]-6-[(trans-4-hydroxycyclohexyl)amino]pyrimido [5,4-d]pyrimidin
Schmelzpunkt: 220-223°C
$R_f$-Wert: 0,38 (Kieselgel; Petrolether/Essigester/Methanol = 10:8:2)

| | | | |
|---|---|---|---|
| Ber. | C 61,01 | H 5,40 | N 23,71 |
| Gef. | 61,14 | 5,46 | 23,83 |

(112) 4-[(3-Methylphenyl)amino]-6-[(trans-4-methoxycyclohexyl)amino]pyrimido [5,4-d]pyrimidin
Schmelzpunkt: 188-191°C
$R_f$-Wert: 0,53 (Kieselgel; Petrolether/Essigester/Methanol = 10:8:2)

| | | | |
|---|---|---|---|
| Ber. | C 65,91 | H 6,64 | N 23,06 |
| Gef. | 65,75 | 6,79 | 22,83 |

(113) 4-[(3-Methylphenyl)amino]-6-[(trans-4-aminocyclohexyl)amino]pyrimido[5,4-d]pyrimidin
Hergestellt aus den Verbindungen des Beispiels 2 durch Umsetzung mit trans-4-tert.Butyloxy-carbonylamino-cyclohexylamin und anschließende Umsetzung mit etherischer Chlorwasserstoff-Lösung und Methanol.
Schmelzpunkt: >260°C
$R_f$-Wert: 0,28 (Reversed-Phase-Kieselgel; Methanol/5%ige Kochsalzlösung = 10:4)

(114) 4-[(3-Methylphenyl)amino]-6-[(trans-4-methylsulfonylaminocyclohexyl)amino]pyrimido[5,4-d]pyrimidin
Hergestellt aus den Verbindungen des Beispiels 2 durch Umsetzung mit trans-4-tert.Butyloxycarbonylamino-cyclohexylamin und anschließende Umsetzung mit etherischer Chlorwasserstoff-Lösung und Methanol sowie anschließende Umsetzung mit Methansulfonsäurechlorid.
Schmelzpunkt: 192-195°C
$R_f$-Wert: 0,37 (Kieselgel; Petrolether/Essigester/Methanol = 10:8:2)

(115) 4-[(3-Methylphenyl)amino]-6-[(trans-4-acetylaminocyclohexyl)amino]-pyrimido[5,4-d]pyrimidin
Hergestellt aus den Verbindungen des Beispiels 2 durch Umsetzung mit trans-4-tert.Butyloxycarbonylamino-cyclohexylamin und anschließende Umsetzung mit etherischer Chlorwasserstoff-Lösung und Methanol sowie anschließende Umsetzung mit Acetanhydrid und Triethylamin.

Schmelzpunkt: 302-305°C
$R_f$-Wert: 0,38 (Kieselgel; Petrolether/Essigester/Methanol = 10:8:3)

(116) 4-[(3-Methylphenyl)amino]-6-[(trans-4-dimethylaminocyclohexyl)amino]-pyrimido [5,4-d]pyrimidin
Hergestellt aus den Verbindungen des Beispiels 2 durch Umsetzung mit trans-4-tert.Butyloxycarbonylamino-cyclohexylamin und anschließende Umsetzung mit etherischer Chlorwasserstoff-Lösung und Methanol sowie anschließende Umsetzung mit Ameisensäure, Formaldehyd und Natriumhydrogencarbonat.
Schmelzpunkt: 161-165°C
$R_f$-Wert: 0,71 (Aluminiumoxid, Methylenchlorid/Methanol = 20:1)

| | | | |
|---|---|---|---|
| Ber. | C 66,82 | H 7,21 | N 25,97 |
| Gef. | 66,74 | 7,32 | 26,12 |

(117) 4-[(3-Methylphenyl)amino]-6-[(cis-4-hydroxy-4-methylcyclohexyl)amino]-pyrimido[5,4-d]pyrimidin
Hergestellt aus den Verbindungen der Beispiele IX und 2.
Schmelzpunkt: 194-196°C
$R_f$-Wert: 0,36 (Kieselgel; Petrolether/Essigester/Methanol = 10:8:2)

(118) 4-[(3-Methylphenyl)amino]-6-[(trans-4-hydroxy-4-methylcyclohexyl)amino]-pyrimido[5,4-d]pyrimidin
Hergestellt aus den Verbindungen der Beispiele IX(1) und 2.
Schmelzpunkt: 217-221°C
$R_f$-Wert: 0,36 (Kieselgel; Petrolether/Essigester/Methanol = 10:8:2)

| | | | |
|---|---|---|---|
| Ber. | C 65,91 | H 6,64 | N 23,06 |
| Gef. | 65,80 | 6,70 | 23,26 |

(119) 4-[(3-Methylphenyl)amino]-6-(N-isopropyl-N-methyl-amino)-pyrimido [5,4-d]pyrimidin
Schmelzpunkt: 91-96°C
$R_f$-Wert: 0,55 (Kieselgel; Petrolether/Essigester/Methanol = 10:8:2)

| | | | |
|---|---|---|---|
| Ber. | C 66,21 | H 6,54 | N 27,25 |
| Gef. | 66,33 | 6,79 | 26,99 |

(120) 4-[(3-Methylphenyl)amino]-6-(N-tert.butyl-N-methylamino)-pyrimido[5,4-d]pyrimidin
Schmelzpunkt: 136-139°C
$R_f$-Wert: 0,89 (Kieselgel; Petrolether/Essigester/Methanol = 10:8:2)

(121) 4-[(3-Methylphenyl)amino]-6-(N-(trans-4-hydroxycyclohexyl)-N-methyl-amino)-pyrimido[5,4-d]pyrimidin
Schmelzpunkt: 220-222°C
$R_f$-Wert: 0,40 (Kieselgel; Petrolether/Essigester/Methanol = 10:8:2)

(122) 4-[(3-Methylphenyl)amino]-6-(1-hydroxy-2-methyl-2-propylamino)-pyrimido[5,4-d]pyrimidin
Schmelzpunkt: 207-210°C
$R_f$-Wert: 0,47 (Kieselgel; Essigester/Methanol - 20:1)

| | | | |
|---|---|---|---|
| Ber. | C 62,95 | H 6,21 | N 25,91 |
| Gef. | 63,16 | 6,38 | 25,41 |

(123) 4-[(3-Methylphenyl)amino]-6-(cis-2,6-dimethyl-morpholino)-pyrimido[5,4-d]pyrimidin
Schmelzpunkt: 134-139°C
$R_f$-Wert: 0,70 (Kieselgel; Petrolether/Essigester/Methanol = 10:8:2)

| | | | |
|---|---|---|---|
| Ber. | C 65,12 | H 6,33 | N 23,98 |
| Gef. | 65,05 | 6,41 | 24,06 |

(124) 4-[(3-Methylphenyl)amino]-6-(trans-2,6-dimethyl-morpholino)-pyrimido [5,4-d]pyrimidin

(125) 4-[(3-Methylphenyl)amino]-6-(3-methyl-morpholino)-pyrimido[5,4-d]pyrimidin

(126) 4-[(3-methylphenyl)amino]-6-(3,3-dimethyl-morpholino)-pyrimido[5,4-d]pyrimidin

(127) 4-[(3-Methylphenyl)amino]-6-(3,5-dimethyl-morpholino)-pyrimido[5,4-d]pyrimidin

(128) 4-[(3-Methylphenyl)amino]-6-(2-methyl-1-piperidinyl)-pyrimido [5,4-d]pyrimidin
Schmelzpunkt: 131-134°C
$R_f$-Wert: 0,66 (Kieselgel; Petrolether/Essigester/Methanol = 10:5:1)

| | | | |
|---|---|---|---|
| Ber. | C 68,24 | H 6,63 | N 25,13 |
| Gef. | 68,25 | 6,72 | 24,68 |

(129) 4-[(3-Chlor-4-fluor-phenyl)amino]-6-[2-(morpholino)ethylamino]-pyrimido [5,4-d]pyrimidin
Schmelzpunkt: 148-150°C
$R_f$-Wert: 0,48 (Kieselgel; Petrolether/Essigester/Methanol = 10:10:3)

| | | | |
|---|---|---|---|
| Ber. | C 53,53 | H 4,74 | N 24,27 |
| Gef. | 53,43 | 4,83 | 24,00 |

(130) 4-[(3-Methylphenyl)amino]-6-(2-hydroxy-cyclohexylamino)-pyrimido[5,4-d]pyrimidin
Schmelzpunkt: 218-220°C
$R_f$-Wert: 0,36 (Kieselgel; Petrolether/Essigester/Methanol = 10:10:2)

(131) 4-[(3-Methylphenyl)amino]-6-(2-methyl-1-pyrrolidinyl)-pyrimido[5,4-d]pyrimidin

(132) 4-[(3-Methylphenyl)amino]-6-(2,2-dimethyl-1-pyrrolidinyl)-pyrimido[5,4-d]pyrimidin

(133) 4-[(3-Methylphenyl)amino]-6-(2,5-dimethyl-1-pyrrolidinyl)-pyrimido [5,4-d]pyrimidin
Schmelzpunkt: 123 - 125°C
$R_f$-Wert: 0,55 (Kieselgel; Petrolether/Essigester = 10:4)

(134) 4-[(3-Methylphenyl)amino]-6-[trans-4-carboxy-cyclohexylamino]-pyrimido [5,4-d]pyrimidin
Hergestellt aus der Verbindung 135 des Beispiels 1 durch Umsetzung mit methanolischer Natronlauge.
Schmelzpunkt: >325°C
Massenspektrum: $M^+$ = 378

(135) 4-[(3-Methylphenyl)amino]-6-[trans-4-(methoxycarbonyl)cyclohexylamino]-pyrimido[5,4-d]pyrimidin
Schmelzpunkt: 170-174°C
$R_f$-Wert: 0,31 (Kieselgel; Petrolether/Essigester = 3:5)

(136) 4-[(3-Methylphenyl)amino]-6-[trans-4-aminocarbonyl-cyclohexylamino]-pyrimido [5,4-d]pyrimidin
Hergestellt aus der Verbindung 134 des Beispiels 1 durch Umsetzung mit Thionylchlorid und Ammoniak.
Schmelzpunkt: 312 - 315°C
$R_f$-Wert: 0,38 (Kieselgel; Petrolether/Essigester/Methanol = 10:8:3)

(137) 4-[(3-Methylphenyl)amino]-6-[trans-4-(N-methylaminocarbonyl)cyclohexylamino]-pyrimido[5,4-d]pyrimidin
Hergestellt aus der Verbindung 134 des Beispiels 1 durch Umsetzung mit Thionylchlorid und Methylamin.
Schmelzpunkt: 298 - 304°C
$R_f$-Wert: 0,43 (Kieselgel; petrolether/Essigester/Methanol = 10:8:3)

(138) 4-[(3-Methylphenyl)amino]-6-[trans-4-(N,N-dimethylaminocarbonyl)cyclohexylamino]-pyrimido [5,4-d]pyrimidin
Hergestellt aus der Verbindung 135 des Beispiels 1 durch Umsetzung mit O-(Benzotriazol-1-yl)-N,N,N',N'-tetramethyluroniumtetrafluoroborat, Triethylamin und Dimethylamin.
Schmelzpunkt: 214-217°C
$R_f$-Wert: 0,40 (Kieselgel; Petrolether/Essigester/Methanol = 10:8:3)

Massenspektrum: $M^+$ = 405

(139) 4-[(3-Methylphenyl)amino]-6-[trans-4-(pyrrolidinocarbonyl)cyclohexylamino]-pyrimido[5,4-d]pyrimidin
Hergestellt aus der Verbindung 135 des Beispiels 1 durch Umsetzung mit O-(Benzotriazol-1-yl)-N,N,N',N'-tetramethyluroniumtetrafluoroborat, Triethylamin und Pyrrolidin.
Schmelzpunkt: 210-214°C
$R_f$-Wert: 0,47 (Kieselgel; Petrolether/Essigester/Methanol = 10:8:3)
Massenspektrum: $M^+$ = 431

(140) 4-[(3-Methylphenyl)amino]-6-[trans-4-(morpholinocarbonyl)cyclohexylamino]-pyrimido[5,4-d]pyrimidin
Hergestellt aus der Verbindung 135 des Beispiels 1 durch Umsetzung mit N,N'-carbonyldiimidazol und Morpholin.
Schmelzpunkt: 150-160°C
$R_f$-Wert: 0,48 (Kieselgel; Petrolether/Essigester/Methanol = 10:8:3)
Massenspektrum: $M^+$ = 447

(141) 4-[(3-Methylphenyl)amino]-6-[(trans-4-(hydroxymethyl)cyclohexylamino]-pyrimido[5,4-d]pyrimidin
Hergestellt aus den Verbindungen der Beispiele 2 und X.
Schmelzpunkt: 264-267°C
$R_f$-Wert: 0,41 (Kieselgel; Petrolether/Essigester/Methanol = 10:8:2)
Massenspektrum: $M^+$ = 364

(142) 4-[(3-Methylphenyl)amino]-6-(4-piperidinyl-amino)-pyrimido[5,4-d]pyrimidin
Hergestellt aus der Verbindung 194 des Beispiels 1 durch Umsetzung mit Palladiumhydroxid und Wasserstoff zur Benzylgruppenabspaltung. Durch anschließende Behandlung mit Palladium auf Kohle in siedendem Dioxan/Wasser-Gemisch wird der teilweise hydrierte Pyrimidopyrimidin-Grundkörper zurückgebildet.
Schmelzpunkt: 187-192°C
$R_f$-Wert: 0,56 (Aluminiumoxid; Methylenchlorid/Methanol = 20:1,6)
Massenspektrum: $M^+$ = 335

(143) 4-[(3-Methylphenyl)amino]-6-[(1-formyl-4-piperidinyl)amino]-pyrimido[5,4-d]pyrimidin

(144) 4-[(3-Methylphenyl)amino]-6-[(1-acetyl-4-piperidinyl)amino]-pyrimido[5,4-d]pyrimidin
Hergestellt aus der Verbindung 142 des Beispiels 1 durch Umsetzung mit Acetanhydrid und Triethylamin.
Schmelzpunkt: 174 - 177°C
$R_f$-Wert: 0,48 (Kieselgel; Methylenchlorid/Methanol = 10:1)

(145) 4-[(3-Methylphenyl)amino]-6-[(1-methylsulfonyl-4-piperidinyl)amino]-pyrimido[5,4-d]pyrimidin
Hergestellt aus der Verbindung 142 des Beispiels 1 durch Umsetzung mit Methylsulfonylchlorid und Triethylamin.
Schmelzpunkt: 229 - 233°C
$R_f$-Wert: 0,59 (Kieselgel; Methylenchlorid/Methanol = 10:1)

(146) 4-[(3-Methylphenyl)amino]-6-[(1-methoxycarbonyl-4-piperidinyl)amino]-pyrimido[5,4-d]pyrimidin
Hergestellt aus der Verbindung 142 des Beispiels 1 durch Umsetzung mit Chlorameisensäuremethylester und Triethylamin.
Schmelzpunkt: 141 - 146°C
$R_f$-Wert: 0,45 (Kieselgel; Methylenchlorid/Methanol = 20:1)

(147) 4-[(3-Methylphenyl)amino]-6-[(1-cyano-4-piperidinyl)amino]-pyrimido[5,4-d]pyrimidin

(148) 4-[(3-Methylphenyl)amino]-6-[(1-aminocarbonyl-4-piperidinyl)amino]-pyrimido[5,4-d]pyrimidin

(149) 4-[(3-Methylphenyl)amino]-6-[(1-(N-methylamino)carbonyl-4-piperidinyl)amino]-pyrimido [5,4 -d]pyrimidin

(150) 4-[(3-Methylphenyl)amino]-6-[(1-(N,N-dimethylamino)carbonyl-4-piperidinyl)amino]-pyrimido[5,4-d]pyrimidin

(151) 4-[(3-Methylphenyl)amino]-6-(4-methoxycarbonyl-1-piperidinyl)-pyrimido [5,4-d]pyrimidin
Hergestellt aus der Verbindung 46 des Beispiels 1 durch Umsetzung mit Thionylchlorid und Methanol.

Schmelzpunkt: 114-118°C
$R_f$-Wert: 0,50 (Kieselgel; Petrolether/Essigester/Methanol = 10:5:1)
Massenspektrum: $M^+$ = 378

(152) 4-[(3-Methylphenyl) amino]-6-[4-(N-methylamino)carbonyl-1-piperidinyl]-pyrimido[5,4-d]pyrimidin
Hergestellt aus der Verbindung 46 des Beispiels 1 durch Umsetzung mit O-(Benzotriazol-1-yl)-N,N,N',N'-tetramethyluroniumtetrafluoroborat, Triethylamin und Methylamin.
Schmelzpunkt: 226-230°C
$R_f$-Wert: 0,43 (Kieselgel; Petrolether/Essigester/Methanol = 10:8:3)
Massenspektrum: $M^+$ = 377

(153) 4-[(3-Methylphenyl)amino]-6-[4-(N,N-dimethylamino)carbonyl-1-piperidinyl]-pyrimido[5,4-d]pyrimidin
Hergestellt aus der Verbindung 46 des Beispiels 1 durch Umsetzung mit O-(Benzotriazol-1-yl)-N,N,N',N'-tetramethyluroniumtetrafluoroborat, Triethylamin und Dimethylamin.
Schmelzpunkt: 174-177°C
$R_f$-Wert: 0,53 (Kieselgel; Petrolether/Essigester/Methanol = 10:8:4)

(154) 4-[(3-Methylphenyl)amino]-6-[4-(pyrrolidino)carbonyl-1-piperidinyl]-pyrimido[5,4-d]pyrimidin
Hergestellt aus der Verbindung 46 des Beispiels 1 durch Umsetzung mit O-(Benzotriazol-1-yl)-N,N, N',N'-tetramethyluroniumtetrafluoroborat, Triethylamin und Pyrrolidin.
Schmelzpunkt: 181-184°C
$R_f$-Wert: 0,46 (Kieselgel; Petrolether/Essigester/Methanol = 10:8:3)
Massenspektrum: $M^+$ = 417

(155) 4-[(3-Methylphenyl)amino]-6-[4-hydroxymethyl-1-piperidinyl]-pyrimido[5,4-d]pyrimidin
Schmelzpunkt: 170-175°C
$R_f$-Wert: 0,55 (Kieselgel; Petrolether/Essigester/Methanol = 10:7:1,5)

| | | | |
|---|---|---|---|
| Ber. | C 65,12 | H 6,33 | N 23,98 |
| Gef. | 65,07 | 6,52 | 23,80 |

(156) 4-[(3-Methylphenyl)amino]-6-[3-hydroxymethyl-1-piperidinyl]-pyrimido[5,4-d]pyrimidin
Schmelzpunkt: 141-145°C
$R_f$-Wert: 0,50 (Kieselgel; Petrolether/Essigester/Methanol = 10:8:2)

| | | | |
|---|---|---|---|
| Ber. | C 65,12 | H 6,33 | N 23,98 |
| Gef. | 64,96 | 6,47 | 23,88 |

(157) 4-[(3-Methylphenyl)amino]-6-[2-hydroxymethyl-1-piperidinyl]-pyrimido[5,4-d]pyrimidin
Schmelzpunkt: 164-168°C
$R_f$-Wert: 0,40 (Kieselgel; Petrolether/Essigester = 1:1)

| | | | |
|---|---|---|---|
| Ber. | C 65,12 | H 6,33 | N 23,98 |
| Gef. | 64,94 | 6,22 | 24,00 |

(158) 4-[(3-Methylphenyl)amino]-6-(n-propylamino)-pyrimido[5,4-d]pyrimidin
Schmelzpunkt: 145-149°C
$R_f$-Wert: 0,76 (Kieselgel; Petrolether/Essigester/Methanol = 10:8:2)

| | | | |
|---|---|---|---|
| Ber. | C 65,29 | H 6,16 | N 28,55 |
| Gef. | 64,40 | 6,33 | 28,13 |

(159) 4-[(3-Methylphenyl)amino]-6-(n-butylamin-pyrimido[5,4-d]pyrimidin
Schmelzpunkt: 136-140°C
$R_f$-Wert: 0,53 (Kieselgel; Petrolether/Essigester/Methanol = 10:8:2)
Massenspektrum: $M^+$ = 308

| | | | |
|---|---|---|---|
| Ber. | C 66,21 | H 6,54 | N 27,25 |
| Gef. | 65,96 | 6,65 | 27,05 |

(160) 4-[(3-Methylphenyl)amino]-6-(3-phenyl-n-propylamino)-pyrimido[5,4-d]pyrimidin
Schmelzpunkt: 118-122°C
$R_f$-Wert: 0,66 (Kieselgel; Petrolether/Essigester/Methanol = 10:8:2)
Massenspektrum: $M^+$ = 370

| | | | |
|---|---|---|---|
| Ber. | C 71,33 | H 5,99 | N 22,68 |
| Gef. | 71,48 | 6,06 | 22,69 |

(161) 4-[(3-Methylphenyl)amino]-6-[4-hydroxy-4-phenyl-1-piperidinyl]-pyrimido[5,4-d]pyrimidin
Schmelzpunkt: 185-188°C
$R_f$-Wert: 0,50 (Kieselgel; Petrolether/Essigester/Methanol = 10:5:1)

(162) 4-[(3-Methylphenyl)amino]-6-[(1-carboxy-1-methyl)ethylamino]-pyrimido[5,4-d]pyrimidin

(163) 4-[(3-Methylphenyl)amino]-6-[(1-methoxycarbonyl-1-methyl)ethylamino]-pyrimido[5,4-d]pyrimidin

(164) 4-[(3-Methylphenyl)amino]-6-[(1-aminocarbonyl-1-methyl)ethylamino]-pyrimido[5,4-d]pyrimidin

(165) 4-[(3-Methylphenyl)amino]-6-[(1-(N-methylamino)carbonyl-1-methyl)ethylamino]-pyrimido[5,4-d]pyrimidin
Schmelzpunkt: 214-216°C
$R_f$-Wert: 0,41 (Kieselgel; Petrolether/Essigester/Methanol = 10:10:3)
Massenspektrum: $M^+$ = 351

(166) 4-[(3-Methylphenyl)amino]-6-[(1-(N,N-dimethylamino)carbonyl-1-methyl)ethylamino]-pyrimido[5,4-d]pyrimidin

(167) 4-[(3-Methylphenyl)amino]-6-[(1-(1-pyrrolidino)carbonyl 1-methyl)ethylamino]-pyrimido[5,4-d]pyrimidin

(168) 4-[(3-Methylphenyl)amino]-6-[(1-(morpholino)carbonyl-1-methyl)ethylamino]-pyrimido [5,4-d]pyrimidin

(169) 4-[(3-Methylphenyl)amino]-6-(3-tetrahydrofuranylamino)-pyrimido[5,4-d]pyrimidin

(170) 4-[(3-Methylphenyl)amino]-6-(4-tetrahydropyranylamino)-pyrimido[5,4-d]pyrimidin
Schmelzpunkt: 226-228°C
$R_f$-Wert: 0,46 (Kieselgel; Petrolether/Essigester/Methanol = 10:10:1)

| | | | |
|---|---|---|---|
| Ber. | C 64,26 | H 5,99 | N 24,98 |
| Gef. | 64,03 | 5,99 | 24,28 |

(171) 4-[(3-Methylphenyl)amino]-6-(tetrahydrofurfurylamino)-pyrimido[5,4-d]pyrimidin

(172) 4-[(3-Methylphenyl)amino]-6-[(1-(2-hydroxyethyl)aminocarbonyl-1-methyl)ethylamino]-pyrimido[5,4-d]pyrimidin

(173) 4-[(3-Methylphenyl)amino]-6-[(1-desoxy-1-D-sorbityl)amino]-pyrimido[5,4-d]pyrimidin
Schmelzpunkt: 179-182°C
$R_f$-Wert: 0,45 (Kieselgel; Petrolether/Essigester = 1:2)

| | | | |
|---|---|---|---|
| Ber. | C 54,79 | H 5,81 | N 20,18 |
| Gef. | 54,69 | 5,84 | 20,38 |

(174) 4-[(3-Methylphenyl)amino]-6-(p-hydroxyphenyl-amino)-pyrimido[5,4-d]pyrimidin

(175) 4-[(3-Methylphenyl)amino]-6-[(trans-4-(N-oxido-N,N-dimethylamino)-cyclohexyl)-amino]-pyrimido[5,4-d]pyrimidin Schmelzpunkt: 182-184°C
$R_f$-Wert: 0,55 (Kieselgel; Petrolether/Essigester/Methanol = 10:10:4)
Hergestellt aus der Verbindung 116 des Beispiels 1 durch Umsetzung mit Wasserstoffperoxid.

(176) 4-[(3-Methylphenyl)amino]-6-[(1-methyl-N-oxido-4-piperidinyl)-amino]-pyrimido[5,4-d]pyrimidin
Schmelzpunkt: 212-214°C
$R_f$-Wert: 0,53 (Aluminiumoxid; Petrolether/Essigester/Methanol = 2:8:3)
Hergestellt aus der Verbindung 62 des Beispiels 1 durch Umsetzung mit Wasserstoffperoxid.

(177) 4-[(3-Methylphenyl)amino]-6-(4-oxo-1-piperidinyl)-pyrimido[5,4-d]pyrimidin
Hergestellt aus der Verbindung 31 des Beispiels 1 durch Umsetzung mit Pyridiniumdichromat.
Schmelzpunkt: 122-124°C
$R_f$-Wert: 0,40 (Aluminiumoxid; Petrolether/Essigester = 1:1)

(178) 4-[(3-Methylphenyl)amino]-6-[2-(2-hydroxyethyloxy)ethylamino]-pyrimido[5,4-d]pyrimidin
Schmelzpunkt: 99-101°C

| | | | |
|---|---|---|---|
| Ber. | C 59,98 | H 5,92 | N 24,68 |
| Gef. | 59,75 | 6,01 | 24,56 |

(179) 4-[(3-Methylphenyl)amino]-6-(2,3-dihydroxypropylamino)-pyrimido[5,4-d]pyrimidin
Schmelzpunkt: 187-189°C
$R_f$-Wert: 0,39 (Kieselgel; Petrolether/Essigester/Methanol = 10:10:2)

| | | | |
|---|---|---|---|
| Ber. | C 58,88 | H 5,56 | N 25,75 |
| Gef. | 58,85 | 5,60 | 25,50 |

(180) 4-[(3-Methylphenyl)amino]-6-[4-(2-oxo-1-pyrrolidinyl)phenylamino]-pyrimido[5,4-d]pyrimidin
Schmelzpunkt: 212-214°C
$R_f$-Wert: 0,42 (Kieselgel; Petrolether/Essigester/Methanol = 10:10:2)
Massenspektrum: $M^+$ = 303

(181) 4-(Phenylamino)-6-(phenylamino)-pyrimido[5,4-d]pyrimidin

(182) 4-[N-Methyl-N-phenyl-amino]-6-[N-methyl-N-phenyl-amino]-pyrimido[5,4-d]pyrimidin

(183) 4-[(4-Chlor-3-fluor-phenyl)amino]-6-(1,3-dihydroxy-2-methyl-2-propylamino)-pyrimido [5,4-d]pyrimidin
Schmelzpunkt: > 230°C
$R_f$-Wert: 0,30 (Kieselgel; Petrolether/Essigester/Methanol = 10:10:3)
Massenspektrum: $M^+$ = 378/380

(184) 4-[(3-Methylphenyl)amino]-6-[4-(morpholino)carbonyl-1-piperidinyl]-pyrimido[5,4-d]pyrimidin
Hergestellt aus der Verbindung 46 des Beispiels 1 durch Umsetzung mit N,N'-Carbonyldiimidazol und Morpholin.
Schmelzpunkt: 208-212°C
$R_f$-Wert: 0,58 (Kieselgel; Petrolether/Essigester/Methanol = 10:8:4)
Massenspektrum: $M^+$ = 433

(185) 4-[(3-Methylphenyl)amino]-6-(tris-hydroxymethyl-methylamino)-pyrimido [5,4-d]pyrimidin
Schmelzpunkt: 222-224°C
$R_f$-Wert: 0,45 (Kieselgel; Petrolether/Essigester/Methanol = 10:5:2)

| | | | |
|---|---|---|---|
| Ber. | C 57,29 | H 5,65 | N 23,58 |
| Gef. | 57,12 | 5,69 | 23,70 |

(186) 4-[(3-Methylphenyl)amino]-6-(4-acetylamino-1-piperidinyl)-pyrimido [5,4-d]pyrimidin

Hergestellt aus der Verbindung 61 des Beispiels 1 durch Umsetzung mit Triethylamin und Acetanhydrid.
Schmelzpunkt: 225-230°C
$R_f$-Wert: 0,50 (Kieselgel; Methylenchlorid/Methanol = 10:1)

| | | | |
|---|---|---|---|
| Ber. | C 63,64 | H 6,14 | N 26,00 |
| Gef. | 63,52 | 6,18 | 25,62 |

(187) 4-[(3-Methylphenyl)amino]-6-(4-methylsulfonylamino-1-piperidinyl)-pyrimido[5,4-d]pyrimidin
Hergestellt aus der Verbindung 61 des Beispiels 1 durch Umsetzung mit Triethylamin und Methansulfonsäurechlorid.
Schmelzpunkt: 182-187°C
$R_f$-Wert: 0,60 (Kieselgel; Methylenchlorid/Methanol = 10:1)

(188) 4-[(3-Methylphenyl)amino]-6-(4-methoxycarbonylamino-1-piperidinyl)-pyrimido[5,4-d]pyrimidin
Hergestellt aus der Verbindung 61 des Beispiels 1 durch Umsetzung mit Triethylamin und Chlorameisensäuremethylester.
Schmelzpunkt: 178-180°C
$R_f$-Wert: 0,40 (Kieselgel; Methylenchlorid/Methanol = 10:0,75)

(189) 4-[(3-Methylphenyl)amino]-6-[4-(N,N-dimethylaminocarbonyl)amino-1-piperidinyl]-pyrimido [5,4-d]pyrimidin
Hergestellt aus der Verbindung 61 des Beispiels 1 durch Umsetzung mit Triethylamin und N,N-Dimethylcarbamidsäurechlorid.
Schmelzpunkt: 220-227°C
$R_f$-Wert: 0,53 (Kieselgel; Methylenchlorid/Methanol = 10:1)

(190) 4-[(3-Methylphenyl)amino]-6-(4-cyanamino-1-piperidinyl)-pyrimido[5,4-d]pyrimidin
Hergestellt aus der Verbindung 61 des Beispiels 1 durch Umsetzung mit Bromcyan.
Schmelzpunkt: 220-224°C
$R_f$-Wert: 0,45 (Kieselgel; Petrolether/Essigester/Methanol = 10:8:3)
Massenspektrum: $M^+$ = 360

(191) 4-[(3-Methylphenyl)amino]-6-(4-formylamino-1-piperidinyl)-pyrimido[5,4-d]pyrimidin
Hergestellt aus der Verbindung 61 des Beispiels 1 durch Umsetzung mit Ameisensäuremethylester.
Schmelzpunkt: 196-198°C
$R_f$-Wert: 0,58 (Kieselgel; Methylenchlorid/Methanol = 10:1,5)

(192) 4-[(3-Methylphenyl)amino]-6-(4-isopropylaminocarbonylmethyl-1-piperazinyl)-pyrimido[5,4-d]pyrimidin
Schmelzpunkt: 146-151°C
$R_f$-Wert: 0,42 (Kieselgel; Petrolether/Essigester/Methanol = 10:8:2)
Massenspektrum: $M^+$ = 420

(193) 4-[(3-Methylphenyl)amino]-6-[4-(2-hydroxymethyl-1-pyrrolidinyl)phenylamino]-pyrimido[5,4-d]pyrimidin
Hergestellt aus den Verbindungen der Beispiele 2 und XX.
Schmelzpunkt: 208-210°C
$R_f$-Wert: 0,50 (Kieselgel; Petrolether/Essigester/Methanol = 10:8:2)

(194) 4-[(3-Methylphenyl)amino]-6-[(1-benzyl-4-piperidinyl)amino]-pyrimido[5,4-d]pyrimidin
Schmelzpunkt: 143-145°C
$R_f$-Wert: 0,48 (Kieselgel; Petrolether/Essigester/Methanol = 10:8:2)
Massenspektrum: $M^+$ = 425

(195) 4-[(3-Methylphenyl)amino]-6-[(1-ethoxycarbonyl-4-piperidinyl)amino]-pyrimido[5,4-d]pyrimidin
Schmelzpunkt: 160-163°C
$R_f$-Wert: 0,46 (Kieselgel; Petrolether/Essigester/Methanol = 10:9:2)

| | | | |
|---|---|---|---|
| Ber. | C 61,90 | H 6,18 | N 24,06 |
| Gef. | 61,73 | 6,23 | 24,08 |

(196) 4-[(3-Methylphenyl)amino]-6-[3-(2-oxo-1-pyrrolidinyl)propylamino]-pyrimido[5,4-d]pyrimidin
Schmelzpunkt: 118-120°C
$R_f$-Wert: 0,25 (Aluminiumoxid; Petrolether/Essigester/Methanol = 10:10:2)

(197) 4-[(3-Methylphenyl)amino]-6-[2-(1,4,7,10,13-pentaoxacyclopentadecyl)-ethylamino]-pyrimido[5,4-d]pyrimidin
Schmelzpunkt: 145-147°C
$R_f$-Wert: 0,37 (Aluminiumoxid; Petrolether/Essigester/Methanol = 10:10:1)

| | | | |
|---|---|---|---|
| Ber. | C 59,48 | H 6,65 | N 17,34 |
| Gef. | 59,56 | 6,69 | 17,28 |

(198) 4-[(3-Methylphenyl)amino]-6-[2-(1,4,7,10,13,16-hexaoxacyclooctadecyl)-ethylamino]-pyrimido [5,4-d]pyrimidin
Schmelzpunkt: 72-74°C
$R_f$-Wert: 0,29 (Aluminiumoxid; Petrolether/Essigester/Methanol = 10:10:1)

(199) 4-[(2-Cyclopropylphenyl)amino]-6-morpholino-pyrimido[5,4-d]pyrimidin
Schmelzpunkt: 171-173°C

| | | | |
|---|---|---|---|
| Ber. | C 65,49 | H 5,78 | N 24,12 |
| Gef. | 65,24 | 5,84 | 24,06 |

(200) 4-[(3-Methylphenyl)amino]-6-[4-(2-hydroxyethyl)-1-piperazinyl]-pyrimido [5,4-d]pyrimidin
Schmelzpunkt: 140-142°C
$R_f$-Wert: 0,45 (Aluminiumoxid; Petrolether/Essigester/Methanol = 10:10:1)

(201) 4-[(4-Amino-3-cyano-phenyl)amino]-6-[(trans-4-hydroxycyclohexyl)-amino]-pyrimido[5,4-d]pyrimidin
Schmelzpunkt: 200°C
$R_f$-Wert: 0,44 (Kieselgel; Petrolether/Essigester/Methanol = 10:10:3)

(202) 4-[(3,4-Dichlorphenyl)amino]-6-[(trans-4-hydroxycyclohexyl)-amino]-pyrimido[5,4-d]pyrimidin
Schmelzpunkt: 220-222°C
$R_f$-Wert: 0,57 (Aluminiumoxid; Petrolether/Essigester/Methanol = 10:10:1)

(203) 4-[(3-Chlor-4-methoxy-phenyl)amino]-6-[(trans-4-hydroxycyclohexyl)-amino]-pyrimido[5,4-d]pyrimidin
Schmelzpunkt: 197-199°C
$R_f$-Wert: 0,40 (Aluminiumoxid; Petrolether/Essigester/Methanol = 10:10:2)

| | | | |
|---|---|---|---|
| Ber. | C 56,92 | H 5,28 | N 20,96 |
| Gef. | 56,71 | 5,29 | 20,54 |

(204) 4-[(3-Chlor-4-fluor-phenyl)amino]-6-[(trans-4-hydroxycyclohexyl)-amino]-pyrimido[5,4-d]pyrimidin
Schmelzpunkt: 222-224°C

| | | | |
|---|---|---|---|
| Ber. | C 55,60 | H 4,66 | N 21,61 |
| Gef. | 55,40 | 4,75 | 21,35 |

(205) 4-[(4-Amino-3-nitro-phenyl)amino]-6-[(trans-4-hydroxycyclohexyl)-amino] -pyrimido [5,4-d]pyrimidin
Schmelzpunkt: 250-252°C
$R_f$-Wert: 0,32 (Kieselgel; Petrolether/Essigester/Methanol = 10:10:2)

(206) 4-[(4-Chlor-3-nitro-phenyl)amino]-6-[(trans-4-hydroxycyclohexyl)-amino]-pyrimido [5,4-d]pyrimidin
Schmelzpunkt: 235-237°C
$R_f$-Wert: 0,28 (Kieselgel; Petrolether/Essigester/Methanol = 10:10:3)

| | | | |
|---|---|---|---|
| Ber. | C 51,99 | H 4,36 | N 23,57 |
| Gef. | 51,70 | 4,45 | 23,78 |

(207) 4-[(4-Amino-3,5-dichlor-phenyl)amino]-6-[(trans-4-hydroxycyclohexyl)-amino]-pyrimido [5,4-d]pyrimidin
Schmelzpunkt: 250-252°C
$R_f$-Wert: 0,23 (Kieselgel; Petrolether/Essigester/Methanol = 10:10:3)

| | | | |
|---|---|---|---|
| Ber. | C 51,43 | H 4,55 | N 23,32 |
| Gef. | 51,55 | 4,70 | 23,35 |

(208) 4-[(3-Methylphenyl)amino]-6-butyloxy-pyrimido[5,4-d]pyrimidin
Schmelzpunkt: 54-56°C
$R_f$-Wert: 0,67 (Kieselgel; Petrolether/Essigester = 10:7)
Massenspektrum: $M^+$ = 309

(209) 4-[(3-Methylphenyl)amino]-6-(1,3-dihydroxy-2-propylamino)-pyrimido [5,4-d]pyrimidin
Schmelzpunkt: 170-172°C
$R_f$-Wert: 0,27 (Kieselgel; Petrolether/Essigester/Methanol = 10:10:3)

| | | | |
|---|---|---|---|
| Ber. | C 58,88 | H 5,55 | N 25,75 |
| Gef. | 58,65 | 5,57 | 25,80 |

(210) 4-[(3-Chlor-4-fluor-phenyl)amino]-6-[(2-hydroxy-1-phenyl)-1-ethylamino]-pyrimido[5,4-d]pyrimidin

(211) 4-[(3-Chlor-4-fluor-phenyl)amino]-6-[(2-hydroxy-2-phenyl)-1-ethylamino]-pyrimido[5,4-d]pyrimidin

(212) 4-[(3-Chlor-4-fluor-phenyl)amino]-6-[(1-hydroxy-3-phenyl)-2-propylamino]-pyrimido [5,4-d]pyrimidin

(213) 4-[(3-Chlor-4-fluor-phenyl)amino]-6-[(1-hydroxy-1-phenyl)-2-propylamino]-pyrimido [5,4-d]pyrimidin
Schmelzpunkt: 234 - 236°C
$R_f$-Wert: 0,42 (Kieselgel; Petrolether/Essigester/Methanol = 10:10:1)
Massenspektrum: $M^+$ = 424

(214) 4-[(3-Chlor-4-fluor-phenyl)amino]-6-[(1,3-dihydroxy-1-phenyl)-2-propylamino]-pyrimido[5,4-d]pyrimidin
Schmelzpunkt: 176-178°C
$R_f$-Wert: 0,48 (Kieselgel; Petrolether/Essigester/Methanol = 10:10:2)

(215) 4-[(3-Chlor-4-fluor-phenyl)amino]-6-[(1,3-dihydroxy-1-(4-nitrophenyl))-2-propylamino]-pyrimido[5,4-d]pyrimidin

(216) 4-[(3-Chlor-4-fluor-phenyl)amino]-6-[(1-hydroxy-3-methoxy-1-phenyl)-2-propylamino]-pyrimido[5,4-d]pyrimidin

(217) 4-[(3-Chlor-4-fluor-phenyl)amino]-6-[(1-hydroxy-3-methyl)-2-butylamino]-pyrimido [5,4-d]pyrimidin

(218) 4- [(3-Chlor-4-fluor-phenyl)amino]-6-[1-(hydroxymethyl)cyclopentylamino]-pyrimido [5,4-d]pyrimidin

(219) 4-[(3-Chlor-4-fluor-phenyl)amino]-6-[(2-hydroxy-2-(3-hydroxyphenyl))-1-ethylamino]-pyrimido[5,4-d]pyrimidin

(220) 4-[(3-Chlor-4-fluor-phenyl)amino]-6-[(2-hydroxy-2-(4-hydroxyphenyl))-1-ethylamino]-pyrimido[5,4-d]pyrimidin

(221) 4-[(3-chlor-4-fluor-phenyl)amino]-6-[(1-hydroxy-1-(4-hydroxyphenyl))-2-propylamino]-pyrimido[5,4-d]pyrimidin

(222) 4-[(3-chlor-4-fluor-phenyl)amino]-6-[tris-(3-hydroxypropyl)-methylamino]-pyrimido [5,4-d]pyrimidin

(223) 4-[(3-chlor-4-fluor-phenyl)amino]-6-[N-methyl-N-(2-hydroxyethyl)amino]-pyrimido[5,4-d]pyrimidin

(224) 4-[(3-chlor-4-fluor-phenyl)amino]-6-[3-carboxy-1-propylamino]-pyrimido[5,4-d]pyrimidin

(225) 4-[(3-Chlor-4-fluor-phenyl)amino]-6-[3-aminocarbonyl1-propylamino]-pyrimido[5,4-d]pyrimidin

(226) 4-[(3-Chlor-4-fluor-phenyl)amino]-6-[3-(N-methylaminocarbonyl)-1-propylamino]-pyrimido[5,4-d]pyrimidin

(227) 4-[(3-chlor-4-fluor-phenyl)amino]-6-[3-(N,N-dimethylaminocarbonyl)-1-propylamino]-pyrimido[5,4-d]pyrimidin

(228) 4-[(3-chlor-4-fluor-phenyl)amino]-6-[3-(N-formylamino)-1-propylamino]-pyrimido[5,4-d]pyrimidin

(229) 4-[(3-chlor-4-fluor-phenyl)amino]-6-[3-(methoxycarbonylamino)-1-propylamino]-pyrimido[5,4-d]pyrimidin

(230) 4-[(3-chlor-4-fluor-phenyl)amino]-6-[2-(N-acetylamino)-1-ethylamino]pyrimido [5,4-d]pyrimidin

(231) 4-[(3-Chlor-4-fluor-phenyl)amino]-6-[3-amino-2-hydroxy-1-propylamino]-pyrimido[5,4-d]pyrimidin

(232) 4-[(3-Chlor-4-fluor-phenyl)amino]-6-[3-(morpholino)-1-propylamino]-pyrimido[5,4-d]pyrimidin

(233) 4-[(3-Chlor-4-fluor-phenyl)amino]-6-[2-(1-piperazinyl)-1-ethylamino]-pyrimido [5,4-d]pyrimidin

(234) 4-[(3-Chlor-4-fluor-phenyl)amino]-6-[2-(1-pyrrolidinyl)-1-ethylamino]-pyrimido [5,4-d]pyrimidin

(235) 4-[(3-Chlor-4-fluor-phenyl)amino]-6-[3-hydroxymethyl-1-piperidinyl]-pyrimido [5,4-d]pyrimidin

(236) 4-[(3-Chlor-4-fluor-phenyl)amino]-6-[(trans-4-(methoxycarbonyl-methyloxy)cyclohexyl)-amino]-pyrimido [5,4-d] pyrimidin Hergestellt aus den Verbindungen der Beispiele 2 und XXVI.
$R_f$-Wert: 0,32 (Kieselgel; Petrolether/Essigester/Methanol = 10:10:1)
Massenspektrum: $M^+$ = 460

(237) 4-[(3-Chlor-4-fluor-phenyl)amino]-6-[(trans-4-(carboxymethyloxy)cyclohexyl)-amino]-pyrimido [5,4-d]pyrimidin Hergestellt aus der Verbindung 236 des Beispiels 1 durch Umsetzung mit Natronlauge in einem Methanol/Tetrahydrofuran-Gemisch.
Schmelzpunkt: 263 - 265°C
$R_f$-Wert: 0,42 (Kieselgel; Petrolether/Essigester/Methanol = 10:10:3)
Massenspektrum: $M^+$ = 446

(238) 4-[(3-Chlor-4-fluor-phenyl)amino]-6-[(trans-4-(N,N-dimethylaminocarbonyl-methyloxy)cyclohexyl)-amino]-pyrimido[5,4-d]pyrimidin

(239) 4-[(3-Chlor-4-fluor-phenyl)amino]-6-[(trans-4-(morpholinocarbonyl-methyloxy)cyclohexyl)-amino]-pyrimido [5,4-d]pyrimidin

(240) 4-[(3-Chlor-4-fluor-phenyl)amino]-6-[(trans-4-(2-methoxycarbonyl-ethyl)cyclohexyl)-amino]-pyrimido[5,4-d] pyrimidin Hergestellt aus den Verbindungen der Beispiele 2 und XXIII. Schmelzpunkt: 144 - 146°C
$R_f$-Wert: 0,62 (Kieselgel; Petrolether/Essigester/Methanol = 10:10:1)

(241) 4-[(3-Chlor-4-fluor-phenyl)amino]-6-[(trans-4-(2-carboxyethyl)cyclohexyl)-amino]-pyrimido[5,4-d]pyrimidin Hergestellt aus der Verbindung 240 des Beispiels 1 durch Umsetzung mit Natronlauge in einem Methanol/Tetrahydrofuran-Gemisch.
Schmelzpunkt: 298 - 300°C
$R_f$-Wert: 0,25 (Kieselgel; Petrolether/Essigester/Methanol = 10:10:2)

(242) 4-[(3-Chlor-4-fluor-phenyl)amino]-6-[(trans-4-(2-N,N-dimethylaminocarbonyl-ethyl)cyclohexyl)-amino]-pyrimido[5,4-d]pyrimidin

(243) 4-[(3-Chlor-4-fluor-phenyl)amino]-6-[(trans-4-(2-morpholinocarbonyl-ethyl)cyclohexyl)-amino]-pyrimido [5,4-d]pyrimidin

(244) 4-[(3-Chlor-4-fluor-phenyl)amino]-6-[(trans-4-(methoxycarbonyl)cyclohexyl-methyl)-amino]-pyrimido[5,4-d] pyrimidin Hergestellt aus den Verbindungen der Beispiele 2 und XXVII.
Schmelzpunkt: 200 - 202°C
$R_f$-Wert: 0,63 (Kieselgel; Petrolether/Essigester/Methanol = 10:10:1)
Massenspektrum: $M^+$ = 444

(245) 4-[(3-Chlor-4-fluor-phenyl)amino]-6-[(trans-4-carboxycyclohexyl-methyl)-amino]-pyrimido [5,4-d]pyrimidin Hergestellt aus der Verbindung 244 des Beispiels 1 durch Umsetzung mit Natronlauge in einem Methanol/Tetrahydrofuran-Gemisch.
Schmelzpunkt: 269 - 271°C
$R_f$-Wert: 0,58 (Kieselgel; Petrolether/Essigester/Methanol = 10:10:3)
Massenspektrum: $M^+$ = 430

(246) 4-[(3-Chlor-4-fluor-phenyl)amino]-6-[(trans-4-(N,N-dimethylamino-carbonyl)cyclohexyl-methyl)-amino]-pyrimido[5,4-d]pyrimidin

(247) 4-[(3-Chlor-4-fluor-phenyl)amino]-6-[(trans-4-(morpholinocarbonyl)cyclohexyl-methyl)-amino]-pyrimido [5,4-d]pyrimidin

(248) 4-[(3-Chlor-4-fluor-phenyl)amino]-6-isopropoxy-pyrimido[5,4-d]pyrimidin
Schmelzpunkt: 125 - 127°C
$R_f$-Wert: 0,42 (Kieselgel; Petrolether/Essigester = 10:5)
Massenspektrum: $M^+$ = 333

(249) 4-[(3-Chlor-4-fluor-phenyl)amino]-6-(4-tetrahydropyranyloxy)-pyrimido[5,4-d]pyrimidin

(250) 4-[(3-Chlor-4-fluor-phenyl)amino]-6-(3-tetrahydrofuranyloxy)-pyrimido [5,4-d]pyrimidin

(251) 4-[(3-Chlor-4-fluor-phenyl)amino]-6-[trans-4-hydroxycyclohexyloxy]-pyrimido[5,4-d]pyrimidin
Schmelzpunkt: 200 - 202°C
$R_f$-Wert: 0,35 (Kieselgel; Petrolether/Essigester/Methanol = 10:10:1)
Massenspektrum: $M^+$ = 362

(252) 4-[(3-Chlor-4-fluor-phenyl)amino]-6-(2-tetrahydropyranylmethyloxy)-pyrimido[5,4-d]pyrimidin

(253) 4-[(3-Chlor-4-fluor-phenyl)amino]-6-[trans-4-(methoxycarbonyl)-cyclohexyloxy]-pyrimido[5,4-d]pyrimidin

(254) 4-[(3-Chlor-4-fluor-phenyl)amino]-6-[trans-4-carboxycyclohexyloxy]-pyrimido [5,4-d]pyrimidin

(255) 4-[(3-Chlor-4-fluor-phenyl)amino]-6-[trans-4-(N,N-dimethylaminocarbonyl)-cyclohexyloxy]-pyrimido[5,4-d] pyrimidin

(256) 4-[(3-Chlor-4-fluor-phenyl)amino]-6-[trans-4-(N-methylaminocarbonyl)-cyclohexyloxy]-pyrimido[5,4-d]pyrimidin

(257) 4-[(3-Chlor-4-fluor-phenyl)amino]-6-[trans-4-(aminocarbonyl)-cyclohexyloxy]-pyrimido[5,4-d]pyrimidin

(258) 4-[(3-Chlor-4-fluor-phenyl)amino]-6-[trans-4-(N,N-dimethylamino)-cyclohexyloxy]-pyrimido[5,4-d]pyrimidin

(259) 4-[(3-Chlor-4-fluor-phenyl)amino]-6-[trans-4-(N-acetylamino)-cyclohexyloxy]-pyrimido[5,4-d]pyrimidin

(260) 4-[(3-Chlor-4-fluor-phenyl)amino]-6-[trans-4-(N-methylsulfonylamino)-cyclohexyloxy]-pyrimido[5,4-d]pyrimidin

(261) 4-[(3-Chlor-4-fluor-phenyl)amino]-6-[trans-4-(N-methoxycarbonylamino)-cyclohexyloxy]-pyrimido[5,4-d]pyrimidin

(262) 4-[(3-Chlor-4-fluor-phenyl)amino]-6-[trans-4-(N-tert.-butyloxycarbonylamino)-cyclohexyloxy]-pyrimido [5,4-d]pyrimidin

(263) 4-[(3-Chlor-4-fluor-phenyl)amino]-6-[1-methyl-4-piperidinyloxy]-pyrimido [5,4-d]pyrimidin
Schmelzpunkt: 129 - 131°C
$R_f$-Wert: 0,48 (Aluminiumoxid; Petrolether/Essigester = 1:1)
Massenspektrum: $M^+$ = 389

(264) 4-[(3-Chlor-4-fluor-phenyl)amino]-6-[1-acetyl-4-piperidinyloxy]-pyrimido [5,4-d]pyrimidin

(265) 4-[(3-Chlor-4-fluor-phenyl)amino]-6-[1-methylsulfonyl-4-piperidinyloxy]-pyrimido [5,4-d]pyrimidin

(266) 4-[(3-Chlor-4-fluor-phenyl)amino]-6-[1-tert.-butyloxycarbonyl-4-piperidinyloxy]-pyrimido[5,4-d]pyrimidin

(267) 4-[(3-Chlor-4-fluor-phenyl)amino]-6-[1-methoxycarbonyl-4-piperidinyloxy]-pyrimido[5,4-d]pyrimidin

(268) 4-[(3-Chlor-4-fluor-phenyl)amino]-6-[2-methoxyethyloxy]pyrimido [5,4-d]pyrimidin

(269) 4-[(3-Chlor-4-fluor-phenyl)amino]-6-[2-hydroxyethyloxy]pyrimido[5,4-d]pyrimidin

(270) 4-[(3-Chlor-4-fluor-phenyl)amino]-6-[1-methoxy-2-propyloxy]-pyrimido[5,4-d]pyrimidin

(271) 4-[(3-Chlor-4-fluor-phenyl)amino]-6-allyloxy-pyrimido[5,4-d]pyrimidin

(272) 4-[(3-Chlor-4-fluor-phenyl)amino]-6-[cyclobutyloxyl-pyrimido[5,4-d]pyrimidin

(273) 4-[(3-Chlor-4-fluor-phenyl)amino]-6-[3-(3-methyl-oxetanyl)-methyloxy]-pyrimido[5,4-d]pyrimidin

(274) 4-[(3-Chlor-4-fluor-phenyl)amino]-6-[2-tetrahydropyranylmethyloxy]-pyrimido [5,4-d]pyrimidin

(275) 4-[(3-Chlor-4-fluor-phenyl)amino]-6-[3-hydroxy-cyclopentyloxy]-pyrimido [5,4-d]pyrimidin

(276) 4-[(3-Chlor-4-fluor-phenyl)amino]-6-[2-hydroxy-cyclohexyloxy]-pyrimido [5,4-d]pyrimidin

(277) 4-[(3-Chlor-4-fluor-phenyl)amino]-6-[3-hydroxy-cyclohexyloxy]-pyrimido [5,4-d]pyrimidin

(278) 4-[(3-Chlor-4-fluor-phenyl)amino]-6-[1-N,N-dimethylamino-2-propyloxy]-pyrimido[5,4-d]pyrimidin

(279) 4-[(3-Chlor-4-fluor-phenyl)amino]-6-[2-(morpholino)ethyloxy]-pyrimido[5,4-d]pyrimidin

(280) 4-[(3-Chlor-4-fluor-phenyl)amino]-6-[1-methyl-3-pyrrolidinyloxy]-pyrimido[5,4-d]pyrimidin

(281) 4-[(3-Chlor-4-fluor-phenyl)amino]-6-[1-methyl-3-piperidinyloxy]-pyrimido[5,4-d]pyrimidin

(282) 4-[(3-Chlor-4-fluor-phenyl)amino]-6-[1-methoxy-2-methyl-2-propylamino]-pyrimido[5,4-d]pyrimidin

(283) 4-[(3-Chlor-4-fluor-phenyl)amino]-6-[3-amino-1-pyrrolidinyl]-pyrimido[5,4-d]pyrimidin

(284) 4-[(3-Chlor-4-fluor-phenyl)amino]-6-[3-N,N-dimethylamino-1-pyrrolidinyl]-pyrimido[5,4-d]pyrimidin

(285) 4-[(3-Chlor-4-fluor-phenyl)amino]-6-[3-acetylamino-1-pyrrolidinyl]-pyrimido[5,4-d]pyrimidin

(286) 4-[(3-Chlor-4-fluor-phenyl)amino]-6-[3-cyanamino-1-pyrrolidinyl]-pyrimido [5,4-d]pyrimidin

(287) 4-[(3-Chlor-4-fluor-phenyl)amino]-6-[3-methylsulfonylamino-1-pyrrolidinyl]-pyrimido [5,4-d]pyrimidin

(288) 4-[(3-Chlor-4-fluor-phenyl)amino]-6-[3-methoxycarbonylamino-1-pyrrolidinyl]-pyrimido [5,4-d]pyrimidin

(289) 4-[(3-Chlor-4-fluor-phenyl)amino]-6-[3-formylamino-1-pyrrolidinyl]-pyrimido [5,4-d]pyrimidin

(290) 4-[(3-Chlor-4-fluor-phenyl)amino]-6-[3-N,N-dimethylaminocarbonylamino-1-pyrrolidinyl]-pyrimido[5,4-d]pyrimidin

(291) 4-[(3-Chlor-4-fluor-phenyl)amino]-6-[3-amino-1-piperidinyl]-pyrimido[5,4-d]pyrimidin

(292) 4-[(3-Chlor-4-fluor-phenyl)amino]-6-[3-N,N-dimethylamino-1-piperidinyl]-pyrimido[5,4-d]pyrimidin

(293) 4-[(3-Chlor-4-fluor-phenyl)amino]-6-[3-acetylamino-1-piperidinyl]-pyrimido [5,4-d]pyrimidin

(294) 4-[(3-Chlor-4-fluor-phenyl)amino]-6-[3-methoxycarbonylamino-1-piperidinyl]-pyrimido [5,4-d]pyrimidin

(295) 4-[(3-Chlor-4-fluor-phenyl)amino]-6-[3-methylsulfonylamino-1-piperidinyl]-pyrimido[5,4-d]pyrimidin

(296) 4-[(3-Chlor-4-fluor-phenyl)amino]-6-[3-pyrrolidinylamino]-pyrimido[5,4-d]pyrimidin

(297) 4-[(3-Chlor-4-fluor-phenyl)amino]-6-[1-methyl-3-pyrrolidinylamino]-pyrimido[5,4-d]pyrimidin

(298) 4-[(3-Chlor-4-fluor-phenyl)amino]-6-[1-acetyl-3-pyrrolidinylamino]-pyrimido [5,4-d]pyrimidin

(299) 4-[(3-Chlor-4-fluor-phenyl)amino]-6-[1-cyano-3-pyrrolidinylamino]-pyrimido[5,4-d]pyrimidin

(300) 4-[(3-Chlor-4-fluor-phenyl)amino]-6-[1-methylsulfonyl-3-pyrrolidinylamino]-pyrimido [5,4-d]pyrimidin

(301) 4-[(3-Chlor-4-fluor-phenyl)amino]-6-[1-methoxycarbonyl-3-pyrrolidinylamino]-pyrimido [5,4-d]pyrimidin

(302) 4-[(3-Chlor-4-fluor-phenyl)amino]-6-[1-formyl-3-pyrrolidinylamino]-pyrimido [5,4-d]pyrimidin

(303) 4-[(3-Chlor-4-fluor-phenyl)amino]-6-[1-(N,N-dimethylaminocarbonyl)-3-pyrrolidinylamino]-pyrimido[5,4-d] pyrimidin

(304) 4-[(3-Chlor-4-fluor-phenyl)amino]-6-[3-piperidinylamino]-pyrimido[5,4-d]pyrimidin

(305) 4-[(3-Chlor-4-fluor-phenyl)amino]-6-[1-methyl-3-piperidinylamino]-pyrimido [5,4-d]pyrimidin

(306) 4-[(3-Chlor-4-fluor-phenyl)amino]-6-[1-acetyl-3-piperidinylamino]-pyrimido [5,4-d]pyrimidin

(307) 4-[(3-Chlor-4-fluor-phenyl)amino]-6-[1-methoxycarbonyl-3-piperidinylamino]-pyrimido[5,4-d]pyrimidin

(308) 4-[(3-Chlor-4-fluor-phenyl)amino]-6-[1-methylsulfonyl-3-piperidinylamino]-pyrimido[5,4-d]pyrimidin

(309) 4-[(3-Chlor-4-fluor-phenyl)amino]-6-[2,5-dimethyl-1-piperazinyl]-pyrimido [5,4-d]pyrimidin

(310) 4-[(3-Chlor-4-fluor-phenyl)amino]-6-[3-methyl-1-piperazinyl]-pyrimido[5,4-d]pyrimidin

(311) 4-[(3-Chlor-4-fluor-phenyl)amino]-6-[4-(2-aminoethyl)-1-piperazinyl]-pyrimido[5,4-d]pyrimidin

(312) 4-[(3-Chlor-4-fluor-phenyl)amino]-6-[4-(2-(2-hydroxyethyloxy)ethyl)-1-piperazinyl]-pyrimido[5,4-d]pyrimidin

(313) 4-[(3-Chlor-4-fluor-phenyl)amino]-6-[2-butyloxy]-pyrimido[5,4-d]pyrimidin

(314) 4-[(3-Chlor-4-fluor-phenyl)amino]-6-[trans-4-methoxycyclohexyloxy]-pyrimido [5,4-d]pyrimidin

(315) 4-[(3-Chlor-4-fluor-phenyl)amino]-6-(1,3-dihydroxy-2-propylamino)-pyrimido [5,4-d]pyrimidin
Schmelzpunkt: 213 - 218°C

$R_f$-Wert: 0,37 (Kieselgel; Petrolether/Essigester = 10:8)

(316) 4-[(3,4-Dichlor-phenyl)amino]-6-(1,3-dihydroxy-2-propylamino)-pyrimido[5,4-d]pyrimidin

(317) 4-[(3-Chlor-phenyl)amino]-6-(1,3-dihydroxy-2-propylamino)-pyrimido[5,4-d]pyrimidin

(318) 4-[(3-Brom-phenyl)amino]-6-(1,3-dihydroxy-2-propylamino)-pyrimido[5,4-d]pyrimidin

(319) 4-[(3-Nitro-phenyl)amino]-6-(1,3-dihydroxy-2-propylamino)-pyrimido [5,4-d]pyrimidin

(320) 4-[(3-Ethinyl-phenyl)amino]-6-(1,3-dihydroxy-2-propylamino)-pyrimido[5,4-d]pyrimidin

(321) 4-[(3,4-Dichlor-phenyl)amino]-6-(1,3-dihydroxy-2-methyl-2-propylamino)-pyrimido[5,4-d]pyrimidin

(322) 4-[(3-Chlor-phenyl)amino]-6-(1,3-dihydroxy-2-methyl-2-propylamino)-pyrimido[5,4-d]pyrimidin

(323) 4-[(3-Brom-phenyl)amino]-6-(1,3-dihydroxy-2-methyl-2-propylamino)-pyrimido [5,4-d]pyrimidin

(324) 4-[(3-Nitro-phenyl)amino]-6-(1,3-dihydroxy-2-methyl-2-propylamino)-pyrimido [5,4-d]pyrimidin

(325) 4-[(3-Ethinyl-phenyl)amino]-6-(1,3-dihydroxy-2-methyl-2-propylamino)-pyrimido [5,4-d]pyrimidin

(326) 4-[(3-Methyl-phenyl)amino]-6-(1,3-dihydroxy-2-methyl-2-propylamino)-pyrimido [5,4-d]pyrimidin

(327) 4-[(3-Chlor-4-fluor-phenyl)amino]-6-(1-hydroxy-2-methyl-2-propylamino)-pyrimido [5,4-d]pyrimidin
Schmelzpunkt: 255 - 259°C
$R_f$-Wert: 0,54 (Kieselgel; Essigester/Methanol = 20:1)

(328) 4-[(3,4-Dichlor-phenyl)amino]-6-(1-hydroxy-2-methyl-2-propylamino)-pyrimido[5,4-d]pyrimidin

(329) 4-[(3-Chlor-phenyl)amino]-6-(1-hydroxy-2-methyl-2-propylamino)-pyrimido [5,4-d]pyrimidin

(330) 4-[(3-Brom-phenyl)amino]-6-(1-hydroxy-2-methyl-2-propylamino)-pyrimido [5,4-d]pyrimidin

(331) 4-[(3-Nitro-phenyl)amino]-6-(1-hydroxy-2-methyl-2-propylamino)-pyrimido[5, 4-d]pyrimidin

(332) 4-[(3-Ethinyl-phenyl)amino]-6-(1-hydroxy-2-methyl-2-propylamino)-pyrimido [5,4-d]pyrimidin

(333) 4-[(3-Nitro-phenyl)amino]-6-[(trans-4-hydroxycyclohexyl)amino]-pyrimido [5, 4-d]pyrimidin

(334) 4-[(3-Ethinyl-phenyl) amino]-6-[(trans-4-hydroxycyclohexyl)-amino]-pyrimido [5,4-d]pyrimidin

(335) 4-[(3-Chlor-4-fluor-phenyl)amino]-6-[(4-aminocyclohexyl)amino]-pyrimido [5,4-d]pyrimidin

(336) 4-[(3,4-Dichlor-phenyl)amino]-6-[(trans-4-aminocyclohexyl)amino]-pyrimido[5,4-d]pyrimidin

(337) 4-[(3-Chlor-phenyl)amino]-6-[(trans-4-aminocyclohexyl)amino]-pyrimido [5,4-d]pyrimidin

(338) 4-[(3-Brom-phenyl)amino]-6-[(trans-4-aminocyclohexyl)amino]-pyrimido[5,4-d]pyrimidin

(339) 4-[(3-Nitro-phenyl)amino]-6-[(trans-4-aminocyclohexyl)amino]-pyrimido[5,4-d]pyrimidin

(340) 4-[(3-Ethinyl-phenyl)amino]-6-[(trans-4-aminocyclohexyl)amino]-pyrimido[5,4-d]pyrimidin

(341) 4-[(3-Chlor-4-fluor-phenyl)amino]-6-[(trans-4-dimethylaminocyclohexyl)amino]-pyrimido[5,4-d]pyrimidin

(342) 4-[(3,4-Dichlor-phenyl)amino]-6-[(trans-4-dimethylaminocyclohexyl)amino]-pyrimido[5,4-d]pyrimidin

(343) 4-[(3-Chlor-phenyl)amino]-6-[(trans-4-dimethylaminocyclohexyl)amino]-pyrimido [5,4-d]pyrimidin

(344) 4-[(3-Brom-phenyl)amino]-6-[(trans-4-dimethylaminocyclohexyl)amino]-pyrimido[5,4-d]pyrimidin

(345) 4-[(3-Nitro-phenyl)amino]-6-[(trans-4-dimethylaminocyclohexyl)amino]-pyrimido [5,4-d]pyrimidin

(346) 4-[(3-Ethinyl-phenyl)amino]-6-[(trans-4-dimethylaminocyclohexyl)amino]-pyrimido [5,4-d]pyrimidin

(347) 4-[(3-Chlor-4-fluor-phenyl)amino]-6-[(trans-4-acetylaminocyclohexyl)amino]-pyrimido [5,4-d]pyrimidin

(348) 4-[(3,4-Dichlor-phenyl)amino]-6-[(trans-4-acetylaminocyclohexyl)amino]-pyrimido [5,4-d]pyrimidin

(349) 4-[(3-Chlor-phenyl)amino]-6-[(trans-4-acetylaminocyclohexyl)amino]-pyrimido [5,4-d]pyrimidin

(350) 4-[(3-Brom-phenyl)amino]-6-[(trans-4-acetylaminocyclohexyl)amino]-pyrimido[5,4-d]pyrimidin

(351) 4-[(3-Nitro-phenyl)amino]-6-[(trans-4-acetylaminocyclohexyl)amino]-pyrimido [5,4-d]pyrimidin

(352) 4-[(3-Ethinyl-phenyl)amino]-6-[(trans-4-acetylaminocyclohexyl)amino]-pyrimido [5,4-d]pyrimidin

(353) 4-[(3-Chlor-4-fluor-phenyl)amino]-6-[(trans-4-methylsulfonylaminocyclohexyl)amino]-pyrimido[5,4-d]pyrimidin

(354) 4-[(3,4-Dichlor-phenyl)amino]-6-[(trans-4-methylsulfonylaminocyclohexyl)amino]-pyrimido[5,4-d]pyrimidin

(355) 4-[(3-Chlor-phenyl)amino]-6-[(trans-4-methylsulfonylaminocyclohexyl)amino]-pyrimido[5,4-d]pyrimidin

(356) 4-[(3-Brom-phenyl)amino]-6-[(trans-4-methylsulfonylaminocyclohexyl)amino]-pyrimido[5,4-d]pyrimidin

(357) 4-[(3-Nitro-phenyl)amino]-6-[(trans-4-methylsulfonylaminocyclohexyl)amino]-pyrimido [5,4-d]pyrimidin

(358) 4-[(3-Ethinyl-phenyl)amino]-6-[(trans-4-methylsulfonylaminocyclohexyl)amino]-pyrimido [5,4-d]pyrimidin

(359) 4-[(3-Chlor-4-fluor-phenyl)amino]-6-[(trans-4-methoxycyclohexyl)amino]-pyrimido [5,4-d]pyrimidin

(360) 4-[(3,4-Dichlor-phenyl)amino]-6-[(trans-4-methoxycyclohexyl)amino]-pyrimido [5,4-d]pyrimidin

(361) 4-[(3-Chlor-phenyl)amino]-6-[(trans-4-methoxycyclohexyl)amino]-pyrimido[5,4-d]pyrimidin

(362) 4-[(3-Brom-phenyl)amino]-6-[(trans-4-methoxycyclohexyl)amino]-pyrimido[5,4-d]pyrimidin

(363) 4-[(3-Nitro-phenyl)amino]-6-[(trans-4-methoxycyclohexyl)amino]-pyrimido [5,4-d]pyrimidin

(364) 4-[(3-Ethinyl-phenyl)amino]-6-[(trans-4-methoxycyclohexyl)amino]-pyrimido [5,4-d]pyrimidin

(365) 4-[(3-Chlor-4-fluor-phenyl)amino]-6-[trans-4-carboxycyclohexylamino]-pyrimido[5,4-d]pyrimidin

(366) 4-[(3,4-Dichlor-phenyl)amino]-6-[trans-4-carboxy-cyclohexylamino]-pyrimido [5,4-d]pyrimidin

(367) 4-[(3-Chlor-phenyl)amino]-6-[trans-4-carboxy-cyclohexylamino]-pyrimido[5,4-d]pyrimidin

(368) 4-[(3-Brom-phenyl)amino]-6-[trans-4-carboxy-cyclohexylamino]-pyrimido[5,4-d]pyrimidin

(369) 4-[(3-Nitro-phenyl)amino]-6-[trans-4-carboxy-cyclohexylamino]-pyrimido[5,4-d]pyrimidin

(370) 4-[(3-Ethinyl-phenyl)amino]-6-[trans-4-carboxy-cyclohexylamino]-pyrimido [5,4-d]pyrimidin

(371) 4-[(3-Chlor-4-fluor-phenyl)amino]-6-[trans-4-(N,N-dimethylamino-carbonyl)cyclohexylamino]-pyrimido[5,4-d]pyrimidin

(372) 4-[(3,4-Dichlor-phenyl)amino]-6-[trans-4-(N,N-dimethylamino-carbonyl)cyclohexylamino]-pyrimido[5,4-d] pyrimidin

(373) 4-[(3-Chlor-phenyl)amino]-6-[trans-4-(N,N-dimethylaminocarbonyl)cyclohexylamino]-pyrimido[5,4-d]pyrimidin

(374) 4-[(3-Brom-phenyl)amino]-6-[trans-4-(N,N-dimethylaminocarbonyl)cyclohexylamino]-pyrimido[5,4-d]pyrimidin

(375) 4-[(3-Nitro-phenyl)amino]-6-[trans-4-(N,N-dimethylaminocarbonyl)cyclohexylamino]-pyrimido [5,4-d]pyrimidin

(376) 4-[(3-Ethinyl-phenyl)amino]-6-[trans-4-(N,N-dimethylamino-carbonyl)cyclohexylamino]-pyrimido[5,4-d]pyrimidin

(377) 4-[(3-Chlor-4-fluor-phenyl)amino]-6-[trans-4-(morpholinocarbonyl)cyclohexylamino]-pyrimido [5,4-d]pyrimidin

(378) 4-[(3,4-Dichlor-phenyl)amino]-6-[trans-4-(morpholinocarbonyl)cyclohexylamino]-pyrimido[5,4-d]pyrimidin

(379) 4-[(3-Chlor-phenyl)amino]-6-[trans-4-(morpholinocarbonyl)cycldhexylamino]-pyrimido [5,4-d]pyrimidin

(380) 4-[(3-Brom-phenyl)amino]-6-[trans-4-(morpholinocarbonyl)cyclohexylamino]-pyrimido[5,4-d]pyrimidin

(381) 4-[(3-Nitro-phenyl)amino]-6-[trans-4-(morpholinocarbonyl)cyclohexylamino]-pyrimido[5,4-d]pyrimidin

(382) 4-[(3-Ethinyl-phenyl)amino]-6-[trans-4-(morpholinocarbonyl)cyclohexylamino]-pyrimido[5,4-d]pyrimidin

(383) 4-[(3-Chlor-4-fluor-phenyl)amino]-6-[trans-4-(pyrrolidinocarbonyl)cyclohexylamino]-pyrimido[5,4-d]pyrimidin

(384) 4-[(3,4-Dichlor-phenyl)amino]-6-[trans-4-(pyrrolidinocarbonyl)cyclohexylamino]-pyrimido [5,4-d]pyrimidin

(385) 4-[(3-Chlor-phenyl)amino]-6-[trans-4-(pyrrolidinocarbonyl)cyclohexylamino]-pyrimido [5,4-d]pyrimidin

(386) 4-[(3-Brom-phenyl)amino]-6-[trans-4-(pyrrolidinocarbonyl)cyclohexylamino]-pyrimido[5,4-d]pyrimidin

(387) 4-[(3-Nitro-phenyl)amino]-6-[trans-4-(pyrrolidinocarbonyl)cyclohexylamino]-pyrimido[5,4-d]pyrimidin

(388) 4-[(3-Ethinyl-phenyl)amino]-6-[trans-4-(pyrrolidinocarbonyl)cyclohexylamino]-pyrimido [5,4-d]pyrimidin

(389) 4-[(3-Chlor-4-fluor-phenyl)amino]-6-[(cis-4-hydroxy-cyclohexyl)amino]-pyrimido[5,4-d]pyrimidin

(390) 4-[(3,4-Dichlor-phenyl)amino]-6-[(cis-4-hydroxy-cyclohexyl)amino]-pyrimido[5,4-d]pyrimidin

(391) 4-[(3-Chlor-phenyl)amino]-6-[(cis-4-hydroxy-cyclohöxyl)amino]-pyrimido[5,4-d]pyrimidin

(392) 4-[(3-Brom-phenyl)amino]-6-[(cis-4-hydroxy-cyclohexyl)amino]-pyrimido[5,4-d]pyrimidin

(393) 4-[(3-Nitro-phenyl)amino]-6-[(cis-4-hydroxy-cyclohexyl)amino]-pyrimido [5,4-d]pyrimidin

(394) 4-[(3-Ethinyl-phenyl)amino]-6-[(cis-4-hydroxy-cyclohexyl)amino]-pyrimido [5,4-d]pyrimidin

(395) 4-[(3-Chlor-4-fluor-phenyl)amino]-6-[N-(trans-4-hydroxy-cyclohexyl)-N-methyl-amino]-pyrimido [5,4-d]pyrimidin
Schmelzpunkt: 243 - 246°C

$R_f$-Wert: 0,51 (Kieselgel; Petrolether/Essigester/Methanol = 10:8:2)

(396) 4-[(3,4-Dichlor-phenyl)amino]-6-[N-(trans-4-hydroxy-cyclohexyl)-N-methyl-amino]-pyrimido [5,4-d]pyrimidin

(397) 4-[(3-Chlor-phenyl)amino]-6-[N-(trans-4-hydroxy-cyclohexyl)-N-methyl-amino]-pyrimido[5,4-d]pyrimidin

(398) 4-[(3-Brom-phenyl)amino]-6-[N-(trans-4-hydroxy-cyclohexyl)-N-methyl-amino]-pyrimido [5,4-d]pyrimidin

(399) 4-[(3-Nitro-phenyl)amino]-6-[N-(trans-4-hydroxy-cyclohexyl)-N-methyl-amino]-pyrimido[5,4-d]pyrimidin

(400) 4-[(3-Ethinyl-phenyl)amino]-6-[N-(trans-4-hydroxy-cyclohexyl)-N-methyl-amino]-pyrimido[5,4-d]pyrimidin

(401) 4-[(3-chlor-4-fluor-phenyl)amino]-6-[(trans-4-hydroxy-4-methylcyclohexyl)amino]-pyrimido [5,4-d]pyrimidin

(402) 4-[(3,4-Dichlor-phenyl)amino]-6-[(trans-4-hydroxy-4-methylcyclohexyl)amino]-pyrimido [5,4-d]pyrimidin

(403) 4-[(3-Chlor-phenyl)amino]-6-[(trans-4-hydroxy-4-methylcyclohexyl)amino]-pyrimido [5,4-d]pyrimidin

(404) 4-[(3-Brom-phenyl)amino]-6-[(trans-4-hydroxy-4-methylcyclohexyl)amino]-pyrimido [5,4-d]pyrimidin

(405) 4-[(3-Nitro-phenyl)amino]-6-[(trans-4-hydroxy-4-methylcyclohexyl)amino]-pyrimido[5,4-d]pyrimidin

(406) 4-[(3-Ethinyl-phenyl)amino]-6-[(trans-4-hydroxy-4-methylcyclohexyl)amino]-pyrimido [5,4-d]pyrimidin

(407) 4-[(3-Chlor-4-fluor-phenyl)amino]-6-[(cis-4-hydroxy-4-methylcyclohexyl)amino]-pyrimido[5,4-d]pyrimidin

(408) 4-[(3,4-Dichlor-phenyl)amino]-6-[(cis-4-hydroxy-4-methylcyclohexyl)amino]-pyrimido[5,4-d]pyrimidin

(409) 4-[(3-Chlor-phenyl)amino]-6-[(cis-4-hydroxy-4-methylcyclohexyl)amino]-pyrimido[5,4-d]pyrimidin

(410) 4-[(3-Brom-phenyl)amino]-6-[(cis-4-hydroxy-4-methylcyclohexyl)amino]-pyrimido [5,4-d]pyrimidin

(411) 4-[(3-Nitro-phenyl)amino]-6-[(cis-4-hydroxy-4-methylcyclohexyl)amino]-pyrimido[5,4-d]pyrimidin

(412) 4-[(3-Ethinyl-phenyl)amino]-6-[(cis-4-hydroxy-4-methylcyclohexyl)amino]-pyrimido [5,4-d]pyrimidin

(413) 4-[(3-Chlor-4-fluor-phenyl)amino]-6-[4-tetrahydropyranylamino]-pyrimido [5,4-d]pyrimidin

(414) 4-[(3,4-Dichlor-phenyl)amino]-6-[4-tetrahydropyranylamino]-pyrimido [5,4-d]pyrimidin

(415) 4-[(3-Chlor-phenyl)amino]-6-[4-tetrahydropyranylamino]-pyrimido[5,4-d]pyrimidin

(416) 4-[(3-Brom-phenyl)amino]-6-[4-tetrahydropyranylamino]-pyrimido[5,4-d]pyrimidin

(417) 4-[(3-Nitro-phenyl)amino]-6-[4-tetrahydropyranylamino]-pyrimido[5,4-d]pyrimidin

(418) 4-[(3-Ethinyl-phenyl)amino]-6-[4-tetrahydropyranylamino]-pyrimido [5,4-d]pyrimidin

(419) 4-[(3-Chlor-4-fluor-phenyl)amino]-6-[(4-oxo-cyclohexyl)amino]-pyrimido [5,4-d]pyrimidin

(420) 4-[(3,4-Dichlor-phenyl)amino]-6-[(4-oxo-cyclohexyl) amino]-pyrimido [5,4-d]pyrimidin

(421) 4-[(3-Chlor-phenyl)amino]-6-[(4-oxo-cyclohexyl)amino]-pyrimido [5,4-d]pyrimidin

(422) 4-[(3-Brom-phenyl)amino]-6-[(4-oxo-cyclohexyl)amino]-pyrimido [5,4-d]pyrimidin

(423) 4-[(3-Nitro-phenyl)amino]-6-[(4-oxo-cyclohexyl)amino]-pyrimido[5,4-d]pyrimidin

(424) 4-[(3-Ethinyl-phenyl)amino]-6-[(4-oxo-cyclohexyl)amino]-pyrimido[5,4-d]pyrimidin

(425) 4-[(3-Chlor-4-fluor-phenyl)amino]-6-(4-oxo-1-piperidinyl)-pyrimido[5,4-d]pyrimidin

(426) 4-[(3,4-Dichlor-phenyl)amino]-6-(4-oxo-1-piperidinyl)-pyrimido[5,4-d]pyrimidin

(427) 4-[(3-Chlor-phenyl)amino]-6-(4-oxo-1-piperidinyl)-pyrimido[5,4-d]pyrimidin

(428) 4-[(3-Brom-phenyl)amino]-6-(4-oxo-1-piperidinyl)-pyrimido[5,4-d]pyrimidin

(429) 4-[(3-Nitro-phenyl)amino]-6-(4-oxo-1-piperidinyl)-pyrimido[5,4-d]pyrimidin

(430) 4-[(3-Ethinyl-phenyl)amino]-6-(4-oxo-l-piperidinyl)-pyrimido[5,4-d]pyrimidin

(431) 4-[(3-Chlor-4-fluor-phenyl)amino]-6-morpholino-pyrimido[5,4-d]pyrimidin
Schmelzpunkt: 218 - 221°C
$R_f$-Wert: 0,47 (Kieselgel; Petrolether/Essigester/Methanol = 10:5:1)

(432) 4-[(3,4-Dichlor-phenyl)amino]-6-morpholino-pyrimido[5,4-d]pyrimidin

(433) 4- [(3-Nitro-phenyl)amino]-6-morpholino-pyrimido[5,4-d]pyrimidin

(434) 4-[(3-Ethinyl-phenyl)amino]-6-morpholino-pyrimido[5,4-d]pyrimidin

(435) 4-[(3-Chlor-4-fluor-phenyl)amino]-6-(4-piperidinyl-amino)-pyrimido [5,4-d]pyrimidin

(436) 4-[(3,4-Dichlor-phenyl)amino]-6-(4-piperidinyl-amino)-pyrimido [5,4-d]pyrimidin

(437) 4-[(3-Chlor-phenyl)amino]-6-(4-piperidinyl-amino)-pyrimido[5,4-d]pyrimidin

(438) 4-[(3-Brom-phenyl)amino]-6-(4-piperidinyl-amino)-pyrimido[5,4-d]pyrimidin

(439) 4-[(3-Nitro-phenyl)amino]-6-(4-piperidinyl-amino)-pyrimido[5,4-d]pyrimidin

(440) 4-[(3-Ethinyl-phenyl)amino]-6-(4-piperidinyl-amino)-pyrimido[5,4-d]pyrimidin

(441) 4-[(3,4-Dichlor-phenyl)amino]-6-[2-(morpholino)-ethylamino]-pyrimido[5,4-d]pyrimidin

(442) 4-[(3-Chlor-phenyl)amino]-6-[2-(morpholino)-ethylamino]-pyrimido [5,4-d]pyrimidin

(443) 4-[(3-Brom-phenyl)amino]-6-[2-(morpholino)-ethylamino]-pyrimido [5,4-d]pyrimidin

(444) 4-[(3-Nitro-phenyl)amino]-6-[2-(morpholino)-ethylamino]-pyrimido [5,4-d]pyrimidin

(445) 4-[(3-Ethinyl-phenyl)amino]-6-[2-(morpholino)-ethylamino]-pyrimido [5,4-d]pyrimidin

(446) 4-[(3-Methyl-phenyl)amino]-6-[2-(morpholino)-ethylamino]-pyrimido [5,4-d] pyrimidin

(447) 4-[(3-Chlor-4-fluor-phenyl)amino]-6-isopropylamino-pyrimido[5,4-d]pyrimidin

(448) 4-[(3,4-Dichlor-phenyl)amino]-6-isopropylamino-pyrimido[5,4-d]pyrimidin

(449) 4-[(3-Nitro-phenyl)amino]-6-isopropylamino-pyrimido[5,4-d]pyrimidin

(450) 4-[(3-Ethinyl-phenyl)amino]-6-isopropylamino-pyrimido[5,4-d]pyrimidin

(451) 4-[(3-Chlor-4-fluor-phenyl)amino]-6-(tert.-butylamino)-pyrimido[5,4-d]pyrimidin

(452) 4-[(3,4-Dichlor-phenyl)amino]-6-(tert.-butylamino)-pyrimido[5,4-d]pyrimidin

(453) 4-[(3-Chlor-phenyl)amino]-6-(tert.-butylamino)-pyrimido[5,4-d]pyrimidin

(454) 4-[(3-Brom-phenyl)amino]-6-(tert.-butylamino)-pyrimido[5,4-d]pyrimidin

(455) 4-[(3-Nitro-phenyl)amino]-6-(tert.-butylamino)-pyrimido[5,4-d]pyrimidin

(456) 4-[(3-Ethinyl-phenyl)amino]-6-(tert.-butylamino)-pyrimido[5,4-d]pyrimidin

(457) 4-[(3-Chlor-4-fluor-phenyl)amino]-6-(1-hydroxy-2-propylamino)-pyrimido[5,4-d]pyrimidin
Schmelzpunkt: 227 - 231°C
$R_f$-Wert: 0,44 (Kieselgel; Petrolether/Essigester/Methanol = 10:10:2)

(458) 4-[(3,4-Dichlor-phenyl)amino]-6-(1-hydroxy-2-propylamino)-pyrimido [5,4-d]pyrimidin

(459) 4-[(3-Chlor-phenyl)amino]-6-(1-hydroxy-2-propylamino)-pyrimido[5,4-d]pyrimidin

(460) 4-[(3-Brom-phenyl)amino]-6-(1-hydroxy-2-propylamino)-pyrimido[5,4-d]pyrimidin

(461) 4-[(3-Nitro-phenyl)amino]-6-(1-hydroxy-2-propylamino)-pyrimido[5,4-d]pyrimidin

(462) 4-[(3-Ethinyl-phenyl)amino]-6-(1-hydroxy-2-propylamino)-pyrimido[5,4-d]pyrimidin

(463) 4-[(3-Chlor-4-fluor-phenyl)amino]-6-(1-methyl-4-piperidinyl-amino)-pyrimido[5,4-d]pyrimidin

(464) 4- [(3,4-Dichlor-phenyl)amino]-6-(1-methyl-4-piperidinylamino)-pyrimido[5,4-d]pyrimidin

(465) 4-[(3-Chlor-phenyl)amino]-6-(1-methyl-4-piperidinyl-amino)-pyrimido [5,4-d]pyrimidin

(466) 4-[(3-Brom-phenyl)amino]-6-(1-methyl-4-piperidinyl-amino)-pyrimido [5,4-d]pyrimidin

(467) 4-[(3-Nitro-phenyl)amino]-6-(1-methyl-4-piperidinyl-amino)-pyrimido [5,4-d]pyrimidin

(468) 4-[(3-Ethinyl-phenyl)amino]-6-(1-methyl-4-piperidinylamino)-pyrimido [5,4-d]pyrimidin

(469) 4-[(3-Chlor-4-fluor-phenyl)amino]-6-(propargylamino)-pyrimido[5,4-d]pyrimidin

(470) 4-[(3,4-Dichlor-phenyl)amino]-6-(propargylamino)-pyrimido[5,4-d]pyrimidin

(471) 4-[(3-Chlor-phenyl)amino]-6-(propargylamino)-pyrimido[5,4-d]pyrimidin

(472) 4-[(3-Brom-phenyl)amino]-6-(propargylamino)-pyrimido[5,4-d]pyrimidin

(473) 4-[(3-Nitro-phenyl)amino]-6-(propargylamino)-pyrimido[5,4-d]pyrimidin

(474) 4-[(3-Ethinyl-phenyl)amino]-6-(propargylamino)-pyrimido[5,4-d]pyrimidin

(475) 4-[(3-Chlor-4-fluor-phenyl)amino]-6-(1-pyrrolidinyl)-pyrimido[5,4-d]pyrimidin

(476) 4-[(3,4-Dichlor-phenyl)amino]-6-(1-pyrrolidinyl)-pyrimido[5,4-d]pyrimidin

(477) 4-[(3-Nitro-phenyl)amino]-6-(1-pyrrolidinyl)-pyrimido[5,4-d]pyrimidin

(478) 4-[(3-Ethinyl-phenyl)amino]-6-(1-pyrrolidinyl)-pyrimido[5,4-d]pyrimidin

(479) 4-[(3-Chlor-4-fluor-phenyl)amino]-6-(tetrahydrofurfurylamino)-pyrimido [5,4-d]pyrimidin
Schmelzpunkt: 158 -160°C

$R_f$-Wert: 0,40 (Kieselgel; Petrolether/Essigester = 1:1)

(480) 4-[(3,4-Dichlor-phenyl)amino]-6-(tetrahydrofurfurylamino)-pyrimido[5,4-d]pyrimidin

(481) 4-[(3-Chlor-phenyl)amino]-6-(tetrahydrofurfurylamino)-pyrimido[5,4-d]pyrimidin

(482) 4-[(3-Brom-phenyl)amino]-6-(tetrahydrofurfurylamino)-pyrimido [5,4-d]pyrimidin

(483) 4-[(3-Nitro-phenyl)amino]-6-(tetrahydrofurfurylamino)-pyrimido[5,4-d]pyrimidin

(484) 4-[(3-Ethinyl-phenyl)amino]-6-(tetrahydrofurfurylamino)-pyrimido[5,4-d]pyrimidin

(485) 4-[(3-Chlor-4-fluor-phenyl)amino]-6-(3-tetrahydrofuranylamino)-pyrimido [5,4-d]pyrimidin
Hergestellt aus den Verbindungen der Beispiele 2 und XIX.
Schmelzpunkt: 239 - 241°C
$R_f$-Wert: 0,51 (Kieselgel; Petrolether/Essigester/Methanol = 10:10:1)

(486) 4-[(3,4-Dichlor-phenyl)amino]-6-(3-tetrahydrofuranylamino)-pyrimido [5,4-d]pyrimidin

(487) 4-[(3-chlor-phenyl)amino]-6-(3-tetrahydrofuranylamino)-pyrimido[5,4-d]pyrimidin

(488) 4-[(3-Brom-phenyl)amino]-6-(3-tetrahydrofuranylamino)-pyrimido [5,4-d]pyrimidin

(489) 4-[(3-Nitro-phenyl)amino]-6-(3-tetrahydrofuranylamino)-pyrimido [5,4-d]pyrimidin

(490) 4-[(3-Ethinyl-phenyl)amino]-6-(3-tetrahydrofuranylamino)-pyrimido[5,4-d]pyrimidin

(491) 4-[(3-Chlor-4-fluor-phenyl)amino]-6-[2-(1-methyl-4-piperazinyl)ethylamino]-pyrimido[5,4-d]pyrimidin

(492) 4-[(3-Chlor-4-fluor-phenyl)amino]-6-[2-(1-acetyl-4-piperazinyl)ethylamino]-pyrimido[5,4-d]pyrimidin

(493) 4-[(3-Chlor-4-fluor-phenyl)amino]-6-[2-(1-methylsulfonyl-4-piperazinyl)ethylamino]-pyrimido[5,4-d]pyrimidin

(494) 4-[(3-Chlor-4-fluor-phenyl)amino]-6-[2-(1-methoxycarbonyl-4-piperazinyl)ethylamino]-pyrimido[5,4-d]pyrimidin

(495) 4-[(3-Chlor-4-fluor-phenyl)amino]-6-[2-(1-cyan-4-piperazinyl)ethylamino]-pyrimido [5,4-d]pyrimidin

(496) 4-[(3-Chlor-4-fluor-phenyl)amino]-6-[2-(1-dimethylaminocarbonyl-4-piperazinyl)ethylamino]-pyrimido[5,4-d] pyrimidin

(497) 4-[(4-Amino-3,5-dibrom-phenyl)amino]-6-[(trans-4-hydroxycyclohexyl)amino]-pyrimido[5,4-d]pyrimidin
Schmelzpunkt: 228 - 230°C
$R_f$-Wert: 0,40 (Kieselgel; Petrolether/Essigester/Methanol = 10:10:3)

| | | | |
|---|---|---|---|
| Ber. | C 42,45 | H 3,76 | N 19,25 |
| Gef. | 42,59 | 4,10 | 19,06 |

Massenspektrum: $M^+$ = 507

(498) 4-[(4-Amino-5-brom-3-chlor-phenyl)amino]-6-[(trans-4-hydroxy-cyclohexyl)amino]-pyrimido [5,4-d]pyrimidin

(499) 4-[(3,5-Dichlor-4-dimethylamino-phenyl)amino]-6-[(trans-4-hydroxy-cyclohexyl)amino]-pyrimido[5,4-d]pyrimidin

(500) 4-[(4-Acetylamino-3,5-dichlor-phenyl)amino]-6-[(trans-4-hydroxy-cyclohexyl)amino]-pyrimido[5,4-d]pyrimidin

(501) 4-[(4-Methylsulfonylamino-3,5-dichlor-phenyl)amino]-6-[(trans-4-hydroxy-cyclohexyl)amino]-pyrimido [5,4-d]pyrimidin

(502) 4-[(4-Trifluormethylsulfonylamino-3,5-dichlor-phenyl)amino]-6-[(trans-4-hydroxy-cyclohexyl)amino]-pyrimido[5,4-d]pyrimidin

(503) 4-[(3,5-Dibrom-4-hydroxy-phenyl)amino]-6-[(trans-4-hydroxy-cyclohexyl)amino]-pyrimido[5,4-d]pyrimidin

(504) 4-[(3,5-Dichlor-4-hydroxy-phenyl)amino]-6-[(trans-4-hydroxy-cyclohexyl)amino]-pyrimido [5,4-d]pyrimidin

(505) 4-[(3,5-Dichlor-4-methoxy-phenyl)amino]-6-[(trans-4-hydroxy-cyclohexyl)amino]-pyrimido[5,4-d]pyrimidin

(506) 4-[(3-Chlor-4-fluor-phenyl)amino]-6-[3-hydroxycyclopentylamino]-pyrimido[5,4-d]pyrimidin

(507) 4-[(3-Chlor-4-fluor-phenyl)amino]-6-[1-hydroxy-3-methyl-3-butylamino]-pyrimido[5,4-d]pyrimidin

(508) 4-[(3-Chlor-4-fluor-phenyl)amino]-6-[1-hydroxy-4-methyl-4-pentylamino]-pyrimido [5,4-d]pyrimidin

(509) 4-[(3-Chlor-4-fluor-phenyl)amino]-6-methyl-pyrimido[5,4-d]pyrimidin

(510) 4-[(3-Chlor-4-fluor-phenyl)amino]-6-phenyl-pyrimido[5,4-d]pyrimidin

(511) 4-[(3-Chlor-4-fluor-phenyl)amino]-6-(4-methoxyphenyl)-pyrimido[5,4-d]pyrimidin

(512) 4-[(3-Chlor-4-fluor-phenyl)amino]-6-[4-(2-hydroxymethyl-1-pyrrolidinyl)cyclohexylamino]-pyrimido[5,4-d]pyrimidin

(513) 4-[(3-Chlor-4-fluor-phenyl)amino]-6-[4-(2-oxo-1-pyrrolidinyl)cyclohexylamino]-pyrimido[5,4-d]pyrimidin

(514) 4-[(3-Chlor-4-fluor-phenyl)amino]-6-[4- (N-acetyl-N-methylamino)cyclohexylamino]-pyrimido[5,4-d]pyrimidin

(515) 4-[(3-Chlor-4-fluor-phenyl)amino]-6-[4-(N,N-bis-(2-hydroxyethyl)amino)cyclohexylamino]-pyrimido[5,4-d] pyrimidin

(516) 4-[(3-Chlor-4-fluor-phenyl)amino]-6-[4-(N-ethyl-N-(2-hydroxyethyl)amino)cyclohexylamino]-pyrimido[5,4-d] pyrimidin

(517) 4-[(3-Chlor-4-fluor-phenyl)amino]-6-[4-(1-pyrrolidinyl)cyclohexylamino]-pyrimido[5,4-d]pyrimidin

(518) 4-[(3-Chlor-4-fluor-phenyl)amino]-6-[4-(2-oxo-1-imidazolidinyl)cyclohexylamino]-pyrimido[5,4-d]pyrimidin

(519) 4-[(3-Chlor-4-fluor-phenyl)amino]-6-[4-(3-methyl-2-oxo-1-imidazolidinyl)cyclohexylamino]-pyrimido[5,4-d] pyrimidin

(520) 4-[(3-Chlor-4-fluor-phenyl)amino]-6-[4-(N-acetyl-N-methylamino)phenylamino]-pyrimido[5,4-d]pyrimidin

(521) 4-[(3-Chlor-4-fluor-phenyl)amino]-6-[4-(N,N-bis-(2-hydroxyethyl)amino)phenylamino]-pyrimido[5,4-d]pyrimidin

(522) 4-[(3-Chlor-4-fluor-phenyl)amino]-6-[4-(N-ethyl-N-(2-hydroxyethyl)amino)phenylamino]-pyrimido[5,4-d]pyrimidin

(523) 4-[(3-Chlor-4-fluor-phenyl)amino]-6- [4-1-pyrrolidinyl)phenylamino]-pyrimido [5,4-d]pyrimidin

(524) 4-[(3-Chlor-4-fluor-phenyl)amino]-6-[4-(2-oxo-1-imidazolidinyl)phenylamino]-pyrimido [5,4-d]pyrimidin

(525) 4-[(3-Chlor-4-fluor-phenyl)amino]-6-[4-(3-methyl-2-oxo-1-imidazolidinyl)phenylamino]-pyrimido [5,4-d]pyrimidin

(526) 4-[(3-Difluormethoxy-phenyl)amino]-6-[(trans-4-hydroxycyclohexyl)amino]-pyrimido [5,4-d]pyrimidin

(527) 4-[(3-chlor-4-fluor-phenyl)amino]-6-[4-morpholino-cyclohexylamino]-pyrimido [5,4-d]pyrimidin

(528) 4-[(3-Chlor-4-fluor-phenyl)amino]-6-[4-morpholino-phenylamino]-pyrimido [5,4-d]pyrimidin
Schmelzpunkt: 250 - 254°C
$R_f$-Wert: 0,49 (Kieselgel; Petrolether/Essigester/Methanol = 10:10:2)

(529) 4-[(3-Chlor-4-fluor-phenyl)amino]-6-dimethylaminopyrimido[5,4-d]pyrimidin
Schmelzpunkt: 203 - 205°C
$R_f$-Wert: 0,85 (Kieselgel; Petrolether/Essigester/Methanol = 10:10:2)
Massenspektrum: $M^+$ = 318

(530) 4-[(3-Chlor-4-fluor-phenyl)amino]-6-[4-amino-1-piperidinyl]-pyrimido[5,4-d]pyrimidin
Schmelzpunkt: 169 - 174°C
$R_f$-Wert: 0,50 (Kieselgel;
Methylenchlorid/Methanol/konz.Ammoniak = 100:30:1)

(531) 4-[(3-Chlor-4-fluor-phenyl)amino]-6-[4-dimethylamino-1-piperidinyl]-pyrimido[5,4-d]pyrimidin
Schmelzpunkt: 162 - 165°C
$R_f$-Wert: 0,48 (Kieselgel; Methylenchlorid/Methanol = 10:3)

(532) 4-[(3-Chlor-4-fluor-phenyl)amino]-6-[2-dimethylamino-1-ethoxy]-pyrimido[5,4-d]pyrimidin
Schmelzpunkt: 98 - 100°C
$R_f$-Wert: 0,38 (Kieselgel; Petrolether/Essigester/Methanol = 10:10:4)

Beispiel 2

4-[(3-Methylphenyl)amino]-6-methylsulfinyl-pyrimido[5,4-d]pyrimidin und 4-[(3-Methylphenyl)amino]-6-methylsulfonyl-pyrimido [5,4-d]pyrimidin

3,9 g 4-[(3-Methylphenyl)amino]-6-methylthio-pyrimido[5,4-d]-pyrimidin und 6,28 g 3-Chlorperoxybenzoesäure (50%ig) in 100 ml Methylenchlorid werden 75 Minuten unter Rückfluß erhitzt. Nach dem Abkühlen wird mit Wasser versetzt, die organische Phase wird abgetrennt, mit Natriumbicarbonatlösung und Wasser gewaschen, getrocknet und einrotiert. Der Rückstand wird mit Diethylether gerührt, der Feststoff abgesaugt und getrocknet.
Ausbeute: 4,2 g,
$R_f$-Wert: 0,38 und 0,54 (Kieselgel; Petrolether/Essigester = 10:10:1)

Die Verbindungen wurden chromatographisch getrennt und charakterisiert:

a) 4-[(3-Methylphenyl)amino]-6-methylsulfinyl-pyrimido[5,4-d]-pyrimidin
Schmelzpunkt: 118°C
$R_f$-Wert: 0,73 (Kieselgel; Essigester/Methanol = 10:1)
Massenspektrum: $M^+$ = 299

b) 4-[(3-Methylphenyl)amino]-6-methylsulfonyl-pyrimido[5,4-d]pyrimidin
Schmelzpunkt: 220°C
$R_f$-Wert: 0,60 (Kieselgel; Essigester)
Massenspektrum: $M^+$ = 315

Analog Beispiel 2 werden folgende Verbindungen erhalten:

(1) 4-(Phenylamino)-6-methylsulfonyl-pyrimido[5,4-d]pyrimidin Es wird ein Überschuß an 3-Chlorperoxybenzoesäure verwendet.
$R_f$-Wert: 0,36 (Kieselgel; Petrolether/Essigester/Methanol = 10:10:1)

(2) 4-(N-Methyl-N-phenyl-amino)-6-methylsulfonyl-pyrimido[5,4-d]pyrimidin

Es wird ein Überschuß an 3-Chlorperoxybenzoesäure verwendet.
$R_f$-Wert: 0,32 (Kieselgel; Petrolether/Essigester/Methanol = 10:10:1)

(3) 4-[(3-Fluorphenyl)amino]-6-methylsulfonyl-pyrimido[5,4-d]pyrimidin
$R_f$-Wert: 0,64 (Kieselgel; Petrolether/Essigester/Methanol = 10:10:1)

(4) 4- [(3-Chlorphenyl)amino]-6-methylsulfonyl-pyrimido[5,4-d]pyrimidin
$R_f$-Wert: 0,50 (Kieselgel; Petrolether/Essigester/Methanol = 10:10:1)

(5) 4-[(3-Bromphenyl)amino]-6-methylsulfonyl-pyrimido[5,4-d]pyrimidin
$R_f$-Wert: 0,38 (Kieselgel; Petrolether/Essigester/Methanol = 10:10:1)

(6) 4-[(3-Trifluormethylphenyl)amino]-6-methylsulfonyl-pyrimido[5,4-d]pyrimidin
$R_f$-Wert: 0,54 (Kieselgel; Petrolether/Essigester/Methanol = 10:10:1)

(7) 4-[(3-Methoxyphenyl)amino]-6-methylsulfonyl-pyrimido[5,4-d]pyrimidin
$R_f$-Wert: 0,49 (Kieselgel; Petrolether/Essigester/Methanol = 10:10:1)

(8) 4-[(3-Ethylphenyl)amino]-6-methylsulfonyl-pyrimido[5,4-d]pyrimidin
$R_f$-Wert: 0,37 (Kieselgel; Petrolether/Essigester = 1:1)

(9) 4-[(3,4-Dichlorphenyl)amino]-6-methylsulfonyl-pyrimido[5,4-d]pyrimidin
$R_f$-Wert: 0,45 (Kieselgel; Petrolether/Essigester = 1:1)

(10) 4-[(3-Chlor-4-fluor-phenyl)amino]-6-methylsulfonyl-pyrimido[5,4-d]pyrimidin
$R_f$-Wert: 0,45 (Kieselgel; Petrolether/Essigester/Methanol = 10:8:2)

(11) 4-[(3-Chlor-4-methoxy-phenyl)amino]-6-methylsulfonyl-pyrimido [5,4-d]pyrimidin
$R_f$-Wert: 0,60 (Kieselgel; Methylenchlorid/Essigester/Methanol = 10:4:3)

(12) 4-[(4-Amino-3-nitro-phenyl)amino]-6-methylsulfonyl-pyrimido[5,4-d]pyrimidin
$R_f$-Wert: 0,19 (Kieselgel; Methylenchlorid/Essigester/Methanol = 10:4:2)

(13) 4-[(4-Chlor-3-nitro-phenyl)amino]-6-methylsulfonyl-pyrimido[5,4-d]pyrimidin
$R_f$-Wert: 0,38 (Kieselgel; Petrolether/Essigester/Methanol = 10:10:3)

(14) 4-[(4-Amino-3-cyano-phenyl)amino]-6-methylsulfonyl-pyrimido[5,4-d]pyrimidin
$R_f$-Wert: 0,25 (Kieselgel; Petrolether/Essigester/Methanol = 10:10:2)

(15) 4-[(4-Amino-3,5-dichlor-phenyl)amino]-6-methylsulfonyl-pyrimido[5,4-d]pyrimidin
$R_f$-Wert: 0,40 (Kieselgel; Petrolether/Essigester/Methanol = 10:10:2)

(16) 4-[(4-Amino-3,5-dibrom-phenyl)amino]-6-methylsulfonyl-pyrimido [5,4-d]pyrimidin
$R_f$-Wert: 0,53 (Kieselgel; Petrolether/Essigester/Methanol = 10:10:2)

Beispiel 3

4-[(3-Methylphenyl)amino]-6-methylthio-pyrimido[5,4-d]pyrimidin

4,9 g 4-Chlor-6-methylthio-pyrimido[5,4-d]pyrimidin, 2,0 g 3-Methylanilin, 2,4 ml Triethylamin und 100 ml Dioxan werden 3 Stunden auf 100°C erhitzt. Nach dem Abkühlen wird das Reaktionsgemisch eingeengt und zwischen Wasser und Methylenchlorid verteilt. Die organische Phase wird abgetrennt, getrocknet und eingeengt und der Rückstand durch Chromatographie über eine Kieselgelsäule (Petrolether/Essigester = 2:1) gereinigt. Der Feststoff wird mit Diethylether verrieben, abgesaugt und getrocknet.
Ausbeute: 2,8 g (43 % der Theorie),
Schmelzpunkt: 118-120°C
$R_f$-Wert: 0,55 (Kieselgel; Petrolether/Essigester = 2:1)
Analog Beispiel 3 werden folgende Verbindungen erhalten:

(1) 4-(Phenylamino)-6-methylthio-pyrimido[5,4-d]pyrimidin $R_f$-Wert: 0,63 (Kieselgel; Petrolether/Essigester = 2:1)

(2) 4-[(4-Fluorphenyl)amino)-6-(cyclopropylamino)-pyrimido[5,4-d]pyrimidin
Ausgangsmaterial: Verbindung des Beispiels IV, 2 1/2 tägiges Erhitzen.
Schmelzpunkt: 200-202°C
$R_f$-Wert: 0,44 (Kieselgel; Petrolether/Essigester = 1:1)

| | | | |
|---|---|---|---|
| Ber. | C 60,80 | H 4,42 | N 28,36 |
| Gef. | 60,77 | 4,52 | 28,60 |

(3) 4-(N-Methyl-N-phenyl-amino)-6-methylthio-pyrimido[5,4-d]-pyrimidin
$R_f$-Wert: 0,45 (Kieselgel; Petrolether/Essigester = 2:1)

(4) 4-[(3,4-Difluorphenyl)amino]-6-(cyclopropylamino)-pyrimido[5,4-d]pyrimidin
Schmelzpunkt: 212-214°C
$R_f$-Wert: 0,51 (Kieselgel; Petrolether/Essigester = 1:1)

(5) 4-[(3,5-Difluorphenyl)amino]-6-(cyclopropylamino)-pyrimido [5,4-d]pyrimidin
Durchführung in Butanol bei Rückflußtemperatur in Gegenwart vor N-Ethyl-diisopropylamin.
Schmelzpunkt: 199-201°C
$R_f$-Wert: 0,62 (Kieselgel; Petrolether/Essigester = 1:1)

(6) 4-[(3-Chlor-4-fluor-phenyl)amino]-6-(cyclopropylamino)-pyrimido [5,4-d]pyrimidin
Schmelzpunkt: 197-199°C

(7) 4- [[3,5-Bis- (trifluormethyl)-phenyl]amino]-6-(cyclopropylamino)-pyrimido [5,4-d]pyrimidin
Schmelzpunkt: 228-230°C
$R_f$-Wert: 0,68 (Kieselgel; Petrolether/Essigester = 1:1)

(8) 4-[(4-Fluor-3-trifluormethylphenyl)amino]-6-(cyclopropylamino)-pyrimido[5,4-d]pyrimidin
Schmelzpunkt: 174-176°C
$R_f$-Wert: 0,50 (Kieselgel; Petrolether/Essigester = 1:1)

| | | | |
|---|---|---|---|
| Ber. | C 52,75 | H 3,23 | N 23,06 |
| Gef. | 52,52 | 3,52 | 22,66 |

(9) 4-[(4-Fluor-3-methylphenyl)amino]-6-(cyclopropylamino)-pyrimido[5,4-d]pyrimidin
Schmelzpunkt: 180-182°C
$R_f$-Wert: 0,40 (Kieselgel; Petrolether/Essigester = 1:1)

| | | | |
|---|---|---|---|
| Ber. | C 61,92 | H 4,87 | N 27,08 |
| Gef. | 61,71 | 4,96 | 26,82 |

(10) 4-[(4-Trifluormethylphenyl)amino]-6-(cyclopropylamino)-pyrimido [5,4-d]pyrimidin
Schmelzpunkt: 229-231°C
$R_f$-Wert: 0,23 (Kieselgel; Petrolether/Essigester = 2:1)

(11) 4-[(3-Fluorphenyl)amino]-6-methylthio-pyrimido[5,4-d]pyrimidin
$R_f$-Wert: 0,52 (Kieselgel; Petrolether/Essigester = 2:1)

(12) 4-[(3-Chlorphenyl)amino]-6-methylthio-pyrimido[5,4-d]pyrimidin
$R_f$-Wert: 0,54 (Kieselgel; Petrolether/Essigester = 2:1)

(13) 4-[(3-Bromphenyl)amino]-6-methylthio-pyrimido[5,4-d]pyrimidin
$R_f$-Wert: 0,60 (Kieselgel; Petrolether/Essigester = 2:1)

(14) 4-[(3-Trifluormethylphenyl)amino]-6-methylthio-pyrimido[5,4-d]pyrimidin
$R_f$-Wert: 0,62 (Kieselgel; Petrolether/Essigester = 2:1)

(15) 4-[(3-Methoxyphenyl)amino]-6-methylthio-pyrimido[5,4-d]pyrimidin
Schmelzpunkt: 168-170°C
$R_f$-Wert: 0,49 (Kieselgel; Petrolether/Essigester = 2:1)

(16) 4-[(3-Ethylphenyl)amino]-6-methylthio-pyrimido[5,4-d]pyrimidin
$R_f$-Wert: 0,53 (Kieselgel; Petrolether/Essigester = 2:1)

(17) 4-[(3-Iodphenyl)amino]-6-(cyclopropylamino)-pyrimido[5,4-d]pyrimidin
Schmelzpunkt: 240-242°C
$R_f$-Wert: 0,47 (Kieselgel; Petrolether/Essigester = 1:1)

| | | | |
|---|---|---|---|
| Ber. | C 44,57 | H 3,42 | N 20,79 |
| Gef. | 44,50 | 3,46 | 20,86 |

(18) 4-[(3-Trifluormethoxyphenyl)amino]-6-(cyclopropylamino)-pyrimido[5,4-d]pyrimidin
Schmelzpunkt: 168-170°C
$R_f$-Wert: 0,55 (Kieselgel; Petrolether/Essigester = 1:1)

(19) 4-[(3-Cyanphenyl)amino]-6-(cyclopropylamino)-pyrimido[5,4-d]pyrimidin
Schmelzpunkt: 240°C
$R_f$-Wert: 0,41 (Kieselgel; Petrolether/Essigester = 1:1)

(20) 4-[(3-Ethoxycarbonylphenyl)amino]-6-(cyclopropylamino)-pyrimido[5,4-d]pyrimidin

(21) 4-[(3-Isopropylphenyl)amino]-6-(cyclopropylamino)-pyrimido[5,4-d]pyrimidin

(22) 4-[(3-Cyclopropylphenyl)amino]-6-(cyclopropylamino)-pyrimido [5,4-d]pyrimidin

(23) 4-[(4-Chlorphenyl)amino]-6-(cyclopropylamino)-pyrimido[5,4-d]pyrimidin
Schmelzpunkt: 218-220°C
$R_f$-Wert: 0,45 (Kieselgel; Petrolether/Essigester = 1:1)

| | | | |
|---|---|---|---|
| Ber. | C 57,60 | H 4,18 | N 26,87 |
| Gef. | 57,71 | 4,32 | 26,57 |

(24) 4-[(4-Methylphenyl)amino]-6-(cyclopropylamino)-pyrimido[5,4-d]pyrimidin
Schmelzpunkt: 192-194°C
$R_f$-Wert: 0,47 (Kieselgel; Petrolether/Essigester = 1:1)

| | | | |
|---|---|---|---|
| Ber. | C 65,73 | H 5,51 | N 28,74 |
| Gef. | 65,67 | 5,65 | 28,51 |

(25) 4-[(4-Methoxyphenyl)amino]-6-(cyclopropylamino)-pyrimido[5,4-d]pyrimidin
Schmelzpunkt: 187-189°C
$R_f$-Wert: 0,45 (Kieselgel; Petrolether/Essigester = 1:1)

| | | | |
|---|---|---|---|
| Ber. | C 62,32 | H 5,23 | N 27,25 |
| Gef. | 62,14 | 5,29 | 26,95 |

(26) 4-[(4-tert.Butylphenyl)amino]-6-(cyclopropylamino)-pyrimido[5,4-d]pyrimidin
Schmelzpunkt: 142-144°C
$R_f$-Wert: 0,55 (Kieselgel; Petrolether/Essigester = 1:1)

| | | | |
|---|---|---|---|
| Ber. | C 68,23 | H 6,63 | N 25,13 |
| Gef. | 68,16 | 6,77 | 24,72 |

(27) 4-[[3-(Cyclopentyloxy)phenyl]amino]-6-(cyclopropylamino)-pyrimido[5,4-d]pyrimidin

(28) 4-[(4-Bromphenyl)amino]-6-(cyclopropylamino)-pyrimido[5,4-d]pyrimidin
Schmelzpunkt: 229-231°C
$R_f$-Wert: 0,42 (Kieselgel; Petrolether/Essigester = 1:1)

| | | | |
|---|---|---|---|
| Ber. | C 50,42 | H 3,66 | N 23,52 |
| Gef. | 50,41 | 3,79 | 23,50 |

(29) 4-[(2-Methylphenyl)amino]-6-(cyclopropylamino)-pyrimido[5,4-d]pyrimidin
Schmelzpunkt: 218-220°C
$R_f$-Wert: 0,40 (Kieselgel; Petrolether/Essigester = 1:1)

| | | | |
|---|---|---|---|
| Ber. | C 65,73 | H 5,51 | N 28,74 |
| Gef. | 65,72 | 5,55 | 28,22 |

(30) 4-[(3,4-Dimethylphenyl)amino]-6-(cyclopropylamino)-pyrimido[5,4-d]pyrimidin
Schmelzpunkt: 180-182°C
$R_f$-Wert: 0,40 (Kieselgel; Petrolether/Essigester = 1:1)

| | | | |
|---|---|---|---|
| Ber. | C 66,64 | H 5,92 | N 27,43 |
| Gef. | 66,56 | 5,99 | 27,51 |

(31) 4-[(5-Indanyl)amino]-6-(cyclopropylamino)-pyrimido[5,4-d]pyrimidin
Schmelzpunkt: 199-201°C
$R_f$-Wert: 0,42 (Kieselgel; Petrolether/Essigester = 1:1)

(32) 4-[(2-Naphthyl)amino]-6-(cyclopropylamino)-pyrimido[5,4-d]pyrimidin
Schmelzpunkt: 187-189°C
$R_f$-Wert: 0,38 (Kieselgel; Petrolether/Essigester = 1:1)

| | | | |
|---|---|---|---|
| Ber. | C 69,49 | H 4,91 | N 25,59 |
| Gef. | 69,22 | 4,96 | 25,87 |

(33) 4-[(1,2,3,4-Tetrahydro-6-naphthyl)amino]-6-(cyclopropylamino)-pyrimido [5,4-d]pyrimidin

(34) 4-[(3,5-Dimethylphenyl)amino]-6-(cyclopropylamino)-pyrimido[5,4-d]pyrimidin
Schmelzpunkt: 210-212°C
$R_f$-Wert: 0,48 (Kieselgel; Petrolether/Essigester = 1:1)

| | | | |
|---|---|---|---|
| Ber. | C 66,64 | H 5,92 | N 27,43 |
| Gef. | 66,71 | 6,07 | 27,60 |

(35) 4-[(2,5-Dimethylphenyl)amino]-6-(cyclopropylamino)-pyrimido[5,4-d]pyrimidin
Schmelzpunkt: 210-212°C
$R_f$-Wert: 0,38 (Kieselgel; Petrolether/Essigester = 1:1)

| | | | |
|---|---|---|---|
| Ber. | C 66,64 | H 5,92 | N 27,43 |
| Gef. | 66,65 | 5,93 | 27,56 |

(36) 4-[(3,4-Dichlorphenyl)amino]-6-(cyclopropylamino)-pyrimido[5,4-d]pyrimidin

Schmelzpunkt: 228-230°C
$R_f$-Wert: 0,40 (Kieselgel; Petrolether/Essigester = 1:1)

| | | | |
|---|---|---|---|
| Ber. | C 51,88 | H 3,48 | N 24,20 |
| Gef. | 51,70 | 3,52 | 23,77 |

(37) 4-[(2-Fluorphenyl)amino]-6-(cyclopropylamino)-pyrimido[5,4-d]pyrimidin
Schmelzpunkt: 198-200°C
$R_f$-Wert: 0,41 (Kieselgel; Petrolether/Essigester = 1:1)

| | | | |
|---|---|---|---|
| Ber. | C 60,80 | H 4,42 | N 28,36 |
| Gef. | 60,60 | 4,61 | 28,16 |

(38) 4-[(2,5-Difluorphenyl)amino]-6-(cyclopropylamino)-pyrimido[5,4-d]pyrimidin
Schmelzpunkt: 238-240°C
$R_f$-Wert: 0,43 (Kieselgel; Petrolether/Essigester = 1:1)

| | | | |
|---|---|---|---|
| Ber. | C 57,32 | H 3,84 | N 26,73 |
| Gef. | 57,57 | 4,05 | 26,23 |

(39) 4-[(2-Fluor-5-methylphenyl)amino]-6-(cyclopropylamino)-pyrimido[5,4-d]pyrimidin
Schmelzpunkt: 200-202°C

| | | | |
|---|---|---|---|
| Ber. | C 61,92 | H 4,87 | N 27,08 |
| Gef. | 61,99 | 4,99 | 27,00 |

(40) 4-[(2-Fluor-3-trifluormethyl-phenyl)amino]-6-(cyclopropylamino)-pyrimido [5,4-d]pyrimidin
Schmelzpunkt: 204-206°C

| | | | |
|---|---|---|---|
| Ber. | C 52,75 | H 3,23 | N 23,06 |
| Gef. | 53,50 | 3,39 | 22,59 |

(41) 4-[(2-Fluor-3-methyl-phenyl)amino]-6-(cyclopropylamino)-pyrimido[5,4-d]pyrimidin

(42) 4-[(2-Fluor-5-trifluormethyl-phenyl)amino]-6-(cyclopropylamino)-pyrimido[5,4-d]pyrimidin
Schmelzpunkt: 210-212°C
$R_f$-Wert: 0,28 (Kieselgel; Petrolether/Essigester = 2:1)

| | | | |
|---|---|---|---|
| Ber. | C 52,75 | H 3,23 | N 23,06 |
| Gef. | 52,37 | 3,51 | 23,27 |

(43) 4-[(2,5-Difluor-4-methyl-phenyl)amino]-6-(cyclopropylamino)-pyrimido[5,4-d]pyrimidin

(44) 4-[(2,4-Difluor-3-methyl-phenyl)amino]-6-(cyclopropylamino)-pyrimido [5,4-d]pyrimidin

(45) 4-[(3-Nitrophenyl)amino]-6-(cyclopropylamino)-pyrimido[5,4-d]pyrimidin
Schmelzpunkt: 208-210°C
$R_f$-Wert: 0,32 (Kieselgel; Petrolether/Essigester = 1:1)

| | | | |
|---|---|---|---|
| Ber. | C 55,72 | H 4,05 | N 30,32 |
| Gef. | 55,58 | 4,12 | 30,42 |

(46) 4-[(3-Ethinylphenyl)amino]-6-(cyclopropylamino)-pyrimido[5,4-d)pyrimidin
Schmelzpunkt: 279-281°C
$R_f$-Wert: 0,43 (Kieselgel; Petrolether/Essigester = 2:1)

| | | | |
|---|---|---|---|
| Ber. | C 67,53 | H 4,66 | N 27,79 |
| Gef. | 67,48 | 4,76 | 28,14 |

(47) 4-(Phenylamino)-6-(morpholino)-pyrimido[5,4-d]pyrimidin Schmelzpunkt: 170-172°C
$R_f$-Wert: 0,47 (Kieselgel; Petrolether/Essigester = 1:2)

| | | | |
|---|---|---|---|
| Ber. | C 62,32 | H 5,23 | N 27,25 |
| Gef. | 62,31 | 5,38 | 27,17 |

(48) 4-[N-Methyl-N-(3-methylphenyl)-amino]-6-(morpholino)-pyrimido[5,4-d]pyrimidin
Schmelzpunkt: 71-73°C
$R_f$-Wert: 0,60 (Kieselgel; Petrolether/Essigester = 1:2)

| | | | |
|---|---|---|---|
| Ber. | C 64,26 | H 5,99 | N 24,98 |
| Gef. | 64,36 | 6,08 | 24,75 |

(49) 4-[N-Methyl-N-phenyl-amino]-6-(morpholino)-pyrimido[5,4-d]pyrimidin
Schmelzpunkt: 132-134°C
$R_f$-Wert: 0,38 (Kieselgel; Petrolether/Essigester = 1:2)

| | | | |
|---|---|---|---|
| Ber. | C 63,33 | H 5,62 | N 26,06 |
| Gef. | 63,59 | 5,79 | 25,82 |

(50) 4-[(3-Cyclopropylphenyl)amino]-6-(morpholino)-pyrimido[5,4-d]pyrimidin

(51) 4-[(3-Cyano-4-hydroxyphenyl)amino]-6-(morpholino)-pyrimido[5,4-d]pyrimidin

(52) 4-[(3-Cyano-4-aminophenyl)amino]-6-(morpholino)-pyrimido[5,4-d]pyrimidin

(53) 4-[(4-Hydroxy-3-nitrophenyl)amino]-6-(morpholino)-pyrimido[5,4-d]pyrimidin

(54) 4-[(4-Amino-3-nitrophenyl)amino]-6-(morpholino)-pyrimido[5,4-d]pyrimidin

(55) 4-[(3,4-Dichlorphenyl)amino]-6-methylthio-pyrimido[5,4-d]pyrimidin
Schmelzpunkt: 158-160°C
$R_f$-Wert: 0,48 (Kieselgel; Petrolether/Essigester = 2:1)

(56) 4-[(3-Chlor-4-fluor-phenyl)amino]-6-methylthio-pyrimido[5,4-d]-pyrimidin
Schmelzpunkt: 140-145°C (Zers.)
$R_f$-Wert: 0,54 (Kieselgel; Petrolether/Essigester = 10:7)
Diese Verbindung läßt sich auch auf folgendem Wege erhalten: Eine Mischung von 10 g 4-Hydroxy-6-methylthio-pyrimido[5,4-d]pyrimidin, 16 ml Hexamethyldisilazan, 22.5 g 3-Chlor-4-fluoranilin und 1 g p-Toluolsulfonsäure-Hydrat wird 12 h zum Sieden erhitzt. Dann werden 500 ml Methanol zugegeben und die Mischung eine weitere Stunde zum Sieden erhitzt. Das Lösungsmittel wird im Rotationsverdampfer abdestilliert, der Rückstand in Methylenchlorid gelöst, die Lösung mit 100 ml 2N Natronlauge extrahiert und über Magnesiumsulfat getrocknet. Nach dem Abdestillieren des Lösungsmittels im Rotationsverdampfer wird der Rückstand zweimal mit je 200 ml Ether verrieben, abfiltriert und mehrfach mit Ether gewaschen.
Nach dem Trocknen erhält man 11.5 g (69 % der Theorie) der Titelverbindung mit den oben angegebenen physikalischen Daten.

(57) 4-[(3-chlor-4-methoxy-phenyl)amino]-6-methylthio-pyrimido[5,4-d]pyrimidin
Schmelzpunkt: 154-156°C
$R_f$-Wert: 0,40 (Kieselgel; Petrolether/Essigester = 1:1)

(58) 4-[(4-Amino-3-nitro-phenyl)amino]-6-methylthio-pyrimido[5,4-d]pyrimidin

Schmelzpunkt: 248-250°C
$R_f$-Wert: 0,48 (Kieselgel; Petrolether/Essigester/Methanol = 10:10:1)

(59) 4-[(4-Chlor-3-nitro-phenyl)amino]-6-methylthio-pyrimido[5,4-d]pyrimidin
Schmelzpunkt: 173-175°C
$R_f$-Wert: 0,58 (Kieselgel; Petrolether/Essigester = 1:1)

(60) 4-[(4-Amino-3-cyano-phenyl)amino]-6-methylthio-pyrimido[5,4-d]pyrimidin
Hergestellt aus der Verbindung des Beispiels XVI.
Schmelzpunkt: 225-227°C

(61) 4-[(4-Amino-3,5-dichlor-phenyl)amino]-6-methylthio-pyrimido[5,4-d]pyrimidin
Schmelzpunkt: 195-197°C
$R_f$-Wert: 0,50 (Kieselgel; Petrolether/Essigester = 1:1)

(62) 4-[(4-Amino-3,5-dibrom-phenyl)amino]-6-methylthio-pyrimido[5,4-d]pyrimidin
Hergestellt aus der Verbindung des Beispiels XVII.
Schmelzpunkt: 245-247°C

(63) 4-[(2-Cyclopropylphenyl)amino]-6-(morpholino)-pyrimido[5,4-d]pyrimidin
Hergestellt aus der Verbindung des Beispiels XV.
Schmelzpunkt: 171-173°C

| | | | |
|---|---|---|---|
| Ber. | C 65,49 | H 5,78 | N 24,12 |
| Gef. | 65,24 | 5,84 | 24,06 |

Beispiel 4

4-(Phenylamino)-pyrimido [5,4-d]pyrimidin

Zu einer Mischung aus 10 ml Jodwasserstoffsäure (67%ig) und 2,4 g Diphosphortetrajodid werden bei 50°C 0,6 g 4-(Phenylamino)-2,8-dichlor-pyrimido[5,4-d]pyrimidin portionsweise unter Rühren zugegeben. Es wird 20 Minuten weitergerührt, auf Eis und Wasser gegossen und mit Natronlauge alkalisch gestellt. Das Gemisch wird dreimal mit Methylenchlorid extrahiert, die vereinigten Extrakte werden getrocknet und eingeengt. Der Rückstand wird in Dioxan gelöst, mit 0,5 g Palladium auf Kohle (10 % Palladium) versetzt und über Nacht unter Rückfluß erhitzt. Es wird vom Katalysator abfiltriert, das Filtrat wird eingeengt und der Rückstand durch Chromatographie über eine Aluminiumoxid-Säule mit Petrolether/Essigester (10:3) gereinigt.
Ausbeute: 0,07 g (16 % der Theorie),
Schmelzpunkt: 110-112°C
$R_f$-Wert: 0,55 (Aluminiumoxid; Petrolether/Essigester = 2:1)
Analog Beispiel 4 werden folgende Verbindungen erhalten:

(1) 4-[(3-Bromphenyl)amino]-pyrimido[5,4-d]pyrimidin
Schmelzpunkt: 208-210°C
$R_f$-Wert: 0,30 (Aluminiumoxid; Petrolether/Essigester = 2:1)

(2) 4-[(3-Chlorphenyl)amino]-pyrimido [5,4-d]pyrimidin Schmelzpunkt: 187-189°C
$R_f$-Wert: 0,35 (Aluminiumoxid; Petrolether/Essigester = 10:4)

(3) 4-[(3-Trifluormethylphenyl)amino]-pyrimido[5,4-d]pyrimidin
$R_f$-Wert: 0,41 (Aluminiumoxid; Petrolether/Essigester = 2:1)
Massenspektrum: $M^+$ = 291

(4) 4-[(3-Methylphenyl)amino]-pyrimido [5,4-d]pyrimidin Schmelzpunkt: 159-160°C
$R_f$-Wert: 0,25 (Kieselgel; Petrolether/Essigester = 2:1)

Beispiel 5

| Dragées mit 75 mg Wirksubstanz | | |
|---|---|---|
| 1 | Dragéekern enthält: | |
| | Wirksubstanz | 75,0 mg |
| | Calciumphosphat | 93,0 mg |
| | Maisstärke | 35,5 mg |
| | Polyvinylpyrrolidon | 10,0 mg |
| | Hydroxypropylmethylcellulose | 15,0 mg |
| | Magnesiumstearat | 1,5 mg |
| | | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht | 230 mg |
| Stempel | 9 mm, gewölbt |

Die so hergestellten Dragéekerne werden mit einem Film überzogen, der im wesentlichen aus Hydroxypropylmethylcellulose besteht. Die fertigen Filmdragees werden mit Bienenwachs geglänzt.

Dragéegewicht: 245 mg.

Beispiel 6

| Tabletten mit 100 mg Wirksubstanz | | |
|---|---|---|
| Zusammensetzung: | | |
| 1 | Tablette enthält: | |
| | Wirksubstanz | 100,0 mg |
| | Milchzucker | 80,0 mg |
| | Maisstärke | 34,0 mg |
| | Polyvinylpyrrolidon | 4,0 mg |
| | Magnesiumstearat | 2,0 mg |
| | | 220,0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht | 220 mg |
| Durchmesser | 10 mm, biplan mit beidseitiger Facette und einseitiger Teilkerbe. |

Beispiel 7

| Tabletten mit 150 mg Wirksubstanz | | |
|---|---|---|
| Zusammensetzung: | | |
| 1 | Tablette enthält: | |
| | Wirksubstanz | 150,0 mg |
| | Milchzucker pulv. | 89,0 mg |
| | Maisstärke | 40,0 mg |
| | Kolloide Kieselgelsäure | 10,0 mg |
| | Polyvinylpyrrolidon | 10,0 mg |
| | Magnesiumstearat | 1,0mg |
| | | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

Beispiel 8

| Hartgelatine-Kapseln mit 150 mg Wirksubstanz | | |
|---|---|---|
| 1 | Kapsel enthält: | |
| | Wirkstoff | 150,0 mg |
| | Maisstärke getr. ca. | 180,0 mg |
| | Milchzucker pulv. ca. | 87,0 mg |
| | Magnesiumstearat | 3,0mg |
| | ca. | 420,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

Beispiel 9

| Suppositorien mit 150 mg Wirksubstanz | | |
|---|---|---|
| 1 | Zäpfchen enthält: | |
| | Wirkstoff | 150,0 mg |
| | Polyäthylenglykol 1500 | 550,0 mg |
| | Polyäthylenglykol 6000 | 460,0 mg |
| | Polyoxyäthylensorbitanmonostearat | 840,0 mg |
| | | 2 000,0 mg |

Herstellung:

Nach dem Aufschmelzen der Suppositorienmasse wird der Wirkstoff darin homogen verteilt und die Schmelze in vorgekühlte Formen gegossen.

Beispiel 10

| Suspension mit 50 mg Wirksubstanz | | |
|---|---|---|
| 100 | ml Suspension enthalten: | |
| | Wirkstoff | 1,00 g |
| | Carboxymethylcellulose-Na-Salz | 0,10 g |
| | p-Hydroxybenzoesäuremethylester | 0,05 g |
| | p-Hydroxybenzoesäurepropylester | 0,01 g |
| | Rohrzucker | 10,00 g |
| | Glycerin | 5,00 g |
| | Sorbitlösung 70%ig | 20,00 g |
| | Aroma | 0,30 g |
| | Wasser dest. ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

Beispiel 11

| Ampullen mit 10 mg Wirksubstanz | |
|---|---|
| Zusammensetzung: | |
| Wirkstoff | 10,0 mg |
| 0,01 n Salzsäure s.q. | |
| Aqua bidest ad | 2,0 ml |

Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

Beispiel 12

| Ampullen mit 50 mg Wirksubstanz | |
|---|---|
| Zusammensetzung: | |
| Wirkstoff | 50,0 mg |
| 0,01 n Salzsäure s.q. | |
| Aqua bidest ad | 10,0 ml |

Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

**Patentansprüche**

**1.** Pyrimido[5,4-d]pyrimidine der allgemeinen Formel

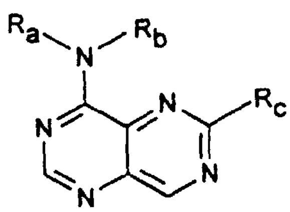

, (I)

in der

$R_a$ ein Wasserstoffatom oder eine Alkylgruppe,

$R_b$ eine durch die Reste $R_1$ bis $R_3$ substituierte Phenylgruppe, wobei

$R_1$ und $R_2$, die gleich oder verschieden sein können, jeweils ein Wasserstoff-, Fluor-, Chlor-, Brom- oder Jodatom,

eine $C_{1-6}$-Alkyl-, Hydroxy- oder $C_{1-6}$-Alkoxygruppe,

eine $C_{3-7}$-Cycloalkyl- oder $C_{4-7}$-Cycloalkoxygruppe, die jeweils durch eine oder zwei Alkylgruppen oder durch eine Arylgruppe substituiert sein können,

eine gegebenenfalls durch eine Arylgruppe substituierte $C_{2-5}$-Alkenyl- oder $C_{3-5}$-Alkenyloxygruppe, wobei der Vinylteil nicht mit dem Sauerstoffatom verknüpft sein kann,

eine gegebenenfalls durch eine Arylgruppe substituierte $C_{2-5}$-Alkinyl- oder $C_{3-5}$-Alkinyloxygruppe, wobei der Ethinylteil nicht mit dem Sauerstoffatom verknüpft sein kann,

eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfinyl-, Trifluormethylsulfonyl-, Arylsulfenyl-, Arylsulfinyl-, Arylsulfonyl-, Aralkylsulfenyl-, Aralkylsulfinyl- oder Aralkylsulfonylgruppe,

eine durch 1 bis 3 Fluoratome substituierte Methyl- oder Methoxygruppe,

eine durch 1 bis 5 Fluoratome substituierte $C_{2-4}$-Alkyl- oder $C_{2-4}$-Alkbxygruppe,

eine Nitro-, Amino-, Alkylamino-, Dialkylamino-, $C_{3-7}$-Cycloalkylamino-, N-Alkyl-$C_{3-7}$-cycloalkylamino-, Arylamino-, N-Alkyl-arylamino-, Aralkylamino- oder N-Alkyl-aralkylaminogruppe,

eine gegebenenfalls durch 1 bis 4 Alkylgruppen substituierte 4-bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein können,

eine gegebenenfalls durch 1 bis 4 Alkylgruppen substituierte (Alkylenimino)carbonyl- oder (Alkylenimino)sulfonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,

eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Perfluoralkylsulfonylamino-, N-Alkyl-perfluoralkylsulfonylamino-, Alkylcarbonyl-, Arylcarbonyl-, Aralkylcarbonyl-, Arylhydroxymethyl-, Aralkyl-hydroxymethyl-, Carboxy-, Alkoxycarbonyl-, Aralkoxycarbonyl-, Aminocarbonyl-, Alkylaminocarbonyl-, Dialkylaminocarbonyl-, Arylaminocarbonyl-, N-Alkyl-arylaminocarbonyl-, Aralkyl aminocarbonyl - , N-Alkylaralkylaminocarbonyl-, N-Hydroxy-aminocarbonyl-, N-Hydroxyalkylaminocarbonyl-, N-Alkoxy-aminocarbonyl-, N-Alkoxy-alkylaminocarbonyl-, Cyano-, Azido-, N-Cyano-amino- oder N-Cyanoalkylaminogruppe,

eine Sulfo-, Alkoxysulfonyl-, Aminosulfonyl-, Alkylaminosulfonyl-, Dialkylaminosulfonyl-, Arylaminosulfonyl-, N-Alkylarylaminosulfonyl-, Aralkylaminosulfonyl- oder N-Alkyl-aralkylaminosulfonylgruppe,

eine Phosphono-, O-Alkyl-phosphono-, O,O'-Dialkyl-phosphono-, O-Aralkyl-phosphono- oder O,O'-Diaralkylphosphonogruppe,

eine durch $R_4$ substituierte Alkyl-oder Alkoxygruppe, wobei

> $R_4$ eine Hydroxy-, Alkoxy-, Aryloxy-, Aralkoxy-, Amino-, Alkylamino-, Dialkylamino-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, Carboxy-, Alkoxycarbonyl-, Aminocarbonyl-, Alkylaminocarbonyl-, Dialkylaminocarbonyl- oder Cyanogruppe,
>
> eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe oder in den vorstehend erwähnten 6- bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein können, oder
>
> eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann, darstellt,

$R_3$ ein Wasserstoff-, Fluor-, Chlor- oder Bromatom, eine Alkyl-, Alkoxy- oder Trifluormethylgruppe oder

$R_2$ zusammen mit $R_3$, sofern diese an benachbarte Kohlenstoffatome gebunden sind, auch eine gegebenenfalls durch eine oder zwei Alkylgruppen substituierte Methylendioxygruppe, eine gegebenenfalls durch ein oder zwei Alkylgruppen substituierte n-$C_{3-6}$-Alkylengruppe oder eine gegebenenfalls durch ein oder zwei Fluor-, Chlor-, Brom- oder Jodatome, durch eine oder zwei Hydroxy-, Alkyl-, Alkoxy-, Trifluormethyl- oder Cyangruppen substituierte 1,3-Butadien-1,4-diylengruppe, wobei die Substituenten gleich oder verschieden sein können, oder

$R_a$ zusammen mit $R_1$, sofern $R_1$ in o-Stellung zu dem durch $R_a$ substituierten Stickstoffatom steht, auch eine gegebenenfalls durch eine oder zwei Alkylgruppen substituierte n-$C_{2-4}$-Alkylengruppe darstellen, und

$R_c$ ein Wasserstoff-, Fluor-, Chlor-, Brom- oder Jodatom,

eine Alkyl-, $C_{3-7}$-Cycloalkyl-, $C_{3-7}$-Cycloalkyl-$C_{1-3}$-alkyl-, Aryl-, Aralkyl-, Hydroxy-, Aryloxy-, Aralkoxy-, Mercapto-, $C_{1-8}$-Alkylsulfenyl-, $C_{1-8}$-Alkylsulfinyl-, $C_{1-8}$-Alkylsulfonyl-, $C_{4-7}$-Cycloalkylsulfenyl-, $C_{4-7}$-Cycloalkylsulfinyl-, $C_{4-7}$-Cycloalkylsulfonyl-, $C_{3-7}$-Cycloalkyl-$C_{1-3}$-alkylsulfenyl-, $C_{3-7}$-Cycloalkyl-$C_{1-3}$-alkylsulfinyl-, $C_{3-7}$-Cycloalkyl-$C_{1-3}$-alkylsulfonyl-, Arylsulfenyl-, Arylsulfinyl-, Arylsulfonyl-, Aralkylsulfenyl-, Aralkylsulfinyl- oder Aralkylsulfonylgruppe,

eine $C_{1-8}$-Alkoxygruppe, die durch eine Alkoxycarbonyl-, Cyano-, Carboxy-, Aminocarbonyl-, Alkylaminocarbonyl- oder Dialkylaminocarbonylgruppe substituiert sein kann,

eine $C_{2-8}$-Alkoxygruppe, die durch eine Hydroxy-, Alkoxy-, Hydroxy-$C_{2-4}$-alkylamino-, Amino-, Alkylamino-, Dialkylamino-, Alkylcarbonylamino-, N-Alkyl-N-(alkylcarbonyl)amino-, Alkylsulfonylamino-, N-Alkyl-N-(alkylsulfonyl)amino-, Alkoxycarbonylamino- oder N-Alkyl-N-(alkoxycarbonyl) aminogruppe, durch eine gegebenenfalls durch 1 bis 4 Alkylgruppen substituierte 5-bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe und zusätzlich in den vorstehend erwähnten 6- bis 7-gliedrigen Alkyleniminogruppen eine Methylengruppe in 4-Stellung durch ein Sauerstoff- oder Schwefelatom, durch eine Carbonyl-, Sulfinyl-, Sulfonyl-, Imino-, N-Alkyl-imino-, N-Alkylcarbonyl-imino-, N-Formyl-imino-, N-Di-alkylaminocarbonyl-imino-, N-Alkoxycarbonyl-imino-, N-Alkylsulfonyl-imino-, N-Aryl-imino- oder N-Aralkyl-iminogruppe ersetzt sein kann, substituiert ist,

eine durch zwei Hydroxy- oder Alkoxygruppen substituierte $C_{3-8}$-Alkoxygruppe,

eine durch eine $C_{3-7}$-Cycloalkylgruppe substituierte $C_{1-8}$-Alkoxygruppe, wobei der Cycloalkylteil jeweils durch 1 bis 4 Alkylgruppen substituiert sein kann und wobei in den vorstehend erwähnten $C_{4-7}$-Cycloalkylteilen jeweils eine Methylengruppe durch ein Sauerstoff- oder Schwefelatom, durch eine Carbonyl-, Sulfinyl-, Sulfonyl-, Imino-, N-Alkyl-imino-, N-Alkylcarbonyl-imino-, N-Alkoxycarbonyl-imino-, N-Alkylsulfonyl-imino-, N-Aryl-imino- oder N-Aralkyl-iminogruppe ersetzt sein kann,

eine gegebenenfalls durch eine oder zwei Hydroxygruppen oder durch eine Alkoxy-, Alkoxycarbonyl-, Cyano-, Carboxy-, Aminocarbonyl-, Alkylaminocarbonyl-, Dialkylaminocarbonyl-, Hydroxy-$C_{2-4}$-alkylamino-, Amino-, Alkylamino-, Dialkylamino-, Alkylcarbonylamino-, N-Alkyl-N-(alkylcarbonyl)amino-, Alkylsulfonylamino-, N-Alkyl-N-(alkylsulfonyl)amino-, Alkoxycarbonylamino- oder N-Alkyl-N-(alkoxycarbonyl)aminogruppe substituierte $C_{4-7}$-Cycloalkoxygruppe, wobei in den vorstehend erwähnten $C_{5-7}$-Cycloalkoxygruppen jeweils eine Methylengruppe durch ein Sauerstoff- oder Schwefelatom, durch eine Sulfinyl-, Sulfonyl-, Imino-, N-Alkyl-imino-, N-Alkylcarbonyl-imino-., N-Alkoxycarbonyl-imino-, N-Alkylsulfonyl-imino-, N-Aryl-imino- oder N-Aralkyl-iminogruppe ersetzt sein kann,

eine gegebenenfalls durch eine Arylgruppe oder $C_{3-7}$-Cycloalkylgruppe substituierte $C_{3-8}$-Alkenyloxygruppe, wobei der Vinylteil nicht mit dem Sauerstoffatom verknüpft sein kann,

eine gegebenenfalls durch eine Arylgruppe oder $C_{3-7}$-Cycloalkylgruppe substituierte $C_{3-8}$-Alkinyloxygruppe, wobei der Ethinylteil nicht mit dem Sauerstoffatom verknüpft sein kann,

eine gegebenenfalls durch 1 bis 4 Alkylgruppen oder 1 bis 2 Arylgruppen substituierte 4- bis 8-gliedrige Alkyleniminogruppe, die zusätzlich durch den Rest $R_5$ substituiert sein kann, wobei

$R_5$ eine Aryl-, Aralkyl-, Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonyloxy-, Arylcarbonyloxy-, Amino-, Alkylamino-, Hydroxy-$C_{2-4}$-alkylamino-, Dialkylamino-, Cyanamino-, Formylamino-, N-(Alkyl)-N-(hydroxy-$C_{2-4}$-alkyl)amino- oder Bis-(hydroxy-$C_{2-4}$-alkyl)aminogruppe,

eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,

eine gegebenenfalls durch 1 bis 4 Alkylgruppen oder eine Hydroxyalkylgruppe substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe ersetzt sein können,

eine gegebenenfalls durch 1 bis 4 Alkylgruppen oder eine Hydroxyalkylgruppe substituierte 6- oder 7-gliedrige Alkyleniminogruppe, wobei jeweils eine Methylengruppe in 4-Stellung des Alkyleniminoteils durch ein Sauerstoff- oder Schwefelatom, durch eine Carbonyl-, Sulfinyl-, Sulfonyl-, Imino-, N-Alkylimino-, N-Alkylcarbonyl-imino-, N-Alkylsulfonyl-imino-, N-Arylimino- oder N-Aralkyl-iminogruppe ersetzt ist und zusätzlich im Alkyleniminoteil der vorstehend erwähnten Gruppen jeweils eine oder zwei der zu den Stickstoffatomen benachbarten Methylengruppen durch eine Carbonylgruppe ersetzt sein können,

eine durch eine Hydroxy-, Alkoxy-, Amino-, Alkylamino-, Dialkylamino-,Alkylcarbonylamino-, N-Alkyl-alkylcarbonylamino-, Alkylsulfonylamino-, N-Alkyl-alkylsulfonylamino- oder eine Hydroxyalkylgruppe substituierte 4- bis 7-gliedrige Alkyleniminogruppe,

eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe,

eine $(NR_7R_8)CONR_6$- oder $(NR_7R_8)SO_2NR_6$-Gruppe, in denen

$R_6$, $R_7$ und $R_8$, die gleich oder verschieden sein können, jeweils ein Wasserstoffatom oder eine Alkylgruppe oder $R_6$ und $R_7$ zusammen eine n-$C_{2-4}$-Alkylengruppe und $R_8$ ein Wasserstoffatom oder eine Alkylgruppe darstellen,

eine Carboxyalkyl-, Alkoxycarbonylalkyl-, Aminocarbonylalkyl-, Alkylaminocarbonylalkyl- oder Dialkylaminocarbonylalkylgruppe,

eine gegebenenfalls durch 1 bis 4 Alkylgruppen substituierte (Alkylenimino)carbonylalkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,

eine (carboxyalkyl)oxy-, (Alkoxycarbonylalkyl)oxy-, (Aminocarbonylalkyl)oxy-, (Alkylaminocarbonylalkyl) oxy- oder (Dialkylaminocarbonylalkyl)oxy-gruppe,

eine gegebenenfalls durch 1 bis 4 Alkylgruppen substituierte [(Alkylenimino)carbonylalkyl]oxy-gruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,

eine Cyanoalkyl-, Hydroxyalkyl-, Alkoxyalkyl-, Aryloxyalkyl-, Aminoalkyl-, Alkylaminoalkyl- oder Dialkylaminoalkylgruppe,

eine gegebenenfalls durch 1 bis 4 Alkylgruppen substituierte (Alkylenimino)alkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,

eine Alkylcarbonylaminoalkyl-, N-Alkyl-alkylcarbonylaminoalkyl-, Alkylsulfonylaminoalkyl-, N-Alkyl-alkylsulfonylaminoalkyl-, Arylcarbonylaminoalkyl-, N-Alkyl-arylcarbonylaminoalkyl-, Arylsulfonylaminoalkyl-, N-Alkyl-arylsulfonylaminoalkyl-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, Alkylsulfenylalkyl-, Alkylsulfinylalkyl-, Alkylsulfonylalkyl-, Arylsulfenylalkyl-, Arylsulfinylalkyl- oder Arylsulfonylalkylgruppe oder

eine $C_{3-7}$-Cycloalkylgruppe, wobei in einer $C_{5-7}$-Cycloalkylgruppe eine Methylengruppe durch ein Sauerstoff- oder Schwefelatom, durch eine Sulfinyl-, Sulfonyl-, Imino- oder N-Alkyl-iminogruppe ersetzt sein kann, darstellt,

oder $R_c$ eine gegebenenfalls durch 1 bis 4 Alkylgruppen oder durch eine Arylgruppe substituierte 6- bis 8-gliedrige Alkyleniminogruppe, die zusätzlich durch den Rest $R_5$ substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl-, N-Oxido-N-alkylimino- oder $R_9$N-Gruppe ersetzt ist, wobei

$R_9$ ein Wasserstoffatom, eine Alkyl-, Hydroxy-$C_{2-4}$-alkyl-, Alkoxy-$C_{2-4}$-alkyl-, Amino-$C_{2-4}$-alkyl-, Alkylamino-$C_{2-4}$-alkyl-, Dialkylamino-$C_{2-4}$-alkyl-, (Hydroxy-$C_{2-4}$-alkoxy)$C_{2-4}$-alkyl-, Aminocarbonylalkyl-, Alkylaminocarbonylalkyl-, Dialkylaminocarbonylalkyl-, Aryl-, Aralkyl-, Formyl-, Alkylcarbonyl-, Alkylsulfonyl-, Arylcarbonyl-, Aryl-sulfonyl-, Aralkylcarbonyl-, Aralkylsulfonyl-, Alkoxycarbonyl-, Cyano-, Aminocarbonyl-, Alkylaminocarbonyl- oder Dialkylaminocarbonylgruppe oder eine (Alkylenimino)carbonylgruppe mit jeweils 4 bis 7 Ringatomen im Alkyleniminoteil, wobei in einem 6- bis 7-gliedrigen Alkyleniminoteil eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann, darstellt,

oder $R_c$ eine gegebenenfalls durch 1 bis 4 Alkylgruppen substituierte 1-Pyrrolidinylgruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 2 bis 4 Kohlenstoffatome enthält, wenn sich die zwei Wasserstoffatome an Kohlenstoffatomen befinden, die durch ein Atom getrennt sind, wobei die vorstehend erwähnten 1-Pyrrolidinylgruppen zusätzlich durch den Rest $R_5$, der wie vorstehend erwähnt definiert ist, substituiert sein können,

eine gegebenenfalls durch 1 bis 4 Alkylgruppen substituierte 1-Piperidinyl- oder 1-Azacyclohept-1-yl-gruppe, in denen zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 1 bis 4 Kohlenstoffatome enthält, wenn sich die zwei Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind, wobei die vorstehend erwähnten 1-Piperidinyl- und 1-Azacyclohept-1-yl-gruppen zusätzlich durch den Rest $R_5$, der wie vorstehend erwähnt definiert ist, substituiert sein können,

eine gegebenenfalls durch 1 bis 4 Alkylgruppen substituierte 1-Pyrrolidinylgruppe, in der zwei Wasserstoffatome in 3-Stellung durch eine $-O-CH_2CH_2-O-$ oder $-O-CH_2CH_2CH_2-O-$Gruppe substituiert sind,

eine gegebenenfalls durch 1 bis 4 Alkylgruppen substituierte 1-Piperidinyl- oder 1-Azacyclohept-1-yl-gruppe, in denen in 3-Stellung oder in 4-Stellung jeweils zwei Wasserstoffatome durch eine $-O-CH_2CH_2-O-$ oder $-O-CH_2CH_2CH_2-O-$Gruppe substituiert sind,

eine Gruppe der Formel

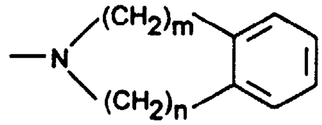

in der

m und n, die gleich oder verschieden sein können, die Zahlen 1 bis 3 oder
m die Zahl 0 und n die Zahl 2, 3 oder 4 bedeuten, wobei zusätzlich der obige Benzoteil durch Fluor-, Chlor-, Brom- oder Jodatome, durch Alkyl-, Trifluormethyl-, Hydroxy-, Alkoxy- oder Cyanogruppen und der obige gesättigte cyclische Iminoteil durch 1 oder 2 Alkylgruppen, wobei die Substituenten jeweils gleich oder verschieden sein können, mono- oder disubstituiert sein kann, oder

eine ($R_{10}NR_{11}$)-Gruppe, in der

$R_{10}$ und $R_{11}$ die gleich oder verschieden sein können, jeweils ein Wasserstoffatom,

eine $C_{1-16}$-Alkylgruppe, die durch 1 oder 2 Aryl- oder $C_{3-7}$-Cycloalkylgruppen, durch eine Carboxy-, Alkoxycarbonyl-, Aminocarbonyl-, Alkylaminocarbonyl-, Dialkylaminocarbonyl-, Hydroxy$C_{2-4}$-alkylaminocarbonyl-, Cyano-, Hydroxy-, Alkoxy-, Aryloxy-, Aralkoxy-, $C_{2-4}$-Alkylendioxy-, Alkylcarbonyloxy-, Arylcarbonyloxy-, Formylamino-, Amino-, Alkylamino- oder Dialkylaminogruppe, durch eine gegebenenfalls durch 1 bis 4 Alkylgruppen substituierte (Alkylenimino)carbonylgruppe mit jeweils 4 bis 7 Ringatomen im Alkyleniminoteil, wobei in einem 6- oder 7-gliedrigen Alkyleniminoteil jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonylimino-, N-Alkylsulfonyl-imino-, N-Aryl-imino- oder N-Aralkyl-iminogruppe ersetzt sein kann,

durch eine gegebenenfalls durch 1 bis 4 Alkylgruppen substituierte 4- bis 7-gliedrige Alkyleniminogruppe, wobei in den vorstehend erwähnten 6- oder 7-gliedrigen Alkyleniminogruppen jeweils eine Methylengruppe in 4-Stellung durch ein Sauerstoff- oder Schwefelatom, durch eine Sulfinyl-, Sulfonyl- oder $R_9N$-Gruppe ersetzt sein kann, wobei $R_9$ wie eingangs definiert ist, und zusätzlich in den vorstehend erwähnten 5- bis 7-gliedrigen Alkyleniminogruppen jeweils eine oder zwei zu den Stickstoffatomen benachbarte Methylengruppen durch eine Carbonylgruppe ersetzt sein können,

durch eine Alkylsulfonylamino-, N-Alkyl-alkylsulfonylamino-, Arylcarbonylamino-, N-Alkyl-arylcarbonylamino-, Alkylcarbonylamino-, N-Alkyl-alkylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe, durch eine ($R_8NR_7$)-CO-$NR_6$- oder ($R_8NR_7$)-$SO_2$- $NR_6$-Gruppe, wobei $R_6$, $R_7$ und $R_8$ wie eingangs definiert sind, durch eine Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe, durch eine durch $R_5$ und gegebenenfalls zusätzlich durch 1 bis 4 Alkylgruppen substituierte $C_{4-7}$-Cycloalkylgruppe, wobei $R_5$ wie eingangs definiert ist, durch eine gegebenenfalls durch 1 bis 4 Alkylgruppen substituierte $C_{5-7}$-Cycloalkylgruppe, in der eine Methylengruppe durch ein Sauerstoff- oder Schwefelatom, durch eine Sulfinyl-, Sulfonyl- oder $NR_9$-Gruppe ersetzt ist oder durch ein Fluor-, Chlor-, Brom- oder Jodatom substituiert sein kann,

eine durch 2 oder 3 Fluoratome substituierte $C_{2-10}$-Alkylgruppe,

eine durch 4 oder 5 Fluoratome substituierte $C_{3-10}$-Alkylgruppe,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine durch 2 bis 5 Hydroxy- oder Alkoxygruppen substituierte $C_{3-10}$-Alkylgruppe,

eine durch eine Arylgruppe und eine Hydroxygruppe substituierte $C_{2-6}$-Alkylgruppe, die zusätzlich durch eine Amino-, Alkylamino-, Dialkylamino-, Hydroxy- oder Alkoxygruppe substituiert sein kann,

eine durch eine Amino-, Alkylamino-, Dialkylamino-, Alkylcarbonylamino- oder Alkoxycarbonylaminogruppe und zusätzlich durch eine Hydroxy- oder Alkoxygruppe substituierte $C_{3-6}$-Alkylgruppe,

eine gegebenenfalls durch eine Arylgruppe oder $C_{3-7}$-Cycloalkylgruppe substituierte Alkenyl- oder Alkinylgruppe mit jeweils 3 bis 6 Kohlenstoffatomen, wobei der Vinyl- oder Ethinylteil nicht mit dem Stickstoffatom verknüpft sein kann,

eine $C_{2-4}$-Alkylgruppe, die durch eine $C_{2-4}$-Alkoxygruppe substituiert ist, welche in ω-Position durch eine Hydroxy- oder Alkoxygruppe substituiert ist,

eine Arylgruppe,

eine Cyclopropylgruppe, die durch 1 oder 2 Alkylgruppen, durch eine Aryl-, Carboxy-, Alkoxycarbonyl-, Aminocarbonyl-, Alkylaminocarbonyl- oder Dialkylaminocarbonylgruppe oder durch eine gegebenenfalls durch 1 bis 4 Alkylgruppen substituierte (Alkylenimino)carbonylgruppe mit jeweils 4 bis 7 Ringatomen im Alkyleniminoteil substituiert sein kann, wobei in den vorstehend erwähnten 6- oder 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkylimino-, N-Alkylcarbonyl-imino-, N-Alkylsulfonyl-imino-, N-Aryl-imino- oder N-Aralkyl-iminogruppe ersetzt sein kann,

eine gegebenenfalls durch 1 bis 4 Alkylgruppen substituierte $C_{4-7}$-Cycloalkylgruppe, die zusätzlich durch $R_5$, der wie eingangs definiert ist, substituiert sein kann,

eine gegebenenfalls durch 1 bis 2 Alkylgruppen substituierte $C_{5-7}$-Cycloalkylgruppe, die zusätzlich durch eine N,N-Dialkyl-N-oxido-aminogruppe substituiert ist,

eine gegebenenfalls durch 1 bis 4 Alkylgruppen substituierte $C_{5-7}$-Cycloalkenylgruppe, wobei der Vinylteil nicht mit dem Stickstoffatom der ($R_{11}NR_{10}$)-Grupppe verknüpft sein kann,

eine gegebenenfalls durch 1 bis 4 Alkylgruppen substituierte $C_{4-7}$-Cycloalkylgruppe, die zusätzlich durch $R_5$ substituiert sein kann, wobei in dem Cycloalkylteil eine Methylengruppe durch ein Sauerstoff- oder Schwefelatom, durch eine Sulfinyl-, Sulfonyl-, N-Alkyl-N-oxido-imino- oder $R_9$N-Gruppe ersetzt ist, wobei $R_5$ und $R_9$ wie eingangs definiert sind,

eine gegebenenfalls durch 1 bis 4 Alkylgruppen substituierte $C_5$-$C_7$-Cycloalkyl- oder $C_5$-$C_7$-cycloalkylalkylgruppe, in denen jeweils eine Methylengruppe im Cycloalkylteil durch eine Carbonylgruppe ersetzt ist,

eine gegebenenfalls durch 1 bis 4 Alkylgruppen substituierte Cyclopentyl- oder Cyclopentylalkylgruppe, in denen jeweils zwei Wasserstoffatome im Cyclopentylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 2 bis 4 Kohlenstoffatome enthält, wenn sich die zwei Wasserstoffatome an Kohlenstoffatomen befinden, die durch ein Kohlenstoffatom getrennt sind, wobei die vorstehend erwähnten Ringe zusätzlich durch den Rest $R_5$, der wie eingangs definiert ist, substituiert sein können,

eine gegebenenfalls durch 1 bis 4 Alkylgruppen substituierte Cyclohexyl-, Cyclohexylalkyl-, Cycloheptyl- oder Cycloheptylalkylgruppe, in denen jeweils zwei Wasserstoffatome im Cycloalkylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 1 bis 4 Kohlenstoffatome enthält, wenn sich die zwei Wasserstoffatome an Kohlenstoffatomen befinden, die durch ein Kohlenstoffatom getrennt sind, oder 1 bis 3 Kohlenstoffatome enthält, wenn sich die zwei Wasserstoffatome an Kohlenstoffatomen befinden, die durch zwei Kohlenstoffatome getrennt sind, wobei die vorstehend erwähnten Ringe zusätzlich durch den Rest $R_5$, der wie eingangs definiert ist, substituiert sein können,

eine gegebenenfalls durch 1 bis 4 Alkylgruppen substituierte 3-Cyclohexen-1-yl- oder 3-Cyclohexen-1-yl-alkylgruppe, in denen im Cyclohexenylteil zwei Wasserstoffatome in 2,5-Stellung durch eine n-$C_{1-3}$-Alkylenbrükke ersetzt sind,

eine 3-Chinuclidinyl-, 4-Chinuclidinyl-, 2-Chinuclidinylalkyl-, 3-Chinuclidinyl-alkyl-, 4-Chinuclidinyl-alkyl-, Azabicyclo[2.2.1]hept-4-yl-, Azabicyclo[2.2.1]hept-4-yl-alkyl- oder Adamantylgruppe, oder

$R_{10}$ ein Wasserstoffatom oder eine Alkylgruppe und $R_{11}$ eine Hydroxy-, Alkoxy- oder Cyanogruppe darstellen, bedeuten,

deren Tautomeren, deren Stereoisomere und deren Salze,

wobei, sofern nichts anderes erwähnt wurde,

unter den bei der Definition der vorstehend erwähnten Reste erwähnten Arylteilen eine Phenylgruppe zu verstehen ist, die jeweils durch $R_{12}$ monosubstituiert, durch $R_{13}$ mono-, di- oder trisubstituiert oder durch $R_{12}$ monosubstituiert und zusätzlich durch $R_{13}$ mono- oder disubstituiert sein kann, wobei die Substituenten gleich oder verschieden sein können, und

$R_{12}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Perfluoralkoxy-, Nitro-, Amino-, Alkylcarbonylamino-, N-Alkyl-alkylcarbonylamino-, Alkylamino-, Dialkylamino-, Hydroxy-$C_{2-4}$-alkylamino-, N-Alkyl- (hydroxy-$C_{2-4}$-alkyl)amino-, Bis- (hydroxy-$C_{2-4}$-alkyl) amino-, Phenylalkylcarbonylamino-, Phenylcarbonylamino-, Alkylsulfonylamino-, Phenylalkylsulfonylamino-, Phenylsulfonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl-, Dialkylaminosulfonyl-, $(R_8NR_7)$-CO-$NR_6$- oder $(R_8NR_7)$- $SO_2$-$NR_6$-Gruppe, wobei $R_6$, $R_7$ und $R_8$ wie eingangs definiert sind,

eine gegebenenfalls durch 1 bis 4 Alkylgruppen oder eine Hydroxyalkylgruppe substituierte 5- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoffatom oder eine $R_9$N-Gruppe ersetzt sein kann, wobei $R_9$ wie eingangs definiert ist,

eine gegebenenfalls durch 1 bis 4 Alkylgruppen oder eine Hydroxyalkylgruppe substituierte 5- bis 7-gliedrige Alkyleniminogruppe, wobei jeweils eine oder zwei zu dem Stickstoffatom benachbarte Methylengruppen jeweils durch eine Carbonylgruppe ersetzt ist, und

$R_{13}$ eine Alkyl-, Hydroxy- oder Alkoxygruppe, ein Fluor-, Chlor-, Brom- oder Jodatom darstellen, wobei zwei Reste $R_{13}$, sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

sowie, soweit nichts anderes erwähnt wurde, die vorstehend erwähnten Alkyl-, Alkylen- und Alkoxyteile jeweils 1 bis 4 Kohlenstoffatome enthalten.

**2.** Pyrimido[5,4-d]pyrimidine der allgemeinen Formel I gemäß Anspruch 1 mit der Maßgabe, daß, sofern nichts anderes erwähnt wurde, jedes Kohlenstoffatom in den im Anspruch 1 erwähnten Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann,

deren Tautomeren, deren Stereoisomere und deren Salze.

**3.** Pyrimido[5,4-d]pyrimidine der allgemeinen Formel I gemäß Anspruch 1 oder 2, in der

$R_a$ ein Wasserstoffatom oder eine Alkylgruppe,

$R_b$ eine durch die Reste $R_1$ bis $R_3$ substituierte Phenylgruppe, wobei

$R_1$ ein Wasserstoff-, Fluor-, Chlor-, Brom- oder Jodatom,

eine $C_{1-6}$-Alkyl-, Hydroxy- oder $C_{1-6}$-Alkoxygruppe,

eine $C_{3-6}$-Cycloalkyl- oder $C_{5-6}$-Cycloalkoxygruppe,

eine $C_{2-5}$-Alkenyl- oder $C_{3-5}$-Alkenyloxygruppe, wobei der Vinylteil nicht mit dem Sauerstoffatom verknüpft sein kann,

eine $C_{2-5}$-Alkinyl- oder $C_{3-5}$-Alkinyloxygruppe, wobei der Ethinylteil nicht mit dem Sauerstoffatom verknüpft sein kann,

eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfonyl-, Arylsulfenyl-, Arylsulfinyl-, Arylsulfonyl-, Aralkylsulfenyl-, Aralkylsulfinyl- oder Aralkylsulfonylgruppe,

eine durch 1 bis 3 Fluoratome substituierte Methyl- oder Methoxygruppe,

eine durch 1 bis 5 Fluoratome substituierte $C_{2-4}$-Alkyl- oder $C_{2-4}$-Alkoxygruppe,

eine Nitro-, Amino-, Alkylamino-, Dialkylamino-, $C_{3-6}$-Cycloalkylamino-, N-Alkyl-$C_{3-6}$-cycloalkylamino-, Arylamino-, N-Alkyl-arylamino-, Aralkylamino- oder N-Alkyl-aralkylaminogruppe,

eine 5- bis 7-gliedrige Alkyleniminogruppe, wobei jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoffatom, durch eine Imino- oder N-Alkyl-iminogruppe ersetzt sein können,

eine (Alkylenimino)carbonyl- oder (Alkylenimino)sulfonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoffatom, durch eine Imino- oder N-Alkyl-iminogruppe ersetzt sein kann,

eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralakylcarbonylamino-, Aralkylsulfonylamino-, N-Alkyl-aralkylsulfonylamino-, Trifluormethylsulfonylamino-, N-Alkyl-trifluormethylsulfonylamino-, Alkylcarbonyl-, Arylcarbonyl-, Aralkylcarbonyl-, Carboxy-, Alkoxycarbonyl-, Aminocarbonyl-, Alkylaminocarbonyl-, Dialkylaminocarbonyl-, Arylaminocarbonyl-, N-Alkylarylaminocarbonyl-, Aralkylaminocarbonyl-, N-Alkyl-aralkylaminocarbonyl-, N-Hydroxy-aminocarbonyl-, N-Hydroxy-alkylaminocarbonyl-, N-Alkoxy-aminocarbonyl-, N-Alkoxy-alkylaminocarbonyl-, Cyano-, Azido-, N-Cyano-amino- oder N-Cyano-alkylaminogruppe,

eine Sulfo-, Aminosulfonyl-, Alkylaminosulfonyl-, Dialkylaminosulfonyl-, Arylaminosulfonyl-, N-Alkyl-arylaminosulfonyl-, Aralkylaminosulfonyl- oder N-Alkyl-aralkylaminosulfonylgruppe,

eine Phosphono-, O-Alkyl-phosphono-, O,O'-Dialkyl-phosphono- oder O,O'-Diaralkyl-phosphonogruppe,

eine durch $R_4$ substituierte Alkyl- oder Alkoxygruppe, wobei

> $R_4$ eine Hydroxy-, Alkoxy-, Aryloxy-, Amino-, Alkylamino-, Dialkylamino-, Alkylsulfenyl-, Alkylsulfinyl-, Alkylsulfonyl-, Arylsulfenyl-, Arylsulfinyl-, Arylsulfonyl-, Carboxy-, Alkoxycarbonyl-, Aminocarbonyl-, Alkylaminocarbonyl-, Dialkylaminocarbonyl- oder Cyanogruppe oder
>
> eine (Alkylenimino)carbonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoffatom, durch eine Imino- oder N-Alkyl-iminogruppe ersetzt sein kann, darstellt,

$R_2$ ein Wasserstoff-, Fluor-, Chlor- oder Bromatom, eine Alkyl-, Trifluormethyl-, Hydroxy-, Alkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Trifluormethylsulfonylamino-, N-Alkyl-trifluormethylsulfonylamino- oder Cyanogruppe und

$R_3$ ein Wasserstoff-, Fluor-, Chlor- oder Bromatom, eine Alkyl-, Trifluormethyl- oder Alkoxygruppe oder

$R_2$ zusammen mit $R_3$, sofern diese an benachbarte Kohlenstoffatome gebunden sind, auch eine gegebenenfalls durch eine oder zwei Alkylgruppen substituierte Methylendioxygruppe, eine gegebenenfalls durch eine

oder zwei Alkylgruppen substituierte n-$C_{3-6}$-Alkylengruppe oder eine gegebenenfalls durch ein Fluor-, Chlor- oder Bromatom, durch eine Hydroxy-, Alkyl-, Alkoxy-, Trifluormethyl- oder Cyangruppe substituierte 1,3-Butadien-1,4-diylengruppe oder

$R_a$ zusammen mit $R_1$, sofern $R_1$ in o-Stellung zu dem durch $R_a$ substituierten Stickstoffatom steht, auch eine n-$C_{2-3}$-Alkylengruppe darstellen, und

$R_c$ ein Wasserstoff- oder Chloratom,

eine Alkyl-, Aryl-, Aralkyl-, Mercapto-, Alkylsulfenyl-, Alkylsulfinyl- oder Alkylsulfonylgruppe,

eine Hydroxy-, Aryloxy- oder Aralkoxygruppe,

eine $C_{1-6}$-Alkoxygruppe, die durch eine Alkoxycarbonyl-, Cyano-, Carboxy-, Aminocarbonyl-, Alkylaminocarbonyl- oder Dialkylaminocarbonylgruppe substituiert sein kann,

eine $C_{2-6}$-Alkoxygruppe, die durch eine Hydroxy-, Alkoxy-, Hydroxy-$C_{2-4}$-alkylamino-, Amino-, Alkylamino-, Dialkylamino-, Alkylcarbonylamino-, Alkylsulfonylamino- oder Alkoxycarbonylaminogruppe, durch eine gegebenenfalls durch 1 bis 2 Alkylgruppen substituierte 5- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoffatom oder durch eine Carbonyl-, Imino-, Alkyl-imino-, Alkylcarbonyl-imino-, Alkoxycarbonyl-imino-, Alkylsulfonylimino-, Formylimino-, Dialkylaminocarbonyl-imino-, Aryl-imino- oder Aralkyl-iminogruppe ersetzt sein kann, substituiert ist,

eine durch zwei Hydroxy- oder Alkoxygruppen substituierte $C_{3-6}$-Alkoxygruppe,

eine durch eine $C_{3-7}$-Cycloalkylgruppe substituierte Alkoxygruppe, wobei der Cycloalkylteil jeweils durch 1 oder 2 Alkylgruppen substituiert sein kann und wobei in den vorstehend erwähnten $C_{4-7}$-Cycloalkylteilen jeweils eine Methylengruppe durch ein Sauerstoffatom, durch eine Imino-, N-Alkyl-imino-, N-Alkylcarbonyl-imino-, N-Alkoxycarbonyl-imino- oder N-Aryl-iminogruppe ersetzt sein kann,

eine gegebenenfalls durch eine Hydroxy-, Alkoxy-, Alkoxycarbonyl-, Cyano-, Carboxy-, Aminocarbonyl-, Alkylaminocarbonyl-, Dialkylaminocarbonyl-, Hydroxy-$C_{2-4}$-alkylamino-, Amino-, Alkylamino-, Dialkylamino-, Alkylcarbonylamino-, Alkylsulfonylamino- oder Alkoxycarbonylaminogruppe substituierte $C_{4-7}$-Cycloalkoxygruppe,

eine $C_{5-7}$-Cycloalkoxygruppe, wobei in der vorstehend erwähnten Cyclopentyloxygruppe jeweils eine Methylengruppe in 3-Stellung, in den vorstehend erwähnten $C_{6-7}$-Cycloalkoxygruppen jeweils eine Methylengruppe in 3- oder 4-Stellung durch ein Sauerstoffatom oder durch eine Imino-, Alkyl-imino-, Alkylcarbonyl-imino-, Alkoxycarbonyl-imino-, Alkylsulfonyl-imino-, Aryl-imino- oder Aralkyl-iminogruppe ersetzt ist,

eine $C_{3-6}$-Alkenyloxygruppe, wobei der Vinylteil nicht mit dem Sauerstoffatom verknüpft sein kann,

eine $C_{3-6}$-Alkinyloxygruppe, wobei der Ethinylteil nicht mit dem Sauerstoffatom verknüpft sein kann,

eine gegebenenfalls durch 1 bis 4 Alkylgruppen oder eine Arylgruppe substituierte 4- bis 8-gliedrige Alkyleniminogruppe, die zusätzlich durch den Rest $R_5$ substituiert sein kann, wobei

$R_5$ eine Aryl-, Aralkyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl-, 4-Alkylpiperazinocarbonyl-, Cyano-, Hydroxy-, Alkoxy-, Aryloxy-, Amino-, Alkylamino-, Dialkylamino-, Hydroxy-$C_{2-4}$-alkylamino-, N-Alkyl-hydroxy-$C_{2-4}$-alkylamino-, Di(hydroxy-$C_{2-4}$-alkyl) amino-, Formylamino-, Cyanamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Carboxyalkyl-, Alkoxycarbonylalkyl-, Aminocarbonylalkyl-, Alkylaminocarbonylalkyl-, Dialkylaminocarbonylalkyl-, Pyrrolidinocarbonylalkyl-, Piperidinocarbonylalkyl-, Morpholinocarbonylalkyl-, Piperazinocarbonylalkyl-, 4-Alkyl-piperazinocarbonylalkyl-, Cyanoalkyl-, Hydroxyalkyl-, Alkoxyalkyl-, Aryloxyalkyl-, Aminoalkyl-, Alkylaminoalkyl-, Dialkylaminoalkyl-, Alkylcarbonylaminoalkyl-, N-Alkyl-alkylcarbonylaminoalkyl-, Alkylsulfonylaminoalkyl-, N-Alkyl-alkylsulfonylaminoalkyl-, Arylcarbonylaminoalkyl-, N-Alkyl-arylcarbonylaminoalkyl-, Arylsulfonylaminoalkyl-, N-Alkyl-arylsulfonylaminoalkyl-, Alkylsulfenyl-, Alkylsulfinyl-, Alkylsulfonyl-, Arylsulfenyl-, Arylsulfinyl-, Arylsulfonyl-, Alkylsulfenylalkyl-, Alkylsulfinylalkyl-, Alkylsulfonylalkyl-, Arylsulfenylalkyl-, Arylsulfinylalkyl-, Arylsulfonylalkyl-, Carboxyalkoxy-, Alkoxycarbonylalkoxy-, Aminocarbonylalkoxy-, Alkylaminocarbonylalkoxy-, Dialkylaminocarbonylalkoxy-, Pyrrolidinocarbonylalkoxy-, Piperidinocarbonylalkoxy-, Morpholinocarbonylalkoxygruppe oder eine $(R_8NR_7)$-CO-$NR_6$-Gruppe, wobei

$R_6$, $R_7$ und $R_8$, die gleich oder verschieden sein können, jeweils ein Wasserstoffatom oder eine Alkylgruppe oder $R_6$ und $R_7$ zusammen eine n-$C_{2-3}$-Alkylengruppe und
$R_8$ ein Wasserstoffatom oder eine Alkylgruppe darstellen,

eine gegebenenfalls durch eine oder zwei Alkylgruppen oder eine Hydroxymethylgruppe substituierte Pyrrolidino-, Piperidino-, Morpholino-, Piperazino-, 4-Alkylpiperazino- oder 4-Alkylcarbonylpiperazinogruppe, wobei im heterocyclischen Teil der vorstehend erwähnten Gruppen jeweils eine oder zwei der zu den Stickstoffatomen benachbarten Methylengruppen durch eine Carbonylgruppe ersetzt sein können,

oder $R_c$ eine gegebenenfalls durch 1 bis 4 Alkylgruppen oder durch eine Arylgruppe substituierte 6- bis 8-gliedrige Alkyleniminogruppe, die zusätzlich durch den Rest $R_5$ substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl-, N-Oxido-N-alkylimino- oder $R_9$N-Gruppe ersetzt ist, wobei

$R_9$ ein Wasserstoffatom, eine Alkyl-, Hydroxy-$C_{2-4}$-alkyl-, Alkoxy-$C_{2-4}$-alkyl-, Hydroxy-$C_{2-4}$-alkoxy-$C_{2-4}$-alkyl-, Aminocarbonylalkyl-, Alkylaminocarbonylalkyl-, Dialkylaminocarbonylalkyl-, Amino-$C_{2-4}$ -alkyl-, Alkylamino-$C_{2-4}$-alkyl-, Dialkylamino-$C_{2-4}$-alkyl-, Aryl-, Aralkyl-, Formyl-, Alkylcarbonyl-, Alkylsulfonyl-, Arylcarbonyl-, Arylsulfonyl-, Aralkylcarbonyl-, Aralkylsulfonyl-, Alkoxycarbonyl-, Cyano-, Aminocarbonyl-, Alkylaminocarbonyl- oder Dialkylaminocarbonylgruppe darstellt,

oder $R_c$ eine gegebenenfalls durch 1 oder 2 Alkylgruppen substituierte 1-Pyrrolidinylgruppe, in der zwei Wasserstoffatome am Kohlenstöffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind,

eine gegebenenfalls durch 1 oder 2 Alkylgruppen substituierte 1-Piperidinyl- oder 1-Azacyclohept-1-yl-gruppe, in denen zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind, oder 1 oder 2 Kohlenstoffatome enthält, wenn sich die zwei Wasserstoffatome an Kohlenstoffatomen befinden, die durch zwei Atome getrennt sind,

eine 1-Pyrrolidinylgruppe, in der zwei Wasserstoffatome in 3-Stellung durch eine -O-$CH_2CH_2$-O- oder -O-$CH_2CH_2CH_2$-O-Gruppe ersetzt sind,

eine 1-Piperidinyl- oder 1-Azacyclohept-1-yl-gruppe, in denen zwei Wasserstoffatome in 3-Stellung oder in 4-Stellung durch eine -O-$CH_2CH_2$-O- oder -O-$CH_2CH_2CH_2$-O-Gruppe ersetzt sind,

eine 2-Isoindolinyl-, 1,2,3,4-Tetrahydro-isochinolin-2-yl- oder 2,3,4,5-Tetrahydro-1H-3-benzazepin-3-yl-Gruppe, wobei der Benzoteil der vorstehend erwähnten Gruppen jeweils durch ein Fluor-, Chlor-, Brom- oder Jodatom, durch eine Trifluormethylgruppe oder durch eine oder zwei Alkyl- oder Alkoxygruppen substituiert sein kann, oder

eine $(R_{10}NR_{11})$-Gruppe darstellt, in der

$R_{10}$ ein Wasserstoffatom oder eine $C_{1-8}$-Alkylgruppe, die ab Position 2 durch eine Hydroxy- oder Alkoxygruppe substituiert sein kann,

$R_{11}$ ein Wasserstoffatom,

eine $C_{1-10}$-Alkylgruppe, die durch eine Aryl-, $C_{3-7}$-Cycloalkyl-, Hydroxy-, Alkoxy-, Aryloxy-, Carboxy-, Alkoxycarbonyl-, Aminocarbonyl-, Alkylaminocarbonyl-, Dialkylaminocarbonyl-, Hydroxy-$C_{2-4}$-alkylaminocarbonyl-, Cyano-, Formylamino-, Amino-, Alkylamino- oder Dialkylaminogruppe, durch eine Pyrrolidinocarbonyl-, Piperidinocarbonyl-, Morpholinocarbonyl-, Piperazinocarbonyl- oder 4-Alkyl-piperazinocarbonylgruppe,

durch eine gegebenenfalls durch 1 oder 2 Alkylgruppen substituierte 5- bis 7-gliedrige Alkyleniminogruppe, wobei in den vorstehend erwähnten 6- oder 7-gliedrigen Alkyleniminoteilen jeweils eine Methylengruppe in 4-Stellung durch ein Sauerstoff- oder Schwefelatom, durch eine Sulfinyl-, Sulfonyl- oder $R_9$N-Gruppe ersetzt sein kann, wobei $R_9$ wie eingangs definiert ist,

durch eine Pyrrolidino-, Piperidino-, Piperazino- oder 4-Alkylpiperazinogruppe, wobei jeweils eine oder zwei der zu den Stickstoffatomen benachbarten Methylengruppen durch eine Carbonylgruppe ersetzt sind,

durch eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino- oder N-Alkyl-alkoxycarbonylaminogruppe, durch eine ($R_8NR_7$)-CO-$NR_6$-Gruppe, wobei $R_6$ bis $R_8$ wie eingangs definiert sind, durch eine Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe oder durch eine $C_{5-7}$-Cycloalkylgruppe, in der eine Methylengruppe durch ein Sauerstoffatom oder durch eine Imino- oder Alkyliminogruppe ersetzt ist, substituiert sein kann,

eine durch ein Chloratom oder ein bis drei Fluoratome substituierte $C_{2-4}$-Alkylgruppe,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine durch 2 bis 5 Hydroxygruppen substituierte $C_{3-10}$-Alkylgruppe,

eine durch eine Hydroxy- und zusätzlich durch eine Arylgruppe substituierte $C_{2-6}$-Alkylgruppe, die gegebenenfalls zusätzlich durch eine Hydroxy- oder Alkoxygruppe substituiert sein kann,

eine durch eine Hydroxy- und zusätzlich durch eine Amino-, Alkylamino- oder Dialkylaminogruppe substituierte $C_{3-6}$-Alkylgruppe,

eine gegebenenfalls durch eine Arylgruppe substituierte Alkenyl- oder Alkinylgruppe mit jeweils 3 bis 6 Kohlenstoffatomen, wobei der Vinyl- oder Ethinylteil nicht mit dem Stickstoffatom verknüpft sein kann,

eine $C_{2-4}$-Alkylgruppe, die durch eine $C_{2-4}$-Alkoxygruppe substituiert ist, welche in ω-Position durch eine Hydroxy- oder Alkoxygruppe substituiert ist,

eine Arylgruppe,

eine Cyclopropylgruppe, die durch 1 oder 2 Alkylgruppen, durch eine Aryl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonylgruppe substituiert sein kann,

eine gegebenenfalls durch 1 oder 2 Alkylgruppen substituierte $C_{4-7}$-Cycloalkylgruppe, die zusätzlich durch $R_5$ substituiert sein kann, wobei $R_5$ wie eingangs definiert ist,

eine gegebenenfalls durch 1 oder 2 Alkylgruppen substituierte $C_{5-7}$-Cycloalkylgruppe, die zusätzlich durch eine N,N-Dialkyl-N-oxido-aminogruppe substituiert ist,

eine gegebenenfalls durch 1 oder 2 Alkylgruppen substituierte $C_{5-7}$-Cycloalkenylgruppe, wobei der Vinylteil nicht mit dem Stickstoffatom der ($R_{11}NR_{10}$)-Grupppe verknüpft sein kann,

eine gegebenenfalls durch 1 oder 2 Alkylgruppen substituierte $C_{5-7}$-Cycloalkylgruppe, wobei in dem Cycloalkylteil jeweils eine Methylengruppe durch ein Sauerstoff- oder Schwefelatom, durch eine Sulfinyl-, Sulfonyl-, N-Alkyl-N-oxido-imino- oder $R_9$N-Gruppe ersetzt ist, wobei $R_9$ wie eingangs definiert ist,

eine gegebenenfalls durch 1 bis 4 Alkylgruppen substituierte $C_{5-7}$-Cycloalkylgruppe, in denen jeweils eine Methylengruppe durch eine Carbonylgruppe ersetzt ist,

eine gegebenenfalls durch 1 oder 2 Alkylgruppen substituierte $C_{4-7}$-Cycloalkylmethylgruppe, die im Cycloalkylteil zusätzlich durch $R_5$ substituiert ist, wobei $R_5$ wie vorstehend erwähnt definiert ist,

eine gegebenenfalls durch 1 bis 4 Alkylgruppen substituierte Cyclopentyl- oder Cyclopentylalkylgruppe, in denen jeweils zwei Wasserstoffatome im Cyclopentylteil durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Kohlenstoffatom getrennt sind,

eine gegebenenfalls durch 1 bis 4 Alkylgruppen substituierte Cyclohexyl-, Cyclohexylalkyl-, Cycloheptyl- oder Cycloheptylalkylgruppe, in denen jeweils zwei Wasserstoffatome im Cycloalkylteil durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Kohlenstoffatom getrennt sind, oder 1 oder 2 Kohlenstoffatome enthält, wenn sich die zwei Wasserstoffatome an Kohlenstoffatomen befinden, die durch zwei Kohlenstoffatome getrennt sind,

eine gegebenenfalls durch 1 bis 4 Alkylgruppen substituierte 5-Norbornen-2-yl- oder 5-Norbornen-2-yl-alkylgruppe,

eine 3-Chinuclidinyl-, 4-Chinuclidinyl-, 3-Chinuclidinyl-alkyl-, 4-Chinuclidinyl-alkyl-, Azabicyclo[2.2.1]hept-4-yl-, Azabicyclo[2.2.1]hept-4-yl-alkyl- oder Adamantylgruppe, oder

$R_{10}$ ein Wasserstoffatom oder eine Alkylgruppe und $R_{11}$ eine Hydroxy- oder Alkoxygruppe darstellen,

deren Tautomere, deren Stereoisomere und deren Salze,

wobei, sofern nichts anderes erwähnt wurde,

unter den bei der Definition der vorstehend erwähnten Reste erwähnten Arylteilen eine Phenylgruppe zu verstehen ist, die jeweils durch $R_{12}$ monosubstituiert, durch $R_{13}$ mono-, di- oder trisubstituiert oder durch $R_{12}$ monosubstituiert und zusätzlich durch $R_{13}$ mono- oder disubstituiert sein kann, wobei die Substituenten gleich oder verschieden sein können, und

$R_{12}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Perfluoralkoxy-, Nitro-, Amino-, Alkylamino-, Hydroxy-$C_{2-4}$-alkylamino-, N-Alkyl-hydroxy-$C_{2-4}$-alkylamino-, Di(hydroxy-$C_{2-4}$-alkyl) amino-, Dialkylamino-, Alkylcarbonylamino-, Phenylalkylcarbonylamino-, Phenylcarbonylamino-, Alkylsulfonylamino-, Phenylalkylsulfonylamino-, Phenylsulfonylamino-, N-Alkyl-alkylcarbonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkylphenylsulfonylamino-, Aminosulfonyl-, Alkylaminosulfonyl- oder Dialkylaminosulfonylgruppe,

eine gegebenenfalls durch 1 bis 2 Alkylgruppen oder eine Hydroxyalkylgruppe substituierte 5- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoffatom oder eine $R_9$N-Gruppe ersetzt sein kann,

eine gegebenenfalls durch 1 bis 2 Alkylgruppen oder eine Hydroxyalkylgruppe substituierte 5- bis 7-gliedrige Alkyleniminogruppe, wobei jeweils eine oder zwei zu dem Stickstoffatom benachbarte Methylengruppen durch jeweils eine Carbonylgruppe ersetzt sind,

eine $(R_8NR_7)$-CO-$NR_6$-Gruppe, wobei $R_6$ bis $R_8$ wie vorstehend erwähnt definiert sind,

$R_{13}$ eine Alkyl-, Hydroxy- oder Alkoxygruppe, ein Fluor-, Chlor-, Brom- oder Jodatom darstellen, wobei zwei Reste $R_{13}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

sowie, soweit nichts anderes erwähnt wurde, die vorstehend erwähnten Alkyl-, Alkylen- und Alkoxyteile jeweils 1 bis 4 Kohlenstoffatome enthalten sowie, sofern nichts anderes erwähnt wurde, jedes Kohlenstoffatom in den vorstehend erwähnten Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

**4.** Pyrimido[5,4-d]pyrimidine der allgemeinen Formel I gemäß Anspruch 1 oder 2, in der

$R_a$ ein Wasserstoffatom oder eine Alkylgruppe,

$R_b$ eine durch die Reste $R_1$ bis $R_3$ substituierte Phenylgruppe, wobei

$R_1$ ein Wasserstoff-, Fluor-, Chlor-, Brom- oder Jodatom,

eine Alkyl-, Hydroxy-, Alkoxy-, $C_{3-6}$-Cycloalkyl- oder $C_{5-6}$-Cycloalkoxygruppe,

eine in 2-Stellung durch eine Hydroxy-, Alkoxy- oder Phenoxygruppe substituierte Ethoxygruppe,

eine $C_{2-5}$-Alkenyl- oder $C_{3-5}$-Alkenyloxygruppe, wobei der Vinylteil nicht mit dem Sauerstoffatom verknüpft sein kann,

eine $C_{2-5}$-Alkinyl- oder $C_{3-5}$-Alkinyloxygruppe, wobei der Ethinylteil nicht mit dem Sauerstoffatom verknüpft sein kann,

eine Phenyl-, Phenoxy-, Phenylalkyl-, Phenylalkoxy-, Alkoxyalkyl-, Phenoxyalkyl-, Carboxyalkyl-, Alkoxycarbonylalkyl-, Aminocarbonylalkyl-, Alkylaminocarbonylalkyl-, Dialkylaminocarbonylalkyl-, Cyanoalkyl-, Alkylsulfenyl-, Alkylsulfinyl-, Alkylsulfonyl-, Alkylsulfonyloxy-, Trifluormethylsulfenyl-, Trifluormethylsulfonyl-, Nitro-, Amino-, Alkylamino-, Dialkylamino-, Pyrrolidino-, Piperidino-, Morpholino-, Alkylcarbonylamino-, N-Alkyl-alkylcarbonylamino-, Alkylsulfonylamino-, N-Alkyl-alkylsulfonylamino-, Trifluormethylsulfonylamino-, N-Alkyl-trifluormethylsulfonylamino-, Alkylcarbonyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl- oder Cyanogruppe,

eine durch 1 bis 3 Fluoratome substituierte Methyl- oder Methoxygruppe,

eine durch 1 bis 5 Fluoratome substituierte Ethyl- oder Ethoxygruppe,

$R_2$ ein Wasserstoff-, Fluor-, Chlor- oder Bromatom, eine Alkyl-, Trifluormethyl-, Amino-, Alkylamino-, Dialkylamino-, Alkylcarbonylamino-, Alkylsulfonylamino-, Trifluormethylsulfonylamino-, Hydroxy- oder Alkoxygruppe,

$R_3$ ein Wasserstoff-, Fluor-, Chlor- oder Bromatom oder eine Alkylgruppe, oder

$R_2$ zusammen mit $R_3$, sofern diese an benachbarte Kohlenstoffatome gebunden sind, auch eine Methylendioxy- oder n-$C_{3-6}$-Alkylengruppe oder eine gegebenenfalls durch ein Fluor-, Chlor- oder Bromatom, durch eine Alkyl-, Alkoxy- oder Trifluormethylgruppe substituierte 1,3-Butadien-1,4-diylengruppe darstellen, bedeuten, und

$R_c$ ein Wasserstoff- oder Chloratom,

eine Alkyl-, Phenyl-, Mercapto-, Alkylsulfenyl-, Alkylsulfinyl- oder Alkylsulfonylgruppe,

eine Hydroxy-, Phenoxy- oder Phenyl-$C_{1-2}$-alkoxygruppe,

eine Alkoxygruppe,

eine $C_{2-4}$-Alkoxygruppe, die durch eine Hydroxy-, Alkoxy-, (2-Hydroxyethyl)amino-, Dialkylamino-, Morpholino-, 1-Pyrrolidinyl-, 1-Piperidinyl-, 4-Methyl-1-piperazinyl-, 4-Acetyl-1-piperazinyl-, 4-Methylsulfonyl-1-piperazinyl-, 4-Methoxycarbonyl-1-piperazinyl-, 4-Formyl-1-piperazinyl- oder 4-Dimethylaminocarbonyl-1-piperazinylgruppe substituiert ist,

eine $C_{3-4}$-Alkoxygruppe, die durch zwei Hydroxygruppen substituiert ist,

eine $C_{1-2}$-Alkoxygruppe, die durch eine gegebenenfalls durch eine oder zwei Methylgruppen substituierte $C_{3-7}$-Cycloalkylgruppe substituiert ist, wobei in den vorstehend erwähnten $C_{4-6}$-Cycloalkylgruppen jeweils eine Methylengruppe durch ein Sauerstoffatom ersetzt sein kann,

eine gegebenenfalls durch eine Hydroxy-, Dialkylamino-, Alkoxy-, Carboxy-, Alkoxycarbonyl-, Aminocarbonyl-, Alkylaminocarbonyl-, Dialkylaminocarbonyl-, Alkylcarbonylamino-, Alkylsulfonylamino- oder Alkoxycarbonylaminogruppe substituierte $C_{4-6}$-Cycloalkoxygruppe,

eine Cyclopentyloxygruppe, in der die Methylengruppe in 3-Stellung durch ein Sauerstoffatom oder durch eine Alkyliminogruppe ersetzt ist,

eine Cyclohexyloxygruppe, in der die Methylengruppe in 3- oder 4-Stellung durch ein Sauerstoffatom oder durch eine Alkylimino-, Alkylcarbonyl-imino-, Alkoxycarbonyl-imino- oder Alkylsulfonyl-iminogruppe ersetzt ist,

eine gegebenenfalls durch eine oder zwei Methylgruppen substituierte Allyloxy- oder Propargyloxygruppe,

eine 1-Azetidinylgruppe,

eine 1-Pyrrolidinylgruppe, die durch 1 bis 2 Alkylgruppen, durch eine Phenyl-, Carboxy-, Hydroxyalkyl-, Aminoalkyl-, Alkylaminoalkyl-, Dialkylaminoalkyl-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonyl-Gruppe oder in 3-Stellung auch durch eine Hydroxy-, Alkoxy-, Amino-, Alkylamino-, Dialkylamino-, Alkylcarbonylamino-, Alkoxycarbonylamino-, Formylamino-, Cyanamino-, Alkylsulfonylamino-, Dialkylaminocarbonylamino-, N-Alkyl-dialkylaminocarbonylamino-, N-Alkyl-dialkylaminocarbonylamino- oder Cyanogruppe substituiert sein kann,

eine 1-Pyrrolidinylgruppe, in der zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind,

eine 1-Piperidinylgruppe, die durch 1 bis 4 Alkylgruppen, durch eine Phenyl-, Hydroxyalkyl-, Aminoalkyl-, Alkylaminoalkyl-, Dialkylaminoalkyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonyl-Gruppe oder in 3-oder 4-Stellung auch durch eine Hydroxy-, Alkoxy-, Amino-, Alkylamino-, Dialkylamino-, Alkylcarbonylamino-, Alkoxycarbonylamino-, Formylamino-, Cyanamino-, Alkylsulfonylamino-, Dialkylaminocarbonylamino-, N-Alkyl-dialkylaminocarbonylamino- oder Cyanogruppe substituiert sein kann,

eine 1-Piperidinylgruppe, die durch 1 bis 2 Alkylgruppen oder eine Phenylgruppe und zusätzlich durch eine Hydroxygruppe substituiert ist,

eine 1-Piperidinylgruppe, in der in 3-Stellung oder in 4-Stellung zwei Wasserstoffatome durch eine $-O-CH_2CH_2-O-$ oder $-O-CH_2CH_2CH_2-O-$Gruppe ersetzt sind,

eine 1-Piperidinylgruppe, in der zwei Wasserstoffatome am Kohlenstoffgerüst durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Atom getrennt sind, oder 1 oder 2 Kohlenstoffatome enthält,

wenn sich die zwei Wasserstoffatome an Kohlenstoffatomen befinden, die durch zwei Atome getrennt sind,

eine gegebenenfalls durch 1 oder 2 Alkylgruppen substituierte 1-Piperidinylgruppe, in der die Methylengruppe in 4-Stellung durch ein Sauerstoff- oder Schwefelatom, durch eine Carbonyl-, Sulfinyl-, Sulfonyl-, Imino-, Alkylimino-, Hydroxy-$C_{2-4}$-alkylimino-, Alkoxy-$C_{2-4}$-alkyl-imino-, Aminocarbonylalkyl-imino-, Alkylaminocarbonylalkyl-imino-, Dialkylaminocarbonylalkylimino-, Amino-$C_{2-4}$-alkyl-imino-, Alkylamino-$C_{2-4}$-alkyl-imino-, Dialkylamino-$C_{2-4}$-alkyl-imino-, Hydroxy-$C_{2-4}$-alkoxy-$C_{2-4}$-alkylimino-, Phenyl-imino-, Phenylalkyl-imino-, Alkylcarbonylimino-, Alkylsulfonyl-imino-, Phenylcarbonyl-imino-, Phenylsulfonyl-imino- oder N-Oxido-N-alkyl-imino-Gruppe ersetzt ist,

eine gegebenenfalls durch 1 oder 2 Alkylgruppen substituierte 1-Azacyclohept-1-yl-Gruppe, in der jeweils die Methylengruppe in 4-Stellung durch ein Sauerstoffatom, durch eine Imino-, N-Alkyl-imino-, N-Phenyl-imino-, N-Phenylalkyl-imino-, N-Alkylcarbonyl-imino-, N-Alkylsulfonyl-imino-, N-Phenylcarbonyl-imino- oder N-Phenylsulfonyl-imino-Gruppe ersetzt sein kann oder zwei Wasserstoffatome in 3,6-Stellung durch eine $-CH_2CH_2-$Gruppe ersetzt sein können,

eine 2-Isoindolinyl-, 1,2,3,4-Tetrahydro-isochinolin-2-yl- oder 2,3,4,5-Tetrahydro-1H-3-benzazepin-3-yl-Gruppe, die jeweils im Benzoteil durch ein Fluor-, Chlor- oder Bromatom, durch eine Alkyl-, Trifluormethyl- oder Alkoxygruppe substituiert sein können, oder

eine ($R_{10}NR_{11}$)-Gruppe darstellen, in der

$R_{10}$ ein Wasserstoffatom oder eine $C_{1-6}$-Alkylgruppe, die ab Position 2 durch eine Hydroxy- oder Alkoxygruppe substituiert sein kann, und

$R_{11}$ ein Wasserstoffatom,

eine $C_{1-8}$-Alkylgruppe, die durch eine Phenyl-, $C_{3-6}$-Cycloalkyl-, Hydroxy-, Alkoxy-, Cyano-, Carboxy-, Alkoxycarbonyl-, Aminocarbonyl-, Alkylaminocarbonyl-, Dialkylaminocarbonyl-, (2-Hydroxyethyl)aminocarbonyl-, Pyrrolidinocarbonyl-, Piperidinocarbonyl-, Morpholinocarbonyl-, 1-Piperazinylcarbonyl-, 4-Alkyl-1-piperazinylcarbonyl-, Amino-, Formylamino-, Alkylamino-, Dialkylamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Phenylcarbonylamino-, N-Alkyl-phenylcarbonylamino-, Phenylsulfonylamino-, N-Alkyl-phenylsulfonylamino-, Alkylsulfenyl-, Alkylsulfinyl-, Alkylsulfonyl-, 1-Pyrrolidinyl-, 2-Oxo-1-pyrrolidinyl-, 1-Piperidinyl-, 2-Oxo-1-piperidinyl-, Morpholino-, 1-Piperazinyl-, 4-Alkyl-1-piperazinyl-, 4-Alkylcarbonyl-1-piperazinyl-, 4-Alkylsulfonyl-1-piperazinyl-, 4-Alkoxycarbonyl-1-piperazinyl-, 4-Cyano-1-piperazinyl-, 4-Formyl-1-piperazinyl-, 4-Aminocarbonyl-1-piperazinyl-, 4-Alkylaminocarbonyl-1-piperazinyl- oder 4-Dialkylaminocarbonyl-1-piperazinyl- oder eine ($R_8NR_7$)-CO-$NR_6$-Gruppe substituiert sein kann, wobei

> $R_6$ und $R_7$ zusammen eine n-$C_{2-3}$-Alkylenbrücke und
> $R_8$ ein Wasserstoffatom oder eine Alkylgruppe darstellen,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine 2,2,2-Trifluorethylgruppe,

eine durch 2 bis 5 Hydroxygruppen substituierte $C_{3-10}$-Alkylgruppe,

eine durch eine Hydroxy- und zusätzlich durch eine Aminogruppe substituierte $C_{3-5}$-Alkylgruppe,

eine durch eine Phenylgruppe und zusätzlich durch eine Hydroxygruppe substituierte $C_{2-4}$-Alkylgruppe, die gegebenenfalls zusätzlich durch eine Hydroxy- oder Alkoxygruppe substituiert sein kann,

eine gegebenenfalls durch eine Phenylgruppe substituierte Alkenyl- oder Alkinylgruppe mit jeweils 3 bis 6 Kohlenstoffatomen, wobei der Vinyl- oder Ethinylteil nicht mit dem Stickstoffatom verknüpft sein kann,

eine $C_{2-4}$-Alkylgruppe, die durch eine $C_{2-4}$-Alkoxygruppe substituiert ist, welche in ω-Position durch eine Hydroxy- oder Alkoxygruppe substituiert ist,

eine Phenylgruppe,

eine Phenylgruppe, die durch eine Alkylcarbonylamino-, N-Alkylalkylcarbonylamino-, (2-Hydroxyethyl)amino-, Di-(2-hydroxyethyl)amino-, N-Alkyl-2-(hydroxyethyl)amino-, Amino-, Alkylamino- oder Dialkylaminogruppe oder durch eine $(R_8NR_7)$-CO-$NR_6$-Gruppe substituiert ist, wobei $R_6$ bis $R_8$ wie vorstehend erwähnt definiert sind,

eine Phenylgruppe, die durch eine Pyrrolidino-, Piperidino-, 2-Oxo-pyrrolidino-, 2-Oxo-piperidino-, Morpholino-, 1-Piperazinyl- oder 4-Alkyl-1-piperazinyl-Gruppe substituiert ist, wobei die vorstehend erwähnten heterocyclischen Teile am Kohlenstoffgerüst jeweils durch 1 oder 2 Alkylgruppen oder durch eine Hydroxyalkylgruppe substituiert sein können,

eine $C_{3-7}$-Cycloalkylgruppe, die durch 1 oder 2 Alkylgruppen, durch eine Phenyl-, Carboxy-, Alkoxycarbonyl-, Aminocarbonyl-, Alkylaminocarbonyl-, Dialkylaminocarbonyl-, Pyrrolidinocarbonyl-, Piperidinocarbonyl-, Morpholinocarbonyl-, Piperazinocarbonyl- oder 4-Alkyl-piperazinocarbonylgruppe substituiert sein kann,

eine gegebenenfalls durch 1 oder 2 Methylgruppen substituierte $C_{5-7}$-Cycloalkylgruppe, die durch eine Hydroxymethyl-, Cyano-, Hydroxy-, Alkoxy-, Amino-, Alkylamino-, Dialkylamino-, 2-Hydroxyethylamino-, Di-(2-hydroxyethyl)amino-, N-Alkyl-2-hydroxyethylamino-, N,N-Dialkyl-N-oxido-amino-, Alkoxycarbonylamino-, N-Alkyl-alkoxycarbonylamino-, Alkylcarbonylamino-, N-Alkylalkylcarbonylamino-, Alkylsulfonylamino-, N-Alkyl-alkylsulfonylamino-, Phenylcarbonylamino-, N-Alkyl-phenylcarbonylamino-, Phenylsulfonylamino-, N-Alkylphenylsulfonylamino- oder durch eine $(R_8NR_7)$-CO-$NR_6$-Gruppe substituiert ist, wobei $R_6$ bis $R_8$ wie vorstehend erwähnt definiert sind,

eine gegebenenfalls durch 1 oder 2 Methylgruppen substituierte $C_{5-7}$-Cycloalkylgruppe, die durch eine Pyrrolidino-, Piperidino-, 2-Oxo-pyrrolidino-, 2-Oxo-piperidino-, Morpholino-, 1-Piperazinyl-, 4-Alkyl-1-piperazinyl- oder 4-Alkylcarbonyl-1-piperazinyl-Gruppe substituiert ist, wobei die vorstehend erwähnten heterocyclischen Teile am Kohlenstoffgerüst jeweils durch 1 oder 2 Alkylgruppen oder durch eine Hydroxymethylgruppe substituiert sein können,

eine gegebenenfalls durch 1 oder 2 Alkylgruppen substituierte $C_{5-7}$-Cycloalkenylgruppe, wobei der Vinylteil nicht an das Stickstoffatom der $(R_{11}NR_{10})$-Gruppe gebunden sein kann,

eine Tetrahydrofurfurylgruppe,

eine Cyclopentylgruppe, in der die Methylengruppe in 3-Stellung durch ein Sauerstoffatom, eine Imino-, Alkylimino-, Alkylcarbonylimino-, Formylimino-, Aminocarbonylimino-, Alkylaminocarbonylimino-, Alkoxycarbonylimino-, Alkylsulfonylimino-, Dialkylaminocarbonylimino- oder Cyaniminogruppe ersetzt ist,

eine Cyclohexylgruppe, in der die Methylengruppe in 3-Stellung durch eine Imino-, Alkyl-imino-, Alkylcarbonyl-imino-, Alkoxycarbonyl-imino- oder Alkylsulfonyl-imino-Gruppe ersetzt ist,

eine Cyclohexylgruppe, in der die Methylengruppe in 4-Stellung durch ein Sauerstoffatom, eine Imino-, N-Alkyl-imino-, N-Phenyl-imino-, N-Phenylalkyl-imino-, N-Formyl-imino-, N-Alkylcarbonyl-imino-, N-Phenylcarbonyl-imino-, N-Alkoxycarbonyl-imino-, N-Cyan-imino-, N-Aminocarbonyl-imino-, N-Alkylaminocarbonyl-imino-, N,N-Dialkylaminocarbonyl-imino-, N-Alkyl-N-oxido-imino-, N-Alkylsulfonyl-imino- oder N-Phenylsulfonyl-imino-Gruppe ersetzt ist,

eine Cyclohexylgruppe, in der eine Methylengruppe durch eine Carbonylgruppe ersetzt ist,

eine gegebenenfalls durch 1 bis 2 Methylgruppen substituierte Cyclopentyl- oder Cyclohexylgruppe, die durch eine Carboxyalkoxy-, Alkoxycarbonylalkoxy-, Aminocarbonylalkoxy-, Alkylaminocarbonylalkoxy-, Dialkylaminocarbonylalkoxy-, Pyrrolidinocarbonylalkoxy-, Piperidinocarbonylalkoxy-, Morpholinocarbonylalkoxy-, Carboxyalkyl-, Alkoxycarbonylalkyl-, Aminocarbonylalkyl-, Alkylaminocarbonylalkyl-, Dialkylaminocarbonylalkyl-, Pyrrolidinocarbonylalkyl-, Piperidinocarbonylalkyl- oder Morpholinocarbonylalkylgruppe substituiert ist,

eine Cyclohexylmethylgruppe, wobei der Cyclohexylteil durch eine Carboxy-, Aminocarbonyl-, Alkylaminocarbonyl-, Dialkylaminocarbonyl-, Pyrrolidinocarbonyl-, Piperidinocarbonyl-, Morpholinocarbonyl-, Alkoxycarbonyl- oder Hydroxymethylgruppe substituiert ist,

eine gegebenenfalls durch 1 bis 3 Methylgruppen substituierte Cyclohexyl- oder Cyclohexylmethylgruppe, in denen jeweils im Cyclohexylteil zwei Wasserstoffatome durch eine geradkettige Alkylenbrücke ersetzt sind, wobei diese Brücke 4 oder 5 Kohlenstoffatome enthält, wenn die zwei Wasserstoffatome sich am selben Kohlenstoffatom befinden, oder 3 oder 4 Kohlenstoffatome enthält, wenn sich die zwei Wasserstoffatome an benachbarten Kohlenstoffatomen befinden, oder 2 oder 3 Kohlenstoffatome enthält, wenn sich die zwei Wasserstoffatome an Kohlenstoffatomen befinden, die durch ein Kohlenstoffatom getrennt sind, oder 1 oder 2 Kohlenstoffatome enthält, wenn sich die zwei Wasserstoffatome an Kohlenstoffatomen befinden, die durch zwei Kohlenstoffatome getrennt sind,

eine gegebenenfalls durch 1 bis 3 Methylgruppen substituierte 5-Norbornen-2-yl- oder 5-Norbornen-2-yl-methylgruppe,

eine 3-Chinuclidinyl-, 4-Chinuclidinyl- oder Adamantylgruppe bedeuten, oder

$R_{10}$ ein Wasserstoffatom oder eine Alkylgruppe und $R_{11}$ eine Hydroxy- oder Alkoxygruppe bedeuten,

deren Tautomere, deren Stereoisomere und deren Salze,

wobei die vorstehend erwähnten Phenylreste jeweils durch ein Fluor-, Chlor- oder Bromatom, durch eine Nitro-, Alkyl-, Alkoxy-, Trifluormethyl- oder Hydroxygruppe substituiert sein können und, sofern nichts anderes erwähnt wurde, die vorstehend erwähnten Alkyl-, Alkylen- und Alkoxyteile jeweils 1 bis 4 Kohlenstoffatome enthalten sowie, sofern nichts anderes erwähnt wurde, jedes Kohlenstoffatom in den vorstehend erwähnten Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

**5.** Pyrimido[5,4-d]pyrimidine der allgemeinen Formel I gemäß Anspruch 1 oder 2, in der

$R_a$ ein Wasserstoffatom oder eine Methylgruppe,

$R_b$ eine 2-Naphthyl-, 1,2,3,4-Tetrahydro-6-naphthyl- oder 5-Indanylgruppe oder eine durch die Reste $R_1$ bis $R_3$ substituierte Phenylgruppe, wobei

$R_1$ ein Wasserstoff-, Fluor-, Chlor-, Brom- oder Jodatom,

eine $C_{1-4}$-Alkyl-, $C_{1-4}$-Alkoxy-, $C_{3-6}$-cycloalkyl-, $C_{5-6}$-Cycloalkoxy-, Cyano-, Methoxycarbonyl-, Ethoxycarbonyl-, Ethinyl- oder Nitrogruppe,

eine durch 1 bis 3 Fluoratome substituierte Methyl- oder Methoxygruppe,

eine durch 1 bis 5 Fluoratome substituierte Ethyl- oder Ethoxygruppe,

$R_2$ ein Wasserstoff-, Fluor- oder Chloratom, eine Methyl-, Hydroxy-, Methoxy-, Amino-, $C_{1-2}$-Alkylamino-, Di-$C_{1-2}$-alkylamino-, $C_{1-2}$-Alkylcarbonylamino-, $C_{1-2}$-Alkylsulfonylamino-, Trifluormethylsulfonylamino- oder Trifluormethylgruppe,

$R_3$ ein Wasserstoff-, Fluor-, Chlor- oder Bromatom oder eine Methylgruppe und

$R_c$ ein Wasserstoffatom, eine Methyl-, Phenyl-, 4-Methoxyphenyl-, Methylsulfenyl-, Methylsulfinyl- oder Me-

thylsulfonylgruppe,

eine Hydroxygruppe,

eine $C_{1-4}$-Alkoxygruppe,

eine Ethoxygruppe, die in 2-Stellung durch eine Hydroxy-, Methoxy-, Morpholino- oder (2-Hydroxyethyl)aminogruppe substituiert ist,

eine 2-Propyloxygruppe, die in 1-Stellung durch eine Methoxy- oder Dimethylaminogruppe substituiert ist,

eine Methoxygruppe, die durch eine 2-Tetrahydrofuryl-, 2-Tetrahydropyranyl- oder 3-Methyl-3-oxetanylgruppe substituiert ist,

eine Cyclobutyloxygruppe,

eine gegebenenfalls in 3-Stellung durch eine Hydroxygruppe substituierte Cyclopentyloxygruppe,

eine Cyclopentyloxygruppe, in der die Methylengruppe in 3-Stellung durch ein Sauerstoffatom oder durch eine Methyl-iminogruppe ersetzt ist,

eine Cyclohexyloxygruppe, die in 2-,3- oder 4-Stellung durch eine Hydroxygruppe oder in 4-Stellung auch durch eine Di($C_{1-2}$-alkyl) amino-, Methoxy-, Carboxy-, Methoxycarbonyl-, Dimethylaminocarbonyl-, Methylaminocarbonyl-, Aminocarbonyl-, Acetylamino-, Methylsulfonylamino-, Methoxycarbonylamino-oder tert.Butyloxycarbonylaminogruppe substituiert sein kann,

eine Cyclohexyloxygruppe, in der die Methylengruppe in 3-Stellung durch eine Methyl-iminogruppe oder die Methylengruppe in 4-Stellung durch ein Sauerstoffatom oder durch eine Methylimino-, Acetyl-imino-, tert.Butyloxycarbonyl-imino-, Methoxycarbonyl-imino- oder Methylsulfonyl-iminogruppe ersetzt ist,

eine Allyloxygruppe,

eine 1-Azetidinylgruppe,

eine 1-Pyrrolidinylgruppe, die durch 1 oder 2 Methylgruppen, durch eine Carboxy-, Methoxycarbonyl-, Ethoxycarbonyl-, Aminocarbonyl-, $C_{1-2}$-Alkylaminocarbonyl- oder Di-($C_{1-2}$)-alkylaminocarbonylgruppe oder in 3-Stellung durch eine Amino-, $C_{1-2}$-Alkylamino-, Di-($C_{1-2}$-alkyl) amino-, $C_{1-2}$-Alkoxycarbonylamino-, $C_{1-2}$-Alkylcarbonylamino-, $C_{1-2}$-Alkylsulfonylamino-, Cyanamino-, Formylamino- oder Dimethylaminocarbonylaminogruppe substituiert sein kann,

eine 1-Pyrrolidinylgruppe, in der in 3-Stellung zwei Wasserstoffatome durch eine n-$C_{4-5}$-Alkylenbrücke ersetzt sind,

eine 1-Piperidinylgruppe, die durch 1 bis 4 Methylgruppen, durch eine Phenyl-, Hydroxy-$C_{1-2}$-alkyl-, Carboxy-, $C_{1-2}$-Alkoxycarbonyl-, Aminocarbonyl-, $C_{1-2}$-Alkylaminocarbonyl-, Di($C_{1-2}$)-alkylaminocarbonyl-, Pyrrolidinocarbonyl- oder Morpholinocarbonylgruppe oder in 3- oder 4-Stellung durch eine Hydroxy-, $C_{1-2}$-Alkoxy-, Amino-, $C_{1-2}$-Alkylamino-, Di-($C_{1-2}$)-Alkylamino-, $C_{1-2}$-Alkylcarbonylamino-, $C_{1-2}$-Alkoxycarbonylamino-, Formylamino-, Cyanamino-, Di-($C_{1-2}$-alkyl)aminocarbonylamino-, $C_{1-2}$-Alkylsulfonylamino- oder Cyanogruppe substituiert sein kann,

eine 1-Piperidinylgruppe, in der in 3-Stellung oder in 4-Stellung zwei Wasserstoffatome durch eine n-$C_{4-5}$-Alkylenbrücke oder durch eine -O-$CH_2CH_2$-O-Brücke ersetzt sind,

eine 1-Piperidinylgruppe, die durch 1 oder 2 Methylgruppen oder eine Phenylgruppe und zusätzlich in 3- oder 4-Stellung durch eine Hydroxygruppe substituiert ist,

eine 1-Piperidinylgruppe, in der in 2,5-Stellung zwei Wasserstoffatome durch eine -$CH_2$- oder -$CH_2CH_2$-Brücke ersetzt sind,

eine gegebenenfalls durch 1 bis 2 Methylgruppen substituierte 1-Piperidinylgruppe, in der die Methylengruppe in 4-Stellung durch ein Sauerstoff- oder Schwefelatom, durch eine Carbonyl-, Sulfinyl-, Sulfonyl-, Imino-, $C_{1-2}$-Alkyl-imino-, (2-Hydroxyethyl)-imino-, 2-(2-Hydroxyethoxy)ethyl-imino-, (2-Aminoethyl)imino-, $C_{1-3}$-Alkylaminocarbonylmethyl-imino-, N-Oxido-N-$C_{1-2}$-alkylimino-, Phenyl-imino-, Benzyl-imino-, Acetyl-imino- oder Methansulfonyl-iminogruppe ersetzt ist,

eine 1-Azacyclohept-1-yl-Gruppe, in der zwei Wasserstoffatome in 3,6-Stellung durch eine -$CH_2CH_2$-Gruppe ersetzt sein können,

eine 1,2,3,4-Tetrahydro-isochinolin-2-yl- oder 2,3,4,5-Tetrahydro-1H-3-benzazepin-3-yl-Gruppe oder

eine ($R_{10}NR_{11}$)-Gruppe darstellen, in der

$R_{10}$ ein Wasserstoffatom, eine $C_{1-4}$-Alkylgruppe oder eine 2-Hydroxyethylgruppe und

$R_{11}$ ein Wasserstoffatom,

eine $C_{1-6}$-Alkyl-, $C_{3-6}$-Cycloalkyl-methyl- oder Phenyl-$C_{1-3}$-alkylgruppe, .

eine gegebenenfalls durch 1 oder 2 Methylgruppen substituierte $C_{3-6}$-Cycloalkyl-, Allyl- oder Propargylgruppe,

eine Phenylgruppe, die durch eine Hydroxy- oder Methylgruppe oder in 4-Stellung durch eine N-$C_{1-2}$-Alkyl-$C_{1-2}$-alkylcarbonylamino-, N-$C_{1-2}$-Alkyl-(2-hydroxyethyl)amino-, Di-(2-hydroxyethyl)amino-, 1-Pyrrolidinyl-, 2-Oxo-1-pyrrolidinyl-, 2-Hydroxymethyl-1-pyrrolidinyl-, 2-Oxo-1-imidazolidinyl-, 3-Methyl-2-oxo-1-imidazolidinyl- oder Morpholinogruppe substituiert sein kann,

eine durch eine Carboxy-, $C_{1-2}$-Alkoxycarbonyl-, Aminocarbonyl-, $C_{1-2}$-Alkylaminocarbonyl-, Di-$C_{1-2}$-alkylaminocarbonyl-, Pyrrolidinocarbonyl-, Piperidinocarbonyl- oder Morpholinocarbonylgruppe substituierte Methylgruppe,

eine gegebenenfalls durch eine Methylgruppe substituierte Ethylgruppe, die in 2-Stellung durch eine Hydroxy-, $C_{1-2}$-Alkoxy-, Amino-, $C_{1-2}$-Alkylamino-, Di-$C_{1-2}$-alkylamino-, Acetylamino-, 1-Pyrrolidinyl-, Morpholino-, 1-Piperazinyl-, 4-Methyl-1-piperazinyl-, 4-Acetyl-1-piperazinyl-, 4-Aminocarbonyl-1-piperazinyl-, 4-Dimethylaminocarbonyl-l-piperazinyl-, 4-Methylaminocarbonyl-1-piperazinyl-, 4-Methylsulfonyl-1-piperazinyl-, 4-Methoxycarbonyl-1-piperazinyl-oder 4-Cyano-1-piperazinylgruppe substituiert ist,

eine 2-Hydroxyethylgruppe, die im Ethylteil durch eine Phenyl-, 3-Hydroxyphenyl-, 4-Hydroxyphenyl-, 4-Nitrophenyl- oder Benzylgruppe substituiert ist, wobei der Ethylteil der vorstehend erwähnten Gruppen zusätzlich durch eine Methyl-, Hydroxymethyl- oder Methoxymethylgruppe substituiert sein kann,

eine durch eine 1,4,7,10-Tetraoxacyclododecyl-, 1,4,7,10,13-Pentaoxacyclopentadecyl- oder eine 1,4,7,10,13,16-Hexaoxacyclooctadecylgruppe substituierte Methylgruppe,

eine 2,2,2-Trifluorethylgruppe,

eine in 3-Stellung durch eine Hydroxy-, Cyano-, Carboxy-, Aminocarbonyl-, Methylaminocarbonyl-, Dimethylaminocarbonyl-, Formylamino, Methoxycarbonylamino-, Morpholino- oder 2-Oxo-1-pyrrolidinylgruppe substituierte Propylgruppe,

eine in 4-Stellung durch eine Hydroxygruppe substituierte Butylgruppe,

eine in 1-Stellung durch eine Hydroxygruppe und zusätzlich in 3-Stellung durch eine Methylgruppe substituierte 3-Butylgruppe,

eine in 1-Stellung durch eine Hydroxygruppe und zusätzlich in 3-Stellung durch eine Methylgruppe substituierte 2-Butylgruppe,

eine in 1-Stellung durch eine Hydroxygruppe und zusätzlich in 4-Stellung durch eine Methylgruppe substituierte 4-Pentylgruppe,

eine 2,3-Dihydroxypropyl-, 3-Amino-2-hydroxy-propyl-, Tris(3-hydroxypropyl)methyl-, 1,3-Dihydroxy-2-propyl-, 1,3-Dihydroxy-2-methyl-2-propyl- oder Tris- (hydroxymethyl)methylgruppe,

eine 2-Propylgruppe, die in 2-Stellung durch eine Hydroxymethyl-, $C_{1-2}$-Alkoxymethyl-, Carboxy-, $C_{1-2}$-Alkoxycarbonyl-, Aminocarbonyl-, N-$C_{1-2}$-Alkylaminocarbonyl-, N,N-Di-$C_{1-2}$-alkylaminocarbonyl-, Pyrrolidinocarbonyl-, Morpholinocarbonyl- oder (2-Hydroxyethyl)aminocarbonylgruppe substituiert ist,

eine 4-Tetrahydropyranyl-, Tetrahydrofurfuryl-, 1-Desoxy-1-D-sorbityl- oder 2-(2-Hydroxyethyloxy)ethylgruppe,

eine in 2- oder 3-Stellung durch eine Hydroxygruppe oder in 1-Stellung durch eine Hydroxymethylgruppe substituierte Cyclopentylgruppe,

eine in 2-, 3- oder 4-Stellung durch eine Hydroxymethyl-, Hydroxy-, $C_{1-2}$-Alkoxy-, ($C_{1-4}$-Alkoxy)carbonylamino-, Amino-, $C_{1-2}$-Alkylamino-, Di-$C_{1-2}$-alkylamino-, Carboxy-, $C_{1-2}$-Alkoxycarbonyl-, Aminocarbonyl-, $C_{1-2}$-Alkylaminocarbonyl-, Di-$C_{1-2}$-Alkylaminocarbonyl-, N-Oxido-di-$C_{1-2}$-alkylamino-, Pyrrolidinocarbonyl-, Morpholinocarbonyl-, $C_{1-2}$-Alkylcarbonylamino- oder $C_{1-2}$-Alkylsulfonylaminogruppe substituierte Cyclohexylgruppe, die zusätzlich durch eine Methylgruppe substituiert sein kann,

eine Cyclohexylgruppe, die in 4-Stellung durch eine 1-Pyrrolidinyl-, 2-Oxo-1-pyrrolidinyl-, 2-Hydroxymethyl-1-pyrrolidinyl-, N-$C_{1-2}$-Alkyl-(2-hydroxyethyl)amino-, Di-(2-hydroxyethyl)amino-, N-$C_{1-2}$-Alkyl-$C_{1-2}$-alkylcarbonylamino-, Morpholino-, 2-Oxo-1-imidazolidinyl-, 3-Methyl-2-oxo-1-imidazolidinyl-, Carboxy-$C_{1-2}$-alkyl-, Carboxy-$C_{1-2}$-alkoxy-, $C_{1-2}$-Alkoxycarbonyl-$C_{1-2}$-alkyl-, $C_{1-2}$-Alkoxycarbonyl-$C_{1-2}$-alkoxy-, Aminocarbonyl-$C_{1-2}$-alkyl-, Aminocarbonyl-$C_{1-2}$-alkoxy-, $C_{1-2}$-Alkylaminocarbonyl-$C_{1-2}$-alkyl-, $C_{1-2}$-Alkylaminocarbonyl-$C_{1-2}$-alkoxy-, Di-$C_{1-2}$-Alkylaminocarbonyl-$C_{1-2}$-alkyl-, Di-$C_{1-2}$-Alkylaminocarbonyl-$C_{1-2}$-alkoxy-, Pyrrolidinocarbonyl-$C_{1-2}$-alkyl-, Pyrrolidinocarbonyl-$C_{1-2}$-alkoxy-, Morpholinocarbonyl-$C_{1-2}$-alkyl-, Morpholinocarbonyl-$C_{1-2}$-alkoxy-, Piperidinocarbonyl-$C_{1-2}$-alkyl- oder Piperidinocarbonyl-$C_{1-2}$-alkoxygruppe substituiert ist, eine Cyclohexylgruppe, in der zwei Wasserstoffatome in 4-Stellung durch eine Oxo-Gruppe oder eine n-$C_{4-5}$-Alkylenbrücke ersetzt sind,

eine Cyclohexylgruppe, in der die Methylengruppe in 4-Stellung durch eine Imino-, $C_{1-2}$-Alkyl-imino-, Phenyl-$C_{1-2}$-alkyl-imino-N-Methyl-N-oxido-imino-, Formyl-imino-, $C_{1-2}$-Alkylcarbonylimino-, $C_{1-2}$-Alkylsulfonyl-imino-, $C_{1-2}$-Alkoxycarbonyl-imino-, Cyan-imino-, Aminocarbonyl-imino-, $C_{1-2}$-Alkylaminocarbonylimino- oder N,N-Di-$C_{1-2}$-alkylaminocarbonyliminogruppe ersetzt ist,

eine Cyclohexylgruppe, in der die Methylengruppe in 3-Stellung durch eine Imino-, $C_{1-2}$-Alkyl-imino-, $C_{1-2}$-Alkylcarbonylimino-, $C_{1-2}$-Alkylsulfonyl-imino- oder $C_{1-2}$-Alkoxycarbonyl-iminogruppe ersetzt ist,

eine Cyclopentylgruppe, in der die Methylengruppe in 3-Stellung durch ein Sauerstoffatom oder eine Imino-, $C_{1-2}$-Alkyl-imino-, Formyl-imino-, $C_{1-2}$-Alkylcarbonyl-imino-, $C_{1-2}$-Alkylsulfonylimino-, $C_{1-2}$-Alkoxycarbonyl-imino-, Cyan-imino- oder N,N-Di-$C_{1-2}$-alkylaminocarbonyliminogruppe ersetzt ist,

eine Cyclohexylmethylgruppe, wobei der Cyclohexylteil in 4-Stellung durch eine Carboxy-, $C_{1-2}$-Alkoxycarbonyl-, N,N-Di-$C_{1-2}$-Alkylaminocarbonyl- oder Morpholinocarbonylgruppe substituiert ist,

eine Norbornan-2-yl-, Norbornan-2-yl-methyl-, 5-Norbornen-2-yl-methyl-, Bornyl-, 3-Chinuclidinyl- oder Adamantylgruppe oder

$R_{10}$ ein Wasserstoffatom oder eine Methylgruppe und $R_{11}$ eine Hydroxy- oder Methoxygruppe bedeuten,

deren Tautomere, deren Stereoisomere und deren Salze.

**6.** Pyrimido[5,4-d]pyrimidine der allgemeinen Formel I gemäß Anspruch 1 oder 2, in der

$R_a$ ein Wasserstoffatom oder eine Methylgruppe,

$R_b$ eine 2-Naphthyl- oder 5-Indanylgruppe oder eine durch die Reste $R_1$ bis $R_3$ substituierte Phenylgruppe, wobei

$R_1$ ein Wasserstoff-, Fluor-, Chlor-, Brom- oder Jodatom, eine Methyl-, Ethyl-, tert.Butyl-, Trifluormethyl-, Ethinyl-, Methoxy-, Cyclopropyl-, Trifluormethoxy-, Cyano-, Ethoxycarbonyl- oder Nitrogruppe,

$R_2$ ein Wasserstoff-, Fluor- oder Chloratom, eine Amino-, Methyl- oder Trifluormethylgruppe und

$R_3$ ein Wasserstoff-, Chlor- oder Bromatom darstellen, und

$R_c$ ein Wasserstoffatom, eine Hydroxy-, Methoxy-, Butyloxy-, Cyclopentyloxy-, 2-[(2-Hydroxyethyl) amino]-ethoxy-, Methylsulfenyl-, Methylsulfinyl- oder Methylsulfonylgruppe,

eine 1-Azetidinyl- oder eine gegebenenfalls durch eine oder zwei Methylgruppen substituierte 1-Pyrrolidinylgruppe,

eine durch eine Hydroxymethylgruppe substituierte 1-Piperidinylgruppe,

eine gegebenenfalls durch eine oder zwei Methylgruppen substituierte 1-Piperidinylgruppe, in der die Methylengruppe in 4-Stellung durch ein Sauerstoff- oder Schwefelatom, durch eine Carbonyl-, Sulfinyl-, Sulfonyl-, Imino-, Methyl-imino-, N-Oxido-N-methyl-imino-, 2-Propylaminocarbonyl-methyl-imino-, Phenyl-imino-, Benzyl-imino-, Acetyl-imino- oder Methylsulfonyl-iminogruppe ersetzt sein kann,

eine in 3-Stellung durch eine Hydroxy- oder Diethylaminocarbonylgruppe oder in 4-Stellung durch eine Hydroxy-, Carboxy-, Methoxycarbonyl-, Aminocarbonyl-, Methylaminocarbonyl-, Dimethylaminocarbonyl-, Pyrrolidinocarbonyl-, Morpholinocarbonyl-, Amino-, Acetylamino-, Methoxycarbonylamino-, Formylamino-, Cyanamino-, Dimethylaminocarbonylamino-, Methylsulfonylamino- oder Phenylgruppe substituierte 1-Piperidinylgruppe,

eine 4-Hydroxy-4-phenyl-1-piperidinylgruppe,

eine 1,2,3,4-Tetrahydro-isochinolin-2-yl- oder 2,3,4,5-Tetrahydro-1H-3-benzazepin-3-yl-Gruppe,

eine 1-Piperidinylgruppe, in der in 4-Stellung zwei Wasserstoffatome durch eine $-OCH_2CH_2-O-$Brücke ersetzt sind,

eine 1-Azacyclohept-1-yl-Gruppe, in der zwei Wasserstoffatome in 3- und 6-Stellung durch eine $-CH_2-CH_2-$Gruppe ersetzt sind, oder

eine $(R_{10}NR_{11})$-Gruppe darstellen, in der

$R_{10}$ ein Wasserstoffatom, eine $C_{1-4}$-Alkylgruppe oder eine 2-Hydroxyethylgruppe und

$R_{11}$ ein Wasserstoffatom,

eine gegebenenfalls durch eine Methylgruppe substituierte Phenylgruppe,

eine Phenylgruppe, die in 4-Stellung durch eine Morpholino- oder 2-(Hydroxymethyl)-1-pyrrolidinylgruppe substituiert ist,

eine $C_{1-6}$-Alkyl-, $C_{3-6}$-Cycloalkyl-, Phenyl-$C_{1-3}$-alkyl-, Cyclopropylmethyl-, Allyl-, Propargyl-, 2-Hydroxyethyl-, 1-Hydroxy-2-propyl-, 3-Hydroxypropyl-, 4-Hydroxybutyl-, 2-Methoxyethyl-, 1-Adamantyl-, Norbornan-2-yl-, Aminocarbonylmethyl-, 2-(Dimethylamino)ethyl-, 3-Chinuclidinyl-, 2,2,2-Trifluorethyl-, 4-Piperidinyl-, 1-Methyl-4-piperidinyl-, 1-Methyl-1-oxido-4-piperidinyl-, 1-Ethoxycarbonyl-4-piperidinyl-, 1-Benzyl-4-piperidinyl-, 2-(2-Hydroxyethoxy)ethyl-, 4-Tetrahydropyranyl-, 1-Hydroxy-2-methyl-2-propyl-, 1-Methoxy-2-methyl-2-propyl-, 2-(Methylaminocarbonyl) -2-propyl-, 2,3-Dihydroxy-1-propyl-, 2-(Morpholino)ethyl-, 1-Desoxy-1-D-sorbityl-, 3-(2-Oxo-1-pyrrolidinyl)-propyl-, Tris-(hydroxymethyl)methyl-, 1,3-Dihydroxy-2-propyl-, 1,3-Dihydroxy-2-methyl-2-propyl- oder Bornylgruppe,

eine 2-Hydroxyethylgruppe, die in 2-Stellung durch eine Phenylgruppe und in 1-Stellung zusätzlich durch eine Methyl- oder Hydroxymethylgruppe substituiert ist,

eine Methylcyclohexyl-, 4-Carboxy-cyclohexyl-, 4-Methoxycarbonyl-cyclohexyl-, 4-Dimethylaminocarbonyl-cyclohexyl-, 4-(1-Pyrrolidinylcarbonyl)-cyclohexyl-, 4-(Morpholinocarbonyl)-cyclohexyl-, 4-[2-(Methoxycarbonyl)ethyl]cyclohexyl-, 4-(2-Carboxy-ethyl)cyclohexyl-, 4-(tert.Butyloxycarbonylamino)-cyclo-hexyl-, 4-Methoxycyclohexyl-, 4-Aminocyclohexyl-, 4-(Dimethylamino)cyclohexyl-, 4-(N,N-Dimethyl-N-oxido-amino)cyclohexyl-, 4-(Acetylamino)-cyclohexyl-, 4-(Methylsulfonylamino)cyclohexyl-, 2-Hydroxycyclohexyl-, 4-Hydroxycyclohexyl-, 4-(Hydroxymethyl)-cyclohexyl-, 4-Hydroxy-4-methyl-cyclohexyl- oder 4-Oxocyclohexylgruppe,

eine durch eine 1,4,7,10,13-Pentaoxacyclopentadecyl- oder eine 1,4,7,10,13,16-Hexaoxacyclooctadecylgruppe substituierte Methylgruppe, oder

$R_{10}$ eine Methylgruppe und $R_{11}$ eine Methoxygruppe darstellen, bedeuten,

deren Tautomere, deren Stereoisomere und deren Salze.

**7.** Folgende Verbindungen der allgemeinen Formel I gemäß Anspruch 1 oder 2:

(1) 4-[(3,4-Dichlorphenyl)amino]-6-[(trans-4-hydroxycyclohexyl)-amino]-pyrimido[5,4-d]pyrimidin,

(2) 4-[(3-Chlor-4-fluor-phenyl)amino]-6-[(trans-4-hydroxycyclohexyl)-amino]-pyrimido[5,4-d]pyrimidin,

(3) 4-[(3-Bromphenyl)amino]-6-[(trans-4-hydroxycyclohexyl)amino]pyrimido[5,4-d]pyrimidin,

(4) 4-[(3-Chlorphenyl)amino]-6-(cyclopropylamino)-pyrimido[5,4-d]pyrimidin,

(5) 4-[(3-Methylphenyl)amino]-6-(4-amino-1-piperidinyl)-pyrimido[5,4-d]pyrimidin,

(6) 4-[(3-Methylphenyl)amino]-6-[(trans-4-aminocyclohexyl)amino]pyrimido[5,4-d]pyrimidin,

(7) 4-[(3-Methylphenyl)amino]-6-(N-(trans-4-hydroxycyclohexyl)-N-methyl-amino)-pyrimido [5,4-d]pyrimidin,

(8) 4-[(3-Methylphenyl)amino]-6-(4-methoxycarbonylamino-1-piperidinyl)-pyrimido [5,4-d]pyrimidin,

(9) 4-[(3-Methylphenyl)amino]-6-[trans-4-(morpholinocarbonyl)cyclohexylamino]-pyrimido[5,4-d]pyrimidin,

(10) 4-[(3-Methylphenyl)amino]-6-[trans-4-(pyrrolidinocarbonyl)cyclohexylamino]-pyrimido[5,4-d]pyrimidin,

(11) 4-[(3-Chlor-4-fluor-phenyl)amino]-6-[N-(trans-4-hydroxycyclohexyl)-N-methyl-amino]-pyrimido[5,4-d] pyrimidin,

(12) 4-[(4-Amino-3,5-dichlor-phenyl)amino]-6-[(trans-4-hydroxycyclohexyl)-amino]-pyrimido[5,4-d]pyrimidin,

(13) 4-[(3-Chlor-4-fluor-phenyl)amino]-6-[2-(morpholino)ethylamino]-pyrimido [5,4-d]pyrimidin,

(14) 4-[(4-Amino-3, 5-dibrom-phenyl) amino]-6-[(trans-4-hydroxycyclohexyl)amino]-pyrimido [5,4-d]pyrimidin,

(15) 4-[(3-Chlor-4-fluor-phenyl)amino]-6-morpholino-pyrimido[5,4-d]pyrimidin,

(16) 4-[(3-Chlor-4-fluor-phenyl)amino]-6-(1-hydroxy-2-methyl-2-propylamino)-pyrimido[5,4-d]pyrimidin,

(17) 4-[(3-Chlor-4-fluor-phenyl)amino]-6-(1-hydroxy-2-propylamino)-pyrimido [5,4-d]pyrimidin,

(18) 4-[(3-Chlor-4-fluor-phenyl)amino]-6-(1,3-dihydroxy2-propylamino)-pyrimido [5,4-d]pyrimidin,

(19) 4- [(3-Chlor-4-fluor-phenyl)amino]-6-[4-amino-1-piperidinyl]-pyrimido[5,4-d]pyrimidin,

(20) 4-[(3-Methylphenyl)amino]-6-(4-piperidinyl-amino)pyrimido[5,4-d]pyrimidin,

(21) 4-[(3-Methylphenyl)amino]-6-(4-formylamino-1-piperidinyl)pyrimido[5,4-d]pyrimidin,

(22) 4-[(3-Methylphenyl)amino]-6-[(1-ethoxycarbonyl-4-piperidinyl)amino]-pyrimido[5,4-d]pyrimidin,

(23) 4-[(3-Methylphenyl)amino]-6-[(3-chinuclidinyl)amino]pyrimido[5,4-d]pyrimidin,

(24) 4-[(3-Chlor-4-fluor-phenyl)amino]-6-[(4-aminocyclohexyl)amino]-pyrimido[5,4-d]pyrimidin,

(25) 4-[(3-Chlor-4-fluor-phenyl)amino]-6-(4-piperidinyl-amino)-pyrimido[5,4-d]pyrimidin,

(26) 4-[(3-Chlor-4-fluor-phenyl)amino]-6-[trans-4-(morpholinocarbonyl)cyclohexylamino]-pyrimido[5,4-d]pyrimidin,

(27) 4-[(3-Chlor-4-fluor-phenyl)amino]-6-[trans-4-(pyrrolidinocarbonyl)cyclohexylamino]-pyrimido[5,4-d]pyrimidin,

(28) 4-[(4-Fluorphenyl)amino)-6-(cyclopropylamino)-pyrimido[5,4-d]pyrimidin,

(29) 4-[(3-Chlor-4-fluor-phenyl)amino]-6-(cyclopropylamino)-pyrimido[5,4-d]pyrimidin,

(30) 4-[(3,4-Dichlorphenyl)amino]-6-(cyclopropylamino)-pyrimido[5,4-d]pyrimidin,

(31) 4-[(3-Chlor-4-fluor-phenyl)amino]-6-(1-methyl-4-piperidinyl-amino)-pyrimido[5,4-d]pyrimidin und

(32) 4-[(3-Chlor-4-fluor-phenyl)amino]-6-[(trans-4-dimethylaminocyclohexyl)amino]-pyrimido[5,4-d]pyrimidin sowie deren Salze.

**8.** 4-[(3-Chlor-4-fluor-phenyl)amino]-6-(1-methyl-4-piperidinylamino)-pyrimido[5,4-d]pyrimidin und dessen Salze.

**9.** 4-[(3-Chlor-4-fluor-phenyl)amino]-6-[(trans-4-dimethylaminocyclohexyl)amino]-pyrimido[5,4-d]pyrimidin pyrimidin und dessen Salze.

**10.** Physiologisch verträgliche Salze der Verbindungen nach mindestens einem der Ansprüche 1 bis 9 mit anorganischen oder organischen Säuren oder Basen.

**11.** Arzneimittel, enthaltend eine Verbindung nach mindestens einem der Ansprüche 1 bis 9 oder ein physiologisch verträgliches Salz gemäß Anspruch 10 neben gegebenenfalls einem oder mehreren inerten Trägerstoffen und/oder Verdünnungsmitteln.

**12.** Verwendung einer Verbindung nach mindestens einem der Ansprüche 1 bis 10 zur Herstellung eines Arzneimittels, das zur Behandlung von benignen oder malignen Tumoren, insbesondere Tumoren epithelialen und neuroepithelialen Ursprungs, der Metastasierung sowie der abnormen Proliferation vaskulärer Endothelzellen (Neoangiogenese), geeignet ist.

**13.** Verfahren zur Herstellung eines Arzneimittels gemäß Anspruch 11, dadurch gekennzeichnet, daß auf nichtchemischem Wege eine Verbindung nach mindestens einem der Ansprüche 1 bis 10 in einen oder mehrere inerte Trägerstoffe und/oder Verdünnungsmittel eingearbeitet wird.

**14.** Verfahren zur Herstellung der Verbindungen der allgemeinen Formel I gemäß den Ansprüchen 1 bis 10, dadurch gekennzeichnet, daß

a) eine Verbindung der allgemeinen Formel

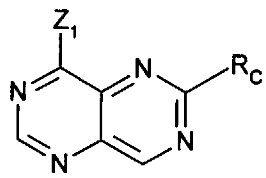

, (II)

in der

$R_c$ wie in den Ansprüchen 1 bis 9 definiert ist und
$Z_1$ eine Austrittsgruppe darstellt, mit einem Amin der allgemeinen Formel

$$H\text{-}(R_aNR_b)\ , \qquad (III)$$

in der
$R_a$ und $R_b$ wie in den Ansprüchen 1 bis 9 definiert sind, umgesetzt wird oder

b) zur Herstellung von Verbindungen der allgemeinen Formel I, in der $R_c$ einen der für $R_c$ in den Ansprüchen 1 bis 8 erwähnten über ein Sauerstoff- oder Stickstoffatom, über eine Mercapto- oder Sulfenylgruppe mit dem Pyrimido[5,4-d]pyrimidin verknüpften Reste darstellt, eine Verbindung der allgemeinen Formel

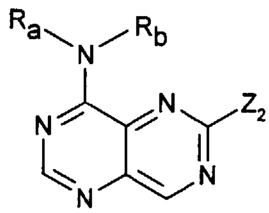

, (IV)

in der

$R_a$ und $R_b$ wie in den Ansprüchen 1 bis 9 definiert sind und
$Z_2$ eine Austrittsgruppe darstellt, mit einer Verbindung der allgemeinen Formel

$$X_1\text{ - H}\ , \qquad (V)$$

in der
$X_1$ einen der für $R_c$ in den Ansprüchen 1 bis 9 erwähnten über ein Sauerstoff- oder Stickstoffatom, über eine Mercapto- oder Sulfenylgruppe mit dem Pyrimido[5,4-d]pyrimidin verknüpften Reste darstellt, umgesetzt wird oder

c) zur Herstellung von Verbindungen der allgemeinen Formel I, in der $R_c$ einen der für $R_c$ in den Ansprüchen 1 bis 9 erwähnten über eine Sulfinyl- oder Sulfonylgruppe mit dem Pyrimido[5,4-d]pyrimidin verknüpften Reste darstellt, eine Verbindung der allgemeinen Formel

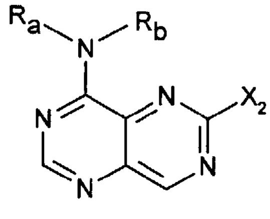

, (VI)

in der

$R_a$ und $R_b$ wie in den Ansprüchen 1 bis 9 definiert sind und
$X_2$ einen der für $R_c$ in den Ansprüchen 1 bis 8 erwähnten über ein Schwefelatom mit dem Pyrimido[5,4-d]pyrimidin verknüpften Reste darstellt, oxidiert wird oder

d) zur Herstellung einer Verbindung der allgemeinen Formel I, in der $R_c$ ein Wasserstoffatom darstellt, eine Verbindung der allgemeinen Formel

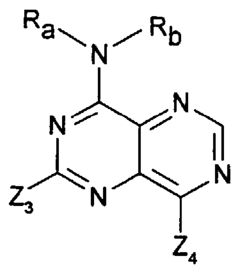

, (VII)

in der

$R_a$ und $R_b$ wie in den Ansprüchen 1 bis 9 definiert sind,

$Z_3$ und $Z_4$, die gleich oder verschieden sein können, jeweils ein Halogenatom darstellen, enthalogeniert wird und

gewünschtenfalls anschließend eine so erhaltene Verbindung der allgemeinen Formel I, die eine Amino-, Alkylamino- oder Iminogruppe enthält, mittels Acylierung oder Sulfonylierung in eine entsprechende Acyl- oder Sulfonylverbindung der allgemeinen Formel I übergeführt wird und/oder

eine so erhaltene Verbindung der allgemeinen Formel I, die eine Amino-, Alkylamino- oder Iminogruppe enthält, mittels Alkylierung oder reduktiver Alkylierung in eine entsprechende Alkylverbindung der allgemeinen Formel I übergeführt werden und/oder

eine so erhaltene Verbindung der allgemeinen Formel I, die eine Carboxygruppe enthält, mittels Veresterung in einen entsprechenden Ester der allgemeinen Formel I übergeführt werden und/oder

eine so erhaltene Verbindung der allgemeinen Formel I, die eine Carboxy- oder Estergruppe enthält, mittels Amidierung in ein entsprechendes Amid der allgemeinen Formel I übergeführt werden und/oder

eine Verbindung der allgemeinen Formel I, die eine primäre oder sekundäre Hydroxygruppe enthält, mittels Oxidation in eine entsprechende Carbonylverbindung der allgemeinen Formel I übergeführt werden und/oder

erforderlichenfalls ein bei den vorstehend beschriebenen Umsetzungen verwendeter Schutzrest wieder abgespalten wird und/oder

gewünschtenfalls eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträgliche Salze übergeführt wird.

**Claims**

**1.** Pyrimido[5,4-d]pyrimidines of the formula

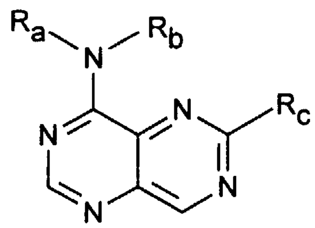

(I),

in which

$R_a$ is a hydrogen atom or an alkyl group,

$R_b$ is a phenyl group which is substituted by the radicals $R_1$ to $R_3$, wherein

$R_1$ and $R_2$, which can be identical or different, are each a hydrogen, fluorine, chlorine, bromine or iodine atom,

a $C_{1-6}$-alkyl, hydroxyl or $C_{1-6}$alkoxy group,

a $C_{3-7}$-cycloalkyl or $C_{4-7}$-Cycloalkoxy group, each of which can be substituted by one or two alkyl groups or by an aryl group,

a $C_{2-5}$-alkenyl or $C_{3-5}$-alkenyloxy group which is optionally substituted by an aryl group, wherein the vinyl part cannot be linked to the oxygen atom,

a $C_{2-5}$-alkynyl or $C_{3-5}$-alkynyloxy group which is optionally substituted by an aryl group, wherein the ethynyl part cannot be linked to the oxygen atom,

an aryl, aryloxy, aralkyl, aralkoxy, alkylsulphenyl, alkylsulphinyl, alkylsulphonyl, alkylsulphonyloxy, trifluoromethylsulphenyl, trifluoromethylsulphinyl, trifluoromethylsulphonyl, arylsulphenyl, arylsulphinyl, arylsulphonyl, aralkylsulphenyl, aralkylsulphinyl or aralkylsulphonyl group,

a methyl or methoxy group which is substituted by 1 to 3 fluorine atoms,

a $C_{2-4}$-alkyl or $C_{2-4}$-alkoxy group which is substituted by 1 to 5 fluorine atoms,

a nitro, amino, alkylamino, dialkylamino, $C_{3-7}$-cycloalkylamino, N-alkyl-$C_{3-7}$-cycloalkylamino, arylamino, N-alkyl-arylamino, aralkylamino or N-alkyl-aralkylamino group,

a 4- to 7-membered alkylenimino group which is optionally substituted by 1 to 4 alkyl groups, wherein, in the abovementioned 5- to 7-membered alkylenimino groups, in each case one or two methylene groups adjacent to the nitrogen atom can be replaced by a carbonyl group, or in the abovementioned 6- to 7-membered alkylenimino groups, a methylene group in the 4-position can be replaced by an oxygen or sulphur atom or by a sulphinyl, sulphonyl, imino, N-alkyl-imino, N-alkyl-carbonyl-imino, N-alkylsulphonyl-imino, N-aryl-imino or N-aralkyl-imino group,

an (alkylenimino)carbonyl or (alkylenimino)sulphonyl group which has in each case 4 to 7 ring atoms in the alkylenimino part and is optionally substituted by 1 to 4 alkyl groups, wherein, in the abovementioned 6- to 7-membered alkylenimino parts, in each case a methylene group in the 4-position can be replaced by an oxygen or sulphur atom or by a sulphinyl, sulphonyl, imino, N-alkyl-imino, N-alkylcarbonyl-imino, N-alkylsulphonylimino, N-aryl-imino or N-aralkyl-imino group,

an alkylcarbonylamino, N-alkyl-alkylcarbonylamino, alkylsulphonylamino, N-alkyl-alkylsulphonylamino, arylcarbonylamino, N-alkyl-arylcarbonylamino, arylsulphonylamino, N-alkyl-aryl-sulphonylamino, aralkylcarbonylamino, N-alkyl-aralkylcarbonylamino, aralkylsulphonylamino, N-alkyl-aralkylsulphonylamino, perfluoroalkylsulphonylamino, N-alkylperfluoroalkylsulphonylamino, alkylcarbonyl, arylcarbonyl, aralkylcarbonyl, aryl-hydroxymethyl, aralkyl-hydroxymethyl, carboxyl, alkoxycarbonyl, aralkoxycarbonyl, aminocarbonyl, alkylaminocarbonyl, dialkylaminocarbonyl, arylaminocarbonyl, N-alkylarylaminocarbonyl, aralkylaminocarbonyl, N-alkylaralkylaminocarbonyl, N-hydroxy-aminocarbonyl, N-hydroxy-alkylaminocarbonyl, N-alkoxy-aminocarbonyl, N-alkoxy-alkylaminocarbonyl, cyano, azido, N-cyano-amino or N-cyano-alkylamino group,

a sulpho, alkoxysulphonyl, aminosulphonyl, alkylaminosulphonyl, dialkylaminosulphonyl, arylaminosulphonyl, N-alkylarylaminosulphonyl, aralkylaminosulphonyl or N-alkyl-aralkylaminosulphonyl group,

a phosphono, O-alkyl-phosphono, O,O'-dialkyl-phosphono, O-aralkyl-phosphono or O,O'-diaralkyl-phosphono group,

an alkyl or alkoxy group which is substituted by $R_4$, wherein

$R_4$ is a hydroxyl, alkoxy, aryloxy, aralkoxy, amino, alkylamino, dialkylamino, alkylsulphenyl, alkylsulphinyl, alkylsulphonyl, arylsulphenyl, arylsulphinyl, arylsulphonyl, aralkylsulphenyl, aralkylsulphinyl, aralkylsulphonyl, carboxyl, alkoxycarbonyl, aminocarbonyl, alkylaminocarbonyl, dialkylaminocarbonyl or cyano group,

a 4- to 7-membered alkylenimino group which is optionally substituted by 1 to 4 alkyl groups, wherein, in the abovementioned 5- to 7-membered alkylenimino groups, in each case one or two methylene groups adjacent to the nitrogen atom can be replaced by a carbonyl group, or in the abovementioned 6- to 7-membered alkylenimino groups, a methylene group in the 4-position can be replaced by an oxygen or sulphur atom or by a sulphinyl, sulphonyl, imino, N-alkyl-imino, N-alkylcarbonylimino, N-alkylsulphonyl-imino, N-aryl-imino or N-aralkyl-imino group, or

an (alkylenimino)carbonyl group which has in each case 4 to 7 ring atoms in the alkylenimino part and is optionally substituted by 1 to 4 alkyl groups, wherein, in the abovementioned 6- to 7-membered alkylenimino parts, in each case a methylene group in the 4-position can be replaced by an oxygen or sulphur atom or by a sulphinyl, sulphonyl, imino, N-alkyl-imino, N-alkylcarbonyl-imino, N-alkylsulphonyl-imino, N-aryl-imino or N-aralkyl-imino group,

$R_3$ is a hydrogen, fluorine, chlorine or bromine atom, an alkyl, alkoxy or trifluoromethyl group or

$R_2$ together with $R_3$, if these are bonded to adjacent carbon atoms, may also denote a methylenedioxy group which is optionally substituted by one or two alkyl groups, an n-$C_{3-6}$-alkylene group which is optionally substituted by one or two alkyl groups, or a 1,3-butadiene-1,4-diylene group which is optionally substituted by one or two fluorine, chlorine, bromine or iodine atoms or by one or two hydroxyl, alkyl, alkoxy, trifluoromethyl or cyano groups, wherein the substituents can be identical or different, or

$R_a$ together with $R_1$, if $R_1$ is in the o-position relative to the nitrogen atom which is substituted by $R_a$, is an n-$C_{2-4}$-alkylene group which is optionally substituted by one or two alkyl groups, and

$R_c$ is a hydrogen, fluorine, chlorine, bromine or iodine atom,

an alkyl, $C_{3-7}$-cycloalkyl, $C_{3-7}$-cycloalkyl-$C_{1-3}$-alkyl, aryl, aralkyl, hydroxyl, aryloxy, aralkoxy, mercapto, $C_{1-8}$-alkylsulphenyl, $C_{1-8}$-alkylsulphinyl, $C_{1-8}$-alkylsulphonyl, $C_{4-7}$-cycloalkylsulphenyl, $C_{4-7}$-cycloalkylsulphinyl, $C_{4-7}$-cycloalkylsulphonyl, $C_{3-7}$-cycloalkyl-$C_{1-3}$-alkylsulphenyl, $C_{3-7}$-cycloalkyl-$C_{1-3}$-alkylsulphinyl, $C_{3-7}$-cycloalkyl-$C_{1-3}$-alkylsulphonyl, arylsulphenyl, arylsulphinyl, arylsulphonyl, aralkylsulphenyl, aralkylsulphinyl or aralkylsulphonyl group,

a $C_{1-8}$-alkoxy group, which can be substituted by an alkoxycarbonyl, cyano, carboxyl, aminocarbonyl, alkylaminocarbonyl or dialkylaminocarbonyl group,

a $C_{2-8}$-alkoxy group substituted by a hydroxyl, alkoxy, hydroxy-$C_{2-4}$-alkylamino, amino, alkylamino, dialkylamino, alkylcarbonylamino, N-alkyl-N-(alkylcarbonyl)amino, alkylsulphonylamino, N-alkyl-N-(alkylsulphonyl)amino, alkoxycarbonylamino or N-alkyl-N-(alkoxycarbonyl)amino group, by a 5- to 7-membered alkylenimino group which is optionally substituted by 1 to 4 alkyl groups, wherein, in the abovementioned 5- to 7-membered

alkylenimino groups, in each case one or two methylene groups adjacent to the nitrogen atom can be replaced by a carbonyl group, and additionally in the abovementioned 6- to 7-membered alkylenimino groups, a methylene group in the 4-position can be replaced by an oxygen or sulphur atom or by a carbonyl, sulphinyl, sulphonyl, imino, N-alkyl-imino, N-alkylcarbonyl-imino, N-formylimino, N-dialkylaminocarbonyl-imino, N-alkoxy-carbonylimino, N-alkylsulphonyl-imino, N-aryl-imino or N-aralkyl-imino group,

a $C_{3-8}$-alkoxy group substituted by two hydroxyl or alkoxy groups,

a $C_{1-8}$-alkoxy group substituted by a $C_{3-7}$-cycloalkyl group wherein in each case the cycloalkyl residue can be substituted by 1 to 4 alkyl groups, and wherein, in the abovementioned $C_{4-7}$-cycloalkyl residues, in each case a methylene group can be replaced by an oxygen or sulphur atom or by a carbonyl, sulphinyl, sulphonyl, imino, N-alkyl-imino, N-alkylcarbonyl-imino, N-alkoxycarbonylimino, N-alkylsulphonyl-imino, N-aryl-imino or N-aralkyl-imino group,

a $C_{4-7}$-cycloalkoxy group which is optionally substituted by one or two hydroxyl groups or by an alkoxy, alkoxycarbonyl, cyano, carboxyl, amino-carbonyl, alkylaminocarbonyl, dialkylaminocarbonyl, hydroxy-$C_{2-4}$-alkylamino, amino, alkylamino, dialkylamino, alkylcarbonyl-amino, N-alkyl-N-(alkylcarbonyl)amino, alkylsulphonylamino, N-alkyl-N-(alkylsulphonyl)amino, alkoxycarbonylamino or N-alkyl-N-(alkoxycarbonyl)amino group, wherein, in the abovementioned $C_{5-7}$-cycloalkoxy groups, in each case a methylene group can be replaced by an oxygen or sulphur atom or by a sulphinyl, sulphonyl, imino, N-alkyl-imino, N-alkylcarbonyl-imino, N-alkoxycarbonyl-imino, N-alkylsulphonyl-imino, N-aryl-imino or N-aralkyl-imino group,

a $C_{3-8}$-alkenyloxy group which is optionally substituted by an aryl group or $C_{3-7}$-cycloalkyl group, wherein the vinyl part cannot be linked to the oxygen atom,

a $C_{3-8}$-alkynyloxy group which is optionally substituted by an aryl group or $C_{3-7}$-cycloalkyl group, wherein the ethynyl part cannot be linked to the oxygen atom,

a 4- to 8-membered alkylenimino group which is optionally substituted by 1 to 4 alkyl groups or 1 to 2 aryl groups, and which can additionally be substituted by the radical $R_5$, wherein

$R_5$ is an aryl, aralkyl, carboxyl, alkoxycarbonyl, amino-carbonyl, alkylaminocarbonyl, dialkylaminocarbonyl, cyano, hydroxyl, alkoxy, aryloxy, aralkoxy, alkylcarbonyloxy, arylcarbonyloxy, amino, alkylamino, hydroxy-$C_{2-4}$-alkylamino, dialkylamino, cyano-amino, formylamino, N-(alkyl)-N-(hydroxy-$C_{2-4}$-alkyl)amino or bis-(hydroxy-$C_{2-4}$-alkyl)amino group,

an (alkylenimino)carbonyl group which has in each case 4 to 7 ring atoms in the alkylenimino part and is optionally substituted by 1 to 4 alkyl groups, wherein, in the abovementioned 6- to 7-membered alkylenimino parts, in each case a methylene group in the 4-position can be replaced by an oxygen or sulphur atom or by a sulphinyl, sulphonyl, imino, N-alkyl-imino, N-alkyl-carbonyl-imino, N-alkylsulphonyl-imino, N-aryl-imino or N-aralkyl-imino group,

a 4- to 7-membered alkylenimino group which is optionally substituted by 1 to 4 alkyl groups or a hydroxyalkyl group, wherein, in the abovementioned 5- to 7-membered alkylenimino groups, in each case one or two methylene groups adjacent to the nitrogen atom can be replaced by a carbonyl group, a 6- or 7-membered alkyleneimino group which is optionally substituted by 1 to 4 alkyl groups or a hydroxyalkyl group, whereby in each case a methylene group in the 4-position of the alkyleneimino residue is replaced by an oxygen or sulphur atom or by a carbonyl, sulphinyl, sulphonyl, imino, N-alkyl-imino, N-alkylcarbonylimino, N-alkyl-sulphonyl-imino, N-aryl-imino or N-aralkyl-imino group, and additionally in the alkyleneimino residue of the abovementioned groups in each case one or two of the methylene groups adjacent to the nitrogen atoms can be replaced by a carbonyl group,

a 4- to 7-membered alkylenimino group which is substituted by a hydroxyl, alkoxy, amino, alkylamino, dialkylamino, alkylcarbonylamino, N-alkyl-alkylcarbonylamino, alkylsulphonylamino, N-alkyl-alkylsulphonyl-amino or hydroxyalkyl group,

an alkylcarbonylamino, N-alkyl-alkylcarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, alkoxycarbonylamino, N-alkyl-alkoxycarbonylamino, aralkoxycarbonylamino or N-alkyl-aralkoxycarbonylamino group,

a $(NR_7R_8)CONR_6$- or $(NR_7R_8)SO_2NR_6$- group, in which

$R_6$, $R_7$ and $R_8$, which can be identical or different, are each a hydrogen atom or an alkyl group or
$R_6$ and $R_7$ together are an n-$C_{2-4}$-alkylene group and
R8 is a hydrogen atom or an alkyl group,

a carboxyalkyl, alkoxycarbonylalkyl, aminocarbonylalkyl, alkylaminocarbonylalkyl or dialkylaminocarbonylalkyl group,

an (alkylenimino)carbonylalkyl group which has in each case 4 to 7 ring atoms in the alkylenimino part and is optionally substituted by 1 to 4 alkyl groups, wherein, in the abovementioned 6- to 7-membered alkylenimino parts, in each case a methylene group in the 4-position can be replaced by an oxygen or sulphur atom or by a sulphinyl, sulphonyl, imino or N-alkyl-imino group,

a (carboxyalkyl)oxy, (alkoxycarbonylalkyl)oxy, (aminocarbonylalkyl)oxy, (alkylaminocarbonylalkyl)oxy or (dialkylaminocarbonylalkyl)oxy group,

an [(alkylenimino)carbonylalkyl]oxy group which has in each case 4 to 7 ring atoms in the alkylenimino part and is optionally substituted by 1 to 4 alkyl groups, wherein, in the abovementioned 6- to 7-membered alkylenimino parts, in each case a methylene group in the 4-position can be replaced by an oxygen or sulphur atom or by a sulphinyl, sulphonyl, imino or N-alkyl-imino group,

a cyanoalkyl, hydroxyalkyl, alkoxyalkyl, aryloxyalkyl, aminoalkyl, alkylaminoalkyl or dialkylaminoalkyl group,

an (alkylenimino)alkyl group which has in each case 4 to 7 ring atoms in the alkylenimino part and is optionally substituted by 1 to 4 alkyl groups, wherein, in the abovementioned 6- to 7-membered alkylenimino parts, in each case a methylene group in the 4-position can be replaced by an oxygen or sulphur atom or by a sulphinyl, sulphonyl, imino or N-alkyl-imino group,

an alkylcarbonylaminoalkyl, N-alkyl-alkylcarbonylaminoalkyl, alkylsulphonylaminoalkyl, N-alkylalkylsulphonylaminoalkyl, arylcarbonylaminoalkyl, N-alkyl-arylcarbonylaminoalkyl, arylsulphonylaminoalkyl, N-alkyl-arylsulphonylaminoalkyl, alkylsulphenyl, alkylsulphinyl, alkylsulphonyl, arylsulphenyl, arylsulphinyl, arylsulphonyl, aralkylsulphenyl, aralkylsulphinyl, aralkylsulphonyl, alkylsulphenylalkyl, alkylsulphinylalkyl, alkylsulphonylalkyl, arylsulphenylalkyl, arylsulphinylalkyl or arylsulphonylalkyl group or

a $C_{3-7}$-cycloalkyl group, wherein a methylene group in any $C_{5-7}$-cycloalkyl group can be replaced by an oxygen or sulphur atom or by a sulphinyl, sulphonyl, imino or N-alkylimino group,

or $R_c$ is a 6- to 8-membered alkylenimino group which is optionally substituted by 1 to 4 alkyl groups or an aryl group and can additionally be substituted by the radical $R_5$, wherein, in the abovementioned alkylenimino groups, in each case a methylene group in the 4-position is replaced by an oxygen or sulphur atom or by a carbonyl, sulphinyl, sulphonyl, N-oxido-N-alkylimino or $R_9N$ group, wherein

$R_9$ is a hydrogen atom, an alkyl, hydroxy-$C_{2-4}$-alkyl, alkoxy-$C_{2-4}$-alkyl, amino-$C_{2-4}$-alkyl, alkylamino-$C_{2-4}$-alkyl, dialkylamino-$C_{2-4}$-alkyl, (hydroxy-$C_{2-4}$-alkoxy)-$C_{2-4}$-alkyl, aminocarbonylalkyl, alkylaminocarbonylalkyl, dialkylaminocarbonylalkyl, aryl, aralkyl, formyl, alkylcarbonyl, alkylsulphonyl, arylcarbonyl, arylsulphonyl, aralkylcarbonyl, aralkylsulphonyl, alkoxycarbonyl, cyano, aminocarbonyl, alkylaminocarbonyl or dialkylaminocarbonyl group or an (alkylenimino)carbonyl group which has in each case 4 to 7 ring atoms in the alkylenimino part, wherein, in a 6- to 7-membered alkylenimino part, a methylene group in the 4-position can be replaced by an oxygen or sulphur atom or by a sulphinyl, sulphonyl, imino or N-alkylimino group,

or $R_c$ is a 1-pyrrolidinyl group which is optionally substituted by 1 to 4 alkyl groups and in which two hydrogen

atoms on the carbon skeleton are replaced by a straight-chain alkylene bridge, wherein this bridge contains 2 to 6 carbon atoms if the two hydrogen atoms are on the same carbon atom, or contains 1 to 5 carbon atoms if the two hydrogen atoms are on adjacent carbon atoms, or contains 2 to 4 carbon atoms if the two hydrogen atoms are on carbon atoms which are separated by an atom, wherein the abovementioned 1-pyrrolidinyl groups can additionally be substituted by the radical $R_5$, which is defined as mentioned above,

a 1-piperidinyl or 1-azacyclohept-1-yl group which is optionally substituted by 1 to 4 alkyl groups and in which two hydrogen atoms on the carbon skeleton are replaced by a straight-chain alkylene bridge, wherein this bridge contains 2 to 6 carbon atoms if the two hydrogen atoms are on the same carbon atom, or contains 1 to 5 carbon atoms if the two hydrogen atoms are on adjacent carbon atoms, or contains 1 to 4 carbon atoms if the two hydrogen atoms are on carbon atoms which are separated by an atom, or contains 1 to 3 carbon atoms if the two hydrogen atoms are on carbon atoms which are separated by two atoms, wherein the abovementioned 1-piperidinyl and 1-azacyclohept-1-yl groups can additionally be substituted by the radical $R_5$, which is defined as mentioned above,

a 1-pyrrolidinyl group which is optionally substituted by 1 to 4 alkyl groups and in which two hydrogen atoms in the 3-position are substituted by a $-O-CH_2CH_2-O-$ or $-O-CH_2CH_2CH_2-O-$ group,

a 1-piperidinyl or 1-azacyclohept-1-yl group which is optionally substituted by 1 to 4 alkyl groups and in which, in the 3-position or in the 4-position, in each case two hydrogen atoms are replaced by a $-O-CH_2CH_2-O-$ or $-O-CH_2CH_2CH_2-O-$ group,

a group of the formula

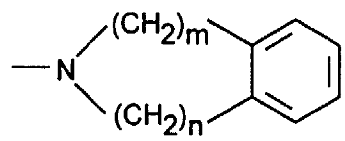

in which

m and n, which can be identical or different, are the numbers 1 to 3, or m is the number 0 and n is the number 2, 3 or 4, wherein, additionally, the above benzo part can be mono- or disubstituted by fluorine, chlorine, bromine or iodine atoms or by alkyl, trifluoromethyl, hydroxyl, alkoxy or cyano groups and the above saturated cyclic imino part can be mono- or disubstituted by 1 or 2 alkyl groups, wherein the substituents can in each case be identical or different, or

an ($R_{10}NR_{11}$) group, in which

$R_{10}$ and $R_{11}$, which can be identical or different, are each a hydrogen atom,

a $C_{1-16}$-alkyl group, which can be substituted by 1 or 2 aryl or $C_{3-7}$-cycloalkyl groups, by a carboxyl, alkoxycarbonyl, aminocarbonyl, alkylaminocarbonyl, dialkylaminocarbonyl, hydroxy-$C_{2-4}$-alkylaminocarbonyl, cyano, hydroxyl, alkoxy, aryloxy, aralkoxy, $C_{2-4}$-alkylenedioxy, alkylcarbonyloxy, arylcarbonyloxy, formylamino, amino, alkylamino or dialkylamino group, by an (alkylenimino)carbonyl group which has in each case 4 to 7 ring atoms in the alkylenimino part and is optionally substituted by 1 to 4 alkyl groups, wherein, in a 6- or 7-membered alkylenimino residue, in each case a methylene group in the 4-position can be replaced by an oxygen or sulphur atom or by a sulphinyl, sulphonyl, imino, N-alkyl-imino, N-alkylcarbonyl-imino, N-alkylsulphonyl-imino, N-aryl-imino or N-aralkyl-imino group,

by a 4- to 7-membered alkylenimino group which is optionally substituted by 1 to 4 alkyl groups, wherein, in the abovementioned 6- or 7-membered alkylenimino groups, in each case a methylene group in the 4-position can be replaced by an oxygen or sulphur atom or by a sulphinyl, sulphonyl or $R_9N$ group, wherein $R_9$ is as defined above, and additionally in the abovementioned 5- to 7-membered alkylenimino groups, in each case one or two methylene groups adjacent to the nitrogen atoms can be replaced by carbonyl groups,

by an alkylsulphonylamino, N-alkyl-alkylsulphonylamino, arylcarbonylamino, N-alkyl-arylcarbonyl-amino, alkylcarbonylamino, N-alkyl-alkylcarbonylamino, arylsulphonylamino, N-alkyl-arylsulphonylamino, aralkylcarbonylamino, N-alkylaralkylcarbonylamino, aralkylsulphonylamino, N-alkyl-aralkylsulphonylamino, alkoxycarbonylamino, N-alkyl-alkoxycarbonylamino, aralkoxycarbonylamino or N-alkyl-aralkoxycarbonylamino group, by an $(R_8NR_7)$-CO-$NR_6$- or $(R_8NR_7)$-$SO_2$-$NR_6$- group, wherein $R_6$, $R_7$ and $R_8$ are as defined above, by an alkylsulphenyl, alkylsulphinyl, alkylsulphonyl, arylsulphenyl, arylsulphinyl, arylsulphonyl, aralkylsulphenyl, aralkylsulphinyl or aralkylsulphonyl group, by a $C_{4-7}$-cycloalkyl group which is substituted by $R_5$ and optionally additionally by 1 to 4 alkyl groups, wherein $R_5$ is as defined above, by a $C_{5-7}$-cycloalkyl group which is optionally substituted by 1 to 4 alkyl groups and in which a methylene group is replaced by an oxygen or sulphur atom or by a sulphinyl, sulphonyl, or $NR_9$ group, or by a fluorine, chlorine, bromine or iodine atom,

a $C_{2-10}$-alkyl group substituted by 2 or 3 fluorine atoms,

a $C_{3-10}$-alkyl group substituted by 4 or 5 fluorine atoms,

a methyl group which is substituted by a 1,4,7,10-tetraoxacyclododecyl, 1,4,7,10,13-pentaoxacyclopentadecyl or a 1,4,7,10,13,16-hexaoxacyclooctadecyl group,

a $C_{3-10}$-alkyl group which is substituted by 2 to 5 hydroxyl or alkoxy groups,

a $C_{2-6}$-alkyl group which is substituted by an aryl group and a hydroxyl group and can additionally be substituted by an amino, alkylamino, dialkylamino, hydroxyl or alkoxy group,

a $C_{3-6}$-alkyl group which is substituted by an amino, alkylamino, dialkylamino, alkylcarbonylamino or alkoxycarbonylamino group and additionally by a hydroxyl or alkoxy group,

an alkenyl or alkynyl group which has in each case 3 to 6 carbon atoms and is optionally substituted by an aryl group or $C_{3-7}$-cycloalkyl group, wherein the vinyl or ethynyl part cannot be linked to the nitrogen atom,

a $C_{2-4}$-alkyl group which is substituted by a $C_{2-4}$-alkoxy group which is substituted in the $\omega$-position by a hydroxyl or alkoxy group,

an aryl group,

a cyclopropyl group which can be substituted by 1 or 2 alkyl groups, by an aryl, carboxyl, alkoxycarbonyl, aminocarbonyl, alkylaminocarbonyl or dialkylaminocarbonyl group or by an (alkylenimino)carbonyl group which has in each case 4 to 7 ring atoms in the alkylenimino part and is optionally substituted by 1 to 4 alkyl groups, wherein, in the abovementioned 6- or 7-membered alkylenimino parts, in each case a methylene group in the 4-position can be replaced by an oxygen or sulphur atom or by a sulphinyl, sulphonyl, imino, N-alkylimino, N-alkylcarbonyl-imino, N-alkylsulphonyl-imino, N-aryl-imino or N-aralkyl-imino group,

a $C_{4-7}$-cycloalkyl group which is optionally substituted by 1 to 4 alkyl groups and can additionally be substituted by $R_5$, which is as defined above,

a $C_{5-7}$-cycloalkyl group which is optionally substituted by 1 to 2 alkyl groups and is additionally substituted by an N,N-dialkyl-N-oxido-amino group,

a $C_{5-7}$-cycloalkenyl group which is optionally substituted by 1 to 4 alkyl groups, wherein the vinyl part cannot be linked to the nitrogen atom of the $(R_{11}NR_{10})$- group,

a $C_{4-7}$-cycloalkyl group which is optionally substituted by 1 to 4 alkyl groups and can additionally be substituted by $R_5$, wherein, in the cycloalkyl part, a methylene group is replaced by an oxygen or sulphur atom or by a sulphinyl, sulphonyl, N-alkyl-N-oxido-imino or $R_9N$ group, wherein $R_5$ and $R_9$ are as defined above,

a $C_5$-$C_7$-cycloalkyl or $C_5$-$C_7$-cycloalkylalkyl group which is optionally substituted by 1 to 4 alkyl groups and in which in each case a methylene group in the cycloalkyl part is replaced by a carbonyl group,

a cyclopentyl or cyclopentylalkyl group which is optionally substituted by 1 to 4 alkyl groups and in which in

each case two hydrogen atoms in the cyclopentyl part are replaced by a straight-chain alkylene bridge, wherein this bridge contains 2 to 6 carbon atoms if the two hydrogen atoms are on the same carbon atom, or contains 1 to 5 carbon atoms if the two hydrogen atoms are on adjacent carbon atoms, or contains 2 to 4 carbon atoms if the two hydrogen atoms are on carbon atoms which are separated by a carbon atom, wherein the abovementioned rings can additionally be substituted by the radical $R_5$, which is as defined above,

a cyclohexyl, cyclohexylalkyl, cycloheptyl or cycloheptylalkyl group which is optionally substituted by 1 to 4 alkyl groups and in which in each case two hydrogen atoms in the cycloalkyl part are replaced by a straight-chain alkylene bridge, wherein this bridge contains 2 to 6 carbon atoms if the two hydrogen atoms are on the same carbon atom, or contains 1 to 5 carbon atoms if the two hydrogen atoms are on adjacent carbon atoms, or contains 1 to 4 carbon atoms if the two hydrogen atoms are on carbon atoms which are separated by a carbon atom, or contains 1 to 3 carbon atoms if the two hydrogen atoms are on carbon atoms which are separated by two carbon atoms, wherein the abovementioned rings can additionally be substituted by the radical $R_5$, which is as defined above,

a 3-cyclohexen-1-yl or 3-cyclohexen-1-yl-alkyl group which is optionally substituted by 1 to 4 alkyl groups and in which two hydrogen atoms in the 2,5-position in the cyclohexenyl part are replaced by an n-$C_{1-3}$-alkylene bridge,

a 3-quinuclidinyl, 4-quinuclidinyl, 2-quinuclidinylalkyl, 3-quinuclidinyl-alkyl, 4-quinuclidinyl-alkyl, aza-bicycle [2.2.1]hept-4-yl, azabicyclo[2.2.1]hept-4-yl-alkyl or adamantyl group, or

$R_{10}$ is a hydrogen atom or an alkyl group and $R_{11}$ is a hydroxyl, alkoxy or cyano group,

their tautomers, their stereoisomers and their salts,

wherein, unless mentioned otherwise,

aryl as mentioned in the definitions of the abovementioned radicals is to be understood as meaning a phenyl group which can be in each case be monosubstituted by $R_{12}$, mono-, di- or trisubstituted by $R_{13}$, or monosubstituted by $R_{12}$ and additionally mono- or disubstituted by $R_{13}$, wherein the substituents can be identical or different, and

$R_{12}$ is a cyano, carboxyl, aminocarbonyl, alkylaminocarbonyl, dialkylaminocarbonyl, alkoxycarbonyl, alkylcarbonyl, alkylsulphenyl, alkylsulphinyl, alkylsulphonyl, alkylsulphonyloxy, perfluoroalkyl, perfluoroalkoxy, nitro, amino, alkylcarbonylamino, N-alkyl-alkylcarbonylamino, alkylamino, dialkylamino, hydroxy-$C_{2-4}$-alkylamino, N-alkyl-(hydroxy-$C_{2-4}$-alkyl)amino, bis-(hydroxy-$C_{2-4}$-alkyl)amino, phenylalkylcarbonylamino, phenylcarbonylamino, alkylsulphonylamino, phenylalkylsulphonylamino, phenylsulphonylamino, N-alkyl-phenylalkylcarbonylamino, N-alkyl-phenylcarbonylamino, N-alkyl-alkylsulphonylamino, N-alkylphenylalkylsulphonylamino, N-alkyl-phenylsulphonylamino, aminosulphonyl, alkylaminosulphonyl, dialkylaminosulphonyl, $(R_8NR_7)$-CO-$NR_6$- or $(R_8NR_7)$-$SO_2$-$NR_6$- group, wherein $R_6$, $R_7$ and $R_8$ are as defined above,

a 5- to 7-membered alkylenimino group which is optionally substituted by 1 to 4 alkyl groups or a hydroxyalkyl group, wherein, in the abovementioned 6- to 7-membered alkylenimino groups, in each case a methylene group in the 4-position can be replaced by an oxygen atom or an $R_9N$ group, wherein $R_9$ is as defined above,

a 5- to 7-membered alkylenimino group which is optionally substituted by 1 to 4 alkyl groups or a hydroxyalkyl group, wherein in each case one or two methylene groups adjacent to the nitrogen atom are replaced by a carbonyl group, and

$R_{13}$ is an alkyl, hydroxyl or alkoxy group or a fluorine, chlorine, bromine or iodine atom, wherein two radicals $R_{13}$, if these are bonded to adjacent carbon atoms, can also be an alkylene group having 3 to 6 carbon atoms, a 1,3-butadiene-1,4-diylene group or a methylenedioxy group,

and, unless mentioned otherwise, the abovementioned alkyl, alkylene and alkoxy parts in each case contain 1 to 4 carbon atoms.

**2.** Pyrimido[5,4-d]pyrimidines of the formula I according to Claim 1 in which, unless mentioned otherwise, each carbon

atom in the alkylene or cycloalkylene parts mentioned in Claim 1 which is bonded to a nitrogen, oxygen or sulphur atom cannot be bonded to a further halogen, nitrogen, oxygen or sulphur atom,

their tautomers, their stereoisomers and their salts.

**3.** Pyrimido[5,4-d]pyrimidines of the formula I according to Claim 1 or 2, in which

$R_a$ is a hydrogen atom or an alkyl group,

$R_b$ is a phenyl group which is substituted by the radicals $R_1$ to $R_3$, wherein

$R_1$ is a hydrogen, fluorine, chlorine, bromine or iodine atom,

a $C_{1-6}$-alkyl, hydroxyl or $C_{1-6}$-alkoxy group,

a $C_{3-6}$-cycloalkyl or $C_{5-6}$-cycloalkoxy group,

a $C_{2-5}$-alkenyl or $C_{3-5}$-alkenyloxy group, wherein the vinyl part cannot be linked to the oxygen atom,

a $C_{2-5}$-alkynyl or $C_{3-5}$-alkynyloxy group, wherein the ethynyl part cannot be linked to the oxygen atom,

an aryl, aryloxy, aralkyl, aralkoxy, alkylsulphenyl, alkylsulphinyl, alkylsulphonyl, alkylsulphonyloxy, trifluoromethylsulphenyl, trifluoromethylsulphonyl, arylsulphenyl, arylsulphinyl, arylsulphonyl, aralkylsulphenyl, aralkylsulphinyl or aralkylsulphonyl group,

a methyl or methoxy group which is substituted by 1 to 3 fluorine atoms,

a $C_{2-4}$-alkyl or $C_{2-4}$-alkoxy group which is substituted by 1 to 5 fluorine atoms,

a nitro, amino, alkylamino, dialkylamino, $C_{3-6}$-cycloalkylamino, N-alkyl-$C_{3-6}$-cycloalkylamino, arylamino, N-alkyl-arylamino, aralkylamino or N-alkyl-aralkylamino group,

a 5- to 7-membered alkylenimino group, wherein in each case one or two methylene groups adjacent to the nitrogen atom can be replaced by a carbonyl group or, in the abovementioned 6- to 7-membered alkylenimino groups, a methylene group in the 4-position can be replaced by an oxygen atom or by an imino or N-alkyl-imino group,

an (alkylenimino)carbonyl or (alkylenimino)sulphonyl group having in each case 5 to 7 ring atoms in the alkylenimino part, wherein, in the abovementioned 6- to 7-membered alkylenimino parts, in each case a methylene group in the 4-position can be replaced by an oxygen atom or by an imino or N-alkyl-imino group,

an alkylcarbonylamino, N-alkyl-alkylcarbonylamino, alkyl-sulphonylamino, N-alkyl-alkylsulphonylamino, arylcarbonyl-amino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, trifluoromethylsulphonylamino, N-alkyltrifluoromethylsulphonylamino, alkylcarbonyl, arylcarbonyl, aralkylcarbonyl, carboxyl, alkoxycarbonyl, aminocarbonyl, alkylaminocarbonyl, dialkylaminocarbonyl, arylaminocarbonyl, N-alkyl-arylaminocarbonyl, aralkylaminocarbonyl, N-alkyl-aralkyl-aminocarbonyl, N-hydroxy-aminocarbonyl, N-hydroxy-alkyl-aminocarbonyl, N-alkoxy-aminocarbonyl, N-alkoxy-alkylaminocarbonyl, cyano, azido, N-cyano-amino or N-cyano-alkylamino group,

a sulpho, aminosulphonyl, alkylaminosulphonyl, dialkylaminosulphonyl, arylaminosulphonyl, N-alkyl-arylaminosulphonyl, aralkylaminosulphonyl or N-alkyl-aralkylaminosulphonyl group,

a phosphono, O-alkyl-phosphono, O,O'-dialkyl-phosphono or O,O'-diaralkyl-phosphono group,

an alkyl or alkoxy group which is substituted by $R_4$, wherein

$R_4$ is a hydroxyl, alkoxy, aryloxy, amino, alkylamino, dialkylamino, alkylsulphenyl, alkylsulphinyl, alkyl-

sulphonyl, arylsulphenyl, arylsulphinyl, arylsulphonyl, carboxyl, alkoxycarbonyl, aminocarbonyl, alkylaminocarbonyl, dialkylaminocarbonyl or cyano group or

an (alkylenimino)carbonyl group having in each case 5 to 7 ring atoms in the alkylenimino part, wherein, in the abovementioned 6- to 7-membered alkylenimino parts, in each case a methylene group in the 4-position can be replaced by an oxygen atom or by an imino or N-alkyl-imino group,

$R_2$ is a hydrogen, fluorine, chlorine or bromine atom or an alkyl, trifluoromethyl, hydroxyl, alkoxy, amino, alkylamino, dialkylamino, alkylcarbonylamino, N-alkylalkylcarbonylamino, alkylsulphonylamino N-alkylalkylsulphonylamino, trifluoromethylsulphonylamino, Nalkyl-trifluoromethyl-sulphonylamino or cyano group and

$R_3$ is a hydrogen, fluorine, chlorine or bromine atom or an alkyl, trifluoromethyl or alkoxy group, or

$R_2$ together with $R_3$, if these are bonded to adjacent carbon atoms, are a methylenedioxy group which is optionally substituted by one or two alkyl groups, an n-$C_{3-6}$-alkylene group which is optionally substituted by one or two alkyl groups, or a 1,3-butadiene-1,4-diylene group which is optionally substituted by a fluorine, chlorine or bromine atom or by a hydroxyl, alkyl, alkoxy, trifluoromethyl or cyano group, or

$R_a$ together with $R_1$, if $R_1$ is in the o-position relative to the nitrogen atom substituted by $R_a$, is an n-$C_{2-3}$-alkylene group, and

$R_c$ is a hydrogen or chlorine atom,

an alkyl, aryl, aralkyl, mercapto, alkylsulphenyl, alkylsulphinyl or alkylsulphonyl group,

a hydroxyl, aryloxy or aralkoxy group,

a $C_{1-6}$-alkoxy group, which can be substituted by an alkoxycarbonyl, cyano, carboxyl, aminocarbonyl, alkylaminocarbonyl or dialkylaminocarbonyl group,

a $C_{2-6}$-alkoxy group substituted by a hydroxyl, alkoxy, hydroxy-$C_{2-4}$-alkylamino, amino, alkylamino, dialkylamino, alkylcarbonylamino, alkylsulphonylamino or alkoxycarbonylamino group, or by a 5- to 7-membered alkylenimino group which is optionally substituted by 1 to 2 alkyl groups, wherein, in the abovementioned 5- to 7-membered alkylenimino groups, in each case one or two methylene groups adjacent to the nitrogen atom can in each case be replaced by a carbonyl group, or in the abovementioned 6- to 7-membered alkylenimino groups, a methylene group in the 4-position can be replaced by an oxygen atom or by a carbonyl, imino, alkyl-imino, alkylcarbonyl-imino, alkoxycarbonyl-imino, alkylsulphonyl-imino, formyl-imino, dialkylaminocarbonyl-imino, aryl-imino or aralkyl-imino group,

a $C_{3-6}$-alkoxy group substituted by two hydroxyl or alkoxy groups,

an alkoxy group which is substituted by a $C_{3-7}$-cycloalkyl group wherein the cycloalkyl residue in each case can be substituted by one or two alkyl groups and wherein, in the abovementioned $C_{4-7}$-cycloalkyl residues, in each case a methylene group can be replaced by an oxygen atom, or by an imino, N-alkyl-imino, N-alkylcarbonyl-imino, N-alkoxycarbonyl-imino or N-aryl-imino group,

a $C_{4-7}$-cycloalkoxy group which is optionally substituted by a hydroxyl, alkoxy, alkoxycarbonyl, cyano, carboxyl, aminocarbonyl, alkylaminocarbonyl, dialkylaminocarbonyl, hydroxy-$C_{2-4}$-alkylamino, amino, alkylamino, dialkylamino, alkylcarbonyl-amino, alkylsulphonylamino or alkoxycarbonylamino group,

a $C_{5-7}$-cycloalkoxy group, wherein, in the abovementioned cyclopentyloxy group, in each case a methylene group in the 3-position, and in the abovementioned $C_{6-7}$-cycloalkoxy groups, in each case a methylene group in the 3- or 4-position is replaced by an oxygen atom or by an imino, alkyl-imino, alkylcarbonyl-imino, alkoxycarbonyl-imino, alkylsulphonyl-imino, aryl-imino or aralkyl-imino group,

a $C_{3-6}$-alkenyloxy group, wherein the vinyl part cannot be linked to the oxygen atom,

a $C_{3-6}$-alkynyloxy group, wherein the ethynyl part cannot be linked to the oxygen atom,

a 4- to 8-membered alkylenimino group which is optionally substituted by 1 to 4 alkyl groups or an aryl group and can additionally be substituted by the radical $R_5$, wherein

$R_5$ is an aryl, aralkyl, carboxyl, alkoxycarbonyl, aminocarbonyl, alkylaminocarbonyl, dialkylaminocarbonyl, pyrrolidinocarbonyl, piperidinocarbonyl, morpholinocarbonyl, piperazinocarbonyl, 4-alkylpiperazinocarbonyl, cyano, hydroxyl, alkoxy, aryloxy, amino, alkylamino, dialkylamino, hydroxy-$C_{2-4}$-alkylamino, N-alkyl-hydroxy-$C_{2-4}$-alkylamino, di(hydroxy- $C_{2-4}$-alkyl)amino, formylamino, cyanoamino, alkylcarbonylamino, N-alkyl-alkylcarbonylamino, alkoxycarbonylamino, N-alkyl-alkoxycarbonyl-amino, alkylsulphonylamino, N-alkyl-alkylsulphonylamino, arylcarbonylamino, N-alkyl-arylcarbonylamino, arylsulphonyl-amino, N-alkyl-arylsulphonylamino, carboxyalkyl, alkoxycarbonylalkyl, aminocarbonylalkyl, alkylaminocarbonylalkyl, dialkylaminocarbonylalkyl, pyrrolidinocarbonylalkyl, piperidinocarbonylalkyl, morpholinocarbonylalkyl, piperazinocarbonylalkyl, 4-alkyl-piperazinocarbonylalkyl, cyanoalkyl, hydroxyalkyl, alkoxyalkyl, aryloxyalkyl, aminoalkyl, alkylaminoalkyl, dialkylaminoalkyl, alkylcarbonylaminoalkyl, N-alkyl-alkylcarbonylaminoalkyl, alkylsulphonylaminoalkyl, N-alkyl-alkylsulphonylaminoalkyl, arylcarbonylaminoalkyl, N-alkyl-arylcarbonylaminoalkyl, arylsulphonylaminoalkyl, N-alkyl-arylsulphonylaminoalkyl, alkylsulphenyl, alkylsulphinyl, alkylsulphonyl, arylsulphenyl, arylsulphinyl, arylsulphonyl, alkylsulphenylalkyl, alkylsulphinylalkyl, alkylsulphonylalkyl, arylsulphenylalkyl, arylsulphinylalkyl, arylsulphonylalkyl, carboxyalkoxy, alkoxycarbonylalkoxy, aminocarbonylalkoxy, alkylaminocarbonylalkoxy, dialkylaminocarbonylalkoxy, pyrrolidinocarbonylalkoxy, piperidinocarbonylalkoxy, morpholinocarbonylalkoxy or an $(R_8NR_7)$-CO-$NR_6$- group, wherein

$R_6$, $R_7$ and $R_8$, which can be identical or different, are each a hydrogen atom or an alkyl group, or
$R_6$ and $R_7$ together are an n-$C_{2-3}$-alkylene group and
$R_8$ is a hydrogen atom or an alkyl group,

a pyrrolidino, piperidino, morpholino, piperazino, 4-alkylpiperazino or 4-alkoxycarbonylpiperazino group, optionally substituted by one or two alkyl groups or a hydroxymethyl group, wherein, in the heterocyclic residue of the abovementioned groups, in each case one or two of the methylene groups adjacent to the nitrogen atoms can be replaced by a carbonyl group,

or $R_c$ is a 6- to 8-membered alkylenimino group which is optionally substituted by 1 to 4 alkyl groups or by an aryl group and can additionally be substituted by the radical $R_5$, wherein, in the abovementioned alkylenimino groups, in each case a methylene group in the 4-position is replaced by an oxygen or sulphur atom or by a carbonyl, sulphinyl, sulphonyl, N-oxido-N-alkylimino or $R_9N$ group, wherein

$R_9$ is a hydrogen atom or an alkyl, hydroxy-$C_{2-4}$-alkyl, alkoxy-$C_{2-4}$-alkyl, hydroxy-$C_{2-4}$-alkoxy-$C_{2-4}$-alkyl, aminocarbonylalkyl, alkylaminocarbonylalkyl, dialkylaminocarbonylalkyl, amino-$C_{2-4}$-alkyl, alkylamino-$C_{2-4}$-alkyl, dialkylamino-$C_{2-4}$-alkyl, aryl, aralkyl, formyl, alkylcarbonyl, alkylsulphonyl, arylcarbonyl, arylsulphonyl, aralkylcarbonyl, aralkylsulphonyl, alkoxycarbonyl, cyano, aminocarbonyl, alkylaminocarbonyl or dialkylaminocarbonyl group,

or $R_c$ is a 1-pyrrolidinyl group which is optionally substituted by 1 or 2 alkyl groups and in which two hydrogen atoms on the carbon skeleton are replaced by a straight-chain alkylene bridge, wherein this bridge contains 4 or 5 carbon atoms if the two hydrogen atoms are on the same carbon atom, or contains 3 or 4 carbon atoms if the two hydrogen atoms are on adjacent carbon atoms, or contains 2 or 3 carbon atoms if the two hydrogen atoms are on carbon atoms which are separated by an atom,

a 1-piperidinyl or 1-azacyclohept-1-yl group which is optionally substituted by 1 or 2 alkyl groups and in which two hydrogen atoms on the carbon skeleton are replaced by a straight-chain alkylene bridge, wherein this bridge contains 4 or 5 carbon atoms if the two hydrogen atoms are on the same carbon atom, or contains 3 or 4 carbon atoms if the two hydrogen atoms are on adjacent carbon atoms, or contains 2 or 3 carbon atoms if the two hydrogen atoms are on carbon atoms which are separated by an atom, or contains 1 or 2 carbon atoms if the two hydrogen atoms are on carbon atoms which are separated by two atoms,

a 1-pyrrolidinyl group in which two hydrogen atoms in the 3-position are replaced by a $-O-CH_2CH_2-O-$ or $-O-CH_2CH_2CH_2-O-$ group,

a 1-piperidinyl or 1-azacyclohept-1-yl group in which two hydrogen atoms in the 3-position or in the 4-position are replaced by a $-O-CH_2CH_2-O-$ or $-O-CH_2CH_2CH_2-O-$group,

a 2-isoindolinyl, 1,2,3,4-tetrahydro-isoquinolin-2-yl or 2,3,4,5-tetrahydro-lH-3-benzazepin-3-yl group, wherein the benzo part of the abovementioned groups can in each case be substituted by a fluorine, chlorine, bromine or iodine atom, by a trifluoromethyl group or by one or two alkyl or alkoxy groups, or

a $(R_{10}NR_{11})$- group, in which

$R_{10}$ is a hydrogen atom or a $C_{1-8}$-alkyl group, which can be substituted from position 2 by a hydroxyl or alkoxy group,

$R_{11}$ is a hydrogen atom,

a $C_{1-10}$-alkyl group, which can be substituted by an aryl, $C_{3-7}$-cycloalkyl, hydroxyl, alkoxy, aryloxy, carboxyl, alkoxycarbonyl, aminocarbonyl, alkylaminocarbonyl, dialkylaminocarbonyl, hydroxy-$C_{2-4}$-alkylaminocarbonyl, cyano, formylamino, amino, alkylamino or dialkylamino group, by a pyrrolidinocarbonyl, piperidinocarbonyl, morpholinocarbonyl, piperazinocarbonyl or 4-alkylpiperazinocarbonyl group,

by a 5- to 7-membered alkylenimino group which is optionally substituted by 1 or 2 alkyl groups, wherein, in the abovementioned 6- or 7-membered alkylenimino residues, in each case a methylene group in the 4-position can be replaced by an oxygen or sulphur atom or by a sulphinyl, sulphonyl or $R_9N$ group, wherein $R_9$ is as defined above,

by a pyrrolidino, piperidino, piperazino or 4-alkylpiperazino group, wherein in each case one or two of the methylene groups adjacent to the nitrogen atoms are replaced by a carbonyl group,

by an alkylcarbonylamino, N-alkyl-alkylcarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, alkoxycarbonylamino or N-alkyl-alkoxycarbonylamino group, by an $(R_8NR_7)$-CO-$NR_6$- group, wherein $R_6$ to $R_8$ are as defined above, by an alkylsulphenyl, alkylsulphinyl, alkylsulphonyl, arylsulphenyl, arylsulphinyl, arylsulphonyl, aralkylsulphenyl, aralkylsulphinyl or aralkylsulphonyl group or by a $C_{5-7}$-cycloalkyl group in which a methylene group is replaced by an oxygen atom or by an imino or alkylimino group,

a $C_{2-4}$-alkyl group substituted by a chlorine atom or by one to three fluorine atoms,

a methyl group which is substituted by a 1,4,7,10-tetraoxacyclododecyl, 1,4,7,10,13-pentaoxacyclopentadecyl or a 1,4,7,10,13,16-hexaoxacyclooctadecyl group,

a $C_{3-10}$-alkyl group which is substituted by 2 to 5 hydroxyl groups,

a $C_{2-6}$-alkyl group which is substituted by a hydroxyl and additionally by an aryl group and which can optionally additionally be substituted by a hydroxyl or alkoxy group,

a $C_{3-6}$-alkyl group which is substituted by a hydroxyl and additionally by an amino, alkylamino or dialkylamino group,

an alkenyl or alkynyl group which has in each case 3 to 6 carbon atoms and is optionally substituted by an aryl group, wherein the vinyl or ethynyl part cannot be linked to the nitrogen atom,

a $C_{2-4}$-alkyl group, which is substituted by a $C_{2-4}$-alkoxy group, which is substituted in the $\omega$-position by a hydroxyl or alkoxy group,

an aryl group,

a cyclopropyl group, which can be substituted by 1 or 2 alkyl groups or by an aryl, carboxyl, alkoxycarbonyl, aminocarbonyl, alkylaminocarbonyl, dialkylaminocarbonyl, pyrrolidinocarbonyl, piperidinocarbonyl, morpholinocarbonyl, piperazinocarbonyl or 4-alkyl-piperazinocarbonyl group,

a $C_{4-7}$-cycloalkyl group which is optionally substituted by 1 or 2 alkyl groups and can additionally be substituted

by $R_5$, wherein $R_5$ is as defined above,

a $C_{5-7}$-cycloalkyl group which is optionally substituted by 1 or 2 alkyl groups and is additionally substituted by an N,N-dialkyl-N-oxido-amino group,

a $C_{5-7}$-cycloalkenyl group which is optionally substituted by 1 or 2 alkyl groups, wherein the vinyl part cannot be linked to the nitrogen atom of the $(R_{11}NR_{10})$- group,

a $C_{5-7}$-cycloalkyl group which is optionally substituted by 1 or 2 alkyl groups, wherein, in the cycloalkyl part, in each case a methylene group is replaced by an oxygen or sulphur atom or by a sulphinyl, sulphonyl, N-alkyl-N-oxido-imino or $R_9N$ group, wherein $R_9$ is as defined above,

a $C_{5-7}$-cycloalkyl group which is optionally substituted by 1 to 4 alkyl groups and in which in each case a methylene group is replaced by a carbonyl group,

a $C_{4-7}$-cycloalkylmethyl group which is optionally substituted by 1 or 2 alkyl groups and is additionally substituted in the cycloalkyl part by $R_5$, wherein $R_5$ is defined as mentioned above,

a cyclopentyl or cyclopentylalkyl group which is optionally substituted by 1 to 4 alkyl groups and in which in each case two hydrogen atoms in the cyclopentyl part are replaced by a straight-chain alkylene bridge, wherein this bridge contains 4 or 5 carbon atoms if the two hydrogen atoms are on the same carbon atom, or contains 3 or 4 carbon atoms if the two hydrogen atoms are on adjacent carbon atoms, or contains 2 or 3 carbon atoms if the two hydrogen atoms are on carbon atoms which are separated by a carbon atom,

a cyclohexyl, cyclohexylalkyl, cycloheptyl or cycloheptylalkyl group which is optionally substituted by 1 to 4 alkyl groups and in which in each case two hydrogen atoms in the cycloalkyl part are replaced by a straight-chain alkylene bridge, wherein this bridge contains 4 or 5 carbon atoms if the two hydrogen atoms are on the same carbon atom, or contains 3 or 4 carbon

atoms if the two hydrogen atoms are on adjacent carbon atoms, or contains 2 or 3 carbon atoms if the two hydrogen atoms are on carbon atoms which are separated by a carbon atom, or contains 1 or 2 carbon atoms if the two hydrogen atoms are on carbon atoms which are separated by two carbon atoms,

a 5-norbornen-2-yl or 5-norbornen-2-yl-alkyl group which is optionally substituted by 1 to 4 alkyl groups,

a 3-quinuclidinyl, 4-quinuclidinyl, 3-quinuclidinylalkyl, 4-quinuclidinyl-alkyl, azabicyclo[2.2.1]hept-4-yl, azabicyclo[2.2.1]hept-4-yl-alkyl or adamantyl group, or

$R_{10}$ is a hydrogen atom or an alkyl group and $R_{11}$ is a hydroxyl or alkoxy group,

their tautomers, their stereoisomers and their salts,

wherein, unless mentioned otherwise,

aryl as mentioned in the definitions of the abovementioned radicals is to be understood as meaning a phenyl group, which can in each case be monosubstituted by $R_{12}$, mono-, di- or trisubstituted by $R_{13}$ or monosubstituted by $R_{12}$ and additionally mono- or disubstituted by $R_{13}$, wherein the substituents can be identical or different, and

$R_{12}$ is a cyano, carboxyl, aminocarbonyl, alkylaminocarbonyl, dialkylaminocarbonyl, alkoxycarbonyl, alkylcarbonyl, alkylsulphenyl, alkylsulphinyl, alkylsulphonyl, alkylsulphonyloxy, perfluoroalkyl, perfluoroalkoxy, nitro, amino, alkylamino, hydroxy-$C_{2-4}$-alkylamino, N-alkyl-hydroxy- $C_{2-4}$-alkylamino, di(hydroxy-$C_{2-4}$-alkyl)amino, dialkylamino, alkylcarbonylamino, phenylalkylcarbonylamino, phenylcarbonylamino, alkylsulphonylamino, phenylalkylsulphonylamino, phenylsulphonylamino, N-alkyl-alkylcarbonylamino, N-alkyl-phenylalkylcarbonylamino, N-alkylphenylcarbonylamino, N-alkyl-alkylsulphonylamino, N-alkyl-phenylalkylsulphonylamino, N-alkylphenylsulphonylamino, aminosulphonyl, alkylaminosulphonyl or dialkylaminosulphonyl group,

a 5- to 7-membered alkylenimino group which is optionally substituted by 1 to 2 alkyl groups or a hydroxyalkyl

group, wherein, in the abovementioned 6- to 7-membered alkylenimino groups, in each case a methylene group in the 4-position can be replaced by an oxygen atom or an $R_9N$ group, wherein

a 5- to 7-membered alkylenimino group which is optionally substituted by 1 to 2 alkyl groups or a hydroxyalkyl group, wherein in each case one or two methylene groups adjacent to the nitrogen atom are replaced by a carbonyl group,

an $(R_8NR_7)$-cO-$NR_6$- group, wherein $R_6$ to $R_8$ are defined as mentioned above,

$R_{13}$ is an alkyl, hydroxyl or alkoxy group or a fluorine, chlorine, bromine or iodine atom, wherein two radicals $R_{13}$, if these are bonded to adjacent carbon atoms, can also be an alkylene group having 3 to 6 carbon atoms, a 1,3-butadiene-1,4-diylene group or a methylenedioxy group,

and, unless mentioned otherwise, the abovementioned alkyl, alkylene and alkoxy parts in each case contain 1 to 4 carbon atoms and, unless mentioned otherwise, each carbon atom in the abovementioned alkylene or cycloalkylene parts which is bonded to a nitrogen, oxygen or sulphur atom cannot be bonded to a further halogen, nitrogen, oxygen or sulphur atom.

**4.** Pyrimido[5,4-d]pyrimidines of the formula I according to Claim 1 or 2, in which

$R_a$ is a hydrogen atom or an alkyl group,

$R_b$ is a phenyl group which is substituted by the radicals $R_1$ to $R_3$, wherein

$R_1$ is a hydrogen, fluorine, chlorine, bromine or iodine atom,

an alkyl, hydroxyl, alkoxy, $C_{3-6}$-cycloalkyl or $C_{5-6}$-cycloalkoxy group,

an ethoxy group which is substituted in the 2-position by a hydroxyl, alkoxy or phenoxy group,

a $C_{2-5}$-alkenyl or $C_{3-5}$-alkenyloxy group, wherein the vinyl part cannot be linked to the oxygen atom,

a $C_{2-5}$-alkynyl or $C_{3-5}$-alkynyloxy group, wherein the ethynyl part cannot be linked to the oxygen atom,

a phenyl, phenoxy, phenylalkyl, phenylalkoxy, alkoxyalkyl, phenoxyalkyl, carboxyalkyl, alkoxycarbonylalkyl, aminocarbonylalkyl, alkylaminocarbonylalkyl, dialkylaminocarbonylalkyl, cyanoalkyl, alkylsulphenyl, alkylsulphinyl, alkylsulphonyl, alkylsulphonyloxy, trifluoromethylsulphenyl, trifluoromethylsulphonyl, nitro, amino, alkylamino, dialkylamino, pyrrolidino, piperidino, morpholino, alkylcarbonylamino, N-alkyl-alkylcarbonylamino, alkylsulphonylamino, N-alkyl-alkylsulphonylamino, trifluoromethylsulphonylamino, N-alkyl-trifluoromethylsulphonylamino, alkylcarbonyl, carboxyl, alkoxycarbonyl, aminocarbonyl, alkylaminocarbonyl, dialkylaminocarbonyl, pyrrolidinocarbonyl, piperidinocarbonyl, morpholinocarbonyl or cyano group,

a methyl or methoxy group which is substituted by 1 to 3 fluorine atoms,

an ethyl or ethoxy group which is substituted by 1 to 5 fluorine atoms,

$R_2$ is a hydrogen, fluorine, chlorine or bromine atom or an alkyl, trifluoromethyl, amino, alkylamino, dialkylamino, alkylcarbonylamino, alkylsulphonylamino, trifluoromethylsulphonylamino, hydroxyl or alkoxy group,

$R_3$ is a hydrogen, fluorine, chlorine or bromine atom or an alkyl group, or

$R_2$ together with $R_3$, if these are bonded to adjacent carbon atoms, is a methylenedioxy or n-$C_{3-6}$-alkylene group or a 1,3-butadiene-1,4-diylene group which is optionally substituted by a fluorine, chlorine or bromine atom or by an alkyl, alkoxy or trifluoromethyl group and

$R_c$ is a hydrogen or chlorine atom,

an alkyl, phenyl, mercapto, alkylsulphenyl, alkylsulphinyl or alkylsulphonyl group,

a hydroxyl, phenoxy or phenyl-$C_{1-2}$-alkoxy group,

an alkoxy group,

a $C_{2-4}$-alkoxy group, which is substituted by a hydroxyl, alkoxy, (2-hydroxyethyl)amino, dialkylamino, morpholino, 1-pyrrolidinyl, 1-piperidinyl, 4-methyl-1-piperazinyl, 4-acetyl-1-piperazinyl, 4-methylsulphonyl-1-piperazinyl, 4-methoxycarbonyl-1-piperazinyl, 4-formyl-1-piperazinyl or 4-dimethylaminocarbonyl-1-piperazinyl group,

a $C_{3-4}$-alkoxy group, which is substituted by two hydroxyl groups,

a $C_{1-2}$-alkoxy group, which is substituted by a $C_{3-7}$-cycloalkyl group which is optionally substituted by one or two methyl groups, wherein, in the abovementioned $C_{4-6}$-cycloalkyl groups, in each case a methylene group can be replaced by an oxygen atom,

a $C_{4-6}$-cycloalkoxy group which is optionally substituted by a hydroxyl, dialkylamino, alkoxy, carboxyl, alkoxycarbonyl, aminocarbonyl, alkylaminocarbonyl, dialkylaminocarbonyl, alkylcarbonylamino, alkylsulphonylamino or alkoxycarbonylamino group,

a cyclopentyloxy group in which the methylene group in the 3-position is replaced by an oxygen atom or by an alkylimino group,

a cyclohexyloxy group in which the methylene group in the 3- or 4-position is replaced by an oxygen atom or by an alkylimino, alkylcarbonyl-imino, alkoxycarbonyl-imino or alkylsulphonyl-imino group,

an allyloxy or propargyloxy group which is optionally substituted by one or two methyl groups,

a 1-azetidinyl group,

a 1-pyrrolidinyl group, which can be substituted by 1 to 2 alkyl groups, by a phenyl, carboxyl, hydroxyalkyl, aminoalkyl, alkylaminoalkyl, dialkylaminoalkyl, alkoxycarbonyl, aminocarbonyl, alkylaminocarbonyl, dialkylaminocarbonyl, pyrrolidinocarbonyl, piperidinocarbonyl, morpholinocarbonyl, piperazinocarbonyl or 4-alkyl-piperazinocarbonyl group or in the 3-position also by a hydroxyl, alkoxy, amino, alkylamino, dialkylamino, alkylcarbonylamino, alkoxycarbonylamino, formylamino, cyanoamino, alkylsulphonylamino, dialkylaminocarbonylamino, N-alkyl-dialkylaminocarbonylamino, N-alkyl-dialkylaminocarbonylamino or cyano group,

a 1-pyrrolidinyl group in which two hydrogen atoms on the carbon skeleton are replaced by a straight-chain alkylene bridge, wherein this bridge contains 4 or 5 carbon atoms if the two hydrogen atoms are on the same carbon atom, or contains 3 or 4 carbon atoms if the two hydrogen atoms are on adjacent carbon atoms, or contains 2 or 3 carbon atoms if the two hydrogen atoms are on carbon atoms which are separated by an atom,

a 1-piperidinyl group, which can be substituted by 1 to 4 alkyl groups, by a phenyl, hydroxyalkyl, aminoalkyl, alkylaminoalkyl, dialkylaminoalkyl, carboxyl, alkoxycarbonyl, aminocarbonyl, alkylaminocarbonyl, dialkylaminocarbonyl, pyrrolidinocarbonyl, piperidinocarbonyl, morpholinocarbonyl, piperazinocarbonyl or 4-alkyl-piperazinocarbonyl group or in the 3- or 4-position also by a hydroxyl, alkoxy, amino, alkylamino, dialkylamino, alkylcarbonylamino, alkoxycarbonylamino, formylamino, cyanoamino, alkylsulphonylamino, dialkylaminocarbonylamino, N-alkyl-dialkylaminocarbonylamino or cyano group,

a 1-piperidinyl group, which is substituted by 1 to 2 alkyl groups or a phenyl group and additionally by a hydroxyl group,

a 1-piperidinyl group in which two hydrogen atoms in the 3-position or in the 4-position are replaced by a $-O-CH_2CH_2-O-$ or $-O-CH_2CH_2CH_2-O-$ group,

a 1-piperidinyl group in which two hydrogen atoms on the carbon skeleton are replaced by a straight-chain alkylene bridge, wherein this bridge contains 4 or 5 carbon atoms if the two hydrogen atoms are on the same carbon atom, or contains 3 or 4 carbon atoms if the two hydrogen atoms are on adjacent carbon atoms, or contains 2 or 3 carbon atoms if the two hydrogen atoms are on carbon atoms which are separated by an atom,

or contains 1 or 2 carbon atoms if the two hydrogen atoms are on carbon atoms which are separated by two atoms,

a 1-piperidinyl group which is optionally substituted by 1 or 2 alkyl groups and in which the methylene group in the 4-position is replaced by an oxygen or sulphur atom or by a carbonyl, sulphinyl, sulphonyl, imino, alkylimino, hydroxy-$C_{2-4}$-alkyl-imino, alkoxy-$C_{2-4}$-alkyl-imino, aminocarbonylalkyl-imino, alkylaminocarbonylalkyl-imino, dialkylaminocarbonylalkyl-imino, amino-$C_{2-4}$-alkyl-imino, alkylamino-$C_{2-4}$-alkyl-imino, dialkylamino-$C_{2-4}$-alkyl-imino, hydroxy-$C_{2-4}$-alkoxy-$C_{2-4}$-alkyl-imino, phenyl-imino, phenylalkyl-imino, alkylcarbonyl-imino, alkylsulphonyl-imino, phenylcarbonyl-imino, phenylsulphonyl-imino or N-oxido-N-alkyl-imino group,

a 1-azacyclohept-1-yl group which is optionally substituted by 1 or 2 alkyl groups and in which in each case the methylene group in the 4-position can be replaced by an oxygen atom or by an imino, N-alkyl-imino, N-phenylimino, N-phenylalkyl-imino, N-alkylcarbonyl-imino, N-alkylsulphonyl-imino, N-phenylcarbonyl-imino or N-phenylsulphonyl-imino group or two hydrogen atoms in the 3,6-position can be replaced by a $-CH_2CH_2-$ group,

a 2-isoindolinyl, 1,2,3,4-tetrahydro-isoquinolin-2-yl or 2,3,4,5-tetrahydro-1H-3-benzazepin-3-yl group, which can in each case be substituted in the benzo part by a fluorine, chlorine or bromine atom or by an alkyl, trifluoromethyl or alkoxy group, or

an $(R_{10}NR_{11})$- group, in which

$R_{10}$ is a hydrogen atom or a $C_{1-6}$-alkyl group, which can be substituted by a hydroxyl or alkoxy group from position 2, and

$R_{11}$ is a hydrogen atom,

a $C_{1-8}$-alkyl group, which can be substituted by a phenyl, $C_{3-6}$-cycloalkyl, hydroxyl, alkoxy, cyano, carboxyl, alkoxycarbonyl, aminocarbonyl, alkylaminocarbonyl, dialkylaminocarbonyl, (2-hydroxyethyl)aminocarbonyl, pyrrolidinocarbonyl, piperidinocarbonyl, morpholinocarbonyl, 1-piperazinylcarbonyl, 4-alkyl-1-piperazinylcarbonyl, amino, formylamino, alkylamino, dialkylamino, alkylcarbonylamino, N-alkyl-alkylcarbonylamino, alkoxycarbonylamino, N-alkyl-alkoxycarbonyl-amino, alkylsulphonylamino, N-alkyl-alkylsulphonylamino, phenylcarbonylamino, N-alkyl-phenylcarbonylamino, phenyl-sulphonylamino, N-alkyl-phenylsulphonylamino, alkylsulphenyl, alkylsulphinyl, alkylsulphonyl, 1-pyrrolidinyl, 2-oxo-1-pyrrolidinyl, 1-piperidinyl, 2-oxo-1-piperidinyl, morpholino, 1-piperazinyl, 4-alkyl-1-piperazinyl, 4-alkylcarbonyl-1-piperazinyl, 4-alkylsulphonyl-1-piperazinyl, 4-alkoxycarbonyl-1-piperazinyl, 4-cyano-1-piperazinyl, 4-formyl-1-piperazinyl, 4-aminocarbonyl-1-piperazinyl, 4-alkylaminocarbonyl-1-piperazinyl or 4-dialkylaminocarbonyl-1-piperazinyl or a $(R_8NR_7)$-CO-$NR_6$- group, wherein

$R_6$ and $R_7$ together are an n-$C_{2-3}$-alkylene bridge and
$R_8$ is a hydrogen atom or an alkyl group,

a methyl group which is substituted by a 1,4,7,10-tetraoxacyclododecyl, 1,4,7,10,13-pentaoxacyclopentadecyl or a 1,4,7,10,13,16-hexaoxacyclooctadecyl group,

a 2,2,2-trifluoroethyl group,

a $C_{3-10}$-alkyl group which is substituted by 2 to 5 hydroxyl groups,

a $C_{3-5}$-alkyl group which is substituted by a hydroxyl and additionally by an amino group,

a $C_{2-4}$-alkyl group which is substituted by a phenyl group and additionally by a hydroxyl group and which can optionally additionally be substituted by a hydroxyl or alkoxy group,

an alkenyl or alkynyl group which has in each case 3 to 6 carbon atoms and is optionally substituted by a phenyl group, wherein the vinyl or ethynyl part cannot be linked to the nitrogen atom,

a $C_{2-4}$-alkyl group which is substituted by a $C_{2-4}$-alkoxy group which is substituted in the ω-position by a hydroxyl or alkoxy group,

a phenyl group,

a phenyl group which is substituted by an alkylcarbonyl-amino, N-alkyl-alkylcarbonylamino, (2-hydroxyethyl) amino, di-(2-hydroxyethyl)amino, N-alkyl-(2-hydroxyethyl)amino, amino, alkylamino or dialkylamino group or by an $(R_8NR_7)$-CO-$NR_6$- group wherein $R_6$ to $R_8$ are defined as mentioned above,

a phenyl group, which is substituted by a pyrrolidino, piperidino, 2-oxo-pyrrolidino, 2-oxo-piperidino, morpholino, 1-piperazinyl or 4-alkyl-1-piperazinyl group, wherein the abovementioned heterocyclic parts can be substituted on the carbon skeleton in each case by 1 or 2 alkyl groups or by a hydroxyalkyl group,

a $C_{3-7}$-cycloalkyl group which can be substituted by 1 or 2 alkyl groups or by a phenyl, carboxyl, alkoxycarbonyl, aminocarbonyl, alkylaminocarbonyl, dialkylaminocarbonyl, pyrrolidinocarbonyl, piperidinocarbonyl, morpholinocarbonyl, piperazinocarbonyl or 4-alkyl-piperazinocarbonyl group,

a $C_{5-7}$-cycloalkyl group which is optionally substituted by 1 or 2 methyl groups and is substituted by a hydroxymethyl, cyano, hydroxyl, alkoxy, amino, alkylamino, dialkylamino, 2-hydroxyethylamino, di-(2-hydroxyethyl)amino, N-alkyl-2-hydroxy-ethylamino, N,N-dialkyl-N-oxido-amino, alkoxycarbonylamino, N-alkyl-alkoxycarbonylamino, alkylcarbonylamino, N-alkyl-alkylcarbonylamino, alkylsulphonylamino, N-alkyl-alkylsulphonylamino, phenylcarbonylamino, N-alkylphenylcarbonyl-amino, phenylsulphonylamino, N-alkylphenylsulphonylamino or by an $(R_8NR_7)$-CO-$NR_6$- group, wherein $R_6$ to $R_8$ are defined as mentioned above,

a $C_{5-7}$-cycloalkyl group which is optionally substituted by 1 or 2 methyl groups and is substituted by a pyrrolidino, piperidino, 2-oxo-pyrrolidino, 2-oxopiperidino, morpholino, 1-piperazinyl, 4-alkyl-1-piperazinyl or 4-alkylcarbonyl-1-piperazinyl group, wherein the abovementioned heterocyclic parts can in each case be substituted on the carbon skeleton by 1 or 2 alkyl groups or by a hydroxymethyl group,

a $C_{5-7}$-cycloalkenyl group which is optionally substituted by 1 or 2 alkyl groups, wherein the vinyl part cannot be bonded to the nitrogen atom of the $(R_{11}NR_{10})$- group,

a tetrahydrofurfuryl group,

a cyclopentyl group in which the methylene group in the 3-position is replaced by an oxygen atom or an imino, alkylimino, alkylcarbonylimino, formylimino, aminocarbonylimino, alkylaminocarbonylimino, alkoxycarbonylimino, alkylsulphonylimino, dialkylaminocarbonylimino or cyanoimino group,

a cyclohexyl group in which the methylene group in the 3-position is replaced by an imino, alkyl-imino, alkylcarbonyl-imino, alkoxycarbonyl-imino or alkylsulphonyl-imino group,

a cyclohexyl group in which the methylene group in the 4-position is replaced by an oxygen atom or an imino, N-alkyl-imino, N-phenyl-imino, N-phenylalkyl-imino, N-formyl-imino, N-alkylcarbonyl-imino, N-phenylcarbonyl-imino, N-alkoxycarbonyl-imino, N-cyano-imino, N-aminocarbonyl-imino, N-alkylamino-carbonyl-imino, N, N-dialkylaminocarbonyl-imino, N-alkyl-N-oxido-imino, N-alkylsulphonyl-imino or N-phenylsulphonylimino group,

a cyclohexyl group in which a methylene group is replaced by a carbonyl group,

a cyclopentyl or cyclohexyl group which is optionally substituted by 1 to 2 methyl groups and is substituted by a carboxyalkoxy, alkoxycarbonylalkoxy, aminocarbonylalkoxy, alkylaminocarbonylalkoxy, dialkylaminocarbonylalkoxy, pyrrolidinocarbonylalkoxy, piperidinocarbonylalkoxy, morpholino-carbonylalkoxy, carboxyalkyl, alkoxycarbonylalkyl, aminocarbonylalkyl, alkylaminocarbonylalkyl, dialkylaminocarbonylalkyl, pyrrolidinocarbonylalkyl, piperidinocarbonylalkyl or morpholinocarbonylalkyl group,

a cyclohexylmethyl group, wherein the cyclohexyl part is substituted by a carboxyl, aminocarbonyl, alkylaminocarbonyl, dialkylaminocarbonyl, pyrrolidinocarbonyl, piperidinocarbonyl, morpholinocarbonyl, alkoxycarbonyl or hydroxymethyl group,

a cyclohexyl or cyclohexylmethyl group which is optionally substituted by 1 to 3 methyl groups and in which in each case two hydrogen atoms in the cyclohexyl part are replaced by a straight-chain alkylene bridge, wherein this bridge contains 4 or 5 carbon atoms if the two hydrogen atoms are on the same carbon atom, or contains 3 or 4 carbon atoms if the two hydrogen atoms are on adjacent carbon atoms, or contains 2 or 3 carbon atoms if the two hydrogen atoms are on carbon atoms which are separated by a carbon atom, or contains 1 or 2 carbon atoms if the two hydrogen atoms are on carbon atoms which are separated by two carbon atoms,

a 5-norbornen-2-yl or 5-norbornen-2-yl-methyl group which is optionally substituted by 1 to 3 methyl groups,

a 3-quinuclidinyl, 4-quinuclidinyl or adamantyl group, or

$R_{10}$ is a hydrogen atom or an alkyl group and $R_{11}$ is a hydroxyl or alkoxy group,

their tautomers, their stereoisomers and their salts,

wherein the abovementioned phenyl radicals in each case can be substituted by a fluorine, chlorine or bromine atom or by a nitro, alkyl, alkoxy, trifluoromethyl or hydroxyl group and, unless mentioned otherwise, the abovementioned alkyl, alkylene and alkoxy parts in each case contain 1 to 4 carbon atoms and, unless mentioned otherwise, each carbon atom in the abovementioned alkylene or cycloalkylene parts which is bonded to a nitrogen, oxygen or sulphur atom cannot be bonded to a further halogen, nitrogen, oxygen or sulphur atom.

**5.** Pyrimido[5,4-d]pyrimidines of the formula I according to Claim 1 or 2, in which

$R_a$ is a hydrogen atom or a methyl group,

$R_b$ is a 2-naphthyl, 1,2,3,4-tetrahydro-6-naphthyl or 5-indanyl group, or a phenyl group which is substituted by the radicals $R_1$ to $R_3$, wherein

$R_1$ is a hydrogen, fluorine, chlorine, bromine or iodine atom,

a $C_{1-4}$-alkyl, $C_{1-4}$-alkoxy, $C_{3-6}$-cycloalkyl, $C_{5-6}$-cycloalkoxy, cyano, methoxycarbonyl, ethoxycarbonyl, ethynyl or nitro group,

a methyl or methoxy group which is substituted by 1 to 3 fluorine atoms,

an ethyl or ethoxy group which is substituted by 1 to 5 fluorine atoms,

$R_2$ is a hydrogen, fluorine or chlorine atom or a methyl, hydroxyl, methoxy, amino, $C_{1-2}$-alkylamino, di-$C_{1-2}$-alkylamino, $C_{1-2}$-alkylcarbonylamino, $C_{1-2}$-alkylsulphonylamino, trifluoromethylsulphonylamino or trifluoromethyl group,

$R_3$ is a hydrogen, fluorine, chlorine or bromine atom or a methyl group and

$R_c$ is a hydrogen atom or a methyl, phenyl, 4-methoxyphenyl, methylsulphenyl, methylsulphinyl or methylsulphonyl group,

a hydroxyl group,

a $C_{1-4}$-alkoxy group,

an ethoxy group which is substituted in the 2-position by a hydroxyl, methoxy, morpholino or (2-hydroxyethyl) amino group,

a 2-propyloxy group which is substituted in the 1-position by a methoxy or dimethylamino group,

a methoxy group which is substituted by a 2-tetrahydrofuryl, 2-tetrahydropyranyl or 3-methyl-3-oxetanyl group,

a cyclobutyloxy group,

a cyclopentyloxy group which is optionally substituted in the 3-position by a hydroxyl group,

a cyclopentyloxy group in which the methylene group in the 3-position is replaced by an oxygen atom or by a methyl-imino group,

a cyclohexyloxy group which can be substituted in the 2-, 3- or 4-position by a hydroxyl group or in the 4-position also by a di-($C_{1-2}$-alkyl)amino, methoxy, carboxyl, methoxycarbonyl, dimethylaminocarbonyl, methylaminocarbonyl, aminocarbonyl, acetylamino, methylsulphonylamino, methoxycarbonylamino or tert-butyloxycarbonylamino group,

a cyclohexyloxy group in which the methylene group in the 3-position is replaced by a methyl-imino group or the methylene group in the 4-position is replaced by an oxygen atom or by a methyl-imino, acetyl-imino, tert-butyloxycarbonyl-imino, methoxycarbonyl-imino or methylsulphonyl-imino group,

an allyloxy group,

a 1-azetidinyl group,

a 1-pyrrolidinyl group which can be substituted by 1 or 2 methyl groups, by a carboxyl, methoxycarbonyl, ethoxycarbonyl, aminocarbonyl, $C_{1-2}$-alkylaminocarbonyl, or di-($C_{1-2}$)-alkylamino-carbonyl group or in the 3-position by an amino, $C_{1-2}$-alkylamino, di-($C_{1-2}$-alkyl)amino, $C_{1-2}$-alkoxycarbonylamino, $C_{1-2}$-alkylcarbonylamino, $C_{1-2}$-alkylsulphonylamino, cyanoamino, formylamino or dimethylaminocarbonylamino group,

a 1-pyrrolidinyl group in which two hydrogen atoms in the 3-position are replaced by an n-$C_{4-5}$-alkylene bridge,

a 1-piperidinyl group which can be substituted by 1 to 4 methyl groups, by a phenyl, hydroxy-$C_{1-2}$-alkyl, carboxyl, $C_{1-2}$-alkoxycarbonyl, aminocarbonyl, $C_{1-2}$-alkylaminocarbonyl, di- ($C_{1-2}$)-alkylaminocarbonyl, pyrrolidinocarbonyl or morpholinocarbonyl group or in the 3- or 4-position by a hydroxyl, $C_{1-2}$-alkoxy, amino, $C_{1-2}$-alkylamino, di-($C_{1-2}$)-alkylamino, $C_{1-2}$-alkylcarbonylamino, $C_{1-2}$-alkoxycarbonylamino, formylamino, cyanoamino, di-($C_{1-2}$-alkyl)aminocarbonylamino, $C_{1-2}$-alkylsulphonylamino or cyano group,

a 1-piperidinyl group in which two hydrogen atoms in the 3-position or in the 4-position are replaced by an n-$C_{4-5}$-alkylene bridge or by an $-O-CH_2CH_2-O-$ bridge,

a 1-piperidinyl group which is substituted by 1 or 2 methyl groups or a phenyl group and additionally in the 3- or 4-position by a hydroxyl group,

a 1-piperidinyl group in which two hydrogen atoms in the 2,5-position are replaced by a $-CH_2-$ or $-CH_2CH_2-$ bridge,

a 1-piperidinyl group which is optionally substituted by 1 to 2 methyl groups and in which the methylene group in the 4-position is replaced by an oxygen or sulphur atom or by a carbonyl, sulphinyl, sulphonyl, imino, $C_{1-2}$-alkyl-imino, (2-hydroxyethyl)-imino, 2-(2-hydroxyethoxy)ethyl-imino, (2-aminoethyl)-imino, $C_{1-3}$-alkylaminocarbonylmethyl-imino, N-oxido-N-$C_{1-2}$-alkylamino, phenyl-imino, benzyl-imino, acetyl-imino or methanesulphonyl-imino group,

a 1-azacyclohept-1-yl group in which two hydrogen atoms in the 3,6-position can be replaced by a $-CH_2CH_2-$ group,

a 1,2,3,4-tetrahydro-isoquinolin-2-yl or 2,3,4,5-tetrahydro-1H-3-benzazepin-3-yl group or

an ($R_{10}NR_{11}$)- group in which

$R_{10}$ is a hydrogen atom, a $C_{1-4}$-alkyl group or 2-hydroxyethyl group and

$R_{11}$ is a hydrogen atom,

a $C_{1-6}$-alkyl, $C_{3-6}$-cycloalkyl-methyl or phenyl-$C_{1-3}$-alkyl group,

a $C_{3-6}$-cycloalkyl, allyl or propargyl group which is optionally substituted by 1 or 2 methyl groups,

a phenyl group which can be substituted by a hydroxyl or methyl group or in the 4-position by an N-$C_{1-2}$-alkyl-$C_{1-2}$-alkylcarbonyl-amino, N-$C_{1-2}$-alkyl-(2-hydroxyethyl)amino, di-(2-hydroxyethyl)amino, 1-pyrrolidinyl, 2-oxo-1-pyrrolidinyl, 2-hydroxymethyl-1-pyrrolidinyl, 2-oxo-1-imidazolidinyl, 3-methyl-2-oxo-1-imidazolidinyl or morpholino group,

a methyl group which is substituted by a carboxyl, $C_{1-2}$-alkoxycarbonyl, aminocarbonyl, $C_{1-2}$-alkylaminocarbonyl, di- $C_{1-2}$-alkylaminocarbonyl, pyrrolidinocarbonyl, piperidinocarbonyl or morpholinocarbonyl group,

an ethyl group which is optionally substituted by a methyl group and is substituted in the 2-position by a hydroxyl, $C_{1-2}$-alkoxy, amino, $C_{1-2}$-alkylamino, di-$C_{1-2}$-alkylamino, acetylamino, 1-pyrrolidinyl, morpholino, 1-piperazinyl, 4-methyl-1-piperazinyl, 4-acetyl-1-piperazinyl, 4-aminocarbonyl-1-piperazinyl, 4-dimethylaminocarbonyl-1-piperazinyl, 4-methylaminocarbonyl-1-piperazinyl, 4-methylsulphonyl-1-piperazinyl, 4-methoxycarbonyl-1-piperazinyl or 4-cyano-1-piperazinyl group,

a 2-hydroxyethyl group which is substituted in the ethyl part by a phenyl, 3-hydroxyphenyl, 4-hydroxyphenyl, 4-nitrophenyl or benzyl group, wherein the ethyl part of the abovementioned groups can additionally be substituted by a methyl, hydroxymethyl or methoxymethyl group,

a methyl group which is substituted by a 1,4,7,10-tetra-oxacyclododecyl, 1,4,7,10,13-pentaoxacyclopentadecyl or a 1,4,7,10,13,16-hexaoxacyclooctadecyl group,

a 2,2,2-trifluoroethyl group,

a propyl group which is substituted in the 3-position by a hydroxyl, cyano, carboxyl, aminocarbonyl, methylaminocarbonyl, dimethylaminocarbonyl, formylamino, methoxycarbonylamino, morpholino or 2-oxo-1-pyrrolidinyl group,

a butyl group which is substituted in the 4-position by a hydroxyl group,

a 3-butyl group which is substituted in the 1-position by a hydroxyl group and additionally in the 3-position by a methyl group,

a 2-butyl group which is substituted in the 1-position by a hydroxyl group and additionally in the 3-position by a methyl group,

a 4-pentyl group which is substituted in the 1-position by a hydroxyl group and additionally in the 4-position by a methyl group,

a 2,3-dihydroxypropyl,3-amino-2-hydroxypropyl, tris-(3-hydroxypropyl)methyl, 1,3-dihydroxy-2-propyl, 1,3-dihydroxy-2-methyl-2-propyl or tris-(hydroxymethyl)methyl group,

a 2-propyl group which is substituted in the 2-position by a hydroxylmethyl, $C_{1-2}$-alkoxymethyl, carboxyl, $C_{1-2}$-alkoxycarbonyl, aminocarbonyl, N-$C_{1-2}$-alkylaminocarbonyl, N,N-di-$C_{1-2}$-alkylaminocarbonyl, pyrrolidinocarbonyl, morpholinocarbonyl or (2-hydroxyethyl)aminocarbonyl group,

a 4-tetrahydropyranyl, tetrahydrofurfuryl, 1-deoxy-1-D-sorbityl or 2-(2-hydroxyethyloxy)ethyl group,

a cyclopentyl group which is substituted in the 2- or 3-position by a hydroxyl group or in the 1-position by a hydroxymethyl group,

a cyclohexyl group which is substituted in the 2-, 3- or 4-position by a hydroxymethyl, hydroxyl, $C_{1-2}$-alkoxy, ($C_{1-4}$-alkoxy)-carbonylamino, amino, $C_{1-2}$-alkylamino, di-$C_{1-2}$-alkylamino, carboxyl, $C_{1-2}$-alkoxycarbonyl, aminocarbonyl, $C_{1-2}$-alkylaminocarbonyl, di-$C_{1-2}$-alkylaminocarbonyl, N-oxido-di- $C_{1-2}$-alkylamino, pyrrolidinocarbonyl, morpholinocarbonyl, $C_{1-2}$-alkylcarbonyl-amino or $C_{1-2}$-alkylsulphonylamino group and can additionally

be substituted by a methyl group,

a cyclohexyl group which is substituted in the 4-position by a 1-pyrrolidinyl, 2-oxo-1-pyrrolidinyl, 2-hydroxymethyl-1-pyrrolidinyl, N-$C_{1-2}$-alkyl-(2-hydroxyethyl)amino, di-(2-hydroxyethyl)amino, N-$C_{1-2}$-alkyl-$C_{1-2}$-alkylcarbonylamino, morpholino, 2-oxo-1-imidazolidinyl, 3-methyl-2-oxo-1-imidazolidinyl, carboxy-$C_{1-2}$-alkyl, carboxy-$C_{1-2}$-alkoxy, $C_{1-2}$-alkoxycarbonyl, $C_{1-2}$-alkyl, $C_{1-2}$-alkoxycarbonyl-$C_{1-2}$-alkoxy, aminocarbonyl-$C_{1-2}$-alkyl, aminocarbonyl-$C_{1-2}$-alkoxy, $C_{1-2}$-alkylamino-carbonyl-$C_{1-2}$-alkyl, $C_{1-2}$-alkylamino-carbonyl-$C_{1-2}$-alkoxy, di-$C_{1-2}$-alkylamino-carbonyl-$C_{1-2}$-alkyl, di-$C_{1-2}$-alkylamino-carbonyl-$C_{1-2}$-alkoxy, pyrrolidinocarbonyl-$C_{1-2}$-alkyl, pyrrolidinocarbonyl-$C_{1-2}$-alkoxy, morpholinocarbonyl- $C_{1-2}$-alkyl, morpholinocarbonyl-$C_{1-2}$-alkoxy, piperidinocarbonyl-$C_{1-2}$-alkyl or piperidinocarbonyl-$C_{1-2}$-alkoxy group,

a cyclohexyl group in which two hydrogen atoms in the 4-position are replaced by an oxo group or an n-$C_{4-5}$-alkylene bridge,

a cyclohexyl group in which the methylene group in the 4-position is replaced by an imino, $C_{1-2}$-alkyl-imino, phenyl-$C_{1-2}$-alkyl-imino, N-methyl-N-oxido-imino, formylimino, $C_{1-2}$-alkylcarbonyl-imino, $C_{1-2}$-alkylsulphonyl-imino, $C_{1-2}$-alkoxycarbonyl-imino, cyano-imino, aminocarbonylimino, $C_{1-2}$-alkylaminocarbonyl-imino or N,N-di-$C_{1-2}$-alkylaminocarbonyl-imino group,

a cyclohexyl group in which the methylene group in the 3-position is replaced by an imino, $C_{1-2}$-alkyl-imino, $C_{1-2}$-alkylcarbonyl-imino, $C_{1-2}$-alkylsulphonyl-imino or $C_{1-2}$-alkoxycarbonyl-imino group,

a cyclopentyl group in which the methylene group in the 3-position is replaced by an oxygen atom or an imino, $C_{1-2}$-alkyl-imino, formyl-imino, $C_{1-2}$-alkylcarbonyl-imino, $C_{1-2}$-alkylsulphonyl-imino, $C_{1-2}$-alkoxycarbonyl-imino, cyano-imino or N,N-di-$C_{1-2}$-alkylaminocarbonylimino group,

a cyclohexylmethyl group, wherein the cyclohexyl part is substituted in the 4-position by a carboxyl, $C_{1-2}$-alkoxycarbonyl, N,N-di-$C_{1-2}$-alkylaminocarbonyl or morpholinocarbonyl group,

a norbornan-2-yl, norbornan-2-yl-methyl, 5-norbornen-2-yl-methyl, bornyl, 3-quinuclidinyl or adamantyl group or

$R_{10}$ is a hydrogen atom or a methyl group and $R_{11}$ is a hydroxyl or methoxy group,

their tautomers, their stereoisomers and their salts.

**6.** Pyrimido[5,4-d]pyrimidines of the general formula I according to Claim 1 or 2, in which

$R_a$ is a hydrogen atom or a methyl group,

$R_b$ is a 2-naphthyl or 5-indanyl group or a phenyl group which is substituted by the radicals $R_1$ to $R_3$, wherein

$R_1$ is a hydrogen, fluorine, chlorine, bromine or iodine atom or a methyl, ethyl, tert-butyl, trifluoromethyl, ethynyl, methoxy, cyclopropyl, trifluoromethoxy, cyano, ethoxycarbonyl or nitro group,

$R_2$ is a hydrogen, fluorine or chlorine atom or an amino, methyl or trifluoromethyl group and

$R_3$ is a hydrogen, chlorine or bromine atom, and

$R_c$ is a hydrogen atom or a hydroxyl, methoxy, butyloxy, cyclopentyloxy, 2-[(2-hydroxyethyl)amino]-ethoxy, methyl-sulphenyl, methylsulphinyl or methylsulphonyl group,

a 1-azetidinyl group or a 1-pyrrolidinyl group which is optionally substituted by one or two methyl groups,

a 1-piperidinyl group which is substituted by a hydroxymethyl group,

a 1-piperidinyl group which is optionally substituted by one or two methyl groups and in which the methylene group in the 4-position can be replaced by an oxygen or sulphur atom or by a carbonyl, sulphinyl, sulphonyl,

imino, methyl-imino, N-oxido-N-methyl-imino, 2-propylaminocarbonyl-methyl-imino, phenyl-imino, benzylimino, acetyl-imino or methylsulphonyl-imino group,

a 1-piperidinyl group which is substituted in the 3-position by a hydroxyl or diethylaminocarbonyl group or in the 4-position by a hydroxyl, carboxyl, methoxycarbonyl, aminocarbonyl, methylaminocarbonyl, dimethylaminocarbonyl, pyrrolidinocarbonyl, morpholinocarbonyl, amino, acetylamino, methoxycarbonylamino, formylamino, cyanoamino, dimethylaminocarbonylamino, methylsulphonylamino or phenyl group,

a 4-hydroxy-4-phenyl-1-piperidinyl group,

a 1,2,3,4-tetrahydro-isoquinolin-2-yl or 2,3,4,5-tetrahydro-lH-3-benzazepin-3-yl group,

a 1-piperidinyl group in which two hydrogen atoms in the 4-position are replaced by an $-OCH_2CH_2-O-$ bridge,

a 1-azacyclohept-1-yl group in which two hydrogen atoms in the 3- and 6-position are replaced by a $-CH_2-CH_2-$group or

an $(R_{10}NR_{11})$- group, in which

$R_{10}$ is a hydrogen atom, a $C_{1-4}$-alkyl group or a 2-hydroxyethyl group and

$R_{11}$ is a hydrogen atom,

a phenyl group which is optionally substituted by a methyl group,

a phenyl group which is substituted in the 4-position by a morpholino or 2-(hydroxymethyl)-1-pyrrolidinyl group,

a $C_{1-6}$-alkyl, $C_{3-6}$-cycloalkyl, phenyl-$C_{1-3}$-alkyl, cyclopropylmethyl, allyl, propargyl, 2-hydroxyethyl, 1-hydroxy-2-propyl, 3-hydroxypropyl, 4-hydroxybutyl, 2-methyoxyethyl, 1-adamantyl, norbornan-2-yl, aminocarbonylmethyl, 2-(dimethylamino)ethyl, 3-quinuclidinyl, 2,2,2-trifluoroethyl, 4-piperidinyl, 1-methyl-4-piperidinyl, 1-methyl-1-oxido-4-piperidinyl, 1-ethoxycarbonyl4-piperidinyl, 1-benzyl-4-piperidinyl, 2-(2-hydroxyethoxy)ethyl, 4-tetrahydropyranyl, 1-hydroxy-2-methyl-2-propyl, 1-methoxy-2-methyl-2-propyl, 2-(methylaminocarbonyl)-2-propyl, 2,3-dihydroxy-1-propyl, 2-(morpholino)ethyl, 1-deoxy-1-D-sorbityl, 3-(2-oxo-1-pyrrolidinyl)-propyl, tris-(hydroxymethyl)methyl, 1,3-dihydroxy-2-propyl, 1,3-dihydroxy-2-methyl-2-propyl or bornyl group,

a 2-hydroxyethyl group which is substituted in the 2-position by a phenyl group and in the 1-position additionally by a methyl or hydroxymethyl group,

a methylcyclohexyl, 4-carboxy-cyclohexyl, 4-methoxycarbonyl-cyclohexyl, 4-dimethylaminocarbonyl-cyclohexyl, 4-(1-pyrrolidinylcarbonyl)-cyclohexyl, 4-(morpholinocarbonyl)-cyclohexyl, 4-12-(methoxycarbonyl) ethyl]cyclohexyl, 4-(2-carboxy-ethyl)cyclohexyl, 4-(tert-butyloxycarbonylamino)-cyclohexyl, 4-methoxycyclohexyl, 4-aminocyclohexyl, 4-(dimethylamino)cyclohexyl, 4-(N,N-dimethyl-N-oxido-amino)cyclohexyl, 4-(acetylamino)cyclohexyl, 4-(methylsulphonylamino)-cyclohexyl, 2-hydroxycyclohexyl, 4-hydroxycyclohexyl, 4-(hydroxymethyl)-cyclohexyl, 4-hydroxy-4-methylcyclohexyl or 4-oxocyclohexyl group,

a methyl group which is substituted by a 1,4,7,10,13-pentaoxacyclopentadecyl or a 1,4,7,10,13,16-hexaoxacyclooctadecyl group, or

$R_{10}$ is a methyl group and $R_{11}$ is a methoxy group,

their tautomers, their stereoisomers and their salts.

**7.** The following compounds of the general formula I according to Claim 1 or 2:

(1) 4-[(3,4-Dichlorophenyl)amino]-6-[(trans-4-hydroxycyclohexyl)-amino]-pyrimido-[5,4-d]-pyrimidine,

(2) 4-[(3-Chloro-4-fluoro-phenyl)amino]-6-[(trans-4-hydroxycyclohexyl)-amino]-pyrimido-[5,4-d]-pyrimidine,

(3) 4-[(3-Bromophenyl)amino]-6-[(trans-4-hydroxycyclohexyl)-amino]-pyrimido-[5,4-d]-pyrimidine,

(4) 4-[(3-Chlorophenyl)amino]-6-(cyclopropylamino)-pyrimido-[5,4-d]-pyrimidine,

(5) 4-[(3-Methylphenyl)amino]-6-(4-amino-1-piperidinyl)-pyrimido[5,4-d]pyrimidine,

(6) 4-[(3-Methylphenyl)amino]-6-[(trans-4-aminocyclohexyl)-amino]-pyrimido-[5,4-d]-pyrimidine,

(7) 4-[(3-Methylphenyl)amino]-6-(N-(trans-4-hydroxycyclohexyl)-N-methyl-amino)-pyrimido-[5,4-d]-pyrimidine,

(8) 4-[(3-Methylphenyl)amino]-6-(4-methoxycarbonylamino-l-piperidinyl)-pyrimido-[5,4-d]-pyrimidine,

(9) 4-[(3-Methylphenyl)amino]-6-[trans-4-(morpholinocarbonyl)-cyclohexylamino]-pyrimido-[5,4-d]-pyrimidine,

(10) 4-[(3-Methylphenyl)amino]-6-[trans-4-(pyrrolidinocarbonyl)cyclohexylamino]-pyrimido-[5,4-d]-pyrimidine,

(11) 4-[(3-Chloro-4-fluoro-phenyl)amino]-6-[N-(trans-4-hydroxycyclohexyl)-N-methyl-amino]-pyrimido-[5,4-d]-pyrimidine,

(12) 4-[(4-Amino-3,5-dichloro-phenyl)amino]-6-[(trans-4-hydroxycyclohexyl)-amino]-pyrimido-[5,4-d]-pyrimidine,

(13) 4-[(3-Chloro-4-fluoro-phenyl)amino]-6-[2-(morpholino)-ethylamino]-pyrimido-[5,4-d]-pyrimidine,

(14) 4-[(4-Amino-3,5-dibromo-phenyl)amino]-6-[(trans-4-hydroxycyclohexyl)-amino]-pyrimido-[5,4-d]-pyrimidine,

(15) 4-[(3-Chloro-4-fluoro-phenyl)amino]-6-morpholinopyrimido-[5,4-d]-pyrimidine,

(16) 4-[(3-Chloro-4-fluoro-phenyl)amino]-6-(1-hydroxy-2-methyl-2-propylamino)-pyrimido-[5,4-d]-pyrimidine,

(17) 4-[(3-Chloro-4-fluoro-phenyl)amino]-6-(1-hydroxy-2-propylamino)-pyrimido-[5,4-d]-pyrimidine,

(18) 4-[(3-Chloro-4-fluoro-phenyl)amino]-6-(1,3-dihydroxy-2-propylamino)-pyrimido-[5,4-d]-pyrimidine,

(19) 4-[(3-Chloro-4-fluoro-phenyl)amino]-6-[4-amino-1-piperidinyl]-pyrimido-[5,4-d]-pyrimidine,

(20) 4-[(3-Methylphenyl)amino]-6-(4-piperidinyl-amino)-pyrimido[5,4-d]pyrimidine,

(21) 4-[(3-Methylphenyl)amino]-6-(4-formylamino-1-piperidinyl)-pyrimido[5,4-d]pyrimidine,

(22) 4-[(3-Methylphenyl)amino]-6-[(1-ethoxycarbonyl-4-piperidinyl)amino]-pyrimido[5,4-d]pyrimidine,

(23) 4-[(3-Methylphenyl)amino]-6-[(3-quinuclidinyl)amino]-pyrimido[5,4-d]pyrimidine,

(24) 4-[(3-Chloro-4-fluoro-phenyl)amino]-6-[(4-aminocyclohexyl)amino]-pyrimido-[5,4-d]-pyrimidine,

(25) 4-[(3-Chloro-4-fluoro-phenyl)amino]-6-(4-piperidinyl-amino)-pyrimido-[5,4-d]-pyrimidine,

(26) 4-[(3-Chloro-4-fluoro-phenyl)amino]-6-[trans-4-(morpholinocarbonyl)cyclohexylamino]-pyrimido-[5,4-d]-pyrimidine,

(27) 4-[(3-Chloro-4-fluoro-phenyl)amino]-6-[trans-4-(pyrrolidinocarbonyl)cyclohexylamino]-pyrimido-[5,4-d] pyrimidine,

(28) 4-[(4-Fluorophenyl)amino]-6-(cyclopropylamino)-pyrimido-[5,4-d]-pyrimidine,

(29) 4-[(3-Chloro-4-fluoro-phenyl)amino]-6-(cyclopropylamino)-pyrimido-[5,4-d]-pyrimidine,

(30) 4-[(3,4-Dichlorophenyl)amino]-6-(cyclopropylamino)pyrimido-[5,4-d]-pyrimidine,

(31) 4-[(3-Chloro-4-fluorophenyl)amino]-6-(1-methyl-4-piperidinyl-amino)-pyrimido[5,4-d]pyrimidine and

(32) 4-[(3-Chloro-4-fluorophenyl)amino]-6-[(trans-4-dimethylaminocyclohexyl)amino]-pyrimido[5,4-d]pyrimidine and their salts.

**8.** 4-[(3-Chloro-4-fluorophenyl)amino]-6-(1-methyl-4-piperidinyl-amino)-pyrimido[5,4-d]pyrimidine and the salts thereof.

**9.** 4-[(3-Chloro-4-fluorophenyl)amino]-6-(trans-4-dimethylaminocyclohexyl)amino]-pyrimido[5,4-d]pyrimidine and the salts thereof.

**10.** Physiologically tolerated salts of the compounds according to at least one of Claims 1 to 7 with inorganic or organic acids or bases.

**11.** Medicaments comprising a compound according to at least one of Claims 1 to 9 or a physiologically tolerated salt according to Claim 10, if appropriate in addition to one or more inert carrier substances and/or diluents.

**12.** Use of a compound according to at least one of Claims 1 to 10 for the preparation of a medicament which is suitable for treating benign or malignant tumours, particularly tumours of epithelial and neuroepithelial origin, metastatic spread and abnormal proliferation of vascular endothelial cells (neoangiogenesis).

**13.** Process for the preparation of a medicament according to Claim 11, characterized in that a compound according to at least one of Claims 1 to 10 is incorporated by a non-chemical route into one or more inert carrier substances and/or diluents.

**14.** Process for the preparation of compounds of the formula I according to Claims 1 to 10, characterized in that

a) a compound of the formula

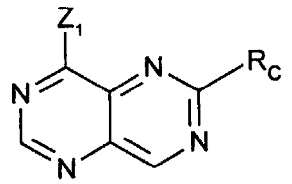

(II),

in which

$R_c$ is as defined in Claims 1 to 10 and
$Z_1$ is a leaving group, is reacted with an amine of the formula

$$H\text{-}(R_aNR_b) \quad \text{(III)}$$

in which
$R_a$ and $R_b$ are as defined in Claims 1 to 9, or

b) to prepare compounds of the formula I in which $R_c$ is one of the radicals mentioned for $R_c$ in Claims 1 to 8 linked to the pyrimido[5,4-d]pyrimidine via an oxygen or nitrogen atom or via a mercapto or sulphenyl group,

a compound of the formula

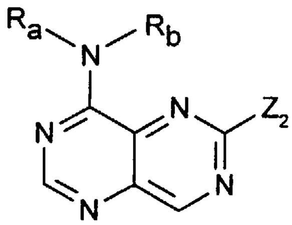

(IV),

in which

$R_a$ and $R_b$ are as defined in Claims 1 to 9 and
$Z_2$ is a leaving group, is reacted with a compound of the formula

$$X_1 - H \quad (V)$$

in which
$X_1$ is one of the radicals mentioned for $R_c$ in Claims 1 to 9 linked to the pyrimido[5,4-d]pyrimidine via an oxygen or nitrogen atom or via a mercapto or sulphenyl group, or

c) to prepare compounds of the general formula I in which $R_c$ is one of the radicals mentioned for $R_c$ in Claims 1 to 9 linked to the pyrimido[5,4-d]pyrimidine via a sulphinyl or sulphonyl group, a compound of the formula

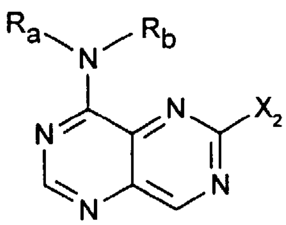

(VI),

in which

$R_a$ and $R_b$ are as defined in Claims 1 to 9 and
$X_2$ is one of the radicals mentioned for $R_c$ in Claims 1 to 8 linked to the pyrimido[5,4-d]pyrimidine via a sulphur atom, is oxidized, or

d) to prepare a compound of the formula I in which $R_c$ is a hydrogen atom, a compound of the formula

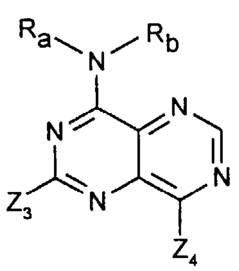

, (VII)

in which

$R_a$ and $R_b$ are as defined in Claims 1 to 9 and
$Z_3$ and $Z_4$, which can be identical or different, are each a halogen atom, is dehalogenated, and

thereafter, if desired, a compound of the formula I thus obtained which contains an amino, alkylamino or imino group is converted into a corresponding acyl or sulphonyl compound of the formula I by means of acylation or sulphonylation, and/or

a compound of the formula I thus obtained which contains an amino, alkylamino or imino group is converted into a corresponding alkyl compound of the formula I by means of alkylation or reductive alkylation, and/or

a compound of the formula 1 thus obtained which contains a carboxyl group is converted into a corresponding ester of the formula I by means of esterification, and/or

a compound of the formula I thus obtained which contains a carboxyl or ester group is converted into a corresponding amide of the formula I by means of amidation, and/or

a compound of the formula I which contains a primary or secondary hydroxyl group is converted into a corresponding carbonyl compound of the formula I by means of oxidation, and/or

if required, a protective radical used in the reactions described above is split off again, and/or

if desired, a compound of the formula I thus obtained is separated into its stereoisomers, and/or

a compound of the formula I thus obtained is converted into a salt thereof, in particular for pharmaceutical use into a physiologically tolerable salt thereof.

## Revendications

**1.** Pyrimido[5,4-d]pyrimidines de formule générale

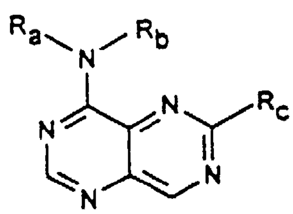

(I)

dans laquelle

$R_a$ représente un atome d'hydrogène ou un groupe alkyle,
$R_b$ représente un groupe phényle substitué par les restes $R_1$ à $R_3$, où

$R_1$ et $R_2$, qui peuvent être identiques ou différents, représentent chacun un atome d'hydrogène, de fluor, de chlore, de brome ou d'iode,
un groupe alkyle en $C_1$-$C_6$, hydroxy ou alcoxy en $C_1$-$C_6$,
un groupe cycloalkyle en $C_3$-$C_7$ ou cycloalcoxy en $C_4$-$C_7$, pouvant chacun être substitué par un ou deux groupes alkyle ou par un groupe aryle,
un groupe alcényle en $C_2$-$C_5$ ou alcényloxy en $C_3$-$C_5$ éventuellement substitué par un groupe aryle, la partie vinylique n'étant pas liée à l'atome d'oxygène,
un groupe alcynyle en $C_2$-$C_5$ ou alcynyloxy en $C_3$-$C_5$ éventuellement substitué par un groupe aryle, la partie éthynyle n'étant pas liée à l'atome d'oxygène,
un groupe aryle, aryloxy, aralkyle, aralcoxy, alkylsulfényle, alkylsulfinyle, alkylsulfonyle, alkylsulfonyloxy, trifluorométhylsulfényle, trifluorométhylsulfinyle, trifluorométhylsulfonyle, arylsulfényle, arylsulfinyle, arylsulfonyle, aralkylsulfényle, aralkylsulfinyle ou aralkylsulfonyle,
un groupe méthyle ou méthoxy substitué par 1 à 3 atomes de fluor,

un groupe alkyle en $C_2$-$C_4$ ou alcoxy en $C_2$-$C_4$ substitué par 1 à 5 atomes de fluor, un groupe nitro, amino, alkylamino, dialkylamino, cycloalkylamino en $C_3$-$C_7$, N-alkyl-(cycloalkyl en $C_3$-$C_7$)amino, arylamino, N-alkyl-arylamino, aralkylamino ou N-alkyl-aralkylamino,
un groupe alkylène-imino de 4 à 7 chaînons éventuellement substitué par 1 à 4 groupes alkyle, un ou deux des groupes méthylène adjacents à l'atome d'azote des groupes alkylène-imino de 5 à 7 chaînons précités pouvant être remplacés par un groupe carbonyle, ou un groupe méthylène en position 4 des groupes alkylène-imino de 6 à 7 chaînons précités pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,
un groupe (alkylène-imino)carbonyle ou (alkylène-imino)sulfonyle ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,
un groupe alkylcarbonylamino, N-alkyl-alkylcarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, perfluoroalkylsulfonylamino, N-alkyl-perfluoroalkylsulfonylamino, alkylcarbonyle, arylcarbonyle, aralkylcarbonyle, aryl-hydroxyméthyle, aralkyl-hydroxyméthyle, carboxy, alcoxycarbonyle, aralcoxycarbonyle, aminocarbonyle, alkylaminocarbonyle, dialkylaminocarbonyle, arylaminocarbonyle, N-alkyl-arylaminocarbonyle, aralkylaminocarbonyle, N-alkyl-aralkylaminocarbonyle, N-hydroxyaminocarbonyle, N-hydroxyalkylaminocarbonyle, N-alcoxyaminocarbonyle, N-alcoxy-alkylaminocarbonyle, cyano, azido, N-cyanoamino ou N-cyanoalkylamino,
un groupe sulfo, alcoxysulfonyle, aminosulfonyle, alkylaminosulfonyle, dialkylaminosulfonyle, arylaminosulfonyle, N-alkyl-arylaminosulfonyle, aralkylaminosulfonyle ou N-alkyl-aralkylaminosulfonyle,
un groupe phosphono, O-alkylphosphono, O,O'-dialkylphosphono, O-aralkylphosphono ou O,O'-diaralkylphosphono,
un groupe alkyle ou alcoxy substitué par $R_4$, où

> $R_4$ représente un groupe hydroxy, alcoxy, aryloxy, aralcoxy, amino, alkylamino, dialkylamino, alkylsulfényle, alkylsulfinyle, alkylsulfonyle, arylsulfényle, arylsulfinyle, arylsulfonyle, aralkylsulfényle, aralkylsulfinyle, aralkylsulfonyle, carboxy, alcoxycarbonyle, aminocarbonyle, alkylaminocarbonyle, dialkylaminocarbonyle ou cyano,
> un groupe alkylène-imino de 4 à 7 chaînons éventuellement substitué par 1 à 4 groupes alkyle, un ou deux des groupes méthylène adjacents à l'atome d'azote des groupes alkylène-imino de 5 à 7 chaînons précités pouvant être remplacés par un groupe carbonyle, ou un groupe méthylène en position 4 des groupes alkylène-imino de 6 à 7 chaînons précités pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,
> un groupe (alkylène-imino)carbonyle ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,

$R_3$ représente un atome d'hydrogène, de fluor, de chlore ou de brome ou un groupe alkyle, alcoxy ou trifluorométhyle, ou
$R_2$ et $R_3$, dans la mesure où ils sont liés à des atomes de carbone adjacents, peuvent aussi représenter ensemble un groupe méthylènedioxy éventuellement substitué par un ou deux groupes alkyle, un groupe n-alkylène en $C_3$-$C_6$ éventuellement substitué par un ou deux groupes alkyle ou un groupe 1,3-butadiène-1,4-diylène éventuellement substitué par un ou deux atomes de fluor, de chlore, de brome ou d'iode, par un ou deux groupes hydroxy, alkyle, alcoxy, trifluorométhyle ou cyano, les substituants pouvant être identiques ou différents, ou

$R_a$ et $R_1$, dans la mesure où $R_1$ se trouve en position ortho par rapport à l'atome d'azote substitué par $R_a$, représentent aussi ensemble un groupe n-alkylène en $C_2$-$C_4$ éventuellement substitué par un ou deux groupes alkyle, et
$R_c$ représente un atome d'hydrogène, de fluor, de chlore, de brome ou d'iode,

un groupe alkyle, cycloalkyle en $C_3$-$C_7$, (cycloalkyle en $C_3$-$C_7$)-(alkyle en $C_1$-$C_3$), aryle, aralkyle, hydroxy, aryloxy, aralcoxy, mercapto, alkylsulfényle en $C_1$-$C_8$, alkylsulfinyle en $C_1$-$C_8$, alkylsulfonyle en $C_1$-$C_8$, cycloalkylsulfényle en $C_4$-$C_7$, cycloalkylsulfinyle en $C_4$-$C_7$, cycloalkylsulfonyle en $C_4$-$C_7$, (cycloalkyl en $C_3$-$C_7$)(alkylsulfényle en $C_1$-$C_3$), (cycloalkyl en $C_3$-$C_7$)-(alkylsulfinyle en $C_1$-$C_3$), (cycloalkyl en $C_3$-$C_7$)-(alkylsulfonyle en $C_1$-$C_3$), arylsulfényle, arylsulfinyle, arylsulfonyle, aralkylsulfényle, aralkylsulfinyle ou aralkylsulfonyle,
un groupe alcoxy en $C_1$-$C_8$ pouvant être substitué par un groupe alcoxycarbonyle, cyano, carboxy, aminocarbonyle, alkylaminocarbonyle ou dialkylaminocarbonyle,
un groupe alcoxy en $C_2$-$C_8$ substitué par un groupe hydroxy, alcoxy, hydroxyalkylamino en $C_2$-$C_4$, amino, alkylamino, dialkylamino, alkylcarbonylamino, N-alkyl-N-(alkylcarbonyl)amino, alkylsulfonylamino, N-alkyl-N-(alkylsulfonyl)amino, alcoxycarbonylamino ou N-alkyl-N-(alcoxycarbonyl)amino, par un groupe alkylène-imino de 5 à 7 chaînons éventuellement substitué par 1 à 4 groupes alkyle, un ou deux groupes méthylène adjacents à l'atome d'azote des groupes alkylène-imino de 5 à 7 chaînons précités pouvant être remplacés par un groupe carbonyle, et en outre un groupe méthylène en position 4 des groupes alkylène-imino de 6 à 7 chaînons précités pouvant être remplacé par un atome d'oxygène ou de soufre ou par un groupe carbonyle, sulfinyle, sulfonyle, imino, N-alkylimino, N-alkylcarbonylimino, N-formylimino, N-dialkylaminocarbonylimino, N-alcoxycarbonylimino, N-alkylsulfonylimino, N-arylimino ou N-aralkylimino,
un groupe alcoxy en $C_3$-$C_8$ substitué par deux groupes hydroxy ou alcoxy,
un groupe alcoxy en $C_1$-$C_8$ substitué par un groupe cycloalkyle en $C_3$-$C_7$, la partie cycloalkyle pouvant être substituée par 1 à 4 groupes alkyle et un groupe méthylène des parties cycloalkyle en $C_4$-$C_7$ précitées pouvant être remplacé par un atome d'oxygène ou de soufre ou par un groupe carbonyle, sulfinyle, sulfonyle, imino, N-alkylimino, N-alkylcarbonylimino, N-alcoxycarbonyl-imino, N-alkylsulfonylimino, N-arylimino ou N-aralkylimino,
un groupe cycloalcoxy en $C_4$-$C_7$ éventuellement substitué par un ou deux groupes hydroxy ou par un groupe alcoxy, alcoxycarbonyle, cyano, carboxy, aminocarbonyle, alkylaminocarbonyle, dialkylaminocarbonyle, hydroxyalkylamino en $C_2$-$C_4$, amino, alkylamino, dialkylamino, alkylcarbonylamino, N-alkyl-N-(alkylcarbonyl) amino, alkylsulfonylamino, N-alkyl-N-(alkylsulfonyl)amino, alcoxycarbonylamino ou N-alkyl-N-(alcoxycarbonyl)amino, un groupe méthylène des parties cycloalkyle en $C_5$-$C_7$ précitées pouvant être remplacé par un atome d'oxygène ou de soufre ou par un groupe sulfinyle, sulfonyle, imino, N-alkylimino, N-alkylcarbonylimino, N-alcoxycarbonylimino, N-alkylsulfonylimino, N-arylimino ou N-aralkylimino,
un groupe alcényloxy en $C_3$-$C_8$ éventuellement substitué par un groupe aryle ou un groupe cycloalkyle en $C_3$-$C_7$, la partie vinylique ne pouvant pas être liée à l'atome d'oxygène,
un groupe alcynyloxy en $C_3$-$C_8$ éventuellement substitué par un groupe aryle ou un groupe cycloalkyle en $C_3$-$C_7$, la partie éthynyle ne pouvant pas être liée à l'atome d'oxygène,
un groupe alkylène-imino de 4 à 8 chaînons éventuellement substitué par 1 à 4 groupes alkyle ou 1 à 2 groupes aryle, et qui peut en outre être substitué par le reste $R_5$, où

$R_5$ représente un groupe aryle, aralkyle, carboxy, alcoxycarbonyle, aminocarbonyle, alkylaminocarbonyle, dialkylaminocarbonyle, cyano, hydroxy, alcoxy, aryloxy, aralcoxy, alkylcarbonyloxy, arylcarbonyloxy, amino, alkylamino, hydroxyalkylamino en $C_2$-$C_4$, dialkylamino, cyanoamino, formylamino, N-alkyl-N-(hydroxyalkyl en $C_2$-$C_4$)amino ou bis(hydroxyalkyl en $C_2$-$C_4$)amino,
un groupe (alkylène-imino)carbonyle ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,
un groupe alkylène-imino de 4 à 7 chaînons éventuellement substitué par 1 à 4 groupes alkyle ou par un groupe hydroxyalkyle, un ou deux groupes méthylène adjacents à l'atome d'azote des groupes alkylène-imino de 5 à 7 chaînons précités pouvant être remplacés par un groupe carbonyle,
un groupe alkylène-imino de 6 ou 7 chaînons éventuellement substitué par 1 à 4 groupes alkyle ou par un groupe hydroxyalkyle, un groupe méthylène en position 4 de la partie alkylène-imino étant remplacé par un atome d'oxygène ou de soufre ou par un groupe carbonyle, sulfinyle, sulfonyle, imino, N-alkylimino, N-alkylcarbonylimino, N-alkylsulfonylimino, N-arylimino ou N-aralkylimino, et, de plus, un ou deux des groupes méthylène adjacents à l'atome d'azote dans la partie alkylène-imino des groupes précités pouvant être remplacés par un groupe carbonyle,
un groupe alkylène-imino de 4 à 7 chaînons substitué par un groupe hydroxy, alcoxy, amino, alkylamino, dialkylamino, alkylcarbonylamino, N-alkyl-alkylcarbonylamino, alkylsulfonylamino, N-alkyl-alkylsulfonylamino ou hydroxyalkyle,
un groupe alkylcarbonylamino, N-alkyl-alkylcarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino, N-alkyl-alcoxycarbonylamino, aralcoxycarbonylamino ou N-alkyl-aralcoxycarbonylamino, un groupe $(NR_7R_8)CONR_6$ ou $(NR_7R_8)SO_2NR_6$, dans lesquels

$R_6$, $R_7$ et $R_8$, qui peuvent être identiques ou différents, représentent chacun un atome d'hydrogène ou un groupe alkyle, ou $R_6$ et $R_7$ forment ensemble un groupe n-alkylène en $C_2$-$C_4$ et $R_8$ représente un atome d'hydrogène ou un groupe alkyle;

un groupe carboxyalkyle, alcoxycarbonylalkyle, aminocarbonylalkyle, alkylaminocarbonylalkyle ou dialkylaminocarbonylalkyle,
un groupe (alkylène-imino)carbonylalkyle ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino ou N-alkylimino,
un groupe carboxyalcoxy, alcoxycarbonylalcoxy, aminocarbonylalcoxy, alkylaminocarbonylalcoxy ou dialkylaminocarbonylalcoxy,
un groupe (alkylène-imino)carbonylalcoxy ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino ou N-alkylimino,
un groupe cyanoalkyle, hydroxyalkyle, alcoxyalkyle, aryloxyalkyle, aminoalkyle, alkylaminoalkyle ou dialkylaminoalkyle,
un groupe (alkylène-imino)alkyle ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino ou N-alkylimino,
un groupe alkylcarbonylaminoalkyle, N-alkyl-alkylcarbonylaminoalkyle, alkylsulfonylaminoalkyle, N-alkyl-alkylsulfonylaminoalkyle, arylcarbonylaminoalkyle, N-alkyl-arylcarbonylaminoalkyle, arylsulfonylaminoalkyle, N-alkyl-arylsulfonylanùnoalkyle, alkylsulfényle, alkylsulfinyle, alkylsulfonyle, arylsulfényle, arylsulfinyle, arylsulfonyle, aralkylsulfényle, aralkylsulfinyle, aralkylsulfonyle, alkylsulfénylalkyle, alkylsulfinylalkyle, alkylsulfonylalkyle, arylsulfénylalkyle, arylsulfinylalkyle ou arylsulfonylalkyle ou
un groupe cycloalkyle en $C_3$-$C_7$, un groupe méthylène d'un groupe cycloalkyle en $C_5$-$C_7$ pouvant être remplacé par un atome d'oxygène ou de soufre ou par un groupe sulfinyle, sulfonyle, imino ou N-alkylimino,

ou $R_c$ représente un groupe alkylène-imino de 6 à 8 chaînons éventuellement substitué par 1 à 4 groupes alkyle ou un groupe aryle, et qui peut être en outre substitué par le reste $R_5$, un groupe méthylène en position 4 des groupes alkylène-imino précités pouvant être remplacé par un atome d'oxygène ou de soufre ou par un groupe carbonyle, sulfinyle, sulfonyle, N-oxydo-N-alkylimino ou $R_9N$, où

$R_9$ représente un atome d'hydrogène, un groupe alkyle, hydroxyalkyle en $C_2$-$C_4$, alcoxy(alkyle en $C_2$-$C_4$), aminoalkyle en $C_2$-$C_4$, alkylamino(alkyle en $C_2$-$C_4$), dialkylamino(alkyle en $C_2$-$C_4$), (hydroxyalkoxy en $C_2$-$C_4$)alkyle en $C_2$-$C_4$, aminocarbonylalkyle, alkylaminocarbonylalkyle, dialkylaminocarbonylalkyle, aryle, aralkyle, formyle, alkylcarbonyle, alkylsulfonyle, arylcarbonyle, arylsulfonyle, aralkylcarbonyle, aralkylsulfonyle, alcoxycarbonyle, cyano, aminocarbonyle, alkylaminocarbonyle ou dialkylaminocarbonyle, ou un groupe (alkylène-imino)carbonyle ayant 4 à 7 atomes cycliques dans la partie alkylène-imino, un groupe méthylène en position 4 des parties alkylène-imino de 6 à 7 chaînons précitées pouvant être remplacé par un atome d'oxygène ou de soufre ou par un groupe sulfinyle, sulfonyle, imino ou N-alkylimino,

ou $R_c$ représente un groupe 1-pyrrolidinyle éventuellement substitué par 1 à 4 groupes alkyle, et dans lequel deux atomes d'hydrogène liés au squelette carboné sont remplacés par un pont alkylène linéaire, ce pont contenant 2 à 6 atomes de carbone lorsque les deux atomes d'hydrogène se trouvent sur le même atome de carbone, ou 1 à 5 atomes de carbone lorsque les deux atomes d'hydrogène se trouvent sur des atomes de carbone adjacents, ou 2 à 4 atomes de carbone lorsque les deux atomes d'hydrogène se trouvent sur des atomes de carbone séparés par un atome, les groupes 1-pyrrolidinyle précités pouvant en outre être substitués par le reste $R_5$ défini de la manière indiquée précédemment,
un groupe 1-pipéridinyle ou 1-azacyclohept-1-yle éventuellement substitué par 1 à 4 groupes alkyle, et dans lequel deux atomes d'hydrogène liés au squelette carboné sont remplacés par un pont alkylène linéaire, ce

pont contenant 2 à 6 atomes de carbone lorsque les deux atomes d'hydrogène se trouvent sur le même atome de carbone, ou 1 à 5 atomes de carbone lorsque les deux atomes d'hydrogène se trouvent sur des atomes de carbone adjacents, ou 1 à 4 atomes de carbone lorsque les deux atomes d'hydrogène se trouvent sur des atomes de carbone séparés par un atome, ou 1 à 3 atomes de carbone lorsque les deux atomes d'hydrogène se trouvent sur des atomes de carbone séparés par deux atomes, les groupes 1-pipéridinyle et 1-azacyclohept-1-yle précités pouvant en outre être substitués par le reste $R_5$ défini de la manière indiquée précédemment,
un groupe 1-pyrrolidinyle éventuellement substitué par 1 à 4 groupes alkyle, dans lequel les deux atomes d'hydrogène en position 3 sont remplacés par un groupe $-O-CH_2CH_2-O-$ ou $-O-CH_2CH_2CH_2-O-$,
un groupe 1-pipéridinyle ou 1-azacyclohept-1-yle éventuellement substitué par 1 à 4 groupes alkyle, dans lequel les deux atomes d'hydrogène en position 3 ou en position 4 sont remplacés par un groupe $-O-CH_2CH_2-O-$ ou $-O-CH_2CH_2CH_2-O-$,
un groupe de formule

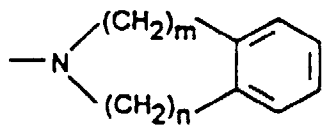

dans lequel m et n, qui peuvent être identiques ou différents, représentent les chiffres 1 à 3 ou
m est égal à 0 et n est égal à 2, 3 ou 4, et la partie benzo ci-dessus peut en outre être mono- ou disubstituée par des atomes de fluor, de chlore, de brome ou d'iode, par des groupes alkyle, trifluorométhyle, hydroxy, alcoxy ou cyano, et la partie imino cyclique saturée ci-dessus peut être substituée par 1 ou 2 groupes alkyle, les substituants pouvant à chaque fois être identiques ou différents,
un groupe $(R_{10}NR_{11})$, dans lequel

$R_{10}$ et $R_{11}$, qui peuvent être identiques ou différents, représentent chacun un atome d'hydrogène,
un groupe alkyle en $C_1$-$C_{16}$ pouvant être substitué

par un ou deux groupes aryle ou cycloalkyle en $C_3$-$C_7$, par un groupe carboxy, alcoxycarbonyle, aminocarbonyle, alkylaminocarbonyle, dialkylaminocarbonyle, hydroxy(alkyl en $C_1$-$C_4$)aminocarbonyle, cyano, hydroxy, alcoxy, aryloxy, aralcoxy, alkylènedioxy en $C_2$-$C_4$, alkylcarbonyloxy, arylcarbonyloxy, formylamino, amino, alkylamino ou dialkylamino, par un groupe (alkylène-imino)carbonyle ayant 4 à 7 atomes cycliques dans la partie alkylène-imino, éventuellement substitué par 1 à 4 groupes alkyle, un groupe méthylène en position 4 d'une partie alkylène-imino de 6 à 7 chaînons pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,
par un groupe alkylène-imino de 4 à 7 chaînons éventuellement substitué par 1 à 4 groupes alkyle, un groupe méthylène en position 4 des groupes alkylène-imino de 6 ou 7 chaînons précités pouvant être remplacé par un atome d'oxygène ou de soufre ou par un groupe sulfinyle, sulfonyle ou $R_9N$, $R_9$ ayant la définition donnée précédemment, et, de plus, un ou deux groupes méthylène adjacents à l'atome d'azote dans les groupes alkylène-imino de 5 à 7 chaînons précités pouvant être remplacés par un groupe carbonyle,
par un groupe alkylsulfonylamino, N-alkyl-alkylsulfonylamino, arylcarbonylamino, N-alkyl-arylcarbonylamino, alkylcarbonylamino, N-alkyl-alkylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino, N-alkyl-alcoxycarbonylamino, aralcoxycarbonylamino ou N-alkyl-aralcoxycarbonylamino, par un groupe $(R_8NR_7)$-CO-$NR_6$ ou $(R_8NR_7)$-$SO_2$-$NR_6$, où $R_6$, $R_7$ et $R_8$ ont la définition donnée précédemment,
par un groupe alkylsulfényle, alkylsulfinyle, alkylsulfonyle, arylsulfényle, arylsulfinyle, arylsulfonyle, aralkylsulfényle, aralkylsulfinyle, ou aralkylsulfonyle, par un groupe cycloalkyle en $C_4$-$C_7$ substitué par $R_5$ et éventuellement substitué en outre par 1 à 4 groupes alkyle, $R_5$ ayant la définition indiquée précédemment, par un groupe cycloalkyle en $C_5$-$C_7$ éventuellement substitué par 1 à 4 groupes alkyle, dans lequel un groupe méthylène est remplacé par un atome d'oxygène ou de soufre ou par un groupe sulfinyle, sulfonyle ou $NR_9$, ou
par un atome de fluor, de chlore, de brome ou d'iode,

un groupe alkyle en $C_1$-$C_{10}$ substitué par 2 ou 3 atomes de fluor,
un groupe alkyle en $C_3$-$C_{10}$ substitué par 4 ou 5 atomes de fluor,
un groupe méthyle substitué par un groupe 1,4,7,10-tétraoxacyclododécyle, 1,4,7,10,13-pentaoxacyclopentadécyle ou 1,4,7,10,13,16-hexaoxacyclooctadécyle, un groupe alkyle en $C_3$-$C_{10}$ substitué par 2 à 5 groupes hydroxy ou alcoxy,
un groupe alkyle en $C_2$-$C_6$ substitué par un groupe aryle et un groupe hydroxy, et qui peut être substitué en plus par un groupe amino, alkylamino, dialkylamino, hydroxy ou alcoxy,
un groupe alkyle en $C_3$-$C_6$ substitué par un groupe amino, alkylamino, dialkylamino, alkylcarbonylamino ou alcoxycarbonylamino et en outre par un groupe hydroxy ou alcoxy,
un groupe alcényle ou alcynyle de 3 à 6 atomes de carbone éventuellement substitué par un groupe aryle ou un groupe cycloalkyle en $C_3$-$C_7$, la partie vinyle ou éthynyle ne pouvant pas être liée à l'atome d'azote,
un groupe alkyle en $C_2$-$C_4$ substitué par un groupe alcoxy en $C_2$-$C_4$ qui est substitué en position $\omega$ par un groupe hydroxy ou alcoxy,
un groupe aryle,
un groupe cyclopropyle pouvant être substitué par 1 ou 2 groupes alkyle, par un groupe aryle, carboxy, alcoxycarbonyle, aminocarbonyle, alkylaminocarbonyle ou dialkylaminocarbonyle ou par un groupe (alkylène-imino)carbonyle ayant 4 à 7 atomes cycliques dans la partie alkylène-imino, éventuellement substitué par 1 à 4 groupes alkyle, un groupe méthylène en position 4 des parties alkylène-imino de 6 à 7 chaînons précitées pouvant être remplacé par un atome d'oxygène ou de soufre ou par un groupe sulfinyle, sulfonyle, imino, N-alkylimino, N-alkylcarbonylimino, N-alkylsulfonylimino, N-arylimino ou N-aralkylimino,
un groupe cycloalkyle en $C_4$-$C_7$ éventuellement substitué par 1 à 4 groupes alkyle, et qui peut être substitué en outre par $R_5$ tel que défini précédemment,
un groupe cycloalkyle en $C_5$-$C_7$ éventuellement substitué par 1 ou 2 groupes alkyle, et qui est substitué en outre par un groupe N,N-dialkyl-N-oxydoamino,
un groupe cycloalcényle en $C_5$-$C_7$ éventuellement substitué par 1 à 4 groupes alkyle, la partie vinylique ne pouvant pas être liée à l'atome d'azote du groupe ($R_{11}NR_{10}$),
un groupe cycloalkyle en $C_4$-$C_7$ éventuellement substitué par 1 à 4 groupes alkyle, qui peut être substitué en outre par $R_5$, un groupe méthylène de la partie cycloalkyle étant remplacé par un atome d'oxygène ou de soufre ou par un groupe sulfinyle, sulfonyle, N-alkyl-N-oxydoimino ou $R_9N$, où $R_5$ et $R_9$ ont la définition indiquée précédemment,
un groupe cycloalkyle en $C_5$-$C_7$ ou (cycloalkyl en $C_5$-$C_7$)alkyle éventuellement substitué par 1 à 4 groupes alkyle, dans lesquels un groupe méthylène de la partie cycloalkyle est remplacé par un groupe carbonyle,
un groupe cyclopentyle ou cyclopentylalkyle éventuellement substitué par 1 à 4 groupes alkyle, dans lequel deux atomes d'hydrogène de la partie cyclopentyle sont remplacés par un pont alkylène linéaire, ce pont contenant 2 à 6 atomes de carbone lorsque les deux atomes d'hydrogène se trouvent sur le même atome de carbone, ou 1 à 5 atomes de carbone lorsque les deux atomes d'hydrogène se trouvent sur des atomes de carbone adjacents, ou 2 à 4 atomes de carbone lorsque les deux atomes d'hydrogène se trouvent sur des atomes de carbone séparés par un atome de carbone, les cycles précités pouvant en outre être substitués par le reste $R_5$ défini de la manière indiquée précédemment,
un groupe cyclohexyle, cyclohexylalkyle, cycloheptyle ou cycloheptylalkyle éventuellement substitué par 1 à 4 groupes alkyle, dans lequel deux atomes d'hydrogène de la partie cycloalkyle sont remplacés par un pont alkylène linéaire, ce pont contenant 2 à 6 atomes de carbone lorsque les deux atomes d'hydrogène se trouvent sur le même atome de carbone, ou 1 à 5 atomes de carbone lorsque les deux atomes d'hydrogène se trouvent sur des atomes de carbone adjacents, ou 1 à 4 atomes de carbone lorsque les deux atomes d'hydrogène se trouvent sur des atomes de carbone séparés par un atome de carbone, ou 1 à 3 atomes de carbone lorsque les deux atomes d'hydrogène se trouvent sur des atomes de carbone séparés par deux atomes de carbone, les cycles précités pouvant en outre être substitués par le reste $R_5$ défini de la manière indiquée précédemment,
un groupe 3-cyclohexène-1-yle ou 3-cyclohexène-1-ylalkyle éventuellement substitué par 1 à 4 groupes alkyle, dans lequel deux atomes d'hydrogène en position 2,5 de la partie cyclohexényle sont remplacés par un pont n-alkylène en $C_1$-$C_3$,
un groupe 3-quinuclidinyle, 4-quinuclidinyle, 2-quinuclidinylalkyle, 3-quinuclidinylalkyle, 4-quinuclidinylalkyle, azabicyclo[2.2.1]hept-4-yle, azabicyclo[2.2.1]hept-4-ylalkyle ou adamantyle, ou
$R_{10}$ est un atome d'hydrogène ou un groupe alkyle et $R_{11}$ représente un groupe hydroxy, alcoxy ou cyano,

leurs tautomères, leurs stéréo isomères et leurs sels,
et où, sauf indication contraire,
les parties aryle évoquées dans la définition des restes précités désignent un groupe phényle qui peut être

monosubstitué par $R_{12}$, mono-, di- ou trisubstitué par $R_{13}$, ou monosubstitué par $R_{12}$ et mono- ou disubstitué en outre par $R_{13}$, les substituants pouvant être identiques ou différents, et
$R_{12}$ représente un groupe cyano, carboxy, aminocarbonyle, alkylaminocarbonyle, dialkylaminocarbonyle, alcoxycarbonyle, alkylcarbonyle, alkylsulfényle, alkylsulfinyle, alkylsulfonyle, alkylsulfonyloxy, perfluoroalkyle, perfluoroalcoxy, nitro, amino, alkylcarbonylamino, N-alkyl-alkylcarbonylamino, alkylamino, dialkylamino, hydroxyalkylamino en $C_2$-$C_4$, N-alkyl-(hydroxyalkyl en $C_2$-$C_4$)amino, bis(hydroxyalkyl en $C_2$-$C_4$) amino, phénylalkylcarbonylamino, phénylcarbonylamino, alkylsulfonylamino, phénylalkylsulfonylamino, phénylsulfonylamino, N-alkyl-phénylalkylcarbonylamino, N-alkyl-phénylcarbonylamino, N-alkyl-alkylsulfonylamino, N-alkyl-phénylalkylsulfonylamino, N-alkyl-phénylsulfonylamino, aminosulfonyle, alkylaminosulfonyle, dialkylaminosulfonyle, $(R_8NR_7)$-CO-$NR_6$ ou $(R_8NR_7)$-$SO_2$-$NR_6$, où $R_6$, $R_7$ et $R_8$ ont la définition donnée précédemment,
un groupe alkylène-imino de 5 à 7 chaînons éventuellement substitué par 1 à 4 groupes alkyle ou un groupe hydroxyalkyle, un groupe méthylène en position 4 des groupes alkylène-imino de 6 à 7 chaînons précités pouvant être remplacé par un atome d'oxygène ou par un groupe $R_9N$, où $R_9$ a la définition donnée précédemment,
un groupe alkylène-imino de 5 à 7 chaînons éventuellement substitué par 1 à 4 groupes alkyle ou un groupe hydroxyalkyle, dans lequel un ou deux groupes méthylène adjacents à l'atome d'azote sont remplacés par un groupe carbonyle, et
$R_{13}$ représente un groupe alkyle, hydroxy ou alcoxy ou un atome de fluor, de chlore, de brome ou d'iode, et deux restes $R_{13}$, dans la mesure où ils sont liés à des atomes de carbone adjacents, peuvent aussi représenter un groupe alkylène de 3 à 6 atomes de carbone, un groupe 1,3-butadiène-1,4-diylène ou un groupe méthylènedioxy,
et, sauf indication contraire, les parties alkyle, alkylène et alcoxy évoquées ci-dessus contiennent chacune 1 à 4 atomes de carbone.

**2.** Pyrimido[5,4-d]pyrimidines de formule générale I selon la revendication 1, sous réserve que, sauf indication contraire, dans les parties alkylène ou cycloalkylène évoquées dans la revendication 1, tout atome de carbone lié à un atome d'azote, d'oxygène ou de soufre, ne puisse être lié à aucun autre atome d'halogène, d'azote, d'oxygène ou de soufre,

leurs tautomères, leurs stéréoisomères et leurs sels.

**3.** Pyrimido[5,4-d]pyrimidines de formule générale I selon la revendication 1 ou 2, dans lesquelles

$R_a$ représente un atome d'hydrogène ou un groupe alkyle,
$R_b$ représente un groupe phényle substitué par les restes $R_1$ à $R_3$, où

$R_1$ représente un atome d'hydrogène, de fluor, de chlore, de brome ou d'iode,
un groupe alkyle en $C_1$-$C_6$, hydroxy ou alcoxy en $C_1$-$C_6$,
un groupe cycloalkyle en $C_3$-$C_6$ ou cycloalcoxy en $C_5$-$C_6$,
un groupe alcényle en $C_2$-$C_5$ ou alcényloxy en $C_3$-$C_5$, la partie vinylique n'étant pas liée à l'atome d'oxygène,
un groupe alcynyle en $C_2$-$C_5$ ou alcynyloxy en $C_3$-$C_5$, la partie éthynyle n'étant pas liée à l'atome d'oxygène,
un groupe aryle, aryloxy, aralkyle, aralcoxy, alkylsulfényle, alkylsulfinyle, alkylsulfonyle, alkylsulfonyloxy, tTifluorométhylsulfényle, trifluorométhylsulfonyle, arylsulfényle, arylsulfinyle, arylsulfonyle, aralkylsulfényle, aralkylsulfinyle ou aralkylsulfonyle,
un groupe méthyle ou méthoxy substitué par 1 à 3 atomes de fluor,
un groupe alkyle en $C_2$-$C_4$ ou alcoxy en $C_2$-$C_4$ substitué par 1 à 5 atomes de fluor, un groupe nitro, amino, alkylamino, dialkylamino, cycloalkylamino en $C_3$-$C_6$, N-alkyl-(cycloalkyl en $C_3$-$C_6$)amino, arylamino, N-alkyl-arylamino, aralkylamino ou N-alkyl-aralkylamino,
un groupe alkylène-imino de 5 à 7 chaînons dans lequel un ou deux des groupes méthylène adjacents à l'atome d'azote peuvent être remplacés par un groupe carbonyle, ou un groupe méthylène en position 4 des groupes alkylène-imino de 6 à 7 chaînons précités peut être remplacé par un atome d'oxygène ou par un groupe imino ou N-alkylimino,
un groupe (alkylène-imino)carbonyle ou (alkylène-imino)sulfonyle ayant 5 à 7 atomes cycliques dans la partie alkylène-imino, un groupe méthylène en position 4 des parties alkylène-imino de 6 à 7 chaînons précitées pouvant être remplacé par un atome d'oxygène ou par un groupe imino ou N-alkylimino,
un groupe alkylcarbonylamino, N-alkyl-alkylcarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, trifluorométhylsulfonylamino, N-alkyl-trifluorométhylsulfonylamino, alkyl-carbonyle, arylcarbonyle, aralkylcarbonyle, carboxy, alcoxycarbonyle, aminocarbonyle, alkylaminocarbonyle, dialkylaminocarbonyle, arylaminocarbonyle, N-alkyl-arylaminocarbonyle, aralkylaminocarbonyle, N-alkyl-aralkylaminocarbonyle, N-hydroxy-aminocarbonyle, N-hydroxyalkylaminocarbonyle, N-alcoxyaminocarbonyle, N-alcoxy-alkylaminocarbonyle, cyano, azido, N-cyanoamino ou N-cyanoalkylamino,
un groupe sulfo, aminosulfonyle, alkylaminosulfonyle, dialkylaminosulfonyle, arylaminosulfonyle, N-alkyl-arylaminosulfonyle, aralkylaminosulfonyle ou N-alkyl-aralkylaminosulfonyle,
un groupe phosphono, O-alkylphosphono, O,O'-dialkylphosphono ou O,O'-diaralkylphosphono,
un groupe alkyle ou alcoxy substitué par $R_4$, où

$R_4$ représente un groupe hydroxy, alcoxy, aryloxy, amino, alkylamino, dialkylamino, alkylsulfényle, alkylsulfinyle, alkylsulfonyle, arylsulfényle, arylsulfinyle, arylsulfonyle, carboxy, alcoxycarbonyle, aminocarbonyle, alkylaminocarbonyle, dialkylaminocarbonyle ou cyano, ou
un groupe (alkylène-imino)carbonyle ayant 5 à 7 atomes cycliques dans la partie alkylène-imino, un groupe méthylène en position 4 des parties alkylène-imino de 6 à 7 chaînons précitées pouvant être remplacé par un atome d'oxygène ou par un groupe imino ou N-alkylimino,

$R_2$ représente un atome d'hydrogène, de fluor, de chlore ou de brome ou un groupe alkyle, trifluorométhyle, hydroxy, alcoxy, amino, alkylamino, dialkylamino, alkylcarbonylamino, N-alkyl-alkylcarbonylamino, alkylsulfonylamino, N-alkyl-alkylsulfonylamino, trifluorométhylsulfonylamino, N-alkyl-trifluorométhylsulfonylamino ou cyano, et
$R_3$ représente un atome d'hydrogène, de fluor, de chlore ou de brome ou un groupe alkyle, alcoxy ou trifluorométhyle, ou
$R_2$ et $R_3$, dans la mesure où ils sont liés à des atomes de carbone adjacents, peuvent aussi représenter ensemble un groupe méthylènedioxy éventuellement substitué par un ou deux groupes alkyle, un groupe n-alkylène en $C_3$-$C_6$ éventuellement substitué par un ou deux groupes alkyle ou un groupe 1,3-butadiène-1,4-diylène éventuellement substitué par un atome de fluor, de chlore ou de brome ou par un groupe hydroxy, alkyle, alcoxy, trifluorométhyle ou cyano, les substituants pouvant être identiques ou différents, ou

$R_a$ et $R_1$, dans la mesure où $R_1$ se trouve en position ortho par rapport à l'atome d'azote substitué par $R_a$, représentent aussi ensemble un groupe n-alkylène en $C_1$-$C_3$, et
$R_c$ représente un atome d'hydrogène ou de chlore,
un groupe alkyle, aryle, aralkyle, mercapto, alkylsulfényle, alkylsulfinyle ou alkylsulfonyle,
un groupe hydroxy, aryloxy ou aralcoxy,
un groupe alcoxy en $C_1$-$C_6$ pouvant être substitué par un groupe alcoxycarbonyle, cyano, carboxy, aminocarbonyle, alkylaminocarbonyle ou dialkylaminocarbonyle,
un groupe alcoxy en $C_2$-$C_6$ substitué par un groupe hydroxy, alcoxy, hydroxyalkylamino en $C_2$-$C_4$, amino, alkylamino, dialkylamino, alkylcarbonylamino, alkylsulfonylamino ou alcoxycarbonylamino, par un groupe alkylène-imino de 5 à 7 chaînons éventuellement substitué par 1 à 2 groupes alkyle, un ou deux groupes méthylène adjacents à l'atome d'azote des groupes alkylène-imino de 5 à 7 chaînons précités pouvant être remplacés par un groupe carbonyle, ou un groupe méthylène en position 4 des groupes alkylène-imino de 6 à 7 chaînons précités pouvant être remplacé par un atome d'oxygène ou par un groupe carbonyle, imino, alkylimino, alkylcarbonylimino, alcoxycarbonylimino, alkylsulfonylimino, formylimino, dialkylaminocarbonylimino, arylimino ou aralkylimino,
un groupe alcoxy en $C_3$-$C_6$ substitué par deux groupes hydroxy ou alcoxy,
un groupe alcoxy substitué par un groupe cycloalkyle en $C_3$-$C_7$, la partie cycloalkyle pouvant être substituée par 1 ou 2 groupes alkyle et un groupe méthylène des parties cycloalkyle en $C_4$-$C_7$ précitées pouvant être remplacé par un atome d'oxygène ou par un groupe imino, N-alkylimino, N-alkylcarbonylimino, N-alcoxycarbonyl-imino ou N-arylimino,
un groupe cycloalcoxy en $C_4$-$C_7$ éventuellement substitué par un groupe hydroxy, alcoxy, alcoxycarbonyle, cyano, carboxy, aminocarbonyle, alkylaminocarbonyle, dialkylaminocarbonyle, hydroxyalkylamino en $C_2$-$C_4$, amino, alkylamino, dialkylamino, alkylcarbonylamino, alkylsulfonylamino ou alcoxycarbonylamino,
un groupe cycloalcoxy en $C_5$-$C_7$, un groupe méthylène en position 3 du noyau cyclopentyloxy précité, ou un groupe méthylène en position 3 ou 4 des parties cycloalcoxy en $C_6$-$C_7$ précitées pouvant être remplacé par un atome d'oxygène ou par un groupe imino, alkylimino, alkylcarbonylimino, alcoxycarbonyl-imino, alkylsulfonylimino, arylimino ou aralkylimino,

un groupe alcényloxy en $C_3$-$C_6$, la partie vinylique ne pouvant pas être liée à l'atome d'oxygène,
un groupe alcynyloxy en $C_3$-$C_6$, la partie éthynyle ne pouvant pas être liée à l'atome d'oxygène,
un groupe alkylène-imino de 4 à 8 chaînons éventuellement substitué par 1 à 4 groupes alkyle ou 1 groupe aryle, et qui peut en outre être substitué par le reste $R_5$, où

$R_5$ représente un groupe aryle, aralkyle, carboxy, alcoxycarbonyle, aminocarbonyle, alkylaminocarbonyle, dialkylaminocarbonyle, pyrrolidinocarbonyle, pipéridinocarbonyle, morpholinocarbonyle, pipérazinocarbonyle, 4-alkylpipérazinocarbonyle, cyano, hydroxy, alcoxy, aryloxy, amino, alkylamino, dialkylamino, hydroxyalkylamino en $C_2$-$C_4$, N-alkyl-(hydroxyalkyl en $C_2$-$C_4$)amino, di(hydroxyalkyl en $C_2$-$C_4$)amino, formylamino, cyanoamino, alkyl-carbonylamino, N-alkyl-alkylcarbonylamino, alcoxycarbonylamino, N-alkyl-alcoxycarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, carboxyalkyle, alcoxycarbonylalkyle, aminocarbonylalkyle, alkylaminocarbonylalkyle, dialkylaminocarbonylalkyle, pyrrolidinocarbonylalkyle, pipéridinocarbonylalkyle, morpholinocarbonylalkyle, pipérazinocarbonylalkyle, 4-alkylpipérazinocarbonylalkyle, cyanoalkyle, hydroxyalkyle, alcoxyalkyle, aryloxyalkyle, aminoalkyle, alkylaminoalkyle, dialkylaminoalkyle, alkylcarbonylaminoalkyle, N-alkyl-alkylcarbonylaminoalkyle, alkylsulfonylaminoalkyle, N-alkyl-alkylsulfonylaminoalkyle, arylcarbonylarnbJoalkyle, N-alkyl-arylcarbonylaninoalkyle, arylsulfonylaninoalkyle, N-alkyl-arylsulfonylaminoalkyle, alkylsulfényle, alkylsulfinyle, alkylsulfonyle, arylsulfényle, arylsulfinyle, arylsulfonyle, alkylsulfénylalkyle, alkylsulfinylalkyle, alkylsulfonylalkyle, arylsulfénylalkyle, arylsulfinylalkyle, arylsulfonylalkyle, carboxyalcoxy, alcoxycarbonylalcoxy, aminocarbonylalcoxy, alkylaminocarbonylalcoxy, dialkylaminocarbonylalcoxy, pyrrolidinocarbonylalcoxy, pipéridinocarbonylalcoxy, morpholinocarbonylalcoxy, ou un groupe $(NR_7R_8)CONR_6$ dans lequel

$R_6$, $R_7$ et $R_8$, qui peuvent être identiques ou différents, représentent chacun un atome d'hydrogène ou un groupe alkyle, ou $R_6$ et $R_7$ forment ensemble un groupe n-alkylène en $C_2$-$C_3$ et $R_8$ représente un atome d'hydrogène ou un groupe alkyle;

un groupe pyrrolidino, pipéridino, morpholino, pipérazino, 4-alkylpipérazino ou 4-alkylcarbonylpipérazino éventuellement substitué par un ou deux groupes alkyle, un ou deux groupes méthylène adjacents à l'atome d'azote dans la partie hétérocyclique des groupes précités pouvant être remplacés par un groupe carbonyle,

ou $R_c$ représente un groupe alkylène-imino de 6 à 8 chaînons éventuellement substitué par 1 à 4 groupes alkyle ou par un groupe aryle, et qui peut être en outre substitué par le reste $R_5$, un groupe méthylène en position 4 des groupes alkylène-imino précités étant remplacé par un atome d'oxygène ou de soufre ou par un groupe carbonyle, sulfinyle, sulfonyle, N-oxydo-N-alkylimino ou $R_9N$, où

$R_9$ représente un atome d'hydrogène, un groupe alkyle, hydroxyalkyle en $C_2$-$C_4$, alcoxy(alkyle en $C_2$-$C_4$), (hydroxyalkoxy en $C_2$-$C_4$)alkyle en $C_2$-$C_4$, aminocarbonylalkyle, alkylaminocarbonylalkyle, dialkylaminocarbonylalkyle, aminoalkyle en $C_2$-$C_4$, alkylamino(alkyle en $C_2$-$C_4$), dialkylamino(alkyle en $C_2$-$C_4$), aryle, aralkyle, formyle, alkylcarbonyle, alkylsulfonyle, arylcarbonyle, arylsulfonyle, aralkylcarbonyle, aralkylsulfonyle, alcoxycarbonyle, cyano, aminocarbonyle, alkylaminocarbonyle ou dialkylaminocarbonyle,

ou $R_c$ représente un groupe 1-pyrrolidinyle éventuellement substitué par 1 à 2 groupes alkyle, et dans lequel deux atomes d'hydrogène liés au squelette carboné sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome,
un groupe 1-pipéridinyle ou 1-azacyclohept-1-yle éventuellement substitué par 1 à 2 groupes alkyle, et dans lequel deux atomes d'hydrogène liés au squelette carboné sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome, ou 1 ou 2 atomes de carbone lorsque les deux atomes d'hydrogène se trouvent sur des atomes de carbone séparés par deux atomes,
un groupe 1-pyrrolidinyle dans lequel les deux atomes d'hydrogène en position 3 sont remplacés par un groupe $-O-CH_2CH_2-O-$ ou $-O-CH_2CH_2CH_2-O-$,

un groupe 1-pipéridinyle ou 1-azacyclohept-1-yle dans lequel les deux atomes d'hydrogène en position 3 ou en position 4 sont remplacés par un groupe $-O-CH_2CH_2-O-$ ou $-O-CH_2CH_2CH_2-O-$,
un groupe 2-isoindolinyle, 1,2,3,4-tétrahydroisoquinoléine-2-yle ou 2,3,4,5-tétrahydro-1H-3-benzazépine-3-yle, la partie benzo des groupes précités pouvant être substituée par un atome de fluor, de chlore, de brome ou d'iode, par un groupe trifluorométhyle ou par un ou deux groupes alkyle ou alcoxy, ou
un groupe ($R_{10}NR_{11}$), dans lequel

$R_{10}$ représente un atome d'hydrogène ou un groupe alkyle en $C_1$-$C_8$ pouvant être substitué en position 2 par un groupe hydroxy ou alcoxy,
$R_{11}$ représente un atome d'hydrogène,
un groupe alkyle en $C_1$-$C_{10}$ pouvant être substitué

par un groupe aryle, cycloalkyle en $C_3$-$C_7$, hydroxy, alcoxy, aryloxy, carboxy, alcoxycarbonyle, aminocarbonyle, alkylaminocarbonyle, dialkylaminocarbonyle, hydroxy(alkyl en $C_1$-$C_4$)aminocarbonyle, cyano, formylamino, amino, alkylamino ou dialkylamino, par un groupe pyrrolidinocarbonyle, pipéridinocarbonyle, morpholinocarbonyle, pipérazinocarbonyle ou 4-alkylpipérazinocarbonyle,
par un groupe alkylène-imino de 5 à 7 chaînons éventuellement substitué par 1 à 2 groupes alkyle, un groupe méthylène en position 4 des groupes alkylène-imino de 6 ou 7 chaînons précités pouvant être remplacé par un atome d'oxygène ou de soufre ou par un groupe sulfinyle, sulfonyle ou $R_9N$, $R_9$ ayant la définition donnée précédemment,
par un groupe pyrrolidino, pipéridino, pipérazino ou 4-alkylpipérazino, dans lequel un ou deux des groupes méthylène adjacents à l'atome d'azote sont remplacés par un groupe carbonyle,
par un groupe alkylcarbonylamino, N-alkyl-alkylcarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino ou N-alkyl-alcoxycarbonylamino, par un groupe ($R_8NR_7$)-CO-$NR_6$ où les $R_6$ à $R_8$ ont la définition donnée précédemment, par un groupe alkylsulfényle, alkylsulfinyle, alkylsulfonyle, arylsulfényle, arylsulfinyle, arylsulfonyle, aralkylsulfényle, aralkylsulfinyle ou aralkylsulfonyle ou par un groupe cycloalkyle en $C_5$-$C_7$ dans lequel un groupe méthylène est remplacé par un atome d'oxygène ou par un groupe imino ou alkylimino,

un groupe alkyle en $C_2$-$C_4$ substitué par un atome de chlore ou un ou trois atomes de fluor,
un groupe méthyle substitué par un groupe 1,4,7,10-tétraoxacyclododécyle, 1,4,7,10,13-pentaoxacyclopentadécyle ou 1,4,7,10,13,16-hexaoxacyclooctadécyle, un groupe alkyle en $C_3$-$C_{10}$ substitué par 2 à 5 groupes hydroxy,
un groupe alkyle en $C_2$-$C_6$ substitué un groupe hydroxy et en outre par un groupe aryle, et qui peut être substitué en plus par un groupe hydroxy ou alcoxy,
un groupe alkyle en $C_3$-$C_6$ substitué par un groupe hydroxy et en outre par un groupe amino, alkylamino ou dialkylamino,
un groupe alcényle ou alcynyle de 3 à 6 atomes de carbone éventuellement substitué par un groupe aryle, la partie vinyle ou éthynyle ne pouvant pas être liée à l'atome d'azote,
un groupe alkyle en $C_2$-$C_4$ substitué par un groupe alcoxy en $C_2$-$C_4$ qui est substitué en position $\omega$ par un groupe hydroxy ou alcoxy,
un groupe aryle,
un groupe cyclopropyle pouvant être substitué par 1 ou 2 groupes alkyle, par un groupe aryle, carboxy, alcoxycarbonyle, aminocarbonyle, alkylaminocarbonyle, dialkylaminocarbonyle, pyrrolidinocarbonyle, pipéridinocarbonyle, morpholinocarbonyle, pipérazinocarbonyle ou 4-alkylpipérazinocarbonyle,
un groupe cycloalkyle en $C_4$-$C_7$ éventuellement substitué par 1 ou 2 groupes alkyle, et qui peut être substitué en outre par $R_5$ tel que défini précédemment,
un groupe cycloalkyle en $C_5$-$C_7$ éventuellement substitué par 1 ou 2 groupes alkyle, et qui est substitué en outre par un groupe N,N-dialkyl-N-oxydoamino,
un groupe cycloalcényle en $C_5$-$C_7$ éventuellement substitué par 1 ou 2 groupes alkyle, la partie vinylique ne pouvant pas être liée à l'atome d'azote du groupe ($R_{11}NR_{10}$),
un groupe cycloalkyle en $C_5$-$C_7$ éventuellement substitué par 1 ou 2 groupes alkyle, un groupe méthylène de la partie cycloalkyle étant remplacé par un atome d'oxygène ou de soufre ou par un groupe sulfinyle, sulfonyle, N-alkyl-N-oxydoimino ou $R_9N$, où $R_9$ a la définition indiquée précédemment,
un groupe cycloalkyle en $C_5$-$C_7$ éventuellement substitué par 1 à 4 groupes alkyle, dans lequel un groupe méthylène est remplacé par un groupe carbonyle,

un groupe (cycloalkyl en $C_4$-$C_7$)méthyle éventuellement substitué par 1 ou 2 groupes alkyle, qui est en outre substitué dans la partie cycloalkyle par $R_5$, $R_5$ ayant la définition indiquée précédemment,
un groupe cyclopentyle ou cyclopentylalkyle éventuellement substitué par 1 à 4 groupes alkyle, dans lequel deux atomes d'hydrogène de la partie cyclopentyle sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome de carbone,
un groupe cyclohexyle, cyclohexylalkyle, cycloheptyle ou cycloheptylalkyle éventuellement substitué par 1 à 4 groupes alkyle, dans lequel deux atomes d'hydrogène de la partie cycloalkyle sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome de carbone, ou 1 ou 2 atomes de carbone lorsque les deux atomes d'hydrogène se trouvent sur des atomes de carbone séparés par deux atomes de carbone,
un groupe 5-norbornène-2-yle ou 5-norbornène-2-ylalkyle éventuellement substitué par 1 à 4 groupes alkyle,
un groupe 3-quinuclidinyle, 4-quinuclidinyle, 3-quinuclidinylalkyle, 4-quinuclidinylalkyle, azabicyclo[2.2.1]hept-4-yle, azabicyclo[2.2.1]hept-4-ylalkyle ou adamantyle, ou
$R_{10}$ est un atome d'hydrogène ou un groupe alkyle et $R_{11}$ représente un groupe hydroxy ou alcoxy,

leurs tautomères, leurs stéréoisomères et leurs sels,
et où, sauf indication contraire,
les parties aryle évoquées dans la définition des restes précités désignent un groupe phényle qui peut être monosubstitué par $R_{12}$, mono-, di- ou trisubstitué par $R_{13}$, ou monosubstitué par $R_{12}$ et mono- ou disubstitué en outre par $R_{13}$, les substituants pouvant être identiques ou différents, et
$R_{12}$ représente un groupe cyano, carboxy, aninocarbonyle, alkylaninocarbonyle, dialkylaninocarbonyle, alcoxycarbonyle, alkylcarbonyle, alkylsulfényle, alkylsulfinyle, alkylsulfonyle, alkylsulfonyloxy, perfluoroalkyle, perfluoroalcoxy, nitro, amino, alkylamino, hydroxyalkylamino en $C_2$-$C_4$, N-alkyl-(hydroxyalkyl en $C_2$-$C_4$)amino, di(hydroxyalkyl en $C_2$-$C_4$)amino, dialkylamino, alkylcarbonylamino, phénylalkylcarbonylamino, phénylcarbonylamino, alkylsulfonylamino, phénylalkylsulfonylamino, phénylsulfonylamino, N-alkyl-alkylcarbonylamino, N-alkylphénylalkylcarbonylamino, N-alkyl-phénylcarbonylamino, N-alkyl-alkylsulfonylamino, N-alkyl-phénylalkylsulfonylamino, N-alkyl-phénylsulfonylanino, aminosulfonyle, alkylaminosulfonyle ou dialkylaminosulfonyle,
un groupe alkylène-imino de 5 à 7 chaînons éventuellement substitué par 1 ou 2 groupes alkyle ou un groupe hydroxyalkyle, un groupe méthylène en position 4 des groupes alkylène-imino de 6 à 7 chaînons précités pouvant être remplacé par un atome d'oxygène ou par un groupe $R_9N$,
un groupe alkylène-imino de 5 à 7 chaînons éventuellement substitué par 1 à 2 groupes alkyle ou un groupe hydroxyalkyle, dans lequel un ou deux groupes méthylène adjacents à l'atome d'azote sont remplacés par un groupe carbonyle,
un groupe $(R_8NR_7)$-CO-$NR_6$, où les $R_6$ à $R_8$ ont la définition indiquée précédemment, et
$R_{13}$ représente un groupe alkyle, hydroxy ou alcoxy ou un atome de fluor, de chlore, de brome ou d'iode, et deux restes $R_{13}$, dans la mesure où ils sont liés à des atomes de carbone adjacents, peuvent aussi représenter un groupe alkylène de 3 à 6 atomes de carbone, un groupe 1,3-butadiène-1,4-diylène ou un groupe méthylènedioxy,

et, sauf indication contraire, les parties alkyle, alkylène et alcoxy évoquées ci-dessus contiennent chacune 1 à 4 atomes de carbone et, sauf indication contraire, tout atome de carbone lié à un atome d'azote, un atome d'oxygène ou un atome de soufre dans les parties alkylène ou cycloalkylène évoquées précédemment ne peut être lié à aucun autre atome d'halogène, d'azote, d'oxygène ou de soufre.

**4.** Pyrimido[5,4-d]pyrimidines de formule générale I selon la revendication 1 ou 2, dans lesquelles

$R_a$ représente un atome d'hydrogène ou un groupe alkyle,
$R_b$ représente un groupe phényle substitué par les restes $R_1$ à $R_3$, où

$R_1$ représente un atome d'hydrogène, de fluor, de chlore, de brome ou d'iode,
un groupe alkyle, hydroxy, alcoxy, cycloalkyle en $C_3$-$C_6$ ou cycloalcoxy en $C_5$-$C_6$,

un groupe éthoxy substitué en position 2 par un groupe hydroxy, alcoxy ou phénoxy,
un groupe alcényle en $C_2$-$C_5$ ou alcényloxy en $C_3$-$C_5$, la partie vinylique n'étant pas liée à l'atome d'oxygène,
un groupe alcynyle en $C_2$-$C_5$ ou alcynyloxy en $C_3$-$C_5$, la partie éthynyle n'étant pas liée à l'atome d'oxygène,
un groupe phényle, phényloxy, phénylalkyle, phénylalcoxy, alcoxyalkyle, phénoxyalkyle, carboxyalkyle, alcoxycarbonylalkyle, aminocarbonylalkyle, alkylaminocarbonylalkyle, dialkylaminocarbonylalkyle, cyanoalkyle, alkylsulfényle, alkylsulfinyle, alkylsulfonyle, alkylsulfonyloxy, trifluorométhylsulfényle, trifluorométhylsulfonyle, nitro, amino, alkylamino, dialkylamino, pyrrolidino, pipéridino, morpholino, alkylcarbonylamino, N-alkyl-alkylcarbonylamino, alkylsulfonylamino, N-alkyl-alkylsulfonylamino, trifluorométhylsulfonylamino, N-alkyl-trifluorométhylsulfonylamino, alkylcarbonyle, carboxy, alcoxycarbonyle, aminocarbonyle, alkylaminocarbonyle, dialkylaminocarbonyle, pyrrolidinocarbonyle, pipéridinocarbonyle, morpholinocarbonyle ou cyano,
un groupe méthyle ou méthoxy substitué par 1 à 3 atomes de fluor,
un groupe éthyle ou éthoxy substitué par 1 à 5 atomes de fluor,
$R_2$ représente un atome d'hydrogène, de fluor, de chlore ou de brome ou un groupe alkyle, trifluorométhyle, amino, alkylamino, dialkylamino, alkylcarbonylamino, alkylsulfonylamino, trifluorométhylsulfonylamino, hydroxy ou alcoxy,
$R_3$ représente un atome d'hydrogène, de fluor, de chlore ou de brome ou un groupe alkyle, ou
$R_2$ et $R_3$, dans la mesure où ils sont liés à des atomes de carbone adjacents, représentent aussi ensemble un groupe méthylènedioxy ou n-alkylène en $C_3$-$C_6$ ou un groupe 1,3-butadiène-1,4-diylène éventuellement substitué par un atome de fluor, de chlore ou de brome ou par un groupe alkyle, alcoxy ou trifluorométhyle, et

$R_c$ représente un atome d'hydrogène ou de chlore,
un groupe alkyle, phényle, mercapto, alkylsulfényle, alkylsulfinyle ou alkylsulfonyle,
un groupe hydroxy, phénoxy ou phényl(alcoxy en $C_1$-$C_2$),
un groupe alcoxy,
un groupe alcoxy en $C_2$-$C_4$ substitué par un groupe hydroxy, alcoxy, (2-hydroxyéthyl)amino, dialkylamino, morpholino, 1-pyrrolidinyle, 1-pipéridinyle, 4-méthyl-1-pipérazinyle, 4-acétyl-1-pipérazinyle, 4-méthylsulfonyl-l-pipérazinyle, 4-méthoxycarbonyl-1-pipérazinyle, 4-formyl-1-pipérazinyle ou 4-diméthylaminocarbonyl-1-pipérazinyle,
un groupe alcoxy en $C_3$-$C_4$ substitué par deux groupes hydroxy,
un groupe alcoxy en $C_1$-$C_2$ substitué par un groupe cycloalkyle en $C_3$-$C_7$ éventuellement substitué par un ou deux groupes méthyle, un groupe méthylène des groupes cycloalkyle en $C_4$-$C_6$ précités pouvant être remplacé par un atome d'oxygène,
un groupe cycloalcoxy en $C_4$-$C_6$ éventuellement substitué par un groupe hydroxy, dialkylamino, alcoxy, carboxy, alcoxycarbonyle, aminocarbonyle, alkylaminocarbonyle, dialkylaminocarbonyle, alkylcarbonylamino, alkylsulfonylamino ou alcoxycarbonylamino,
un groupe cyclopentyloxy dans lequel le groupe méthylène en position 3 est remplacé par un atome d'oxygène ou par un groupe alkylimino,
un groupe cyclohexyloxy dans lequel le groupe méthylène en position 3 ou 4 est remplacé par un atome d'oxygène ou par un groupe alkylimino, alkylcarbonylimino, alcoxycarbonylimino ou alkylsulfonylimino,
un groupe allyloxy ou propargyloxy éventuellement substitué par un ou deux groupes méthyle,
un groupe 1-azétidinyle,
un groupe 1-pyrrolidinyle pouvant être substitué par 1 à 2 groupes alkyle, par un groupe phényle, carboxy, hydroxyalkyle, aminoalkyle, alkylaminoalkyle, dialkylaminoalkyle, alcoxycarbonyle, aminocarbonyle, alkylaminocarbonyle, dialkylaminocarbonyle, pyrrolidinocarbonyle, pipéridinocarbonyle, morpholinocarbonyle, pipérazinocarbonyle ou 4-alkylpipérazinocarbonyle, ou encore en position 3 par un groupe hydroxy, alcoxy, amino, alkylamino, dialkylamino, alkylcarbonylamino, alcoxycarbonylamino, formylamino, cyanoamino, alkylsulfonylamino, dialkylaminocarbonylamino, N-alkyl-dialkylaminocarbonylamino, N-alkyl-dialkylaminocarbonylamino ou cyano,
un groupe 1-pyrrolidinyle dans lequel deux atomes d'hydrogène liés au squelette carboné sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome,
un groupe 1-pipéridinyle pouvant être substitué par 1 à 4 groupes alkyle, par un groupe phényle, hydroxyalkyle,

aminoalkyle, alkylaminoalkyle, dialkylaminoalkyle, carboxy, alcoxycarbonyle, aminocarbonyle, alkylaminocarbonyle, dialkylaminocarbonyle, pyrrolidinocarbonyle, pipéridinocarbonyle, morpholinocarbonyle, pipérazinocarbonyle ou 4-alkylpipérazinocarbonyle, ou encore en position 3 ou 4 par un groupe hydroxy, alcoxy, amino, alkylamino, dialkylamino, alkylcarbonylamino, alcoxycarbonylamino, formylamino, cyanoamino, alkylsulfonylamino, dialkylaminocarbonylamino, N-alkyl-dialkylaminocarbonylamino ou cyano,
un groupe 1-pipéridinyle substitué par 1 à 2 groupes alkyle ou par un groupe phényle, et en outre par un groupe hydroxy,
un groupe 1-pipéridinyle dans lequel les deux atomes d'hydrogène en position 3 ou en position 4 sont remplacés par un groupe $-O-CH_2CH_2-O-$ ou $-O-CH_2CH_2CH_2O-$,
un groupe 1-pipéridinyle dans lequel deux atomes d'hydrogène liés au squelette carboné sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome, ou 1 ou 2 atomes de carbone lorsque les deux atomes d'hydrogène se trouvent sur des atomes de carbone séparés par deux atomes,
un groupe 1-pipéridinyle éventuellement substitué par 1 ou 2 groupes alkyle, dans lequel le groupe méthylène en position 4 est remplacé par un atome d'oxygène ou de soufre ou par un groupe carbonyle, sulfinyle, sulfonyle, imino, alkylimino, hydroxyalkylimino en $C_2$-$C_4$, alcoxy(alkyl en $C_2$-$C_4$)imino, aminocarbonylalkylimino, alkylaminocarbonylalkylimino, dialkylaminocarbonylalkylimino, aminoalkylimino en $C_2$-$C_4$, alkylamino(alkyl en $C_2$-$C_4$)imino, dialkylamino(alkyl en $C_2$-$C_4$)imino, hydroxy(alcoxy en $C_2$-$C_4$)(alkyl en $C_2$-$C_4$)imino, phénylimino, phénylalkylimino, alkylcarbonylimino, alkylsufonylimino, phénylcarbonylimino, phénylsulfonylimino ou N-oxydo-N-alkylimino,
un groupe 1-azacyclohept-1-yle éventuellement substitué par 1 ou 2 groupes alkyle, dans lequel le groupe méthylène en position 4 peut être remplacé par un atome d'oxygène ou par un groupe imino, N-alkylimino, N-phénylimino, N-phénylalkylimino, N-alkylcarbonylimino, N-alkylsufonylimino, N-phénylcarbonylimino ou N-phénylsulfonylimino, ou deux atomes d'hydrogène en position 3,6 peuvent être remplacés par un groupe $-CH_2CH_2-$,
un groupe 2-isoindolinyle, 1,2,3,4-tétrahydroisoquinoléine-2-yle ou 2,3,4,5-tétrahydro-1H-3-benzazépine-3-yle, pouvant être substitués dans la partie benzo par un atome de fluor, de chlore ou de brome ou par un groupe alkyle, trifluorométhyle ou alcoxy, ou
un groupe ($R_{10}NR_{11}$), dans lequel

$R_{10}$ représente un atome d'hydrogène ou un groupe alkyle en $C_1$-$C_6$ pouvant être substitué en position 2 par un groupe hydroxy ou alcoxy,
$R_{11}$ représente un atome d'hydrogène,
un groupe alkyle en $C_1$-$C_8$ pouvant être substitué par un groupe phényle, cycloalkyle en $C_3$-$C_6$, hydroxy, alcoxy, cyano, carboxy, alcoxycarbonyle, aminocarbonyle, alkylaminocarbonyle, dialkylaminocarbonyle, 2-hydroxyéthylaminocarbonyle, pyrrolidinocarbonyle, pipéridinocarbonyle, morpholinocarbonyle, 1-pipérazinylcarbonyle, 4-alkyl-1-pipérazinylcarbonyle, amino, formylamino, alkylamino, dialkylamino, alkylcarbonylamino, N-alkyl-alkylcarbonylamino, alcoxycarbonylamino, N-alkyl-alcoxycarbonylamino, alkylsulfonylamino, N-alkyl-alkylsulfonylamino, phénylcarbonylamino, N-alkyl-phénylcarbonylamino, phénylsulfonylamino, N-alkyl-phénylsulfonylamino, alkylsulfényle, alkylsulfinyle, alkylsulfonyle, 1-pyrrolidinyle, 2-oxo-1-pyrrolidinyle, 1-pipéridinyle, 2-oxo-1-pipéridinyle, morpholino, 1-pipérazinyle, 4-alkyl-1-pipérazinyle, 4-alkylcarbonyl-1-pipérazinyle, 4-alkylsulfonyl-1-pipérazinyle, 4-alcoxycarbonyl-1-pipérazinyle, 4-cyano-1-pipérazinyle, 4-formyl-1-pipérazinyle, 4-aminocarbonyl-1-pipérazinyle, 4-alkylaminocarbonyl-1-pipérazinyle ou 4-dialkylaminocarbonyl-1-pipérazinyle, ou par un groupe ($R_8NR_7$)-CO-$NR_6$ dans lequel

$R_6$ et $R_7$ forment ensemble un pont n-alkylène en $C_2$-$C_3$ et
$R_8$ représente un atome d'hydrogène ou un groupe alkyle,

un groupe méthyle substitué par un groupe 1,4,7,10-tétraoxacyclododécyle, 1,4,7,10,13-pentaoxacyclopentadécyle ou 1,4,7,10,13,16-hexaoxacyclooctadécyle,
un groupe 2,2,2-trifluoroéthyle,
un groupe alkyle en $C_3$-$C_{10}$ substitué par 2 à 5 groupes hydroxy,
un groupe alkyle en $C_3$-$C_5$ substitué par un groupe hydroxy et en outre par un groupe amino,
un groupe alkyle en $C_2$-$C_4$ substitué par un groupe phényle et en outre par un groupe hydroxy, et qui peut éventuellement être substitué en plus par un groupe hydroxy ou alcoxy,
un groupe alcényle ou alcynyle de 3 à 6 atomes de carbone éventuellement substitué par un groupe

phényle, la partie vinyle ou éthynyle ne pouvant pas être liée à l'atome d'azote,

un groupe alkyle en $C_2$-$C_4$ substitué par un groupe alcoxy en $C_2$-$C_4$ qui est substitué en position $\omega$ par un groupe hydroxy ou alcoxy,

un groupe phényle,

un groupe phényle substitué par un groupe alkylcarbonylamino, N-alkyl-alkylcarbonylamino, (2-hydroxyéthyl)amino, di(2-hydroxyéthyl)amino, N-alkyl(2-hydroxyéthyl)amino, amino, alkylamino ou dialkylamino, ou par un groupe ($R_8NR_7$)-CO-$NR_6$ dans lequel les $R_6$ à $R_8$ ont la définition indiquée précédemment,

un groupe phényle substitué par un groupe pyrrolidino, pipéridino, 2-oxopyrrolidino, 2-oxopipéridino, morpholino, 1-pipérazinyle ou 4-alkyl-1-pipérazinyle, les parties hétérocycliques précitées pouvant être substituées sur le squelette carboné par 1 ou 2 groupes alkyle ou par un groupe hydroxyalkyle,

un groupe cycloalkyle en $C_3$-$C_7$ pouvant être substitué par 1 ou 2 groupes alkyle, par un groupe phényle, carboxy, alcoxycarbonyle, aminocarbonyle, alkylaminocarbonyle, dialkylaminocarbonyle, pyrrolidinocarbonyle, pipéridinocarbonyle, morpholinocarbonyle, pipérazinocarbonyle ou 4-alkylpipérazinocarbonyle,

un groupe cycloalkyle en $C_5$-$C_7$ éventuellement substitué par 1 ou 2 groupes méthyle, et qui est substitué par un groupe hydroxyméthyle, cyano, hydroxy, alcoxy, amino, alkylamino, dialkylamino, 2-hydroxyéthylamino, di(2-hydroxyéthyl)amino, N-alkyl-2-hydroxyéthylamino, N,N-dialkyl-N-oxydoamino, alcoxycarbonylamino, N-alkyl-alcoxycarbonylamino, alkylcarbonylamino, N-alkyl-alkylcarbonylamino, alkylsulfonylamino, N-alkyl-alkylsulfonylamino, phénylcarbonylamino, N-alkyl-phénylcarbonylamino, phénylsulfonylamino, N-alkyl-phénylsulfonylamino ou par un groupe ($R_8NR_7$)-CO-$NR_6$ dans lequel les $R_6$ à $R_8$ ont la définition indiquée précédemment,

un groupe cycloalkyle en $C_5$-$C_7$ éventuellement substitué par 1 ou 2 groupes méthyle, et qui est substitué par un groupe pyrrolidino, pipéridino, 2-oxopyrrolidino, 2-oxopipéridino, morpholino, 1-pipérazinyle ou 4-alkyl-1-pipérazinyle ou 4-alkylcarbonyl-1-pipérazinyle, les parties hétérocycliques précitées pouvant être substituées sur le squelette carboné par 1 ou 2 groupes alkyle ou par un groupe hydroxyméthyle,

un groupe cycloalcényle en $C_5$-$C_7$ éventuellement substitué par 1 ou 2 groupes alkyle, la partie vinylique ne pouvant pas être liée à l'atome d'azote du groupe ($R_{11}NR_{10}$),

un groupe tétrahydrofurfuryle,

un groupe cyclopentyle dans lequel le groupe méthylène en position 3 est remplacé par un atome d'oxygène ou par un groupe imino, alkylimino, alkylcarbonylimino, formylimino, aminocarbonylimino, alkylaminocarbonylimino, alcoxycarbonylimino, alkylsulfonylimino, dialkylaminocarbonylimino, ou cyanoimino,

un groupe cyclohexyle dans lequel le groupe méthylène en position 3 est remplacé par un groupe imino, alkylimino, alkylcarbonylimino, alcoxycarbonylimino, ou alkylsulfonylimino,

un groupe cyclohexyle dans lequel le groupe méthylène en position 4 est remplacé par un atome d'oxygène ou par un groupe imino, N-alkylimino, N-phénylimino, N-phénylalkylimino, N-formylimino, N-alkylcarbonylimino, N-phénylcarbonylimino, N-alcoxycarbonylimino, N-cyanoimino, N-aminocarbonylimino, N-alkylaminocarbonylimino, N,N-dialkylaminocarbonylimino, N-alkyl-N-oxydoimino, N-alkylsulfonylimino, ou N-phénylsulfonylimino,

un groupe cyclohexyle dans lequel un groupe méthylène est remplacé par un groupe carbonyle,

un groupe cyclopentyle ou cyclohexyle éventuellement substitué par 1 ou 2 groupes méthyle, qui est substitué par un groupe carboxyalcoxy, alcoxycarbonyloxy, alcoxycarbonylalcoxy, aminocarbonylalcoxy, alkylaminocarbonylalcoxy, dialkylaminocarbonylalcoxy, pyrrolidinocarbonylalcoxy, pipéridinocarbonylalcoxy, morpholinocarbonylalcoxy, carboxyalkyle, alcoxycarbonylalkyle, aminocarbonylalkyle, alkylaminocarbonylalkyle, dialkylaminocarbonylalkyle, pyrrolidinocarbonylalkyle, pipéridinocarbonylalkyle ou morpholinocarbonylalkyle,

un groupe cyclohexylméthyle, dans lequel la partie cyclohexyle est substituée par un groupe carboxy, aminocarbonyle, alkylaminocarbonyle, dialkylaminocarbonyle, pyrrolidinocarbonyle, pipéridinocarbonyle, morpholinocarbonyle, alcoxycarbonyle ou hydroxyméthyle,

un groupe cyclohexyle ou cyclohexylméthyle éventuellement substitué par 1 à 3 groupes méthyle, dans lequel deux atomes d'hydrogène de la partie cyclohexyle sont remplacés par un pont alkylène linéaire, ce pont contenant 4 ou 5 atomes de carbone lorsque les deux atomes d'hydrogène se trouvent sur le même atome de carbone, ou 3 ou 4 atomes de carbone lorsque les deux atomes d'hydrogène se trouvent sur des atomes de carbone adjacents, ou 2 ou 3 atomes de carbone lorsque les deux atomes d'hydrogène se trouvent sur des atomes de carbone séparés par un atome de carbone, ou 1 ou 2 atomes de carbone lorsque les deux atomes d'hydrogène se trouvent sur des atomes de carbone séparés par deux atomes de carbone,

un groupe 5-norbornène-2-yle ou 5-norbornène-2-ylméthyle éventuellement substitué par 1 à 3 groupes méthyle,

un groupe 3-quinuclidinyle, 4-quinuclidinyle ou adamantyle, ou

$R_{10}$ est un atome d'hydrogène ou un groupe alkyle et $R_{11}$ représente un groupe hydroxy ou alcoxy,

leurs tautomères, leurs stéréoisomères et leurs sels,
où les restes phényle précités peuvent être substitués par un atome de fluor, de chlore ou de brome ou par un groupe nitro, alkyle, alcoxy, trifluorométhyle ou hydroxy, et, sauf indication contraire, les parties alkyle, alkylène et alcoxy évoquées ci-dessus contiennent chacune 1 à 4 atomes de carbone et, sauf indication contraire, tout atome de carbone lié à un atome d'azote, d'oxygène ou de soufre dans les parties alkylène ou cycloalkylène évoquées précédemment ne peut être lié à aucun autre atome d'halogène, d'azote, d'oxygène ou de soufre.

**5.** Pyfimido[5,4-d]pyrimidines de formule générale I selon la revendication 1 ou 2, dans lesquelles

$R_a$ représente un atome d'hydrogène ou un groupe méthyle,
$R_b$ représente un groupe 2-naphtyle, 1,2,3,4-tétrahydro-6-naphtyle ou 5-indanyle, ou un groupe phényle substitué par les restes $R_1$ à $R_3$, où

$R_1$ représente un atome d'hydrogène, de fluor, de chlore, de brome ou d'iode,
un groupe alkyle en $C_1$-$C_4$, alcoxy en $C_1$-$C_4$, cycloalkyle en $C_3$-$C_6$, cycloalcoxy en $C_5$-$C_6$, cyano, méthoxycarbonyle, éthoxycarbonyle, éthynyle ou nitro,
un groupe méthyle ou méthoxy substitué par 1 à 3 atomes de fluor,
un groupe éthyle ou éthoxy substitué par 1 à 5 atomes de fluor,
$R_2$ représente un atome d'hydrogène, de fluor ou de chlore ou un groupe méthyle, hydroxy, méthoxy, amino, (alkyl en $C_1$-$C_2$)amino, di(alkyl en $C_1$-$C_2$)amino, (alkyl en $C_1$-$C_2$)carbonylamino, (alkyl en $C_1$-$C_2$) sulfonylamino, trifluorométhylsulfonylamino ou trifluorométhyle,
$R_3$ représente un atome d'hydrogène, de fluor, de chlore ou de brome ou un groupe méthyle, et

$R_c$ représente un atome d'hydrogène, un groupe méthyle, phényle, 4-méthoxyphényle, méthylsulfényle, méthylsulfinyle ou méthylsulfonyle,
un groupe hydroxy,
un groupe alcoxy en $C_1$-$C_4$,
un groupe éthoxy substitué en position 2 par un groupe hydroxy, méthoxy, morpholino ou (2-hydroxyéthyl) amino,
un groupe 2-propyloxy substitué en position 1 par un groupe méthoxy ou diméthylamino,
un groupe méthoxy substitué par un groupe 2-tétrahydrofuryle, 2-tétrahydropyranyle ou 3-méthyl-3-oxétanyle,
un groupe cyclobutyloxy,
un groupe cyclopentyloxy éventuellement substitué en position 3 par un groupe hydroxy,
un groupe cyclopentyloxy dans lequel le groupe méthylène en position 3 est remplacé par un atome d'oxygène ou par un groupe méthylimino,
un groupe cyclohexyloxy pouvant être substitué en position 2, 3 ou 4 par un groupe hydroxy ou en position 4 par un groupe di(alkyl en $C_1$-$C_2$)amino, méthoxy, carboxy, méthoxycarbonyle, diméthylaminocarbonyle, méthylaminocarbonyle, aminocarbonyle, acétylamino, méthylsulfonylamino, méthoxycarbonylamino ou tert-butyloxycarbonylamino,
un groupe cyclohexyloxy dans lequel le groupe méthylène en position 3 est remplacé par un groupe méthylimino ou le groupe méthylène en position 4 est remplacé par un atome d'oxygène ou par un groupe méthylimino, acétylimino, tert-butyloxycarbonylimino, méthoxycarbonylimino ou méthylsulfonylimino,
un groupe allyloxy,
un groupe 1-azétidinyle,
un groupe 1-pyrrolidinyle pouvant être substitué par 1 à 2 groupes méthyle, par un groupe carboxy, méthoxycarbonyle, éthoxycarbonyle, aminocarbonyle, (alkyl en $C_1$-$C_2$)aminocarbonyle ou di(alkyl en $C_1$-$C_2$)aminocarbonyle, ou en position 3 par un groupe amino, (alkyl en $C_1$-$C_2$)amino, di(alkyl en $C_1$-$C_2$)amino, (alcoxy en $C_1$-$C_2$)carbonylarnino, (alkyl en $C_1$-$C_2$)carbonylamino, (alkyl en $C_1$-$C_2$)sulfonylamino, cyanoamino, formylamino ou diméthylaminocarbonylamino,
un groupe 1-pyrrolidinyle dans lequel les deux atomes d'hydrogène en position 3 sont remplacés par un pont n-alkylène en $C_4$-$C_5$,
un groupe 1-pipéridinyle éventuellement substitué par 1 à 4 groupes méthyle, par un groupe phényle, hydroxyalkyle en $C_1$-$C_2$, carboxy, (alcoxy en $C_1$-$C_2$)carbonyle, aminocarbonyle, (alkyl en $C_1$-$C_2$)aminocarbonyle, di(alkyl en $C_1$-$C_2$)aminocarbonyle, pyrrolidinocarbonyle ou morpholinocarbonyle, ou en position 3 ou 4 par un groupe hydroxy, alcoxy en $C_1$-$C_2$, amino, (alkyl en $C_1$-$C_2$)amino, di(alkyl en $C_1$-$C_2$)amino, (alkyl en $C_1$-$C_2$)

carbonylamino, (alcoxy en $C_1$-$C_2$)carbonylamino, formylamino, cyanoamino, di(alkyl en $C_1$-$C_2$)aminocarbonylamino, (alkyl en $C_1$-$C_2$)sulfonylamino ou cyano,
un groupe 1-pipéridinyle dans lequel les deux atomes d'hydrogène en position 3 ou en position 4 sont remplacés par un pont n-alkylène en $C_4$-$C_5$ ou par un pont $-O-CH_2CH_2-O-$,
un groupe 1-pipéridinyle substitué par 1 ou 2 groupes méthyle ou par un groupe phényle, et en outre par un groupe hydroxy en position 3 ou 4,
un groupe 1-pipéridinyle dans lequel deux atomes d'hydrogène en position 2,5 sont remplacés par un pont $-CH_2$ ou $-CH_2CH_2-$,
un groupe 1-pipéridinyle éventuellement substitué par 1 ou 2 groupes méthyle, dans lequel le groupe méthylène en position 4 est remplacé par un atome d'oxygène ou de soufre ou par un groupe carbonyle, sulfinyle, sulfonyle, imino, (alkyl en $C_1$-$C_2$)amino, (2-hydroxyéthyl)imino, 2-(2-hydroxyéthoxy)éthylimino, (2-aminoéthyl)imino, (alkyl en $C_1$-$C_3$)aminocarbonylméthylimino, N-oxydo-N-(alkyl en $C_1$-$C_2$)imino, phénylimino, benzylimino, acétylimino ou méthanesulfonylimino,
un groupe 1-azacyclohept-1-yle dans lequel deux atomes d'hydrogène en position 3,6 peuvent être remplacés par un groupe $-CH_2CH_2-$,
un groupe 1,2,3,4-tétrahydroisoquinoléine-2-yle ou 2,3,4,5-tétrahydro-1H-3-benzazépine-3-yle, ou un groupe ($R_{10}NR_{11}$), dans lequel

$R_{10}$ représente un atome d'hydrogène, un groupe alkyle en $C_1$-$C_4$ ou un groupe 2-hydroxyéthyle, et
$R_{11}$ représente un atome d'hydrogène,
un groupe alkyle en $C_1$-$C_6$, (cycloalkyle en $C_3$-$C_6$)méthyle ou phényl(alkyle en $C_1$-$C_3$),
un groupe cycloalkyle en $C_3$-$C_6$, allyle ou propargyle éventuellement substitué par 1 ou 2 groupes méthyle,
un groupe phényle pouvant être substitué par un groupe hydroxy ou méthyle ou en position 4 par un groupe N-(alkyl en $C_1$-$C_2$)-(alkyl en $C_1$-$C_2$)carbonylamino, N-(alkyl en $C_1$-$C_2$)-(2-hydroxyéthyl)amino, di(2-hydroxyéthyl)amino, 1-pyrrolidinyle, 2-oxo-1-pyrrolidinyle, 2-hydroxyméthyl-1-pyrrolidinyle, 2-oxo-1-imidazolidinyle, 3-méthyl-2-oxo-1-imidazolidinyle ou morpholino,
un groupe méthyle substitué par un groupe carboxy, (alcoxy en $C_1$-$C_2$)carbonyle, aminocarbonyle, (alkyl en $C_1$-$C_2$)aminocarbonyle, di(alkyl en $C_1$-$C_2$)aminocarbonyle, pyrrolidinocarbonyle, pipéridinocarbonyle ou morpholinocarbonyle,
un groupe éthyle éventuellement substitué par un groupe méthyle, et qui est substitué en position 2 par un groupe hydroxy, alcoxy en $C_1$-$C_2$, amino, alkylamino en $C_1$-$C_2$, di(alkyl en $C_1$-$C_2$)amino, acétylamino, 1-pyrrolidinyle, morpholino, 1-pipérazinyle, 4-méthyl-1-pipérazinyle, 4-acétyl-1-pipérazinyle, 4-aminocarbonyl-1-pipérazinyle, 4-diméthylaminocarbonyl-1-pipérazinyle, 4-méthylaminocarbonyl-1-pipérazinyle, 4-méthylsulfonyl-1-pipérazinyle, 4-méthoxycarbonyl-1-pipérazinyle ou 4-cyano-1-pipérazinyle,
un groupe 2-hydroxyéthyle substitué dans la partie éthyle par un groupe phényle, 3-hydroxyphényle, 4-hydroxyphényle, 4-nitrophényle ou benzyle, la partie éthyle des groupes précités pouvant être substituée en outre par un groupe méthyle, hydroxyméthyle ou méthoxyméthyle,
un groupe méthyle substitué par un groupe 1,4,7,10-tétraoxacyclododécyle, 1,4,7,10,13-pentaoxacyclopentadécyle ou 1,4,7,10,13,16-hexaoxacyclooctadécyle, un groupe 2,2,2-trifluoroéthyle,
un groupe propyle substitué en position 3 par un groupe hydroxy, cyano, carboxy, aminocarbonyle, méthylaminocarbonyle, diméthylaminocarbonyle, formylamino, méthoxycarbonylamino, morpholino ou 2-oxo-1-pyrrolidinyle,
un groupe butyle substitué en position 4 par un groupe hydroxy,
un groupe 3-butyle substitué en position 1 par un groupe hydroxy et en outre en position 3 par un groupe méthyle,
un groupe 2-butyle substitué en position 1 par un groupe hydroxy et en outre en position 3 par un groupe méthyle,
un groupe 4-pentyle substitué en position 1 par un groupe hydroxy et en outre en position 4 par un groupe méthyle,
un groupe 2,3-dihydroxypropyle, 3-amino-2-hydroxypropyle, tris(3-hydroxypropyl)méthyle, 1,3-dihydroxy-2-propyle, 1,3-dihydroxy-2-méthyl-2-propyle ou tris(hydroxyméthyl)méthyle,
un groupe 2-propyle substitué en position 2 par un groupe hydroxyméthyle (alcoxy en $C_1$-$C_2$)méthyle, carboxy, (alcoxy en $C_1$-$C_2$)carbonyle, aminocarbonyle, N-(alkyl en $C_1$-$C_2$)aminocarbonyle, N,N-di(alkyl en $C_1$-$C_2$)aminocarbonyle, pyrrolidinocarbonyle, morpholinocarbonyle ou (2-hydroxyéthyl)aminocarbonyle, un groupe 4-tétrahydropyranyle, tétrahydrofurfuryle, 1-désoxy-1-D-sorbityle ou 2-(2-hydroxyéthyloxy)éthyle,
un groupe cyclopentyle substitué en position 2 ou 3 par un groupe hydroxy ou en position 1 par un groupe hydroxyméthyle,

un groupe cyclohexyle substitué en position 2, 3 ou 4 par un groupe hydroxyméthyle, hydroxy, alcoxy en $C_1$-$C_2$, (alcoxy en $C_1$-$C_4$)carbonylamino, amino, (alkyl en $C_1$-$C_2$)amino, di(alkyl en $C_1$-$C_2$)amino, carboxy, (alcoxy en $C_1$-$C_2$)carbonyle, aminocarbonyle, (alkyl en $C_1$-$C_2$)aminocarbonyle, di(alkyl en $C_1$-$C_2$)aminocarbonyle, N-oxido-di(alkyl en $C_1$-$C_2$)amino, pyrrolidinocarbonyle, morpholinocarbonyle, (alkyl en $C_1$-$C_2$) carbonylarnhlo ou (alkyl en $C_1$-$C_2$)sulfonylamino, et qui peut être substitué en outre par un groupe méthyle,
un groupe cyclohexyle substitué en position 4 par un groupe 1-pyrrolidinyle, 2-oxo-1-pyrrolidinyle, 2-hydroxyméthyl-1-pyrrolidinyle, N-(alkyl en $C_1$-$C_2$)-(2-hydroxyéthyl) amino, di-(2-hydroxyéthyl)amino, N-(alkyl en $C_i$-$C_i$)-(alkyl en $C_1$-$C_2$)carbonylamino, morpholino, 2-oxo-1-imidazolidinyle, 3-méthyl-2-oxo-1-imidazolidinyle, carboxy(alkyle en $C_1$-$C_2$), carboxy(alcoxy en $C_1$-$C_2$), (alcoxy en $C_1$-$C_2$)carbonyl(alkyle en $C_1$-$C_2$), (alcoxy en $C_1$-$C_2$)carbonyl(alcoxy en $C_1$-$C_2$), aminocarbonyl(alkyle en $C_1$-$C_2$), aminocarbonyl(alcoxy en $C_1$-$C_2$), (alkyl en $C_1$-$C_2$)aminocarbonyl(alkyle en $C_1$-$C_2$), (alkyl en $C_1$-$C_2$)aminocarbonyl(alcoxy en $C_1$-$C_2$), di(alkyl en $C_1$-$C_2$)aminocarbonyl(alkyle en $C_1$-$C_2$), di(alkyl en $C_1$-$C_2$)aminocarbonyl(alcoxy en $C_1$-$C_2$), pyrrolidinocarbonyl(alkyle en $C_1$-$C_2$), pyrrolidinocarbonyl(alcoxy en $C_1$-$C_2$), morpholinocarbonyl(alkyle en $C_1$-$C_2$), morpholinocarbonyl(alcoxy en $C_1$-$C_2$), pipéridinocarbonyl(alkyle en $C_1$-$C_2$) ou pipéridinocarbonyl(alcoxy en $C_1$-$C_2$),
un groupe cyclohexyle dans lequel les deux atomes d'hydrogène en position 4 sont remplacés par un groupe oxo ou par un pont n-alkylène en $C_4$-$C_5$,
un groupe cyclohexyle dans lequel le groupe méthylène en position 4 est remplacé par un groupe imino, alkylimino en $C_1$-$C_2$, phényl(alkyl en $C_1$-$C_2$)imino, N-méthyl-N-oxydo-imino, formylimino, (alkyl en $C_1$-$C_2$) carbonylimino, alkylsulfonylimino en $C_1$-$C_2$, (alcoxy en $C_1$-$C_2$)carbonylimino, cyanoimino, aminocarbonylimino, (alkyl en $C_1$-$C_2$)aminocarbonylimino ou N,N-di(alkyl en $C_1$-$C_2$)aminocarbonylimino,
un groupe cyclohexyle dans lequel le groupe méthylène en position 3 est remplacé par un groupe imino, alkylimino en $C_1$-$C_2$, (alkyl en $C_1$-$C_2$)carbonylimino, alkylsulfonylimino en $C_1$-$C_2$ ou (alcoxy en $C_1$-$C_2$) carbonylimino,
un groupe cyclopentyle dans lequel le groupe méthylène en position 3 est remplacé par un atome d'oxygène ou un groupe imino, alkylimino en $C_1$-$C_2$, formylimino, (alkyl en $C_1$-$C_2$)carbonylimino, alkylsulfonylimino en $C_1$-$C_2$, (alcoxy en $C_1$-$C_2$)carbonylimino, cyanoimino ou N,N-di(alkyl en $C_1$-$C_2$)aminocarbonylimino,
un groupe cyclohexylméthyle dans lequel la partie cyclohexyle est substituée en position 4 par un groupe carboxy, (alcoxy en $C_1$-$C_2$)carbonyle, N,N-di(alkyl en $C_1$-$C_2$)aminocarbonyle ou morpholinocarbonyle,
un groupe norbomane-2-yle, norbomane-2-ylméthyle, 5-norbornène-2-ylméthyle, bornyle, 3-quinuclidinyle ou adamantyle, ou
$R_{10}$ est un atome d'hydrogène ou un groupe méthyle et $R_{11}$ représente un groupe hydroxy ou méthoxy,

leurs tautomères, leurs stéréoisomères et leurs sels.

**6.** Pyrimido[5,4-d]pyrimidines de formule générale I selon la revendication 1 ou 2, dans lesquelles

$R_a$ représente un atome d'hydrogène ou un groupe méthyle,
$R_b$ représente un groupe 2-naphtyle ou 5-indanyle, ou un groupe phényle substitué par les restes $R_1$ à $R_3$, où

$R_1$ représente un atome d'hydrogène, de fluor, de chlore, de brome ou d'iode, ou un groupe méthyle, éthyle, tert-butyle, trifluorométhyle, éthynyle, méthoxy, cyclopropyle, trifluorométhoxy, cyano, éthoxycarbonyle ou nitro,
$R_2$ représente un atome d'hydrogène, de fluor ou de chlore ou un groupe amino, méthyle ou trifluorométhyle,
$R_3$ représente un atome d'hydrogène, de chlore ou de brome, et

$R_c$ représente un atome d'hydrogène, un groupe hydroxy, méthoxy, butyloxy, cyclopentyloxy, 2-[(2-hydroxyéthyl)amino]éthoxy, méthylsulfényle, méthylsulfinyle ou méthylsulfonyle,
un groupe 1-azétidinyle ou un groupe 1-pyrrolidinyle éventuellement substitué par 1 ou 2 groupes méthyle,
un groupe 1-pipéridinyle substitué par un groupe hydroxyméthyle,
un groupe 1-pipéridinyle éventuellement substitué par 1 ou 2 groupes méthyle, dans lequel le groupe méthylène en position 4 peut être remplacé par un atome d'oxygène ou de soufre ou par un groupe carbonyle, sulfinyle, sulfonyle, imino, méthylimino, N-oxydo-N-méthylimino, 2-propylaminocarbonyl-méthylimino, phénylimino, benzylimino, acétylimino ou méthylsulfonylimino,
un groupe 1-pipéridinyle substitué en position 3 par un groupe hydroxy ou diéthylaminocarbonyle ou en position 4 par un groupe hydroxy, carboxy, méthoxycarbonyle, aminocarbonyle, méthylaminocarbonyle, diméthylaminocarbonyle, pyrrolidinocarbonyle, morpholinocarbonyle, amino, acétylamino, méthoxycarbonylamino, formylamino, cyanoamino, diméthylaminocarbonylamino, méthylsulfonylamino ou phényle,
un groupe 4-hydroxy-4-phényl-1-pipéridinyle,
un groupe 1,2,3,4-tétrahydroisoquinoléine-2-yle ou 2,3,4,5-tétrahydro-1H-3-benzazépine-3-yle,
un groupe 1-pipéridinyle dans lequel les deux atomes d'hydrogène en position 4 sont remplacés par un pont $-OCH_2CH_2O-$,
un groupe 1-azacyclohept-1-yle dans lequel deux atomes d'hydrogène en position 3 et 6 sont remplacés par un groupe $-CH_2CH_2-$, ou
un groupe ($R_{10}NR_{11}$), dans lequel

$R_{10}$ représente un atome d'hydrogène, un groupe alkyle en $C_1$-$C_4$ ou un groupe 2-hydroxyéthyle, et
$R_{11}$ représente un atome d'hydrogène,
un groupe phényle éventuellement substitué par un groupe méthyle,
un groupe phényle substitué en position 4 par un groupe morpholino ou 2-hydroxyméthyl-1-pyrrolidinyle,
un groupe alkyle en $C_1$-$C_6$, cycloalkyle en $C_3$-$C_6$, phényl(alkyle en $C_1$-$C_3$), cyclopropylméthyle, allyle, propargyle, 2-hydroxyéthyle, 1-hydroxy-2-propyle, 3-hydroxypropyle, 4-hydroxybutyle, 2-méthoxyéthyle, 1-adamantyle, norbomane-2-yle, aminocarbonylméthyle, 2-(diméthylanùno)éthyle, 3-quinuclidinyle, 2,2,2-trifluoroéthyle, 4-pipéridinyle, 1-méthyl-4-pipéridinyle, 1-méthyl-1-oxydo-4-pipéridinyle, 1-éthoxycarbonyl-4-pipéridinyle, 1-benzyl-4-pipéridinyle, 2-(2-hydroxyéthoxy)éthyle, 4-tétrahydropyranyle, 1-hydroxy-2-méthyl-2-propyle, 1-méthoxy-2-méthyl-2-propyle, 2-(méthylaminocarbonyl)-2-propyle, 2,3-dihydroxy-1-propyle, 2-(morpholino)éthyle, 1-désoxy-1-D-sorbityle, 3-(2-oxo-1-pyrrolidinyl)propyle, tris(hydroxyméthyl)méthyle, 1,3-dihydroxy-2-propyle, 1,3-dihydroxy-2-méthyl-2-propyle ou bomyle,
un groupe 2-hydroxyéthyle substitué en position 2 par un groupe phényle et en outre en position 1 par un groupe méthyle ou hydroxyméthyle,
un groupe méthylcyclohexyle, 4-carboxycyclohexyle, 4-méthoxycarbonylcyclohexyle, 4-diméthylaminocarbonylcyclohexyle, 4-(1-pyrrolidinylcarbonyl)cyclohexyle, 4-(morpholinocarbonyl)cyclohexyle, 4-[2-(méthoxycarbonyl)éthyl]cyclohexyle, 4-(2-carboxyéthyl)cyclohexyle, 4-(tert-butyloxycarbonylamino)cyclohexyle, 4-méthoxycyclohexyle, 4-aminocyclohexyle, 4-(diméthylamino)cyclohexyle, 4-(N,N-diméthyl-N-oxydo-amino)cyclohexyle, 4-(acétylamino)cyclohexyle, 4-(méthylsulfonylamino)cyclohexyle, 2-hydroxycyclohexyle, 4-hydroxycyclohexyle, 4-(hydroxyméthyl)cyclohexyle, 4-hydroxy-4-méthylcyclohexyle ou 4-oxocyclohexyle,
un groupe méthyle substitué par un groupe 1,4,7,10,13-pentaoxacyclopentadécyle ou 1,4,7,10,13,16-hexaoxacyclooctadécyle, ou
$R_{10}$ est un groupe méthyle et $R_{11}$ représente un groupe méthoxy,

leurs tautomères, leurs stéréoisomères et leurs sels.

**7.** Composés ci-dessous de formule générale I selon la revendication 1 ou 2:

(1) la 4-[(3,4-dichlorophényl)amino]-6-[(trans-4-hydroxycyclohexyl)amino]pyrimido[5,4-d]pyrimidine,
(2) la 4-[(3-chloro-4-fluorophényl)amino]-6-[(trans-4-hydroxycyclohexyl)amino]pyrimido[5,4-d]pyrimidine,
(3) la 4-[(3-bromophényl)amino]-6-[(trans-4-hydroxycyclohexyl)amino]pyrimido[5,4-d]pyrimidine,
(4) la 4-[(3-chlorophényl)amino]-6-(cyclopropylamino)pyrimido[5,4-d]pyrimidine,
(5) la 4-[(3-méthylphényl)amino]-6-(4-amino-1-pipéridinyl)pyrimido[5,4-d]pyrimidine,
(6) la 4-[(3-méthylphényl)amino]-6-[(trans-4-aminocyclohexyl)amino]pyrimido[5,4-d]pyrimidine,
(7) la 4-[(3-méthylphényl)amino]-6-(N-(trans-4-hydroxycyclohexyl)-N-méthylamino)pyrimido[5,4-d]pyrimidine,
(8) la 4-[(3-méthylphényl)amino]-6-(4-méthoxycarbonylamino-1-pipéridinyl)pyrimido[5,4-d]pyrimidine,
(9) la 4-[(3-méthylphényl)amino]-6-[trans-4-(morpholinocarbonyl)cyclohexylamino]pyrimido[5,4-d]pyrimidine,
(10) la 4-[(3-méthylphényl)amino]-6-[trans-4-(pyrrolidinocarbonyl)cyclohexylamino]pyrimido[5,4-d]pyrimidine,
(11) la 4-[(3-chloro-4-fluorophényl)amino]-6-[N-(trans-4-hydroxycyclohexyl)-N-méthylamino]pyrimido[5,4-d]pyrimidine,
(12) la 4-[(4-amino-3,5-dichlorophényl)amino]-6-[(trans-4-hydroxycyclohexyl)amino]pyrimido[5,4-d]pyrimidine,
(13) la 4-[(3-chloro-4-fluorophényl)amino]-6-[2-(morpholinoéthylamino]pyrimido[5,4-d]pyrimidine,
(14) la 4-[(4-amino-3,5-dibromophényl)amino]-6-[(trans-4-hydroxycyclohexyl)amino]pyrimido[5,4-d]pyrimidine,
(15) la 4-[(3-chloro-4-fluorophényl)amino]-6-morpholinopyrimido[5,4-d]pyrimidine,

(16) la 4-[(3-chloro-4-fluorophényl]amino]-6-(1-hydroxy-2-méthyl-2-propylamino)pyrimido[5,4-d]pyrimidine,
(17) la 4-[(3-chloro-4-fluorophényl)amino]-6-(1-hydroxy-2-propylamino)pyrimido[5,4-d]pyrimidine,
(18) la 4-[(3-chloro-4-fluorophényl)amino]-6-(1,3-dihydroxy-2-propylamino)pyrimido[5,4-d]pyrimidine,
(19) la 4-[(3-chloro-4-fluorophényl)amino]-6-[4-amino-1-pipéridinyl]pyrimido[5,4-d]pyrimidine,
(20) la 4-[(3-méthylphényl)amino]-6-(4-pipéridinylamino)pyrimido[5,4-d]pyrimidine,
(21) la 4-[(3-méthylphényl)amino]-6-(4-formylamino-1-pipéridinyl)pyrimido[5,4-d]pyrimidine,
(22) la 4-[(3-méthylphényl)amino]-6-[(1-éthoxycarbonyl-4-pipéridinyl)amino]pyrimido[5,4-d]pyrimidine,
(23) la 4-[(3-méthylphényl)amino]-6-[(3-quinuclidinyl)amino]pyrimido[5,4-d]-pyrimidine,
(24) la 4-[(3-chloro-4-fluorophényl)amino]-6-[(4-aminocyclohexyl)amino]pyrimido[5,4-d]pyrimidine,
(25) la 4-[(3-chloro-4-fluorophényl)amino]-6-(4-pipéridinylamino)pyrimido[5,4-d]pyrimidine,
(26) la 4-[(3-chloro-4-fluorophényl)amino]-6-[trans-4-(morpholinocarbonyl)cyclohexylamino]pyrimido[5,4-d]pyrimidine,
(27) la 4-[(3-chloro-4-fluorophényl)amino]-6-[trans-4-(pyrrolidinocarbonyl)cyclohexylamino]pyrimido[5,4-d]pyrimidine,
(28) la 4-[(4-fluorophényl)amino]-6-(cyclopropylamino)pyrimido[5,4-d]pyrimidine,
(29) la 4-[(3-chloro-4-fluorophényl)amino]-6-(cyclopropylamino)pyrimido[5,4-d]pyrimidine,
(30) la 4-[(3,4-dichlorophényl)amino]-6-(cyclopropylamino)pyrimido[5,4-d]pyrimidine,
(31) la 4-[(3-chloro-4-fluorophényl)amino]-6-(1-méthyl-4-pipéridinylamino)pyrimido[5,4-d]pyrimidine, et
(32) la 4-[(3-chloro-4-fluorophényl)amino]-6-[(trans-4-diméthylaminocyclohexyl)amino]pyrimido[5,4-d]pyrimidine, et leurs sels.

**8.** La 4-[(3-chloro-4-fluorophényl)amino]-6-(1-méthyl-4-pipéridinylamino)pyrimido[5,4-d]pyrimidine et ses sels.

**9.** La 4-[(3-chloro-4-fluorophényl)amino]-6-[(trans-4-diméthylaminocyclohexyl)amino]pyrimido[5,4-d]pyrimidine et ses sels.

**10.** Sels physiologiquement acceptables des composés selon l'une au moins des revendications 1 à 9 avec des acides ou des bases inorganiques ou organiques.

**11.** Médicament contenant un composé selon l'une au moins des revendications 1 à 9 ou un sel physiologiquement acceptable selon la revendication 10, avec éventuellement un ou plusieurs supports et/ou diluants inertes.

**12.** Utilisation d'un composé selon l'une au moins des revendications 1 à 10 pour la préparation d'un médicament approprié au traitement de tumeurs bénignes ou malignes, en particulier de tumeurs d'origine épithéliale et neuro-épithéliale, de la formation de métastases et de la prolifération anormale de cellules endothéliales vasculaires (néoangiogenèse).

**13.** Procédé de préparation d'un médicament selon la revendication 11, caractérisé en ce que l'on incorpore par des voies non chimiques un composé selon l'une au moins des revendications 1 à 10 dans un ou plusieurs supports et/ou diluants inertes.

**14.** Procédé de préparation des composés de formule générale I selon les revendications 1 à 10, caractérisé en ce que

a) on fait réagir un composé de formule générale

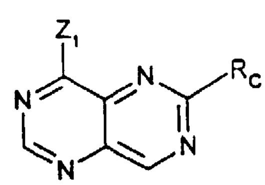

(II)

dans laquelle $R_c$ a la définition indiquée dans les revendications 1 à 9 et $Z_1$ représente un groupe partant, avec une amine de formule générale

$$H\text{-}(R_aNR_b) \qquad (III)$$

dans laquelle $R_a$ et $R_b$ ont la définition indiquée dans les revendications 1 à 9, ou

b) pour préparer des composés de formule générale I dans laquelle $R_c$ représente l'un des restes, cités dans les revendications 1 à 8, reliés à la pyrimido[5,4-d]pyrimidine par l'intermédiaire d'un atome d'oxygène ou d'azote ou d'un groupe mercapto ou sulfényle, on fait réagir un composé de formule générale

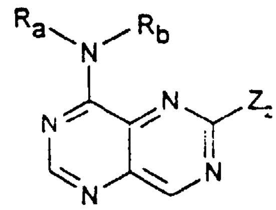

(IV)

dans laquelle $R_a$ et $R_b$ ont la définition indiquée dans les revendications 1 à 9 et $Z_2$ représente un groupe partant, avec un composé de formule générale

$$X_1 - H \qquad (V)$$

dans laquelle $X_1$ représente l'un des restes, cités dans les revendications 1 à 9, reliés à la pyrimido[5,4-d] pyrimidine par l'intermédiaire d'un atome d'oxygène ou d'azote ou d'un groupe mercapto ou sulfényle, ou

c) pour préparer des composés de formule générale I dans laquelle $R_c$ représente l'un des restes, cités dans les revendications 1 à 9, liés à la pyrimido[5,4-d]pyrimidine par l'intermédiaire d'un groupe sulfinyle ou sulfonyle, on oxyde un composé de formule générale

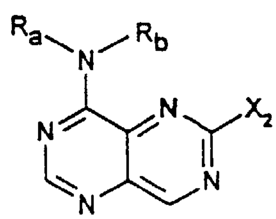

(VI)

dans laquelle $R_a$ et $R_b$ ont la définition indiquée dans les revendications 1 à 9 et $X_2$ représente l'un des restes, cités dans les revendications 1 à 8, liés à la pyrimido[5,4-d]pyrimidine par l'intermédiaire d'un atome de soufre, ou

d) pour préparer un composé de formule générale I dans laquelle $R_c$ représente un atome d'hydrogène, on élimine les atomes d'halogène d'un composé de formule générale

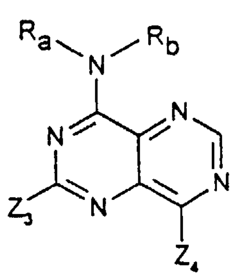

(VII)

dans laquelle $R_a$ et $R_b$ ont la définition donnée dans les revendications 1 à 9 et $Z_3$ et $Z_4$, qui peuvent être

identiques ou différents, représentent chacun un atome d'halogène, et
si désiré, on transforme ensuite par acylation ou sulfonylation un composé de formule générale I ainsi obtenu, qui contient un groupe amino, alkylamino ou imino, en un composé acylé ou sulfonylé correspondant de formule générale I, et/ou
on transforme par alkylation ou alkylation réductrice un composé de formule générale I ainsi obtenu, qui contient un groupe amino, alkylamino ou imino, en un composé alkylé correspondant de formule générale I, et/ou
on transforme par estérification un composé de formule générale I ainsi obtenu, qui contient un groupe carboxy, en un ester de formule générale I correspondant, et/ou
on transforme par amidation un composé de formule générale I ainsi obtenu, qui contient un groupe carboxy ou ester, en un amide de formulé générale I correspondant, et/ou
on transforme par oxydation un composé de formule générale I contenant un groupe hydroxy primaire ou secondaire en un composé carbonylé de formule générale I correspondant, et/ou
si nécessaire, on élimine un reste protecteur utilisé dans les réactions décrites précédemment, et/ou
si désiré, on sépare un composé de formule générale I ainsi obtenu en ses stéréoisomères, et/ou
on transforme un composé de formule générale I ainsi obtenu en ses sels, en particulier, pour l'application pharmaceutique, en ses sels physiologiquement acceptables.